(19) Europäisches Patentamt
European Patent Office
Office européen des brevets

(11) EP 2 350 093 B1

# (12) EUROPÄISCHE PATENTSCHRIFT

(45) Veröffentlichungstag und Bekanntmachung des Hinweises auf die Patenterteilung:
**03.10.2012 Patentblatt 2012/40**

(21) Anmeldenummer: **09747793.9**

(22) Anmeldetag: **22.10.2009**

(51) Int Cl.:
***C07D 495/04*** (2006.01) ***A61K 31/4365*** (2006.01)
***A61P 25/04*** (2006.01) ***A61P 25/08*** (2006.01)

(86) Internationale Anmeldenummer:
**PCT/EP2009/007567**

(87) Internationale Veröffentlichungsnummer:
**WO 2010/046108 (29.04.2010 Gazette 2010/17)**

## (54) SUBSTITUIERTE 4,5,6,7-TETRAHYDROTHIENOPYRIDINE ALS KCNQ2/3 MODULATOREN ZUR BEHANDLUNG VON SCHMERZ, EPILEPSIE UND HARNINKONTINENZ

SUBSTITUTED 4,5,6,7-TETRAHYDROTHIENOPYRIDINES AS KCNQ2/3 MODULATORS FOR TREATING PAIN, EPILEPSY AND URINARY INCONTINENCE

4,5,6,7-TÉTRAHYDROTHIÉNOPYRIDINES SUBSTITUÉES, COMME MODULATEURS DES KCNQ2/3 POUR LE TRAITEMENT DE LA DOULEUR, DE L'ÉPILEPSIE ET DE L'INCONTINENCE URINAIRE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **24.10.2008 EP 08018617**

(43) Veröffentlichungstag der Anmeldung:
**03.08.2011 Patentblatt 2011/31**

(73) Patentinhaber: **Grünenthal GmbH**
**52078 Aachen (DE)**

(72) Erfinder:
- **KÜHNERT, Sven**
  **52355 Düren (DE)**
- **BAHRENBERG, Gregor**
  **52156 Monschau-Konzen (DE)**
- **KLESS, Achim**
  **52072 Aachen (DE)**
- **MERLA, Beatrix**
  **52078 Aachen (DE)**
- **SCHIENE, Klaus**
  **41363 Jüchen (DE)**
- **SCHRÖDER, Wolfgang**
  **52074 Aachen (DE)**

(74) Vertreter: **Bülle, Jan et al**
**Kutzenberger & Wolff**
**Patentanwaltssozietät**
**Theodor-Heuss-Ring 23**
**50668 Köln (DE)**

(56) Entgegenhaltungen:
**WO-A-96/34870 WO-A-2008/046582**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 350 093 B1

## Beschreibung

Die Erfindung betrifft substituierte Tetrahydrothienopyridine, Verfahren zu ihrer Herstellung, Arzneimittel enthaltend diese Verbindungen sowie die Verwendung dieser Verbindungen zur Herstellung von Arzneimitteln.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nociception in letzter Zeit erschienen sind.

Ein pathophysiologisches Merkmal von chronischen Schmerzen besteht in der Übererregbarkeit von Neuronen. Die neuronale Erregbarkeit wird entscheidend von der Aktivität von $K^+$ Kanälen beeinflusst, da diese das Ruhemembranpotential der Zelle und somit die Erregbarkeitsschwelle maßgeblich bestimmen. Heteromere $K^+$ Kanäle vom molekularen Subtyp KCNQ2/3 (Kv7.2/7.3) sind in Neuronen verschiedener Regionen des zentralen (Hippocampus, Amygdala) und peripheren (Hinterwurzelganglien) Nervensystems exprimiert und regulieren deren Erregbarkeit. Die Aktivierung von KCNQ2/3 $K^+$ Kanälen führt zu einer Hyperpolarisation der Zellmembran und damit einhergehend zu einer Abnahme der elektrischen Erregbarkeit dieser Neurone. KCNQ2/3 exprimierende Neurone der Hinterwurzelganglien sind an der Übertragung nociceptiver Erregungen von der Peripherie ins Rückenmark beteiligt (Passmore et al., J Neurosci. 2003; 23(18):7227-36).

Dementsprechend konnte für den KCNQ2/3 Agonisten Retigabine eine analgetische Wirksamkeit in präklinischen Neuropathie- und Entzündungsschmerzmodellen nachgewiesen werden (Blackburn-Munro and Jensen, Eur J Pharmacol. 2003; 460(2-3):109-16; Dost et al., Naunyn Schmiedebergs Arch Pharmacol 2004; 369(4): 382-390).

Der KCNQ2/3 $K^+$ Kanal stellt somit einen geeigneten Ansatzpunkt zur Behandlung von Schmerz; insbesondere von Schmerz ausgewählt aus der Gruppe bestehend aus chronischem Schmerz, neuropathischem Schmerz, inflammatorischem Schmerz und muskulärem Schmerz (Nielsen et al., Eur J Pharmacol. 2004; 487(1-3): 93-103), insbesondere von neuropathischem und inflammatorischem Schmerz dar.

Darüber hinaus ist der KCNQ2/3 $K^+$ Kanal ein geeignetes Target für die Therapie einer Vielzahl weiterer Erkrankungen wie beispielsweise Migräne (US20G2/0128277), kognitive Erkrankungen (Gribkoff, Expert Opin Ther Targets 2003; 7(6): 737-748), Angstzuständen (Korsgaard et al., J Pharmacol Exp Ther. 2005, 14(1): 282-92), Epilepsie (Wickenden et al., Expert Opin Ther Pat 2004, 14(4): 457-469; Gribkoff, Expert Opin Ther Targets 2008, 12(5): 565-81; Miceli et al., Curr Opin Pharmacol 2008, 8(1): 65-74), Harninkontinenz (Streng et al., J Urol 2004; 172: 2054-2058), Abhängigkeit (Hansen et al., Eur J Pharmacol 2007, 570(1-3): 77-88), Manielbipolare Störungen (Dencker et al., Epilepsy Behav 2008, 12(1): 49-53), Dystonie-assozüerte Dyskinesien (Richter et al., Br J Pharmacol 2006, 149(6): 747-53).

Eine Aufgabe der Erfindung bestand darin, neue Verbindungen zur Verfügung zu stellen, welche Vorteile gegenüber den Verbindungen des Standes der Technik aufweisen. Die Verbindungen sollten sich insbesondere als pharmakologische Wirkstoffe in Arzneimitteln eignen, vorzugsweise in Arzneimitteln zur Behandlung von Störungen oder Krankheiten, welche zumindest teilweise durch KCNQ2/3 $K^+$ Kanäle vermittelt werden.

Diese Aufgabe wird durch den Gegenstand der Patentansprüche gelöst.

Es wurde überraschend gefunden, dass sich substituierte Tetrahydrothienopyridine der nachstehend angegebenen allgemeinen Formel (1) zur Behandlung von Schmerzen eignen. Es wurde ferner überraschend gefunden, dass substituierte Tetrahydrothienopyridine der nachstehend angegebenen allgemeinen Formel (1) auch eine ausgezeichnete Affinität zum KCNQ2/3 $K^+$ Kanal aufweisen und sich daher zur Behandlung von Störungen oder Krankheiten eignen, die zumindest teilweise durch KCNQ2/3 $K^+$ Kanäle vermittelt werden. Dabei wirken die substituierten Tetrahydrothienopyridine als Modulatoren, d.h. Agonisten oder Antagonisten, des KCNQ2/3 $K^+$ Kanals.

Aus dem Stand der Technik sind substituierte Tetrahydropyrrolopyrazine bekannt, die eine Affinität zum KCNQ2/3 $K^+$ Kanal aufweisen (WO 2008/046582).

Substituierte Tetrahydrothienopyridine und deren Verwendung in Arzneimitteln werden in WO 96/34870 beschrieben. Ferner sind weitere Tetrahydrothienopyridine bekannt, für die jedoch keine Verwendung in Arzneimitteln beschrieben ist (z.B. CA 940806-85-9; CA 931614-62-9; CA 930990-23-1).

Ein Gegenstand der Erfindung sind substituierte Tetrahydrothienopyridine der allgemeinen Formel (1),

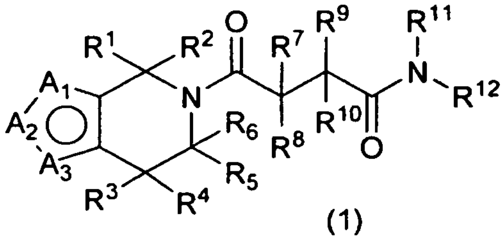

(1)

,

worin

$A_1$ für S, $A_2$ für $CR^{14}$ und $A_3$ für $CR^{15}$ steht; oder
$A_1$ für $CR^{13}$, $A_2$ für S und $A_3$ für $CR^{15}$ steht; oder
A, für $CR^{13}$, $A_2$ für $CR^{14}$ und $A_3$ für S steht;

$R^0$ für $C_{1-8}$-Alkyl, gesättigt oder ungesättigt, verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über $C_{1-8}$-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; steht;

$R^1$ für H; F; Cl; Br; CN; oder $R^0$ steht;

$R^2$ für H, F, Cl, Br oder $C_{1-8}$-Alkyl, gesättigt oder ungesättigt, verzweigt oder unverzweigt, unsubstituiert oder einfach oder mehrfach substituiert; steht;

oder $R^1$ und $R^2$ zusammen mit dem sie verbindenden Kohlenstoffatom als Ringglied ein $C_{3-7}$-Cycloalkyl oder Heterocyclyl bilden, jeweils gesättigt oder ungesättigt, unsubstituiert oder einfach oder mehrfach substituiert, jeweils ggf. mit (Hetero-)aryl kondensiert, unsubstituiert oder einfach oder mehrfach substituiert;

$R^3$, $R^4$, $R^5$, $R^6$ unabhängig voneinander für H; F; Cl; Br; $C_{1-8}$-Alkyl, gesättigt oder ungesättigt, verzweigt oder unverzweigt, unsubstituiert oder einfach oder mehrfach substituiert; oder Phenyl, unsubstituiert oder einfach oder mehrfach substituiert stehen;

$R^7$ für H; F; Cl; Br; CN; $R^0$; $OR^0$; O-C(=O)-$R^0$; O-C(=O)-O-$R^0$; O-(C=O)-NH-$R^0$; O-C(=O)-N($R^0$)$_2$; NH-$R^0$; N($R^0$)$_2$; NH-C(=O)-$R^0$; NH-C(=O)-O-$R^0$; NH-C(=O)-NH-$R^0$; NH-C(=O-N($R^0$)$_2$; $NR^0$-C(=O)-$R^0$; $NR^0$-C(=O)-O-$R^0$; $NR^0$-C(=O)-$NH_2$; $NR^0$-C(=O)-NH-$R^0$; $NR^0$-C(=O)-N($R^0$)$_2$;NH-S(=O)$_2$OH; NH-S(=O)$_2R^0$; NH-S(=O)$_2OR^0$; NH-S(=O)$_2NH_2$; NH-S(=O)$_2NHR^0$; NH-S(=O)$_2$N($R^0$)$_2$; $NR^0$-S(=O)$_2$OH; $NR^0$-S(=O)$_2R^0$; $NR^0$-S(=O)$_2OR^0$; $NR^0$-S(=O)$_2NH_2$; $NR^0$-S(=O)$_2NHR^0$; $NR^0$-S(=O)$_2$N($R^0$)$_2$; C(=O)-OH; C(=O)-$OR^0$; C(=O)-$NH_2$; C(=O)-NH-$R^0$; C(=O)-N($R^0$)$_2$; S(=O)$_2$-OH; S(=O)$_2OR^0$; S(=O)$_2$-$NH_2$; S(=O)$_2$-NH-$R^0$; S(=O)$_2$-N($R^0$)$_2$;

$R^8$ für H; F; Cl; Br; CN; oder $C_{1-8}$-Alkyl, gesättigt oder ungesättigt, verzweigt oder unverzweigt, unsubstituiert oder einfach oder mehrfach substituiert; steht;

$R^9$ für H; F; Cl; Br; CN; $C_{1-8}$-Alkyl, gesättigt oder ungesättigt, verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über $C_{2-8}$-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; O-$C_{1-8}$-Alkyl, gesättigt oder ungesättigt, verzweigt oder unverzweigt, unsubstituiert oder einfach oder mehrfach substituiert; O-$C_{3-7}$-Cycloalkyl oder O-Heterocyclyl, jeweils gesättigt oder ungesättigt, unsubstituiert oder einfach oder mehrfach

substituiert; über O-$C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über O-$C_{1-8}$-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; O-C(=O)-$R^0$; O-C(=O)-O-$R^0$; O-(C=O)-NH-$R^0$; O-C(=O)-N($R^0$)$_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$;NH-S(=O)$_2$OH; NH-S(=O)$_2$$R^0$; NH-S(=O)$_2$O$R^0$; NH-S(=O)$_2$$NH_2$; NH-S(=O)$_2$NH$R^0$; NH-S(=O)$_2$N($R^0$)$_2$; N$R^0$-S(=O)$_2$OH; N$R^0$-S(=O)$_2$$R^0$; N$R^0$-S(=O)$_2$O$R^0$; N$R^0$-S(=O)$_2$$NH_2$; N$R^0$-S(=O)$_2$NH$R^0$; N$R^0$-S(=O)$_2$N($R^0$)$_2$; C(=O)-OH; C(=O)-O$R^0$; C(=O)-$NH_2$; C(=O)-NH-$R^0$; C(=O)-N($R^0$)$_2$; S(=O)$_2$-OH; S(=O)$_2$O$R^0$; S(=O)$_2$-$NH_2$; S(=O)$_2$-NH-$R^0$; S(=O)$_2$-N($R^0$)$_2$ steht;

$R^{10}$ für H; F; Cl; Br; CN; oder $C_{1-8}$-Alkyl, gesättigt oder ungesättigt, verzweigt oder unverzweigt, unsubstituiert oder einfach oder mehrfach substituiert; steht;

oder $R^7$ und $R^9$ zusammen mit den sie verbindenden Kohlenstoffatomen als Ringgliedern ein $C_{3-7}$-Cycloalkyl oder Heterocyclyl bilden, jeweils gesättigt oder ungesättigt, unsubstituiert oder einfach oder mehrfach substituiert, ggf. mit (Hetero-)aryl kondensiert, unsubstituiert oder einfach oder mehrfach substituiert;

oder $R^7$ und $R^8$; oder $R^9$ und $R^{10}$; zusammen mit den sie verbindenden Kohlenstoffatomen als Ringgliedern ein $C_{3-7}$-Cycloalkyl oder Heterocyclyl bilden, jeweils gesättigt oder ungesättigt, unsubstituiert oder einfach oder mehrfach substituiert, jeweils ggf. mit (Hetero-)aryl kondensiert, unsubstituiert oder einfach oder mehrfach substituiert;

$R^{11}$ für H; $C_{1-8}$-Alkyl, gesättigt oder ungesättigt, verzweigt oder unverzweigt, unsubstituiert oder einfach oder mehrfach substituiert; oder $C_{3-7}$-Cycloalkyl, gesättigt oder ungesättigt, unsubstituiert oder einfach oder mehrfach substituiert; steht;

$R^{12}$ für $C_{2-16}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann;

oder $R^{11}$ und $R^{12}$ zusammen mit dem sie verbindenden Stickstoffatom als Ringglied ein Heterocyclyl bilden, gesättigt oder ungesättigt, unsubstituiert oder einfach oder mehrfach substituiert, ggf. mit (Hetero-)aryl kondensiert, unsubstituiert oder einfach oder mehrfach substituiert;

$R^{13}$ $R^{14}$ und $R^{15}$ jeweils unabhängig voneinander H; F; Cl; Br; I; NO; $NO_2$; $CF_3$; CN; $R^0$; C(=O)H; C(=O)$R^0$; $CO_2H$; C(=O)O$R^0$; $CONH_2$; C(=O)NH$R^0$; C(=O)N($R^0$)$_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2$$NH_2$; O-S(=O)$_2$NH$R^0$; O-S(=O)$_2$N($R^0$)$_2$; $NH_2$; NH-$R^0$; N($R^0$)$_2$: NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2$$R^0$; NH-S(=O)$_2$O$R^0$; NH-S(=O)$_2$$NH_2$; NH-S(=O)$_2$NH$R^0$; NH-S(=O)$_2$N($R^0$)$_2$; N$R^0$-S(=O)$_2$OH; N$R^0$-S(=O)$_2$$R^0$, N$R^0$-S(=O)$_2$O$R^0$; N$R^0$-S(=O)$_2$$NH_2$; N$R^0$-S(=O)$_2$NH$R^0$; N$R^0$-S(=O)$_2$N($R^0$)$_2$; SH; S$R^0$; S(=O)$R^0$; S(=O)$_2$$R^0$; S(=O)$_2$OH; S(=O)$_2$O$R^0$; S(=O)$_2$$NH_2$; S(=O)$_2$NH$R^0$; oder S(=O)$_2$N($R^0$)$_2$ bedeuten;

worin "Alkyl substituiert", "Heterocyclyl substituiert" und "Cycloalkyl substituiert" für die Substitution eines oder mehrerer Wasserstoffatome jeweils unabhängig voneinander durch F; Cl; Br; I; CN; $CF_3$; =O; =NH; =C($NH_2$)$_2$; $NO_2$; $R^0$; C(=O)H; C(=O)$R^0$; $CO_2H$; C(=O)O$R^0$; $CONH_2$; C(=O)NH$R^0$; C(=O)N($R^0$)$_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2$$NH_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2$$R^0$NH-S(=O)$_2$O$R^0$; NH-S(=O)$_2$$NH_2$; NH-S(=O)$_2$NH$R^0$; NH-S(=O)$_2$N($R^0$)$_2$; N$R^0$-S(=O)$_2$OH; N$R^0$-S(=O)$_2$$R^0$; N$R^0$-S(=O)$_2$O$R^0$; N$R^0$-S(=O)$_2$$NH_2$; N$R^0$-S(=O)$_2$NH$R^0$; N$R^0$-S(=O)$_2$N($R^0$)$_2$; SH; S$R^0$; S(=O)$R^0$; S(=O)$_2$$R^0$; S(=O)$_2$OH; S(=O)$_2$O$R^0$; S(=O)$_2$$NH_2$; S(=O)$_2$NH$R^0$; S(=O)$_2$N($R^0$)$_2$; steht;

worin "Aryl substituiert" und "Heteroaryl substituiert" für die Substitution eines oder mehrerer Wasserstoffatome jeweils unabhängig voneinander durch F; Cl; Br; I; NO; $NO_2$; $CF_3$; CN; $R^0$; C(=O)H; C(=O)$R^0$; $CO_2H$; C(=O)O$R^0$; $CONH_2$; C(=O)NH$R^0$; C(=O)N$(R^0)_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N$(R^0)_2$; O-S$(=O)_2$-$R^0$; O-S$(=O)_2$OH; O-S$(=O)_2$O$R^0$; O-S$(=O)_2$$NH_2$; O-S$(=O)_2$NH$R^0$; O-S$(=O)_2$N$(R^0)_2$; $NH_2$; NH-$R^0$; N$(R^0)_2$; NH-C(=O)-$R^0$; NH-C(=O)-O-$R^0$; NH-C(=O)-NH-$R^0$; NH-C(=O)-N$(R^0)_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N$(R^0)_2$; NH-S$(=O)_2$OH; NH-S$(=O)_2$$R^0$; NH-S$(=O)_2$O$R^0$; NH-S$(=O)_2$$NH_2$; NH-S$(=O)_2$NH$R^0$; NH-S$(=O)_2$N$(R^0)_2$; N$R^0$-S$(=O)_2$OH; N$R^0$-S$(=O)_2$$R^0$; N$R^0$-S$(=O)_2$O$R^0$; N$R^0$-S$(=O)_2$$NH_2$; N$R^0$-S$(=O)_2$NH$R^0$; N$R^0$-S$(=O)_2$N$(R^0)_2$; SH; S$R^0$; S(=O)$R^0$; S$(=O)_2$$R^0$; S$(=O)_2$OH; S$(=O)_2$O$R^0$; S$(=O)_2$$NH_2$; S$(=O)_2$NH$R^0$; S$(=O)_2$N$(R^0)_2$; steht;

mit Ausnahme der folgenden Verbindungen:

- 1-Morpholino-4-(4-(thiophen-2-yl)-6,7-dihydrothieno[3,2-c]pyridin-5(4H)-yl)butan-1,4-dion,
- 1-(4-Acetylpiperazin-1-yl)-4-(4-(thiophen-2-yl)-6,7-dihydrothieno[3,2-c]pyridin-5(4H)-yl)butan-1,4-dion und
- 1-(3-Phenyl-4,5-dihydropyrazol-1-yl)-4-(4-(thiophen-2-yl)-6,7-dihydrothieno[3,2-c]pyridin-5(4H)-yl)butan-1,4-dion;

in Form der freien Verbindungen oder Salze physiologisch verträglicher Säuren oder Basen.

Der an den Tetrahydropyridin-Ring ankondensierte fünfgliedrige aromatische Ring enthält gemäß der allgemeinen Formel (1) immer ein Schwefelatom und stellt somit ein Thienyl dar, welches zweifach substituiert ist mit den Substituenten $R^{13}$ und/oder $R^{14}$ und/oder $R^{15}$, welche jeweils ggf. H bedeuten können.

Die Ausdrücke "$C_{1-2}$-Alkyl", "$C_{1-8}$-Alkyl", "$C_{1-4}$-Alkyl", "$C_{2-8}$-Alkyl", "$C_{2-16}$-Alkyl", "$C_{4-16}$-Alkyl", umfassen im Sinne dieser Erfindung acyclische gesättigte oder ungesättigte Kohlenwasserstoffreste, die verzweigt oder unverzweigt sowie unsubstituiert oder ein- oder mehrfach substituiert sein können, mit 1 bis 2 bzw. 1 bis 4 bzw. 1 bis 8 bzw. 2 bis 8 bzw. 2 bis 16 bzw. 4 bis 16 C-Atomen, d.h. $C_{1-2}$-Alkanyle und $C_{1-2}$-Alkenyle bzw. $C_{1-4}$-Alkanyle, $C_{1-4}$-Alkenyle und $C_{2-4}$-Alkinyle bzw. $C_{1-8}$-Alkanyle, $C_{1-8}$-Alkenyle und $C_{2-8}$-Alkinyle bzw. $C_{2-8}$-Alkanyle, $C_{2-8}$-Alkenyle und $C_{2-8}$-Alkinyle bzw. $C_{2-16}$-Alkanyle, $C_{2-16}$-Alkenyle und $C_{2-16}$-Alkinyle bzw. $C_{4-16}$-Alkanyle, $C_{4-16}$-Alkenyle und $C_{4-16}$-Alkinyle. Dabei weisen Alkenyle mindestens eine C-C-Doppelbindung und Alkinyle mindestens eine C-C-Dreifachbindung auf. Bevorzugt ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, Ethylenyl (Vinyl), Ethinyl, Propenyl (-$CH_2CH=CH_2$, -$CH=CH-CH_3$, -$C(=CH_2)-CH_3$), Propinyl (-$CH-C\equiv CH$, -$C\equiv C-CH_3$), Butenyl, Butinyl, Pentenyl, Pentinyl, Hexenyl und Hexinyl, Heptenyl, Heptinyl, Octenyl, Octinyl, Nonenyl, Noninyl, Decenyl, Decinyl, Undecenyl, Undecinyl, Dodecenyl, Dodecinyl, Tridecenyl, Tridecinyl, Tetradecenyl, Tetradecinyl, Pentadecenyl, Pentadecinyl, Hexadecenyl und Hexadecinyl umfasst.

Der Ausdruck "Cycloalkyl" oder "$C_{3-7}$-Cycloalkyl" bedeutet für die Zwecke dieser Erfindung cyclische Kohlenwasserstoffe mit 3, 4, 5, 6 oder 7 Kohlenstoffatomen, wobei die Kohlenwasserstoffe gesättigt oder ungesättigt (aber nicht aromatisch), unsubstituiert oder ein- oder mehrfach substituiert sein können. Die Bindung des Cycloalkyls an die an die übergeordnete allgemeine Struktur kann über jedes beliebige und mögliche Ringglied des Cycloalkyl-Restes erfolgen. Die Cycloalkyl-Reste können auch mit weiteren gesättigten, (partiell) ungesättigten, heterocyclischen, aromatischen oder heteroaromatischen Ringsystemen kondensiert sein, die wiederum unsubstituiert oder einfach oder mehrfach substituiert sein können. Die Cycloalkyl-Reste können weiterhin einfach oder mehrfach verbrückt sein wie bspw. im Fall von Adamantyl oder Dicyclopentadienyl. Bevorzugt ist $C_{3-7}$-Cycloalkyl aus der Gruppe ausgewählt, die Cyclopropyl, Cyclobutyl, Cyclopentyl, Cyclohexyl, Cycloheptyl, Cyclopentenyl, Cyclohexenyl und Cycloheptenyl enthält.

Der Begriff "Heterocyclyl" umfasst gesättigte oder ungesättigte (aber nicht aromatische) Cycloalkyle mit drei bis sieben Ringgliedern, in denen ein, zwei oder drei Kohlenstoffatome durch ein Heteroatom jeweils unabhängig voneinander ausgewählt aus der Gruppe S, N oder O ersetzt sind, wobei die Ringglieder unsubstituiert oder ein- oder mehrfach substituiert sein können. Die Bindung des Heterocyclyls an die übergeordnete allgemeine Struktur kann über jedes beliebige und mögliche Ringglied des Heterocyclyl-Restes erfolgen. Die Heterocyclyl-Reste können auch mit weiteren gesättigten, (partiell) ungesättigten oder aromatischen oder heteroaromatischen Ringsystemen kondensiert sein, die wiederum unsubstituiert oder einfach oder mehrfach substituiert sein können. Bevorzugt sind Heterocyclyl-Reste aus der Gruppe Azetidinyl, Aziridinyl, Azepanyl, Chinolinyl, Dioxanyl, Dioxolanyl, Furanyl, Imidazolidinyl, Isoxazolidinyl, Isochinolinyl, Indolinyl, Morpholinyl, Pyranyl, Pyrrolyl, Pyridinyl, Pyrrolyl, Pyrrolidinyl, Piperazinyl, Piperidinyl, Pyrazolidinyl, Pyrazolinonyl oder Thiomorpholinyl. Besonders bevorzugt sind Heterocyclyl-Reste aus der Gruppe Morpholinyl, Piperazinyl oder Piperidinyl.

Der Begriff "Aryl" bedeutet im Sinne dieser Erfindung aromatische Kohlenwasserstoffe mit bis zu 14 Ringgliedern, u.a. Phenyle und Naphthyle. Jeder Aryl-Rest kann unsubstituiert oder einfach oder mehrfach substituiert vorliegen, wobei die ArylSubstituenten gleich oder verschieden und in jeder beliebigen und möglichen Position des Aryls sein können. Die Bindung des Aryls an die übergeordnete allgemeine Struktur kann über jedes beliebige und mögliche Ringglied des ArylRestes erfolgen. Die Aryl-Reste können auch mit weiteren gesättigten, (partiell) ungesättigten, heterocyclischen, aromatischen oder heteroaromatischen Ringsystemen kondensiert sein, die wiederum unsubstituiert oder einfach oder mehrfach substituiert sein können. Bevorzugt ist Aryl aus der Gruppe ausgewählt, die Phenyl, 1-Naphthyl und 2-Naphthyl enthält, welche jeweils unsubstituiert oder ein- oder mehrfach substituiert sein können. Ein besonders bevorzugtes Aryl ist Phenyl, unsubstituiert oder einfach oder mehrfach substituiert.

Der Begriff "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome jeweils unabhängig voneinander ausgewählt sind aus der Gruppe S, N oder O und der Heteroaryl-Rest unsubstituiert oder ein- oder mehrfach substituiert sein kann; im Falle der Substitution am Heteroaryl können die Substituenten gleich oder verschieden sein und in jeder beliebigen und möglichen Position des Heteroaryls sein. Bevorzugte Heteroatome sind S, N und O. Besonders bevorzugt sind S und N. Die Bindung an die übergeordnete allgemeine Struktur kann über jedes beliebige und mögliche Ringglied des Heteroaryl-Restes erfolgen. Das Heteroaryl kann auch Teil eines bi- oder polycyclischen Systems mit bis zu 14 Ringgliedern sein, wobei das Ringsystem mit weiteren gesättigten, (partiell) ungesättigten, heterocyclischen oder aromatischen oder heteroaromatischen Ringen gebildet werden kann, die wiederum unsubstituiert oder einfach oder mehrfach substituiert sein können. Der an den Tetrahydropyridin-Ring ankondensierte Thienyl-Ring der allgemeinen Formel (1) stellt somit im Sinne dieser Erfindung ein Heteroaryl dar und ggf. vorhandene Substituenten sind demnach vorzugsweise definiert wie die vorstehend definierten Substituenten von Heteroaryl. Es ist bevorzugt, dass der Heteroaryl-Rest ausgewählt ist aus der Gruppe, die Benzofuranyl, Benzoimidazolyl, Benzothienyl, Benzothiadiazolyl, Benzothiazolyl, Benzotriazolyl, Benzodioxolanyl, Benzodioxanyl, Chinazolinyl, Carbazolyl, Chinolinyl, Furyl (Furanyl), Imidazolyl, Indazolyl, Indolizinyl, Isochinolinyl, Isoxazoyl, Isothiazolyl, Indolyl, Oxadiazolyl, Phtalazinyl, Pyrazolyl, Pyridyl, Pyrrolyl, Pyridazinyl, Pyrimidinyl, Pyrazinyl, Purinyl, Phenazinyl, Thienyl, Triazolyl, Thiazolyl, Thiadiazolyl oder Triazinyl umfasst. Besonders bevorzugt sind Pyridyl und Thienyl.

Die Ausdrücke "über $C_{1-2}$-Alkyl oder $C_{1-4}$-Alkyl oder $C_{1-8}$-Alkyl oder $C_{2-8}$-Alkyl verbrücktes Aryl, Heteroaryl, Heterocyclyl oder Cycloalkyl" bedeuten im Sinne der Erfindung, dass $C_{1-2}$-Alkyl oder $C_{1-4}$-Alkyl oder $C_{1-8}$-Alkyl oder $C_{2-8}$-Alkyl und Aryl bzw. Heteroaryl bzw. Heterocyclyl bzw. Cycloalkyl die oben definierten Bedeutungen haben und der Aryl- bzw. Heteroaryl- bzw. Heterocyclyl- bzw. Cycloalkyl-Rest über eine $C_{1-2}$-Alkyl- oder eine $C_{1-4}$-Alkyl- oder eine $C_{1-8}$-Alkyl- oder eine $C_{2-8}$-Alkyl-Gruppe an die übergeordnete allgemeine Struktur gebunden ist. Die Alkylkette kann in allen Fällen gesättigt oder ungesättigt, verzweigt oder unverzweigt, unsubstituiert oder einfach oder mehrfach substituiert sein.

Im Zusammenhang mit "Alkyl", "Heterocyclyl" und "Cycloalkyl" versteht man unter dem Begriff "ein- oder mehrfach substituiert" im Sinne dieser Erfindung die ein- oder mehrfache, z.B. zwei-, drei- oder vierfache Substitution eines oder mehrerer Wasserstoffatome jeweils unabhängig voneinander durch Substituenten ausgewählt aus der Gruppe aus F; Cl; Br; I; CN; $CF_3$; =O; =NH; $=C(NH_2)_2$; $NO_2$; $R^0$; C(=O)H; C(=O)$R^0$; $CO_2H$; C(=O)O$R^0$; $CONH_2$; C(=O)NH$R^0$; C(=O)N$(R^0)_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N$(R^0)_2$; O-S$(=O)_2$-$R^0$; O-S$(=O)_2$OH; O-S$(=O)_2$O$R^0$; O-S$(=O)_2$$NH_2$; O-S$(=O)_2$NH$R^0$; O-S$(=O)_2$N$(R^0)_2$; $NH_2$: NH-$R^0$; N$(R^0)_2$; NH-C(=O)-$R^0$; NH-C(=O)-O-$R^0$; NH-C(=O)-NH-$R^0$; NH-C(=O)-N$(R^0)_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N$(R^0)_2$; NH-S$(=O)_2$OH; NH-S$(=O)_2$$R^0$; NH-S$(=O)_2$O$R^0$; NH-S$(=O)_2$$NH_2$; NH-S$(=O)_2$NH$R^0$; NH-S$(=O)_2$N$(R^0)_2$; N$R^0$-S$(=O)_2$OH; N$R^0$-S$(=O)_2$$R^0$; N$R^0$-S$(=O)_2$O$R^0$; N$R^0$-S$(=O)_2$$NH_2$; N$R^0$-S$(=O)_2$NH$R^0$; N$R^0$-S$(=O)_2$N$(R^0)_2$; SH; S$R^0$; S(=O)$R^0$; S$(=O)_2$$R^0$; S$(=O)_2$OH; S$(=O)_2$O$R^0$; S$(=O)_2$$NH_2$; S$(=O)_2$NH$R^0$; S$(=O)_2$N$(R^0)_2$;, wobei unter mehrfach substituierten Resten solche Reste zu verstehen sind, die entweder an verschiedenen oder an gleichen Atomen mehrfach, z.B. zwei-, drei- oder vierfach substituiert sind, beispielsweise dreifach am gleichen C-Atom wie im Falle von $CF_3$ oder $CH_2CF_3$ oder an verschiedenen Stellen wie im Falle von CH(OH)-CH=CH-$CHCl_2$. Ein Substituent kann gegebenenfalls seinerseits wiederum einfach oder mehrfach substituiert sein. Die Mehrfachsubstitution kann mit dem gleichen oder mit verschiedenen Substituenten erfolgen.

Bevorzugte "Alkyl", "Heterocyclyl-" und "Cycloalkyl-" Substituenten sind F; Cl; Br; I; CN; =O; =NH; $=C(NH_2)_2$; $NO_2$; Benzyl; $C_{1-8}$-Alkyl; $CF_3$; C(=O)H; C(=O)OH; C(=O)$C_{1-8}$-Alkyl; C(=O)Aryl; C(=O)Heteroaryl; C(=O)O-$C_{1-8}$-Alkyl; C(=O)O-Aryl; C(=O)O-Heteroaryl; C(=O)$NH_2$; C(=O)NH-$C_{1-8}$-Alkyl; C(=O)N($C_{1-8}$-Alkyl)$_2$; C(=O)NH-Aryl; C(=O)N(Aryl)2; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Aryl)(Heteroaryl); OH; O-$C_{1-8}$-Alkyl; O-$C_{1-8}$-Alkyl-OH; O-($C_{1-8}$-Alkyl)-O; O-($C_{1-8}$-Alkyl)-O-$C_{1-8}$-Alkyl; O$CF_3$; O-Aryl; O-Heteroaryl; O-Benzyl; O-C(=O)-$C_{1-8}$-Alkyl; O-C(=O)-Aryl; O-C(=O)-Heteroaryl; O-S$(=O)_2$OH; O-S$(=O)_2$-O$C_{1-8}$-Alkyl; O-S$(=O)_2$-O-Aryl; O-S$(=O)_2$-O-Heteroaryl; O-S$(=O)_2$$C_{1-8}$-Alkyl; O-S$(=O)_2$Aryl, O-S$(=O)_2$Heteroaryl; O-S$(=O)_2$$NH_2$; O-S$(=O)_2$NH-$C_{1-8}$-Alkyl; O-S$(=O)_2$N($C_{1-8}$-Alkyl)$_2$; O-S$(=O)_2$NH-Aryl; ; O-S$(=O)_2$N(Aryl)$_2$; O-S$(=O)_2$NH-Heteroaryl; O-S$(=O)_2$N(Heteroaryl)$_2$; O-S$(=O)_2$N($C_{1-8}$-Alkyl)(Aryl); O-S$(=O)_2$N(Heteroaryl)(Aryl); O-S$(=O)_2$N($C_{1-8}$-Alkyl)(Heteroaryl); $NH_2$; NH(OH); N=C$(NH_2)_2$; NH-$C_{1-8}$-Alkyl; NH-$C_{1-8}$-Alkyl-OH; N($C_{1-8}$-Alkyl)$_2$; N($C_{1-8}$-Alkyl-OH)$_2$ NH-Aryl; N(Aryl)$_2$; NH-Heteroaryl; N(Heteroaryl)$_2$; N($C_{1-8}$-Alkyl)(Aryl); N($C_{1-8}$-Alkyl)(Heteroaryl); N(Aryl)(Heteroaryl); NH-C(=O)$C_{1-8}$-Alkyl; NH-C(=O)-Aryl; NH-C(=O)-Heteroaryl; N($C_{1-8}$-Alkyl)-C(=O)$C_{1-8}$-Alkyl; N($C_{1-8}$-Alkyl)-C(=O)Aryl; N($C_{1-8}$-Alkyl)-C(=O)Heteroaryl; N(Aryl)-C(=O)$C_{1-8}$-Alkyl; N(Aryl)-C(=O)Aryl; N(Aryl)-C(=O) Heteroaryl; N(Heteroaryl)-C(=O)$C_{1-8}$-Alkyl; N(Heteroaryl)-C(=O)Aryl; N(Heteroaryl)-C(=O)Heteroaryl; NH-S$(=O)_2$OH; NH-S$(=O)_2$$C_{1-8}$-Alkyl; NH-S$(=O)_2$Aryl; NH-S$(=O)_2$Heteroaryl;

NH-S(=O)$_2$OC$_{1-8}$-Alkyl; NH-S(=O)$_2$O-Aryl; NH-S(=O)$_2$O-Heteroaryl; NH-S(=O)$_2$NH$_2$; NH-S(=O)$_2$NH(C$_{1-8}$-Alkyl); NH-S(=O)$_2$NH(Aryl); NH-S(=O)$_2$NH(Heteroaryl); NH-S(=O)$_2$N(C$_{1-8}$-Alkyl)$_2$; NH-S(=O)$_2$N(Aryl)$_2$; NH-S(=O)$_2$N(Heteroaryl)$_2$; NH-S(=O)2N(C$_{1-8}$-Alkyl)(Aryl); NH-S(=O)$_2$N(C$_{1-8}$-Alkyl)(Heteroaryl); NH-S(=O)$_2$N(Aryl)(Heteroaryl); N(C$_{1-8}$-Alkyl)-S(=O)$_2$OH; N(Aryl)-S(=O)$_2$OH; N(Heteroaryl)-S(=O)$_2$OH; N(C$_{1-8}$-Alkyl)-S(=O)$_2$-C$_{1-8}$-Alkyl; N(C$_{1-8}$-Alkyl)-S(=O)$_2$-Aryl; N(C$_{1-8}$-Alkyl)-S(=O)$_2$-Heteroaryl; N(Aryl)-S(=O)$_2$-C$_{1-8}$-Alkyl; N(Aryl)-S(=O)$_2$-Aryl; N(Aryl)-S(=O)$_2$-Heteroaryl; N(Heteroaryl)-S(=O)$_2$-C$_{1-8}$-Alkyl; N(Heteroaryl)-S(=O)$_2$-Aryl; N(Heteroaryl)-S(=O)$_2$-Heteroaryl; N(C$_{1-8}$-Alkyl)-S(=O)$_2$-OC$_{1-8}$-Alkyl; N(C$_{1-8}$-Alkyl)-S(=O)$_2$-O-Aryl; N(C$_{1-8}$-Alkyl)-S(=O)$_2$-O-Heteroaryl; N(Aryl)-S(=O)$_2$-OC$_{1-8}$-Alkyl; N(Aryl)-S(=O)$_2$-O-Aryl-, N(Aryl)-S(=O)$_2$-O-Heteroaryl; N(Heteroaryl)-S(=O)$_2$-OC$_{1-8}$-Alkyl; N(Heteroaryl)-S(=O)$_2$-O-Aryl; N(Heteroaryl)-S(=O)2-O-Heteroaryl; N(C$_{1-8}$-Alkyl)-S(=O)$_2$NH$_2$; N(Aryl)-S(=O)$_2$NH$_2$; N(Heteroaryl)-S(=O)$_2$NH$_2$; N(C$_{1-8}$-Alkyl)-S(=O)$_2$NH(C$_{1-8}$-Alkyl); N(C$_{1-8}$-Alkyl)-S(=O)$_2$NH(Aryl); N(C$_{1-8}$-Alkyl)-S(=O)$_2$NH(Heteroaryl); N(C$_{1-8}$-Alkyl)-S(=O)$_2$N(C$_{1-8}$-Alkyl)$_2$; N(C$_{1-8}$-Alkyl)-S(=O)$_2$N(Aryl)$_2$; N(C$_{1-8}$-Alkyl)-S(=O)$_2$N(Heteroaryl)$_2$; N(C$_{1-8}$-Alkyl)-S(=O)$_2$N(C$_{1-8}$-Alkyl)(Aryl); N(C$_{1-8}$-Alkyl)-S(=O)2N(C$_{1-8}$-Alkyl)(Heteroaryl); N(C$_{1-8}$-Alkyl)-S(=O)$_2$N(Aryl)(Heteroaryl); N(Aryl)-S(=O)$_2$NH(C$_{1-8}$-Alkyl); N(Aryl)-S(=O)$_2$NH(Aryl); N(Aryl)-S(=O)$_2$NH(Heteroaryl); N(Aryl)-S(=O)$_2$N(C$_{1-8}$-Alkyl)$_2$; N(Aryl)-S(=O)$_2$N(Aryl)$_2$; N(Aryl)-S(=O)$_2$N(Heteroaryl)$_2$; N(Aryl)-S(=O)$_2$N(C$_{1-8}$-Alkyl)(Aryl); N(Aryl)-S(=O)$_2$N(C$_{1-8}$-Alkyl)(Heteroaryl); N(Aryl)-S(=O)$_2$N(Aryl)(Heteroaryl); N(Heteroaryl)-S(=O)$_2$NH(C$_{1-8}$-Alkyl); N(Heteroaryl)-S(=O)$_2$NH(Aryl); N(Heteroaryl)-S(=O)$_2$NH(Heteroaryl); N(Heteroaryl)-S(=O)$_2$N(C$_{1-8}$-Alkyl)$_2$; N(Heteroaryl)-S(=O)$_2$N(Aryl)$_2$; N(Heteroaryl)-S(=O)$_2$N(Heteroaryl)$_2$; N(Heteroaryl)-S(=O)$_2$N(C$_{1-8}$-Alkyl)(Aryl); N(Heteroaryl)-S(=O)$_2$N(C$_{1-8}$-Alkyl)(Heteroaryl); N(Heteroaryl)-S(=O)$_2$N(Aryl)(Heteroaryl); SH; SCF$_3$; S-C$_{1-8}$-Alkyl; S-Benzyl; S-Aryl; S-Heteroaryl; S(=O)$_2$OH; S(=O)$_2$-OC$_{1-8}$-Alkyl; S(=O)$_2$-O-Aryl; S(=O)$_2$-O-Heteroaryl; S(=O)C$_{1-8}$-Alkyl; S(=O)Aryl, S(=O)Heteroaryl; S(=O)$_2$C$_{1-8}$-Alkyl; S(=O)$_2$Aryl, S(=O)$_2$Heteroaryl; S(=O)$_2$NH$_2$; S(=O)$_2$NH-C$_{1-8}$-Alkyl: S(=O)$_2$N(C$_{1-8}$-Alkyl)$_2$; S(=O)$_2$NH-Aryl; S(=O)$_2$N(Aryl)$_2$; S(=O)$_2$NH-Heteroaryl; S(=O)$_2$N(Heteroaryl)$_2$; S(=O)$_2$N(C$_{1-8}$-Alkyl)(Aryl); S(=O)$_2$N(Heteroaryl)(Aryl); S(=O)$_2$N(C$_{1-8}$-Alkyl)(Heteroaryl); Aryl, Heteroaryl, C$_{3-7}$-Cycloalkyl, Heterocyclyl oder C$_{1-8}$-Alkyl verbrücktes Aryl, Heteroaryl, C$_{3-7}$-Cycloalkyl oder Heterocyclyl. Ein Substituent kann ggf. seinerseits wiederum einfach oder mehrfach substituiert sein kann. Die Mehrfachsubstitution erfolgt mit dem gleichen oder mit unterschiedlichen Substituenten.

Im Zusammenhang mit "Aryl" und "Heteroaryl" versteht man im Sinne dieser Erfindung unter "ein- oder mehrfach substituiert" die ein- oder mehrfache, z.B. zwei-, drei- oder vierfache Substitution eines oder mehrerer Wasserstoffatome des Ringsystems jeweils unabhängig voneinander durch Substituenten ausgewählt aus der Gruppe aus F; Cl; Br; I; NO; NO$_2$; CF$_3$; CN; R$^0$; C(=O)H; C(=O)R$^0$; CO$_2$H; C(=O)OR$^0$; CONH$_2$; C(=O)NHR$^0$; C(=O)N(R$^0$)$_2$; OH; O-(C$_{1-8}$-Alkyl)-O; OR$^0$; O-C(=O)-R$^0$; O-C(=O)-O-R$^0$; O-(C=O)-NH-R$^0$; O-C(=O)-N(R$^0$)$_2$; O-S(=O)$_2$-R$^0$; O-S(=O)$_2$OH; O-S(=O)$_2$OR$^0$; O-S(=O)$_2$NH$_2$; O-S(=O)$_2$NHR$^0$; O-S(=O)$_2$N(R$^0$)$_2$; NH$_2$; NH-R$^0$; N(R$^0$)$_2$; NH-C(=O)-R$^0$; NH-C(=O)-O-R$^0$; NH-C(=O)-NH-R$^0$; NH-C(=O)-N(R$^0$)$_2$; NR$^0$-C(=O)-R$^0$; NR$^0$-C(=O)-O-R$^0$; NR$^0$-C(=O)-NH$_2$; NR$^0$-C(=O)-NH-R$^0$; NR$^0$-C(=O)-N(R$^0$)$_2$; NH-S(=O)$_2$OH; NH-S(=O)$_2$R$^0$; NH-S(=O)$_2$OR$^0$; NH-S(=O)$_2$NH$_2$; NH-S(=O)$_2$NHR$^0$; NH-S(=O)$_2$N(R$^0$)$_2$; NR$^0$-S(=O)$_2$OH; NR$^0$-S(=O)$_2$R$^0$; NR$^0$-S(=O)$_2$OR$^0$; NR$^0$-S(=O)$_2$NH$_2$; NR$^0$-S(=O)$_2$NHR$^0$; NR$^0$-S(=O)$_2$N(R$^0$)$_2$; SH; SR$^0$; S(=O)R$^0$; S(=O)$_2$R$^0$; S(=O)$_2$O$_H$; S(=O)$_2$OR$^0$; S(=O)$_2$NH$_2$; S(=O)$_2$NHR$^0$; S(=O)$_2$N(R$^0$)$_2$ an einem oder ggf. verschiedenen Atomen, wobei ein Substituent ggf. seinerseits wiederum einfach oder mehrfach substituiert sein kann. Die Mehrfachsubstitution erfolgt mit dem gleichen oder mit unterschiedlichen Substituenten.

Bevorzugte "Aryl-" und "Heteroaryl-" Substituenten sind F; Cl; Br; I; CN; NH$_2$; OCF$_3$; SCF$_3$; S(=O)CF$_3$; S(=O)$_2$CF$_3$; NH(OH); NO; NO$_2$; CF$_2$H; OCF$_2$H; SCF$_2$H; Benzyl; C$_{1-8}$-Alkyl; CF$_3$; C(=O)H; C(=O)OH; C(=O)C$_{1-8}$-Alkyl; C(=O)Aryl; C(=O)Heteroaryl; C(=O)O-C$_{1-8}$-Alkyl; C(=O)O-Aryl; C(=O)O-Heteroaryl; C(=O)NH$_2$; C(=O)NH-C$_{1-8}$-Alkyl; C(=O)N(C$_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N(C$_{1-8}$-Alkyl)(Aryl); C(=O)N(C$_{1-8}$-Alkyl)(Heteroaryl); C(=O)N(Aryl)(Heteroaryl); OH; O-C$_{1-8}$-Alkyl; O-C$_{1-8}$-Alkyl-OH; O-(C$_{1-8}$-Alkyl)-O; O-(C$_{1-8}$-Alkyl)-O-C$_{1-8}$-Alkyl; O-Aryl; O-Heteroaryl; O-Benzyl; O-C(=O)-C$_{1-8}$-Alkyl; O-C(=O)-Aryl; O-C(=O)-Heteroaryl; O-S(=O)$_2$OH; O-S(=O)$_2$-OC$_{1-8}$-Alkyl; O-S(=O)$_2$-O-Aryl; O-S(=O)$_2$-O-Heteroaryl; O-S(=O)$_2$C$_{1-8}$-Alkyl; O-S(=O)$_2$Aryl, O-S(=O)$_2$Heteroaryl; O-S(=O)$_2$NH$_2$-, O-S(=O)$_2$NH-C$_{1-8}$-Alkyl; O-S(=O)$_2$N(C$_{1-8}$-Alkyl)$_2$; O-S(=O)$_2$NH-Aryl; O-S(=O)$_2$N(Aryl)$_2$., O-S(=O)$_2$NH-Heteroaryl; O-S(=O)$_2$N(Heteroaryl)$_2$; O-S(=O)$_2$N(C$_{1-8}$-Alkyl)(Aryl); O-S(=O)$_2$N(Heteroaryl)(Aryl); O-S(=O)$_2$N(C$_{1-8}$-Alkyl)(Heteroaryl); NH$_2$; NH(OH); N=C(NH$_2$)$_2$; NH-C$_{1-8}$-Alkyl; NH-C$_{1-8}$-Alkyl-OH; N(C$_{1-8}$-Alkyl)$_2$; N(C$_{1-8}$-Alkyl-OH)$_2$; NH-Aryl; N(Aryl)$_2$; NH-Heteroaryl; N(Heteroaryl)$_2$; N(C$_{1-8}$-Alkyl)(Aryl); N(C$_{1-8}$-Alkyl)(Heteroaryl); N(Aryl)(Heteroaryl); NH-C(=O)C$_{1-8}$-Alkyl; NH-C(=O)-Aryl; NH-C(=O)-Heteroaryl; N(C$_{1-8}$-Alkyl)-C(=O)C$_{1-8}$-Alkyl; N(C$_{1-8}$-Alkyl)-C(=O)Aryl; N(C$_{1-8}$-Alkyl)-C(=O)Heteroaryl; N(Aryl)-C(=O)C$_{1-8}$-Alkyl; N(Aryl)-C(=O)Aryl; N(Aryl)-C(=O)Heteroaryl; N(Heteroaryl)-C(=O)C$_{1-8}$-Alkyl; N(Heteroaryl)-C(=O)Aryl; N(Heteroaryl)-C(=O)Heteroaryl; NH-S(=O)$_2$OH; NH-S(=O)$_2$C$_{1-8}$-Alkyl; NH-S(=O)$_2$Aryl; NH-S(=O)$_2$Heteroaryl; NH-S(=O)$_2$OC$_{1-8}$-Alkyl; NH-S(=O)$_2$O-Aryl; NH-S(=O)$_2$O-Heteroaryl; NH-S(=O)$_2$NH$_2$; NH-S(=O)$_2$NH(C$_{1-8}$-Alkyl); NH-S(=O)$_2$NH(Aryl); NH-S(=O)$_2$NH(Heteroaryl); NH-S(=O)$_2$N(C$_{1-8}$-Alkyl)$_2$; NH-S(=O)$_2$N(Aryl)$_2$; NH-S(=O)$_2$N(Heteroaryl)$_2$; NH-S(=O)$_2$N(C$_{1-8}$-Alkyl)(Aryl); NH-S(=O)$_2$N(C$_{1-8}$-Alkyl)(Heteroaryl); NH-S(=O)$_2$N(Aryl)(Heteroaryl); N(C$_{1-8}$-Alkyl)-S(=O)$_2$OH; N(Aryl)-S(=O)$_2$OH; N(Heteroaryl)-S(=O)$_2$OH; N(C$_{1-8}$-Alkyl)-S(=O)$_2$-C$_{1-8}$-Alkyl; N(C$_{1-8}$-Alkyl)-S(=O)$_2$-Aryl; N(C$_{1-8}$-Alkyl)-S(=O)$_2$-Heteroaryl; N(Aryl)-S(=O)$_2$-C$_{1-8}$-Alkyl; N(Aryl)-S(=O)$_2$-Aryl; N(Aryl)-S(=O)$_2$-Heteroaryl; N(Heteroaryl)-S(=O)$_2$-C$_{1-8}$-Alkyl; N(Heteroaryl)-S(=O)$_2$-Aryl; N(Heteroaryl)-S(=O)$_2$-Heteroaryl; N(C$_{1-8}$-Al-

kyl)-$S(=O)_2$-$OC_{1-8}$-Alkyl; N($C_{1-8}$-Alkyl)-$S(=O)_2$-O-Aryl; N($C_{1-8}$-Alkyl)-$S(=O)_2$-O-Heteroaryl; N(Aryl)-$S(=O)_2$-$OC_{1-8}$-Alkyl; N(Aryl)-$S(=O)_2$-O-Aryl-, N(Aryl)-$S(=O)_2$-O-Heteroaryl; N(Heteroaryl)-$S(=O)_2$-$OC_{1-8}$-Alkyl; N(Heteroaryl)-$S(=O)_2$-O-Aryl; N(Heteroaryl)-$S(=O)_2$-O-Heteroaryl; N($C_{1-8}$-Alkyl)-$S(=O)_2NH_2$; N(Aryl)-$S(=O)_2NH_2$; N(Heteroaryl)-$S(=O)_2NH_2$; N($C_{1-8}$-Alkyl)-$S(=O)_2$NH($C_{1-8}$-Alkyl); N($C_{1-8}$-Alkyl)-$S(=O)_2$NH(Aryl); N($C_{1-8}$-Alkyl)-$S(=O)_2$NH(Heteroaryl); N($C_{1-8}$-Alkyl)-$S(=O)_2$N($C_{1-8}$-Alkyl)$_2$; N($C_{1-8}$-Alkyl)-$S(=O)_2$N(Aryl)$_2$; N($C_{1-8}$-Alkyl)-$S(=O)_2$N(Heteroaryl)$_2$; N($C_{1-8}$-Alkyl)-$S(=O)_2$N($C_{1-8}$-Alkyl)(Aryl); N($C_{1-8}$-Alkyl)-$S(=O)_2$N($C_{1-8}$-Alkyl)(Heteroaryl); N($C_{1-8}$-Alkyl)-$S(=O)_2$N(Aryl)(Heteroaryl); N(Aryl)-$S(=O)_2$NH($C_{1-8}$-Alkyl); N(Aryl)-$S(=O)_2$NH(Aryl); N(Aryl)-$S(=O)_2$NH(Heteroaryl); N(Aryl)-$S(=O)_2$N($C_{1-8}$-Alkyl)$_2$, N(Aryl)-$S(=O)_2$N(Aryl)$_2$; N(Aryl)-$S(=O)_2$N(Heteroaryl)$_2$; N(Aryl)-$S(=O)_2$N($C_{1-8}$-Alkyl)(Aryl); N (Aryl)-$S(=O)_2$N($C_{1-8}$-Alkyl)(Heteroaryl); N(Aryl)-$S(=O)_2$N(Aryl)(Heteroaryl); N(Heteroaryl)-$S(=O)_2$NH($C_{1-8}$-Alkyl); N(Heteroaryl)-$S(=O)_2$NH(Aryl); N(Heteroaryl)-$S(=O)_2$NH(Heteroaryl); N(Heteroaryl)-$S(=O)_2$N($C_1$-8-Alkyl)$_2$; N(Heteroaryl)-$S(=O)_2$N(Aryl)$_2$; N(Heteroaryl)-$S(=O)_2$N(Heteroaryl)$_2$; N(Heteroaryl)-$S(=O)_2$N($C_{1-8}$-Alkyl)(Aryl); N(Heteroaryl)-$S(=O)_2$N($C_{1-8}$-Alkyl)(Heteroaryl); N(Heteroaryl)-$S(=O)_2$N(Aryl)(Heteroaryl); SH; S-$C_{1-8}$-Alkyl; S-Benzyl; S-Aryl; S-Heteroaryl; $S(=O)_2OH$; $S(=O)_2$-$OC_{1-8}$-Alkyl; $S(=O)_2$-O-Aryl; $S(=O)_2$-O-Heteroaryl; $S(=O)_2C_{1-8}$-Alkyl; $S(=O)_2$Aryl, $S(=O)_2$Heteroaryl; $S(=O)C_{1-8}$-Alkyl; S(=O)Aryl, S(=O)Heteroaryl; $S(=O)_2NH_2$; $S(=O)_2NH$-$C_{1-8}$-Alkyl; $S(=O)_2$N($C_{1-8}$-Alkyl)$_2$; $S(=O)_2$NH-Aryl; $S(=O)_2$N(Aryl)$_2$; $S(=O)_2$NH-Heteroaryl; $S(=O)_2$N(Heteroaryl)$_2$; $S(=O)_2$N($C_{1-8}$-Alkyl)(Aryl); $S(=O)_2$N(Heteroaryl)(Aryl); $S(=O)_2$N($C_{1-8}$-Alkyl)(Heteroaryl); Aryl, Heteroaryl, $C_{3-7}$-Cycloalkyl, Heterocyclyl oder $C_{1-8}$-Alkyl verbrücktem Aryl, Heteroaryl, $C_{3-7}$-Cycloalkyl oder Heterocyclyl. Ein Substituent kann ggf. seinerseits wiederum einfach oder mehrfach substituiert sein kann. Die Mehrfachsubstitution erfolgt mit dem gleichen oder mit unterschiedlichen Substituenten. Besonders bevorzugte Substituenten sind F, Cl, $OCH_3$, $CF_3$, $OCF_3$, $SCF_3$ und $CH_3$.

Die erfindungsgemäßen Verbindungen sind durch Substituenten definiert, beispielsweise durch $R^1$, $R^2$ und $R^3$ (Substituenten der 1. Generation), welche ihrerseits ggf. substituiert sind (Substituenten der 2. Generation). Je nach Definition können diese Substituenten der Substituenten ihrerseits erneut substituiert sein (Substituenten der 3. Generation). Ist beispielsweise $R^1 = R^0$ wobei $R^0$ = Aryl (Substituent der 1. Generation), so kann Aryl seinerseits substituiert sein, z.B. mit $NHR^0$, wobei $R^0 = C_{1-8}$-Alkyl (Substituent der 2. Generation). Es ergibt sich daraus die funktionelle Gruppe Aryl-$NHC_{1-8}$-Alkyl. $C_{1-8}$-Alkyl kann dann seinerseits erneut substituiert sein, z.B. mit Cl (Substituent der 3. Generation). Es ergibt sich daraus dann insgesamt die funktionelle Gruppe Aryl-$NHC_{1-8}$-Alkyl -Cl.

In einer bevorzugten Ausführungsform können die Substituenten der 3. Generation jedoch nicht erneut substituiert sein, d.h. es gibt dann keine Substituenten der 4. Generation.

In einer anderen bevorzugten Ausführungsform können die Substituenten der 2. Generation nicht erneut substituiert sein, d.h. es gibt dann bereits keine Substituenten der 3. Generation. Mit anderen Worten können in dieser Ausführungsform die funktionellen Gruppen für $R^0$ bis $R^{35}$ jeweils ggf. substituiert sein, die jeweiligen Substituenten können dann ihrerseits jedoch nicht erneut substituiert sein.

Wenn ein Rest innerhalb eines Moleküls mehrfach vorkommt, wie z.B. der Rest $R^0$, dann kann dieser Rest für verschiedene Substituenten jeweils unterschiedliche Bedeutungen haben: sind beispielsweise sowohl $R^1 = R^0$ als auch $R^7 = R^0$, so kann $R^0$ für $R^1$ = Aryl und $R^0$ für $R^7 = C_{1-8}$-Alkyl bedeuten.

In einigen Fällen sind die erfindungsgemäßen Verbindungen durch Substituenten definiert, die zusammen mit dem oder den sie verbindenden Kohlenstoff- oder Heteroatom(en) als Ringglied oder als Ringgliedern einen Ring bilden, beispielsweise ein $C_{3-7}$-Cycloalkyl oder ein Heterocyclyl, jeweils gesättigt oder ungesättigt, unsubstituiert oder einfach oder mehrfach substituiert. Diese so gebildeten Ringsysteme können ggf. mit (Hetero-)aryl kondensiert sein, d.h. mit einem Aryl wie Phenyl oder einem Heteroaryl wie Pyridyl, wobei der (Hetero-)aryl-Rest unsubstituiert oder einfach oder mehrfach substituiert sein kann. Vorzugsweise sind die so gebildeten Ringsysteme mit einem Aryl kondensiert, besonders bevorzugt mit Phenyl. Bilden die Substituenten $R^{11}$ und $R^{12}$ beispielsweise mit dem sie verbindenden Stickstoffatom einen Piperidin-Ring, so kann dieser Piperidin-Ring mit Phenyl zu Tetrahydroisochinolinyl kondensiert sein.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, $\alpha$-Liponsäure, Acetylglycin, Hippursäure, Phosphorsäure und/oder Asparaginsäure. Besonders bevorzugt sind die Zitronensäure und die Salzsäure.

Physiologisch verträgliche Salze mit Kationen oder Basen sind Salze der jeweiligen Verbindung - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calcium-Salze.

Bevorzugte Ausführungsformen der erfindungsgemäßen Verbindungen der allgemeinen Formel (1) haben die

allgemeine Formel (1a), (1b) oder (1c):

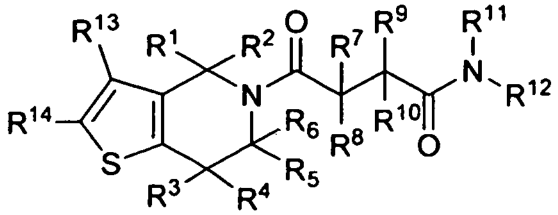

(1a)

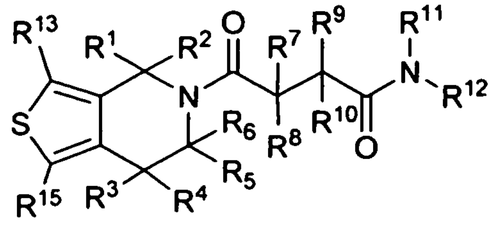

(1b)

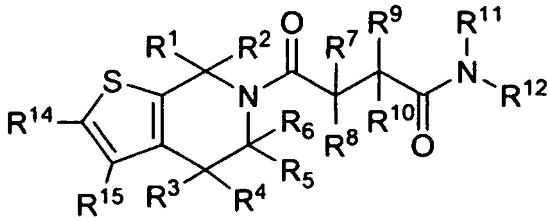

(1c)

worin $R^{13}$, $R^{14}$ und $R^{15}$ jeweils unabhängig voneinander H; F; Cl; Br; I; NO; $NO_2$; $CF_3$; CN; $R^0$; C(=O)H; C(=O)$R^0$; $CO_2H$; C(=O)O$R^0$; $CONH_2$; C(=O)NH$R^0$; C(=O)N$(R^0)_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N$(R^0)_2$; O-S$(=O)_2$-$R^0$; O-S$(=O)_2$OH; O-S$(=O)_2$O$R^0$; O-S$(=O)_2NH_2$; O-S$(=O)_2$NH$R^0$; O-S$(=O)_2$N$(R^0)_2$; $NH_2$; NH-$R^0$; N$(R^0)_2$; NH-C(=O)-$R^0$; NH-C(=O)-O-$R^0$; NH-C(=O)-NH-$R^0$; NH-C(=O)-N$(R^0)_2$; N$R^0$-C(=O)-$R^0$; N$R^0$-C(=O)-O-$R^0$; N$R^0$-C(=O)-$NH_2$-, N$R^0$-C(=O)-NH-$R^0$; N$R^0$-C(=O)-N$(R^0)_2$; NH-S$(=O)_2$OH; NH-S$(=O)_2R^0$; NH-S$(=O)_2$O$R^0$; NH-S$(=O)_2NH_2$; NH-S$(=O)_2$NH$R^0$; NH-S$(=O)_2$N$(R^0)_2$; N$R^0$-S$(=O)_2$OH; N$R^0$-S$(=O)_2R^0$; N$R^0$-S$(=O)_2$O$R^0$; N$R^0$-S$(=O)_2NH_2$; N$R^0$-S$(=O)_2$NH$R^0$; N$R^0$-S$(=O)_2$N$(R^0)_2$; SH; S$R^0$; S(=O)$R^0$; S$(=O)_2R^0$; S$(=O)_2$OH; S$(=O)_2$O$R^0$; S$(=O)_2NH_2$; S$(=O)_2$NH$R^0$; oder S$(=O)_2$N$(R^0)_2$ bedeuten;

Besonders bevorzugt sind Verbindungen der allgemeinen Formeln (1a) und (1c). Ganz besonders bevorzugt sind Verbindungen der allgemeinen Formel (1a).

Weitere bevorzugte Ausführungsformen der erfindungsgemäßen Verbindungen der allgemeinen Formel (1) und der allgemeinen Formeln (1a), (1b) und (1c) besitzen die allgemeine Formel (2a), (2b) oder (2c):

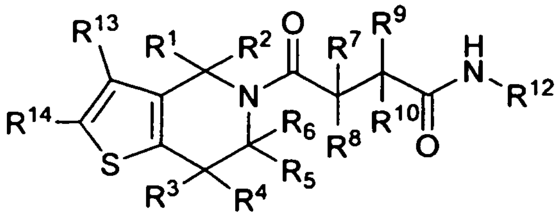

(2a)

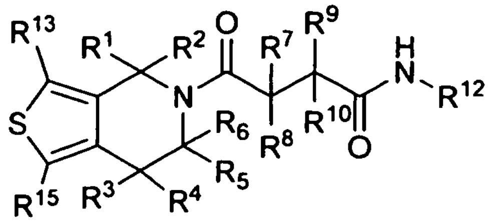

(2b)

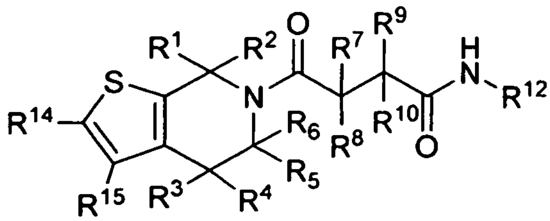

(2c)

$R^{13}$, $R^{14}$ und $R^{15}$ jeweils unabhängig voneinander H; F; Cl; Br; I; NO; $NO_2$; $CF_3$; CN; $R^0$; C(=O)H; C(=O)$R^0$; $CO_2H$; C(=O)O$R^0$; $CONH_2$; C(=O)NH$R^0$; C(=O)N$(R^0)_2$; OH; O$R^0$; O-($C_{1-8}$-Alkyl)-O; O-C(=O)-$R^0$; O-C(=O)-O-$R^0$;

O-(C=O)-NH-$R^0$; O-C(=O)-N($R^0$)2; O-S(=O)$_2$-$R^0$; O-S(=O)$_2$OH; O-S(=O)$_2$O$R^0$; O-S(=O)$_2$NH$_2$; O-S(=O)$_2$NH$R^0$; O-S(=O)$_2$N($R^0$)$_2$; NH$_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-NH$_2$; N$R^0$-C(=O)-NH-$R^0$; N$R^0$-C(=O)-N($R^0$)$_2$; NH-S(=O)$_2$OH; NH-S(=O)$_2$$R^0$; NH-S(=O)$_2$O$R^0$; NH-S(=O)$_2$NH$_2$; NH-S(=O)$_2$NH$R^0$; NH-S(=O)$_2$N($R^0$)$_2$; N$R^0$-S(=O)$_2$OH; N$R^0$-S(=O)$_2$$R^0$; N$R^0$-S(=O)$_2$O$R^0$; N$R^0$-S(=O)$_2$NH$_2$; N$R^0$-S(=O)$_2$NH$R^0$; N$R^0$-S(=O)$_2$N($R^0$)$_2$; SH; S$R^0$; S(=O)$R^0$; S(=O)$_2$$R^0$; S(=O)$_2$OH; S(=O)$_2$O$R^0$; S(=O)$_2$NH$_2$; S(=O)$_2$NH$R^0$; oder S(=O)$_2$N($R^0$)$_2$ bedeuten;

Besonders bevorzugt sind Verbindungen der allgemeinen Formeln (2a) und (2c). Ganz besonders bevorzugt sind Verbindungen der allgemeinen Formel (2a).

In einer weiteren bevorzugten Ausführungsform der erfindungsgemäßen Verbindungen sind $R^{13}$, $R^{14}$ und $R^{15}$ gemäß einer der allgemeinen Formeln (1), (1a), (1 b), (1c), (2a), (2b) oder (2c) jeweils unabhängig voneinander ausgewählt aus der Gruppe bestehend aus H; F; Cl; Br; I; NO; NO$_2$; CN; NH$_2$; NH-C$_{1-8}$-Alkyl; N(C$_{1-8}$-Alkyl)$_2$; NH-C(=O)C$_{1-8}$-Alkyl; NH-C(=O)-Aryl; NH-C(=O)-Heteroaryl; C$_{1-8}$-Alkyl; CF$_3$; CHO; C(=O)C$_{1-8}$-Alkyl; C(=O)Aryl; C(=O)Heteroaryl; CO$_2$H; C(=O)O-C$_{1-8}$-Alkyl; C(=O)O-Aryl; C(=O)O-Heteroaryl; CONH$_2$; C(=O)NH-C$_{1-8}$-Alkyl; C(=O)N(C$_{1-8}$-Alkyl)$_2$-; C(=O)NH-Aryl; C(=O)N(Aryl)$_2$; C(=O)NH-Heteroaryl; C(=O)N(Heteroaryl)$_2$; C(=O)N(C$_{1-8}$-Alkyl)(Aryl); C(=O)N(C$_{1-8}$-Alkyl)(Heteroaryl); C(=O)N(Heteroaryl)(Aryl); OH; O-C$_{1-8}$-Alkyl; OCF$_3$; O-(C$_{1-8}$-Alkyl)-O; O-(C$_{1-8}$-Alkyl)-O-C$_{1-8}$-Alkyl; O-Benzyl; O-Aryl; O-Heteroaryl; O-C(=O)C$_{1-8}$-Alkyl; O-C(=O)Aryl; O-C(=O)Heteroaryl; SH; S-C$_{1-8}$-Alkyl; SCF$_3$; S-Benzyl; S-Aryl; S-Heteroaryl; Aryl; Heteroaryl; C$_{3-7}$-Cycloalkyl; Heterocyclyl oder C$_{1-8}$-Alkyl verbrücktes Aryl, Heteroaryl, C$_{3-7}$-Cycloalkyl oder Heterocyclyl.

Bevorzugter sind die Substituenten $R^{13}$, $R^{14}$ und $R^{15}$ gemäß einer der allgemeinen Formeln (1), (1a), (1b), (1c), (2a), (2b) oder (2c) jeweils unabhängig voneinander ausgewählt aus der Gruppe bestehend aus H; F; Cl; Br; CN; NH$_2$; NH-C(=O)C$_{1-8}$-Alkyl; NH-C(=O)-Aryl; NH-C(=O)-Heteroaryl; C$_{1-8}$-Alkyl; CF$_3$; CONH$_2$; C(=O)NH-C$_{1-8-Alkyl}$; C(=O)N(C$_{1-8}$-Alkyl)2; C(=O)NH-Aryl; C(=O)N(Aryl)$_2$; C(=O)NH-Heteroaryl; C(=O)N(Heteroaryl)$_2$; C(=O)N(C$_{1-8}$-Alkyl)(Aryl); C(=O)N(C$_{1-8}$-Alkyl)(Heteroaryl); C(=O)N(Heteroaryl)(Aryl); OH; O-C$_{1-8}$-Alkyl; OCF$_3$; O-Benzyl; O-Aryl; O-Heteroaryl; SH; S-C$_{1-8}$-Alkyl; SCF$_3$; S-Benzyl; S-Aryl; S-Heteroaryl; Aryl; Heteroaryl; C$_{3-7}$-Cycloalkyl; Heterocyclyl oder C$_{1-8}$-Alkyl verbrücktes Aryl, Heteroaryl, C$_{3-7}$-Cycloalkyl oder Heterocyclyl.

Noch bevorzugter sind die Substituenten $R^{13}$, $R^{14}$ und $R^{15}$ gemäß einer der allgemeinen Formeln (1), (1a), (1b), (1c), (2a), (2b) oder (2c) jeweils unabhängig voneinander ausgewählt aus der Gruppe bestehend aus H; F; Cl; Br; CN; CF$_3$; OCF$_3$; SCF$_3$; C$_{1-8}$-Alkyl; O-C$_{1-8}$-Alkyl; CONH$_2$; C(=O)NH-C$_{1-8}$-Alkyl; NH$_2$; NH-C(=O)C$_{1-8}$-Alkyl;

Besonders bevorzugt sind die Substituenten $R^{13}$, $R^{14}$ und $R^{15}$ jeweils unabhängig voneinander ausgewählt aus der Gruppe bestehend aus H; F, Cl; Br; CN; OCH$_3$; OCF$_3$; CF$_3$ und C$_{1-8}$-Alkyl.

Ganz besonders bevorzugt bedeuten die Substituenten $R^{13}$, $R^{14}$ und $R^{15}$ gemäß einer der allgemeinen Formeln (1), (1a), (1b), (1c), (2a), (2b) oder (2c) jeweils unabhängig voneinander H oder CH$_3$.

Insbesondere bevorzugt sind Verbindungen, bei denen einer der Reste $R^{13}$ und $R^{14}$; oder $R^{13}$ und $R^{15}$; oder $R^{14}$ und $R^{15}$ gemäß einer der allgemeinen Formeln (1), (1a), (1b), (1c), (2a), (2b) oder (2c) für CH$_3$ steht und der verbleibende Rest H bedeutet.

In einer weiteren bevorzugten Ausführungsform ist der Substituent $R^1$ ausgewählt aus der Gruppe bestehend aus H; F; Cl; Br; CN; C$_{1-8}$-Alkyl, gesättigt oder ungesättigt, verzweigt oder unverzweigt, unsubstituiert oder einfach oder mehrfach substituiert; C$_{3-7}$-Cycloalkyl, gesättigt oder ungesättigt, unsubstituiert oder einfach oder mehrfach substituiert; Aryl oder Heteroaryl, jeweils unsubstituiert oder einfach oder mehrfach substituiert; über C$_{1-8}$-Alkyl verbrücktes C$_{3-7}$-Cycloalkyl, gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über C$_{1-8}$-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann;

und der Substituent $R^2$ ausgewählt aus der Gruppe bestehend aus H oder C$_{1-8}$-Alkyl, gesättigt oder ungesättigt, verzweigt oder unverzweigt, unsubstituiert oder einfach oder mehrfach substituiert;

oder $R^1$ und $R^2$ bilden zusammen mit dem sie verbindenden Kohlenstoffatom als Ringglied ein C$_{3-7}$-Cycloalkyl oder Heterocyclyl, jeweils gesättigt oder ungesättigt, unsubstituiert oder einfach oder mehrfach substituiert, jeweils ggf. mit (Hetero-)aryl kondensiert, unsubstituiert oder einfach oder mehrfach substituiert.

In einer weiteren bevorzugten Ausführungsform ist der Substituent $R^1$ ausgewählt aus der Gruppe bestehend aus H; C$_{1-8}$-Alkyl, gesättigt oder ungesättigt, verzweigt oder unverzweigt, unsubstituiert oder einfach oder mehrfach substituiert; C$_{3-7}$-Cycloalkyl, gesättigt oder ungesättigt, unsubstituiert oder einfach oder mehrfach substituiert; Aryl oder Heteroaryl, jeweils unsubstituiert oder einfach oder mehrfach substituiert; über C$_{1-8}$-Alkyl verbrücktes C$_{3-7}$-Cycloalkyl, gesättigt oder ungesättigt; oder über C$_{1-8}$-Alkyl verbrücktes Aryl oder Heteroaryl, unsubstituiert oder einfach oder mehrfach substituiert;

und der Substituent $R^2$ ausgewählt aus der Gruppe bestehend aus H oder C$_{1-8}$-Alkyl, gesättigt oder ungesättigt, verzweigt oder unverzweigt;

oder $R^1$ und $R^2$ bilden zusammen mit dem sie verbindenden Kohlenstoffatom als Ringglied ein C$_{3-7}$-Cycloalkyl, gesättigt

oder ungesättigt, unsubstituiert oder einfach oder mehrfach substituiert, ggf. mit (Hetero-)aryl kondensiert, unsubstituiert oder einfach oder mehrfach substituiert.

In einer weiteren bevorzugten Ausführungsform ist der Substituent $R^1$ ausgewählt aus der Gruppe bestehend aus H; $C_{1-8}$-Alkyl, gesättigt oder ungesättigt, verzweigt oder unverzweigt, unsubstituiert oder einfach oder mehrfach substituiert; $C_{3-7}$-Cycloalkyl, gesättigt oder ungesättigt, unsubstituiert oder einfach oder mehrfach substituiert; Phenyl, Pyridyl oder Thienyl, jeweils unsubstituiert oder einfach oder mehrfach substituiert; über $C_{1-8}$-Alkyl verbrücktes $C_{3-7}$-Cycloalkyl, gesättigt oder ungesättigt; oder über $C_{1-8}$-Alkyl verbrücktes Phenyl, Pyridyl oder Thienyl, jeweils unsubstituiert oder einfach oder mehrfach substituiert;

und der Substituent $R^2$ ausgewählt aus der Gruppe bestehend aus H oder $C_{1-8}$-Alkyl, gesättigt oder ungesättigt, verzweigt oder unverzweigt.

In einer weiteren bevorzugten Ausführungsform ist der Substituent $R^1$ ausgewählt aus der Gruppe bestehend aus H; $C_{1-8}$-Alkyl, gesättigt oder ungesättigt, verzweigt oder unverzweigt, unsubstituiert oder einfach oder mehrfach substituiert; $C_{3-7}$-Cycloalkyl, gesättigt oder ungesättigt, unsubstituiert oder einfach oder mehrfach substituiert; Phenyl oder Thienyl, jeweils unsubstituiert oder einfach oder mehrfach substituiert; über $C_{1-8}$-Alkyl verbrücktes $C_{3-7}$-Cycloalkyl, gesättigt oder ungesättigt; oder über $C_{1-8}$-Alkyl verbrücktes Phenyl oder Thienyl; jeweils unsubstituiert oder einfach oder mehrfach substituiert;

und der Substituent $R^2$ steht für H.

In einer weiteren bevorzugten Ausführungsform ist der Substituent $R^1$ ausgewählt aus der Gruppe bestehend aus H; $C_{1-8}$-Alkyl, gesättigt, verzweigt oder unverzweigt, unsubstituiert oder einfach oder mehrfach substituiert; $C_{3-7}$-Cycloalkyl, gesättigt, unsubstituiert oder einfach oder mehrfach substituiert; Phenyl oder Thienyl, jeweils unsubstituiert oder einfach oder mehrfach substituiert; oder über $C_{1-8}$-Alkyl verbrücktes Phenyl, unsubstituiert.

und der Substituent $R^2$ steht für H.

In einer weiteren bevorzugten Ausführungsform ist der Substituent $R^1$ ausgewählt aus der Gruppe bestehend aus

$C_{1-8}$-Alkyl, gesättigt, verzweigt oder unverzweigt, unsubstituiert;

über $C_{1-8}$-Alkyl verbrücktes Phenyl, unsubstituiert; oder

bedeutet gemäß einer der nachstehenden allgemeinen Formeln (3a) oder (4a) $(CH_2)_e$-verbrücktes Phenyl,

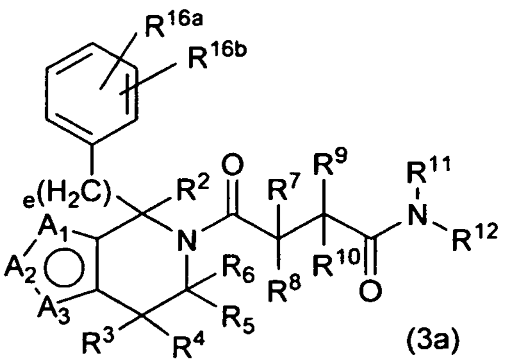
(3a)

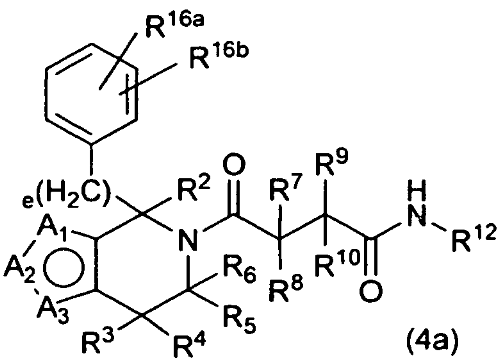
(4a)
,

einfach oder zweifach substituiert mit $R^{16a}$ und/oder $R^{16b}$;

oder bedeutet gemäß einer der nachstehenden allgemeinen Formeln (3b) oder (4b) $(CH_2)_e$-verbrücktes Thienyl,

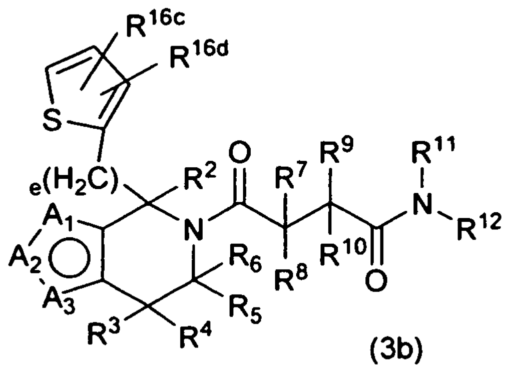
(3b)

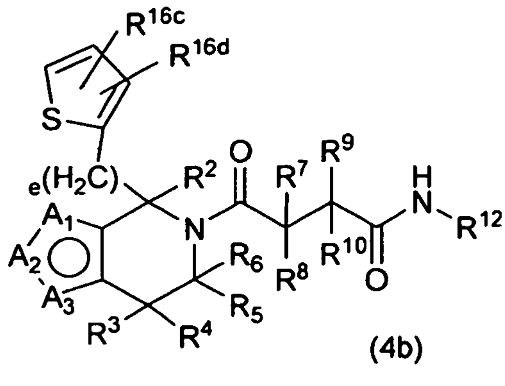
(4b)

einfach oder zweifach substituiert mit $R^{16c}$ und/oder $R^{16d}$;

oder bedeutet gemäß einer der nachstehenden allgemeinen Formeln (3c) oder (4c) $(CH_2)_e$-verbrücktes $C_{3-7}$-Cycloalkyl,

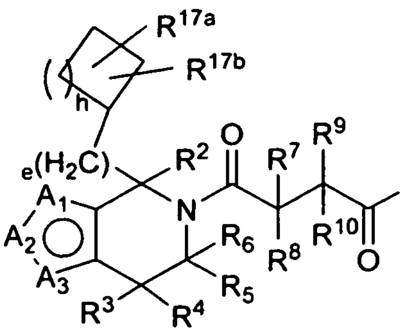
(3c)

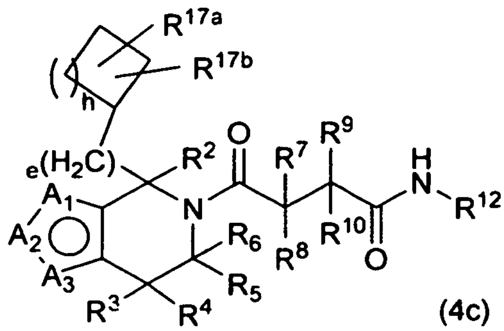
(4c)

einfach oder zweifach substituiert mit $R^{17a}$ und/oder $R^{17b}$;
und der Substituent $R^2$ steht für H;
wobei e für 0, 1, 2, 3 oder 4, bevorzugt für 0 steht;
$R^{16a}$ und $R^{16b}$ unabhängig voneinander ausgewählt sind aus der Gruppe bestehend aus H, F, Cl, Br, CN, $NH_2$, $OCF_3$, $SCF_3$, $CF_3$, $C_{1-8}$-Alkyl, gesättigt oder ungesättigt, verzweigt oder unverzweigt, unsubstituiert oder einfach oder mehrfach substituiert; Aryl, Heteroaryl, jeweils unsubstituiert oder einfach oder mehrfach substituiert; $C_{3-7}$-Cycloalkyl oder Heterocyclyl , jeweils gesättigt oder ungesättigt, unsubstituiert oder einfach oder mehrfach substituiert;
$R^{16c}$ und $R^{16d}$ unabhängig voneinander ausgewählt sind aus der Gruppe bestehend aus H, F, Cl, Br, CN, $NH_2$, $OCF_3$, $SCF_3$, $CF_3$, $C_{1-8}$-Alkyl, gesättigt oder ungesättigt, verzweigt oder unverzweigt, unsubstituiert oder einfach oder mehrfach substituiert; Aryl, Heteroaryl, jeweils unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert.
h 0, 1, 2, 3 oder 4 bedeutet;
$R^{17a}$ und $R^{17b}$ unabhängig voneinander ausgewählt sind aus der Gruppe bestehend aus H, F, Cl, Br, CN, $NH_2$, $OCF_3$, $SCF_3$, $CF_3$, $C_{1-8}$-Alkyl, gesättigt oder ungesättigt, verzweigt oder unverzweigt, unsubstituiert oder einfach oder mehrfach substituiert; Aryl, Heteroaryl, jeweils unsubstituiert oder einfach oder mehrfach substituiert;

Bevorzugt sind $R^{16a}$ und $R^{16b}$ unabhängig voneinander ausgewählt aus der Gruppe bestehend aus H, F, Cl, Br, $CH_3$, $C_2H_5$, iso-Propyl, $OCH_3$ und $CF_3$.

Bevorzugt sind $R^{16c}$ und $R^{16d}$ unabhängig voneinander ausgewählt aus der Gruppe bestehend aus H, F, Cl, Br, $CH_3$, $OCH_3$ und $CF_3$.

Besonders bevorzugt stehen $R^{16c}$ und $R^{16d}$ unabhängig voneinander für H oder $CH_3$.

Bevorzugt steht h für 2 oder 3, besonders bevorzugt für 3.

Bevorzugt sind $R^{17a}$ und $R^{17b}$ unabhängig voneinander ausgewählt aus der Gruppe bestehend aus H, F, Cl, Br, $CH_3$, $OCH_3$ und $CF_3$.

Besonders bevorzugt stehen $R^{17a}$ und $R^{17b}$ jeweils für H.

Ganz besonders bevorzugt sind Verbindungen der Formeln (3a), (4a), (3b) und (4b). Insbesondere bevorzugt sind Verbindungen der Formeln (4a) und (4b).

In einer anderen bevorzugten Ausführungsform stehen $R^1$ und $R^2$ jeweils für H;
oder $R^2$ bedeutet H und $R^1$ ist ungleich H.

In einer weiteren bevorzugten Ausführungsform sind die Substituenten $R^3$, $R^4$, $R^5$ und $R^6$ unabhängig voneinander ausgewählt aus der Gruppe bestehend aus H, $C_{1-8}$-Alkyl, gesättigt oder ungesättigt, verzweigt oder unverzweigt, unsubstituiert oder einfach oder mehrfach substituiert; Phenyl, unsubstituiert oder einfach oder mehrfach substituiert.

Bevorzugt stehen $R^3$, $R^4$, $R^5$ und $R^6$ jeweils unabhängig voneinander für H; $CH_3$; oder Phenyl.

Besonders bevorzugt bedeuten $R^3$, $R^4$, $R^5$ jeweils H und $R^6$ ist ausgewählt aus H oder Phenyl.

In einer weiteren bevorzugten Ausführungsform ist der Substituent $R^7$ ausgewählt aus der Gruppe bestehend aus H; F; Cl; Br; CN; OH; $NH_2$; $C_{1-8}$-Alkyl, O-$C_{1-8}$-Alkyl, NH-$C_{1-8}$-Alkyl, N($C_{1-8}$-Alkyl)$_2$, jeweils gesättigt oder ungesättigt, verzweigt oder unverzweigt, unsubstituiert oder einfach oder mehrfach substituiert; Phenyl oder Heteroaryl, jeweils unsubstituiert oder einfach oder mehrfach substituiert; über $C_{1-2}$-Alkyl verbrücktes Phenyl oder Heteroaryl, jeweils unsubstituiert oder einfach oder mehrfach substituiert, wobei die Alkylkette jeweils verzweigt oder unverzweigt, gesättigt oder ungesättigt, unsubstituiert oder einfach oder mehrfach substituiert sein kann;
und der Rest $R^9$ ausgewählt aus der Gruppe bestehend aus H; F; Cl; Br; CN; OH; $NH_2$; $C_{1-8}$-Alkyl, O-$C_{1-8}$-Alkyl, NH-$C_{1-8}$-Alkyl, N($C_{1-8}$-Alkyl)$_2$, jeweils gesättigt oder ungesättigt, verzweigt oder unverzweigt, unsubstituiert oder einfach oder mehrfach substituiert; Phenyl oder Heteroaryl, jeweils unsubstituiert oder einfach oder mehrfach substituiert; über $C_2$-Alkyl verbrücktes Phenyl oder Heteroaryl, jeweils unsubstituiert oder einfach oder mehrfach substituiert, wobei die Alkylkette jeweils verzweigt oder unverzweigt, gesättigt oder ungesättigt, unsubstituiert oder einfach oder mehrfach

substituiert sein kann.

Bevorzugt ist $R^7$ ausgewählt aus der Gruppe bestehend aus H; $C_{1-8}$-Alkyl, NH-$C_{1-8}$-Alkyl, N($C_{1-8}$-Alkyl)$_2$, jeweils gesättigt oder ungesättigt, verzweigt oder unverzweigt, unsubstituiert oder einfach oder mehrfach substituiert; Phenyl oder Heteroaryl, jeweils unsubstituiert oder einfach oder mehrfach substituiert; über $C_{1-2}$-Alkyl verbrücktes Phenyl oder Heteroaryl, jeweils unsubstituiert oder einfach oder mehrfach substituiert, wobei die Alkylkette jeweils verzweigt oder unverzweigt, gesättigt oder ungesättigt, unsubstituiert oder einfach oder mehrfach substituiert sein kann;
und der Rest $R^9$ ausgewählt aus der Gruppe bestehend aus H; $C_{1-8}$-Alkyl, NH-$C_{1-8}$-Alkyl, N($C_{1-8}$-Alkyl)$_2$, jeweils gesättigt oder ungesättigt, verzweigt oder unverzweigt, unsubstituiert oder einfach oder mehrfach substituiert; Phenyl oder Heteroaryl, jeweils unsubstituiert oder einfach oder mehrfach substituiert; über $C_2$-Alkyl verbrücktes Phenyl oder Heteroaryl, jeweils unsubstituiert oder einfach oder mehrfach substituiert, wobei die Alkylkette jeweils verzweigt oder unverzweigt, gesättigt oder ungesättigt, unsubstituiert oder einfach oder mehrfach substituiert sein kann.

Bevorzugter ist $R^7$ ausgewählt aus der Gruppe bestehend aus H; $C_{1-8}$-Alkyl, jeweils gesättigt oder ungesättigt, verzweigt oder unverzweigt, unsubstituiert oder einfach oder mehrfach substituiert; Phenyl, unsubstituiert oder einfach oder mehrfach substituiert; über $C_{1-2}$-Alkyl verbrücktes Phenyl, unsubstituiert oder einfach oder mehrfach substituiert, wobei die Alkylkette jeweils verzweigt oder unverzweigt, gesättigt oder ungesättigt sein kann;
und der Rest $R^9$ ausgewählt aus der Gruppe bestehend aus H; $C_{1-8}$-Alkyl, jeweils gesättigt oder ungesättigt, verzweigt oder unverzweigt, unsubstituiert oder einfach oder mehrfach substituiert; Phenyl, unsubstituiert oder einfach oder mehrfach substituiert; über $C_2$-Alkyl verbrücktes Phenyl, unsubstituiert oder einfach oder mehrfach substituiert, wobei die Alkylkette jeweils verzweigt oder unverzweigt, gesättigt oder ungesättigt sein kann.

Noch bevorzugter ist $R^7$ ausgewählt aus der Gruppe bestehend aus H; $C_{1-8}$-Alkyl, jeweils gesättigt oder ungesättigt, verzweigt oder unverzweigt; Phenyl oder Benzyl, jeweils unsubstituiert oder einfach oder mehrfach substituiert; und der Rest $R^9$ ausgewählt aus der Gruppe bestehend aus H; $C_{1-8}$-Alkyl, jeweils gesättigt oder ungesättigt, verzweigt oder unverzweigt; Phenyl, unsubstituiert oder einfach oder mehrfach substituiert;

Besonders bevorzugt ist einer der Reste $R^7$ und $R^9$ ausgewählt aus der Gruppe bestehend aus H; $CH_3$; Phenyl, unsubstituiert oder einfach oder mehrfach substituiert; und der verbleibende Rest steht für H.

Insbesondere bevorzugt ist einer der Reste $R^7$ und $R^9$ ausgewählt aus der Gruppe bestehend aus H; $CH_3$; Phenyl, unsubstituiert; und der verbleibende Rest steht für H.

In einer anderen bevorzugten Ausführungsform bilden $R^7$ und $R^9$ zusammen mit den sie verbindenden Kohlenstoffatomen als Ringgliedern ein $C_{3-7}$-Cycloalkyl oder Piperidinyl, jeweils gesättigt oder ungesättigt, unsubstituiert oder einfach oder mehrfach substituiert, ggf. mit (Hetero-)aryl kondensiert, unsubstituiert oder einfach oder mehrfach substituiert.

Bevorzugt bilden $R^7$ und $R^9$ zusammen mit den sie verbindenden Kohlenstoffatomen als Ringgliedern ein $C_{3-7}$-Cycloalkyl, gesättigt oder ungesättigt, unsubstituiert oder einfach oder mehrfach substituiert, ggf. mit Aryl kondensiert, unsubstituiert oder einfach oder mehrfach substituiert.

Besonders bevorzugt bilden $R^7$ und $R^9$ zusammen mit den sie verbindenden Kohlenstoffatomen als Ringgliedern ein $C_{3-7}$-Cycloalkyl, gesättigt oder ungesättigt, ggf. mit Phenyl kondensiert.

In einer weiteren bevorzugten Ausführungsform ist $R^8$ ausgewählt aus der Gruppe bestehend aus H; oder $C_{1-8}$-Alkyl, gesättigt oder ungesättigt, verzweigt oder unverzweigt, unsubstituiert oder einfach oder mehrfach substituiert.

Bevorzugt ist Rest $R^8$ ausgewählt aus H oder $C_{1-8}$-Alkyl, gesättigt.

Bevorzugter steht der Rest $R^8$ für H oder $CH_3$.

Besonders bevorzugt bedeutet $R^8$ H.

In einer weiteren bevorzugten Ausführungsform ist $R^{10}$ ausgewählt aus der Gruppe bestehend aus H; oder $C_{1-8}$-Alkyl, gesättigt oder ungesättigt, verzweigt oder unverzweigt, unsubstituiert oder einfach oder mehrfach substituiert.

Bevorzugt ist Rest $R^{10}$ ausgewählt aus H oder $C_{1-8}$-Alkyl, gesättigt.

Bevorzugter steht der Rest $R^{10}$ für H oder $CH_3$.

Besonders bevorzugt bedeutet $R^{10}$ H.

In einer weiteren bevorzugten Ausführungsform bilden $R^7$ und $R^8$; oder $R^9$ und $R^{10}$; zusammen mit den sie verbindenden Kohlenstoffatomen als Ringgliedern ein $C_{3-7}$-Cycloalkyl oder Piperidinyl, jeweils gesättigt oder ungesättigt, unsubstituiert oder einfach oder mehrfach substituiert, ggf. mit (Hetero-)aryl kondensiert, unsubstituiert oder einfach oder mehrfach substituiert.

Bevorzugt bilden $R^7$ und $R^8$; oder $R^9$ und $R^{10}$; zusammen mit den sie verbindenden Kohlenstoffatomen als Ringgliedern ein $C_{3-7}$-Cycloalkyl, gesättigt oder ungesättigt, unsubstituiert oder einfach oder mehrfach substituiert, ggf. mit Aryl kondensiert, unsubstituiert oder einfach oder mehrfach substituiert.

Besonders bevorzugt bilden $R^7$ und $R^8$; oder $R^9$ und $R^{10}$; zusammen mit den sie verbindenden Kohlenstoffatomen als Ringgliedern ein $C_{3-7}$-Cycloalkyl, gesättigt oder ungesättigt, ggf. mit Phenyl kondensiert.

In einer weiteren bevorzugten Ausführungsform ist der Substituent $R^{11}$ ausgewählt aus der Gruppe bestehend aus H; $C_{1-8}$-Alkyl, gesättigt oder ungesättigt, verzweigt oder unverzweigt, $C_{3-7}$-Cycloalkyl, gesättigt oder ungesättigt; oder Benzyl, unsubstituiert oder einfach oder mehrfach substituiert.

Bevorzugt steht der Rest $R^{11}$ für H; $C_{1-8}$-Alkyl, gesättigt oder ungesättigt, verzweigt oder unverzweigt; oder Benzyl.

Bevorzugter bedeutet der Rest $R^{11}$ H oder $CH_3$.

Besonders bevorzugt steht $R^{11}$ für H.

In einer weiteren bevorzugten Ausführungsform ist der Rest $R^{12}$ ausgewählt aus der Gruppe bestehend aus $C_{4-16}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann.

Bevorzugter ist der Rest $R^{12}$ ausgewählt aus der Gruppe bestehend aus $C_{4-16}$-Alkyl, gesättigt oder ungesättigt, verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; mit der Maßgabe, dass, wenn $R^{12}$ Heterocyclyl oder Heteroaryl bedeutet, die Bindung des Heterocyclyls oder Heteroaryls über ein Kohlenstoffatom des Heterocyclyls oder Heteroaryls erfolgt.

Noch bevorzugter steht der Rest $R^{12}$ für $C_{4-16}$-Alkyl, gesättigt oder ungesättigt, verzweigt oder unverzweigt, unsubstituiert oder einfach oder mehrfach substituiert; oder ist ausgewählt aus den folgenden Teilstrukturen A, B oder C,

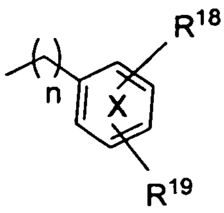
A

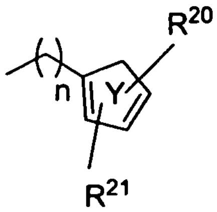
B

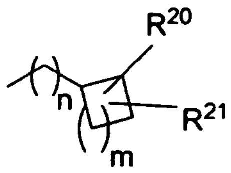
C

wobei

n = 0, 1, 2, 3, 4, 5, 6, 7 oder 8; besonders bevorzugt 1,2 oder 3, insbesondere 1;

m = 0, 1, 2 oder 3; bedeutet;

der Ring X ein oder zwei N-Atome als Ringglied(er) enthalten kann;

der Ring Y mindestens 1 Heteroatom ausgewählt aus N, O oder S enthält und bis zu 3 Heteroatome unabhängig voneinander ausgewählt aus N, O oder S enthalten kann;

$R^{18}$ und $R^{19}$ unabhängig voneinander H; F; Cl; Br; I; NO; $NO_2$; $CF_3$; CN; $R^0$; C(=O)H; C(=O)$R^0$; $CO_2H$; C(=O)O$R^0$; $CONH_2$; C(=O)NH$R^0$; C(=O)N$(R^0)_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N$(R^0)_2$; O-S$(=O)_2$-$R^0$; O-S$(=O)_2$OH; O-S$(=O)_2$O$R^0$; O-S$(=O)_2NH_2$; O-S$(=O)_2$NH$R^0$; O-S$(=O)_2$N$(R^0)_2$; $NH_2$; NH-$R^0$; N$(R^0)_2$; NH-C(=O)-$R^0$; NH-C(=O)-O-$R^0$; NH-C(=O)-NH-$R^0$; NH-C(=O)-N$(R^0)_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N$(R^0)_2$; NH-S$(=O)_2$OH; NH-S$(=O)_2R^0$; NH-S$(=O)_2$O$R^0$; NH-S$(=O)_2NH_2$; NH-S$(=O)_2$NH$R^0$; NH-S$(=O)_2$N$(R^0)_2$; N$R^0$-S$(=O)_2$OH; N$R^0$-S$(=O)_2R^0$; N$R^0$-S$(=O)_2$O$R^0$; N$R^0$-S$(=O)_2NH_2$; N$R^0$-S$(=O)_2$NH$R^0$; N$R^0$-S$(=O)_2$N$(R^0)_2$; SH; S$R^0$; S(=O)$R^0$; S$(=O)_2R^0$; S$(=O)_2$OH; S$(=O)_2$O$R^0$; S$(=O)_2NH_2$; S$(=O)_2$NH$R^0$; oder S$(=O)_2$N$(R^0)_2$ bedeuten;

oder $R^{18}$ und $R^{19}$ zusammen mit den sie verbindenden Kohlenstoff- oder Stickstoffatomen als Ringgliedern ein an

den Phenyl-Ring kondensiertes Aryl oder Heteroaryl, jeweils unsubstituiert oder einfach oder mehrfach substituiert; oder ein an den Phenyl-Ring kondensiertes $C_{3-7}$-Cycloalkyl oder Heterocyclyl, jeweils gesättigt oder ungesättigt, unsubstituiert oder einfach oder mehrfach substituiert; bilden;

$R^{20}$ und $R^{21}$ unabhängig voneinander H oder $C_{1-8}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; bedeuten.

Besonders bevorzugt sind Verbindungen, in denen $R^{12}$ die Bedeutung der Teilstruktur **A** hat.

Ganz besonders bevorzugt sind Verbindungen, in denen $R^{12}$ die Bedeutung der Teilstruktur **A** hat, worin $R^{18}$ und $R^{19}$ unabhängig voneinander H; F; Cl; Br; I; CN; $NH_2$; $OCF_3$; $SCF_3$; $S(=O)CF_3$; $S(=O)_2CF_3$; NH(OH); NO; $NO_2$; $CF_2H$; $OCF_2H$; $SCF_2H$; Benzyl; $C_{1-8}$-Alkyl; $CF_3$; C(=O)H; C(=O)OH; C(=O)$C_{1-8}$-Alkyl; C(=O)Aryl; C(=O)Heteroaryl; C(=O)O-$C_{1-8}$-Alkyl; C(=O)O-Aryl; C(=O)O-Heteroaryl; C(=O)$NH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Aryl)(Heteroaryl); OH; O-$C_{1-8}$-Alkyl; O-$C_{1-8}$-Alkyl-OH; O-($C_{1-8}$-Alkyl)-O; O-($C_{1-8}$-Alkyl)-O-$C_{1-8}$-Alkyl; O-Aryl; O-Heteroaryl; O-Benzyl; O-C(=O)-$C_{1-8}$-Alkyl; O-C(=O)-Aryl; O-C(=O)-Heteroaryl; O-S(=O)$_2$OH; O-S(=O)$_2$-O$C_{1-8}$-Alkyl; O-S(=O)$_2$-O-Aryl; O-S(=O)$_2$-O-Heteroaryl; O-S(=O)$_2C_{1-8}$-Alkyl; O-S(=O)$_2$Aryl, O-S(=O)$_2$Heteroaryl; O-S(=O)$_2NH_2$; O-S(=O)$_2$NH-$C_{1-8}$-Alkyl; O-S(=O)$_2$N($C_{1-8}$-Alkyl)$_2$; O-S(=O)$_2$NH-Aryl; 0-S(=O)$_2$N(Aryl)$_2$; O-S(=O)$_2$NH-Heteroaryl; O-S(=O)$_2$N(Heteroaryl)$_2$; O-S(=O)$_2$N($C_{1-8}$-Alkyl)(Aryl); O-S(=O)$_2$N(Heteroaryl)(Aryl); O-S(=O)$_2$N($C_{1-8}$-Alkyl)(Heteroaryl); $NH_2$; NH(OH); N=C($NH_2$)$_2$; NH-$C_{1-8}$-Alkyl; NH-$C_{1-8}$-Alkyl-OH; N($C_{1-8}$-Alkyl)$_2$; N($C_{1-8}$-Alkyl-OH)$_2$; NH-Aryl; N(Aryl)$_2$; NH-Heteroaryl; N(Heteroaryl)$_2$; N($C_{1-8}$-Alkyl)(Aryl); N($C_{1-8}$-Alkyl)(Heteroaryl); N(Aryl)(Heteroaryl); NH-C(=O)$C_{1-8}$-Alkyl; NH-C(=O)-Aryl; NH-C(=O)-Heteroaryl; N($C_{1-8}$-Alkyl)-C(=O)$C_{1-8}$-Alkyl; N($C_{1-8}$-Alkyl)-C(=O)Ary); N($C_{1-8}$-Alkyl)-C(=O)Heteroaryl; N(Aryl)-C(=O)$C_{1-8}$-Alkyl; N(Aryl)-C(=O)Aryl; N(Aryl)-C(=O)Heteroaryl; N(Heteroaryl)-C(=O)$C_{1-8}$-Alkyl; N(Heteroaryl)-C(=O)Aryl; N(Heteroaryl)-C(=O)Heteroaryl; NH-S(=O)$_2$OH; NH-S(=O)$_2C_{1-8}$-Alkyl; NH-S(=O)$_2$Aryl; NH-S(=O)$_2$Heteroaryl; NH-S(=O)$_2$O$C_{1-8}$-Alkyl; NH-S(=O)$_2$O-Aryl; NH-S(=O)$_2$O-Heteroaryl; NH-S(=O)$_2NH_2$; NH-S(=O)$_2$NH($C_{1-8}$-Alkyl); NH-S(=O)$_2$NH(Aryl); NH-S(=O)$_2$NH(Heteroaryl); NH-S(=O)$_2$N($C_{1-8}$-Alkyl)$_2$; NH-S(=O)$_2$N(Aryl)$_2$; NH-S(=O)$_2$N(Heteroaryl)$_2$; NH-S(=O)$_2$N($C_{1-8}$-Alkyl)(Aryl); NH-S(=O)$_2$N($C_{1-8}$-Alkyl)(Heteroaryl); NH-S(=O)$_2$N(Aryl)(Heteroaryl); N($C_{1-8}$-Alkyl)-S(=O)$_2$OH; N(Aryl)-S(=O)$_2$OH; N(Heteroaryl)-S(=O)$_2$OH; N($C_{1-8}$-Alkyl)-S(=O)$_2$-$C_{1-8}$-Alkyl; N($C_{1-8}$-Alkyl)-S(=O)$_2$-Aryl; N($C_{1-8}$-Alkyl)-S(=O)$_2$-Heteroaryl; N(Aryl)-S(=O)$_2$-$C_{1-8}$-Alkyl; N(Aryl)-S(=O)$_2$-Aryl; N(Aryl)-S(=O)$_2$-Heteroaryl; N(Heteroaryl)-S(=O)$_2$-$C_{1-8}$-Alkyl; N(Heteroaryl)-S(=O)$_2$-Aryl; N(Heteroaryl)-S(=O)$_2$-Heteroaryl; N($C_{1-8}$-Alkyl)-S(=O)$_2$-O$C_{1-8}$-Alkyl; N($C_{1-8}$-Alkyl)-S(=O)$_2$-O-Aryl; N($C_{1-8}$-Alkyl)-S(=O)$_2$-O-Heteroaryl; N(Aryl)-S(=O)$_2$-O$C_{1-8}$-Alkyl; N(Aryl)-S(=O)$_2$-O-Aryl; N(Aryl)-S(=O)$_2$-O-Heteroaryl; N(Heteroaryl)-S(=O)$_2$-O$C_{1-8}$-Alkyl; N(Heteroaryl)-S(=O)$_2$-O-Aryl; N(Heteroaryl)-S(=O)$_2$-O-Heteroaryl; N($C_{1-8}$-Alkyl)-S(=O)$_2NH_2$; N(Aryl)-S(=O)$_2NH_2$; N(Heteroaryl)-S(=O)$_2NH_2$; N($C_{1-8}$-Alkyl)-S(=O)$_2$NH($C_{1-8}$-Alkyl); N($C_{1-8}$-Alkyl)-S(=O)$_2$NH(Aryl); N($C_{1-8}$-Alkyl)-S(=O)$_2$NH(Heteroaryl); N($C_{1-8}$-Alkyl)-S(=O)$_2$N($C_{1-8}$-Alkyl)$_2$; N($C_{1-8}$-Alkyl)-S(=O)$_2$N(Aryl)$_2$; N($C_{1-8}$-Alkyl)-S(=O)$_2$N(Heteroaryl)$_2$; N($C_{1-8}$-Alkyl)-S(=O)$_2$N($C_{1-8}$-Alkyl)(Aryl); N($C_{1-8}$-Alkyl)-S(=O)$_2$N($C_{1-8}$-Alkyl)(Heteroaryl); N($C_{1-8}$-Alkyl)-S(=O)$_2$N(Aryl)(Heteroaryl); N(Aryl)-S(=O)$_2$NH($C_{1-8}$-Alkyl); N(Aryl)-S(=O)$_2$NH(Aryl); N(Aryl)-S(=O)$_2$NH(Heteroaryl); N(Aryl)-S(=O)$_2$N($C_{1-8}$-Alkyl)$_2$; N(Aryl)-S(=O)$_2$N(Aryl)$_2$; N(Aryl)-S(=O)$_2$N(Heteroaryl)$_2$; N(Aryl)-S(=O)$_2$N($C_{1-8}$-Alkyl)(Aryl); N(Aryl)-S(=O)$_2$N($C_{1-8}$-Alkyl)(Heteroaryl); N(Aryl)-S(=O)$_2$N(Aryl)(Heteroaryl); N(Heteroaryl)-S(=O)$_2$NH($C_{1-8}$-Alkyl); N(Heteroaryl)-S(=O)$_2$NH(Aryl); N(Heteroaryl)-S(=O)$_2$NH(Heteroaryl); N(Heteroaryl)-S(=O)$_2$N($C_{1-8}$-Alkyl)$_2$; N(Heteroaryl)-S(=O)$_2$N(Aryl)$_2$; N(Heteroaryl)-S(=O)$_2$N(Heteroaryl)2; N(Heteroaryl)-S(=O)$_2$N($C_{1-8}$-Alkyl)(Aryl); N(Heteroaryl)-S(=O)$_2$N($C_{1-8}$-Alkyl)(Heteroaryl); N(Heteroaryl)-S(=O)$_2$N(Aryl)(Heteroaryl); SH; S-$C_{1-8}$-Alkyl; S-Benzyl; S-Aryl; S-Heteroaryl; S(=O)$_2$OH; S(=O)$_2$-O$C_{1-8}$-Alkyl; S(=O)$_2$-O-Aryl; S(=O)$_2$-O-Heteroaryl; S(=O)$_2C_{1-8}$-Alkyl; S(=O)$_2$Aryl, S(=O)$_2$Heteroaryl; S(=O)$C_{1-8}$-Alkyl; S(=O)Aryl, S(=O)Heteroaryl; S(=O)$_2NH_2$; S(=O)$_2$NH-$C_{1-8}$-Alkyl; S(=O)$_2$N($C_{1-8}$-Alkyl)$_2$; S(=O)$_2$NH-Aryl; S(=O)$_2$N(Aryl)$_2$; S(=O)$_2$NH-Heteroaryl; S(=O)$_2$N(Heteroaryl)$_2$; S(=O)$_2$N($C_{1-8}$-Alkyl)(Aryl); S(=O)$_2$N(Heteroaryl)(Aryl); S(=O)$_2$N($C_{1-8}$-Alkyl)(Heteroaryl); Aryl, Heteroaryl, $C_{3-7}$-Cycloalkyl, Heterocyclyl oder $C_{1-8}$-Alkyl verbrücktem Aryl, Heteroaryl, $C_{3-7}$-Cycloalkyl oder Heterocyclyl bedeuten;
oder $R^{18}$ und $R^{19}$ zusammen mit den sie verbindenden Kohlenstoff- oder Stickstoffatomen als Ringgliedern ein an den Phenyl-Ring kondensiertes Aryl oder Heteroaryl, jeweils unsubstituiert oder einfach oder mehrfach substituiert; oder ein an den Phenyl-Ring kondensiertes $C_{3-7}$-Cycloalkyl oder Heterocyclyl, jeweils gesättigt oder ungesättigt, unsubstituiert oder einfach oder mehrfach substituiert; bilden;
In einer weiteren bevorzugten Ausführungsform steht der Rest $R^{12}$ für $C_{4-16}$-Alkyl, gesättigt oder ungesättigt, verzweigt oder unverzweigt;
oder ist ausgewählt aus den folgenden Teilstrukturen **A**, **B** oder **C**,

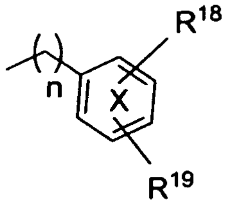
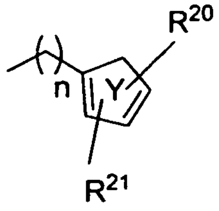
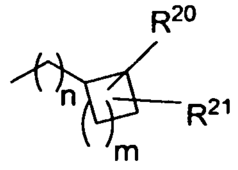

**A** **B** **C**

, wobei

n = 0, 1, 2, 3, 4, 5, 6, 7 oder 8; besonders bevorzugt 1,2 oder 3, insbesondere 1;

m = 0, 1, 2 oder 3; bedeutet;

der Ring X ein N-Atom als Ringglied enthalten kann;

der Ring Y mindestens 1 Heteroatom ausgewählt aus N, O oder S enthält und bis zu 3 Heteroatome unabhängig voneinander ausgewählt aus N, O oder S enthalten kann;

$R^{18}$ und $R^{19}$ unabhängig voneinander H; F; Cl; Br; I; NO; $NO_2$; CN; $NH_2$; NH-$C_{1-8}$-Alkyl; N($C_{1-8}$-Alkyl)$_2$; NH-C(=O)$C_{1-8}$-Alkyl; NH-C(=O)-Aryl; NH-C(=O)-Heteroaryl; $C_{1-8}$-Alkyl; $CF_3$; C(=O)H; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Aryl)(Heteroaryl); OH; O-$C_{1-8}$-Alkyl; O-$C_{1-8}$-Alkyl-OH; $OCF_3$; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; SH; S-$C_{1-8}$-Alkyl; $SCF_3$; S-Benzyl; S-Aryl; S-Heteroaryl; Aryl; Heteroaryl; $C_{3-7}$-Cycloalkyl; Heterocyclyl; oder $C_{1-8}$-Alkyl verbrücktes Aryl, Heteroaryl, $C_{3-7}$-Cycloalkyl oder Heterocyclyl, bedeuten;

oder $R^{18}$ und $R^{19}$ zusammen mit den sie verbindenden Kohlenstoff- oder Stickstoffatomen als Ringgliedern ein an den Phenyl-Ring kondensiertes $C_{3-7}$-Cycloalkyl, gesättigt oder ungesättigt, unsubstituiert oder einfach oder mehrfach substituiert; oder ein an den Phenyl-Ring kondensiertes Phenyl, Imidazolyl oder Thiadiazolyl, jeweils unsubstituiert oder einfach oder mehrfach substituiert; oder zusammen mit den sie verbindenden Kohlenstoffatomen als Ringgliedern O-$CH_2$-O; oder O-$CH_2$-$CH_2$-O; bilden;

$R^{20}$ und $R^{21}$ unabhängig voneinander H; oder $C_{1-8}$-Alkyl, gesättigt oder ungesättigt; verzweigt oder unverzweigt, bedeuten.

Besonders bevorzugt sind Verbindungen, in denen $R^{12}$ die Bedeutung der Teilstruktur **A** hat.

Ganz besonders bevorzugt sind Verbindungen, in denen $R^{12}$ die Bedeutung der Teilstruktur **A** hat, worin $R^{18}$ und $R^{19}$ unabhängig voneinander H; F; Cl; Br; CN; $NH_2$; $C_{1-8}$-Alkyl; $CF_3$; OH; O-$C_{1-8}$-Alkyl; $OCF_3$; oder $SCF_3$ bedeuten;
oder $R^{18}$ und $R^{19}$ zusammen mit den sie verbindenden Kohlenstoff- oder Stickstoffatomen als Ringgliedern ein an den Phenyl-Ring kondensiertes $C_{3-7}$-Cycloalkyl, gesättigt oder ungesättigt, unsubstituiert oder einfach oder mehrfach substituiert; oder ein an den Phenyl-Ring kondensiertes Phenyl, Imidazolyl oder Thiadiazolyl, jeweils unsubstituiert oder einfach oder mehrfach substituiert; oder zusammen mit den sie verbindenden Kohlenstoffatomen als Ringgliedern O-$CH_2$-O; oder O-$CH_2$-$CH_2$-O; bilden.

In einer weiteren bevorzugten Ausführungsform steht der Rest $R^{12}$ für $C_{4-16}$-Alkyl, gesättigt oder ungesättigt, verzweigt oder unverzweigt;
oder ist ausgewählt aus den folgenden Teilstrukturen **A**, **B** oder **C**,

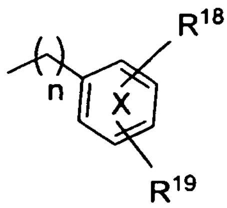
A
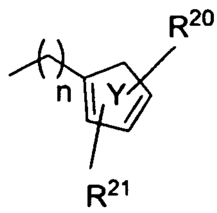
B
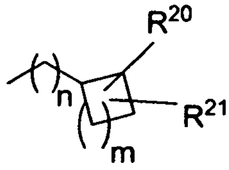
C

, wobei

n = 0, 1, 2, 3, 4, 5 oder 6; besonders bevorzugt 1,2 oder 3, insbesondere 1;

m = 0, 1, 2 oder 3; bedeutet;

der Ring X ein N-Atom als Ringglied enthalten kann;

der Ring Y mindestens 1 Heteroatom ausgewählt aus N, O oder S enthält und bis zu 3 Heteroatome unabhängig voneinander ausgewählt aus N, O oder S enthalten kann;

$R^{18}$ und $R^{19}$ unabhängig voneinander H; F; Cl; CN; $CH_3$; $CF_3$; OH; $OCH_3$; $OCF_3$; oder $SCF_3$ bedeuten;

oder $R^{18}$ und $R^{19}$ zusammen mit den sie verbindenden Kohlenstoff- oder Stickstoffatomen als Ringgliedern ein an den Phenyl-Ring ankondensiertes Phenyl, Imidazol, Thiadiazol, jeweils unsubstituiert oder einfach oder mehrfach substituiert; oder O-$CH_2$-O; oder O-$CH_2$-$CH_2$-O; bilden;

$R^{20}$ und $R^{21}$ unabhängig voneinander H; oder $CH_3$ bedeuten.

Besonders bevorzugt sind Verbindungen, in denen $R^{12}$ die Bedeutung der Teilstruktur **A** hat.

Ganz besonders bevorzugt sind Verbindungen, in denen $R^{12}$ die Bedeutung der Teilstruktur **A** hat, worin $R^{18}$ und $R^{19}$ unabhängig voneinander H; F; Cl; CN; $CH_3$; $CF_3$; OH; $OCH_3$; $OCF_3$; oder $SCF_3$ bedeuten;

In einer anderen bevorzugten Ausführungsform bilden $R^{11}$ und $R^{12}$ zusammen mit dem sie verbindenden Stickstoffatom als Ringglied eine der folgenden Reste,

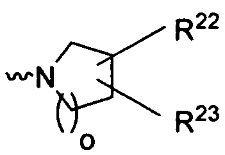
,
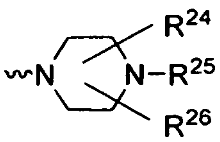
,
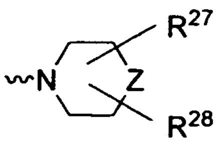
,
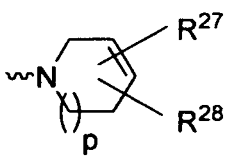
,

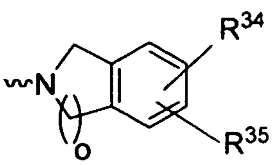
,

worin

Z = O oder S;

o = 0, 1 oder 2;

p = 0 oder 1;

$R^{22}$, $R^{23}$, $R^{24}$, $R^{25}$ und $R^{26}$ unabhängig voneinander H; $C_{1-8}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann;

oder $R^{22}$ und $R^{23}$ mit dem oder den sie verbindenden Kohlenstoffatom(en) als Ringglied(ern) ein $C_{3-7}$-Cycloalkyl oder Heterocyclyl bilden, jeweils gesättigt oder ungesättigt, unsubstituiert oder einfach oder mehrfach substituiert, ggf. mit (Hetero-)aryl kondensiert, unsubstituiert oder einfach oder mehrfach substituiert; oder ein ankondensiertes Aryl oder Heteroaryl bilden, unsubstituiert oder einfach oder mehrfach substituiert;

$R^{27}$ und $R^{28}$ unabhängig voneinander H; $C_{1-8}$-Alkyl, gesättigt oder ungesättigt; verzweigt oder unverzweigt, unsubstituiert oder einfach oder mehrfach substituiert, bedeuten;

$R^{34}$ und $R^{35}$ unabhängig voneinander H; F; Cl; Br; I; NO; $NO_2$; CN; $NH_2$; NH-$C_{1-8}$-Alkyl; N($C_{1-8}$-Alkyl)$_2$; NH-C(=O)$C_{1-8}$-Alkyl; NH-C(=O)-Aryl; NH-C(=O)-Heteroaryl; $C_{1-8}$-Alkyl; $CF_3$; C(=O)H; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl; C(=O)N($C_{1-8}$-Alkyl)$_2$; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Aryl)(Heteroaryl); OH; O-$C_{1-8}$-Alkyl; O-$C_{1-8}$-Alkyl-OH; $OCF_3$; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; SH; S-$C_{1-8}$-Alkyl; $SCF_3$; S-Benzyl; S-Aryl; S-Heteroaryl; Aryl; Heteroaryl; $C_{3-7}$-Cycloalkyl; Heterocyclyl; oder $C_{1-8}$-Alkyl verbrücktes Aryl, Heteroaryl, $C_{3-7}$-Cycloalkyl oder Heterocyclyl, bedeuten.

Bevorzugt bilden $R^{11}$ und $R^{12}$ zusammen mit dem sie verbindenden Stickstoffatom als Ringglied einen der folgenden Reste,

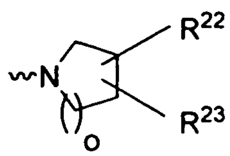
,
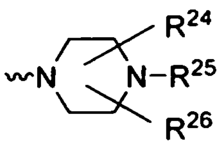
,
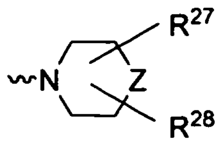
,
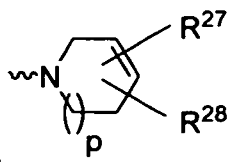
,

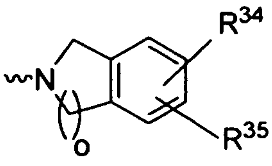
,

worin

Z = O oder S;

o = 0, 1 oder 2;

p = 0 oder 1;

$R^{22}$, $R^{23}$ und $R^{25}$ jeweils unabhängig voneinander ausgewählt sind aus H; $C_{1-8}$-Alkyl, gesättigt oder ungesättigt; verzweigt oder unverzweigt; oder einen der nachfolgenden Reste bilden,

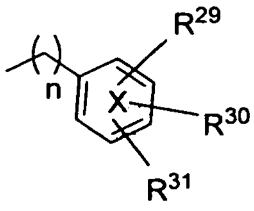 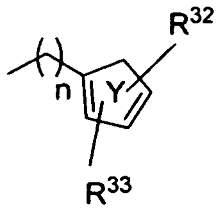 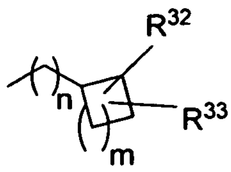

,

worin

n = 0, 1, 2, 3 oder 4;

m = 0, 1, 2 oder 3;

der Ring X ein N-Atom enthalten kann;

der Ring Y bis zu 3 Heteroatome unabhängig voneinander ausgewählt aus N, O oder S enthalten kann;

$R^{29}$ $R^{30}$ und $R^{31}$ unabhängig voneinander H; F; Cl; Br; CN; $NH_2$; $C_{1-8}$-Alkyl; $CF_3$; OH; O-$C_{1-8}$-Alkyl; $OCF_3$; oder $SCF_3$ bedeuten;

oder $R^{29}$ und $R^{30}$ zusammen mit den sie verbindenden Kohlenstoffatomen als Ringgliedern ein an den Phenyl-Ring ankondensiertes $C_{3-7}$-Cycloalkyl, gesättigt oder ungesättigt; oder ein an den Phenyl-Ring ankondensiertes Phenyl, unsubstituiert oder einfach oder mehrfach substituiert; oder O-$CH_2$-O; oder O-$CH_2$-$CH_2$-O; bilden;

$R^{32}$ und $R^{33}$ unabhängig voneinander H; oder $C_{1-8}$-Alkyl, gesättigt oder ungesättigt; verzweigt oder unverzweigt, bedeuten.

oder $R^{22}$ und $R^{23}$ mit dem oder den sie verbindenden Kohlenstoffatom(en) als Ringglied(ern) ein $C_{3-7}$-Cycloalkyl oder Heterocyclyl bilden, jeweils gesättigt oder ungesättigt, ggf. mit (Hetero-)aryl kondensiert, unsubstituiert oder einfach oder mehrfach substituiert; oder ein ankondensiertes Aryl oder Heteroaryl bilden, unsubstituiert oder einfach oder mehrfach substituiert;

$R^{24}$ und $R^{26}$ jeweils unabhängig voneinander H; $C_{1-8}$-Alkyl, gesättigt oder ungesättigt; verzweigt oder unverzweigt, unsubstituiert oder einfach oder mehrfach substituiert; Aryl oder Heteroaryl, jeweils unsubstituiert oder einfach oder mehrfach substituiert; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann;

$R^{27}$ und $R^{28}$ jeweils unabhängig voneinander H; $C_{1-8}$-Alkyl, gesättigt oder ungesättigt; verzweigt oder unverzweigt, unsubstituiert oder einfach oder mehrfach substituiert, bedeuten;

$R^{34}$ und $R^{35}$ unabhängig voneinander H; F; Cl; Br; CN; $NH_2$; $C_{1-8}$-Alkyl; $CF_3$; OH; O-$C_{1-8}$-Alkyl; $OCF_3$; oder $SCF_3$ bedeuten.

Besonders bevorzugt bilden $R^{11}$ und $R^{12}$ zusammen mit dem sie verbindenden Stickstoffatom als Ringglied einen der folgenden Reste

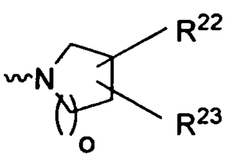 , 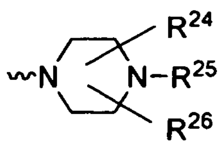 , 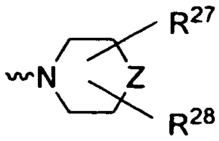 , 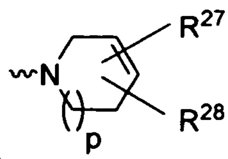 ,

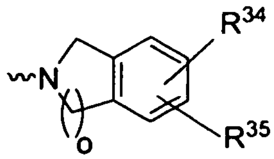

,

worin

Z = O oder S;

o = 0, 1 oder 2;

p = 0 oder 1;

$R^{22}$, $R^{23}$ und $R^{25}$ jeweils unabhängig voneinander ausgewählt sind aus H; $C_{1-8}$-Alkyl, gesättigt oder ungesättigt; verzweigt oder unverzweigt; oder einem der nachfolgenden Reste

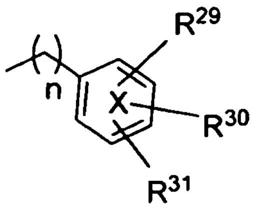 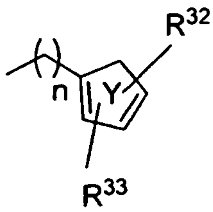 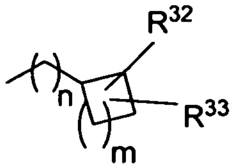

,

worin

n = 0, 1, 2, 3 oder 4;

m = 0, 1, 2 oder 3;

der Ring X ein N-Atom enthalten kann;

der Ring Y bis zu 3 Heteroatome unabhängig voneinander ausgewählt aus N, O oder S enthalten kann;

$R^{29}$, $R^{30}$ und $R^{31}$ unabhängig voneinander H; F; Cl; CN; $CH_3$; $CF_3$; OH; $OCH_3$; $OCF_3$; oder $SCF_3$ bedeuten;

oder $R^{29}$ und $R^{30}$ zusammen mit den sie verbindenden Kohlenstoffatomen als Ringgliedern O-$CH_2$-O; oder O-$CH_2$-$CH_2$-O; bilden;

$R^{32}$ und $R^{33}$ unabhängig voneinander H; oder $CH_3$ bedeuten;

oder $R^{22}$ und $R^{23}$ mit dem oder den sie verbindenden Kohlenstoffatom(en) als Ringglied(ern) ein $C_{3-7}$-Cycloalkyl, gesättigt oder ungesättigt; oder ein ankondensiertes Phenyl oder Thienyl bilden, jeweils unsubstituiert oder einfach oder mehrfach substituiert mit F; Cl; CN; $CH_3$; $CF_3$; OH; $OCH_3$; $OCF_3$; oder $SCF_3$;

$R^{24}$ und $R^{26}$ jeweils unabhängig voneinander H; $C_{1-8}$-Alkyl, gesättigt oder ungesättigt; verzweigt oder unverzweigt; Phenyl, unsubstituiert oder einfach oder mehrfach substituiert; bedeutet;

$R^{27}$ und $R^{28}$ jeweils unabhängig voneinander H; oder $CH_3$ bedeuten;

$R^{34}$ und $R^{35}$ unabhängig voneinander H; F; Cl; CN; $CH_3$; $CF_3$; OH; $OCH_3$; $OCF_3$; oder $SCF_3$ bedeuten.

Besonders bevorzugt sind Verbindungen, bei denen $R^{11}$ und $R^{12}$ zusammen mit dem sie verbindenden Stickstoffatom als Ringglied den Rest

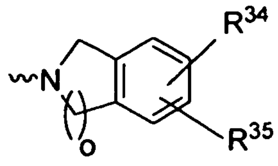

bilden.

Besonders bevorzugt sind Verbindungen der allgemeinen Formel (1), worin
$R^1$ ausgewählt ist aus H; $C_{1-8}$-Alkyl; $C_{3-7}$-Cycloalkyl; über $C_1$-Alkyl verbrücktes $C_{3-7}$-Cycloalkyl; Phenyl, unsubstituiert oder einfach oder zweifach substituiert mit Substituenten unabhängig voneinander ausgewählt aus der Gruppe bestehend aus F, Cl, Br, $CH_3$, $C_2H_5$, iso-Propyl, $OCH_3$ oder $CF_3$; Thienyl, unsubstituiert oder einfach oder zweifach substituiert mit $CH_3$; über $C_{1-3}$-Alkyl verbrücktes Phenyl, unsubstituiert; $R^2$ ausgewählt ist aus H; $CH_3$, oder $C_2H_5$, vorzugsweise aber H bedeutet;
oder $R^1$ und $R^2$ zusammen mit dem sie verbindenden Kohlenstoffatom als Ringglied ein $C_{3-7}$-Cycloalkyl bilden, gesättigt, unsubstituiert, ggf. mit Phenyl kondensiert;
$R^3$, $R^4$, $R^5$ jeweils H bedeuten und $R^6$ H, -$CH_3$ oder Phenyl bedeutet.
einer der Reste $R^7$ oder $R^9$ ausgewählt ist aus der Gruppe bestehend aus H; $CH_3$; Phenyl, unsubstituiert; und der verbleibende Rest für H steht;
oder $R^7$ und $R^9$ zusammen mit den sie verbindenden Kohlenstoffatomen als Ringgliedern ein $C_{3-7}$-Cycloalkyl bilden, gesättigt oder ungesättigt, unsubstituiert, ggf. mit Phenyl kondensiert, unsubstituiert;
$R^8$ und $R^{10}$ jeweils H bedeuten;
oder $R^7$ und $R^8$; oder $R^9$ und R'°; zusammen mit den sie verbindenden Kohlenstoffatomen als Ringgliedem ein $C_{3-7}$-Cycloalkyl bilden, gesättigt oder ungesättigt, unsubstituiert, ggf. mit Phenyl kondensiert, unsubstituiert;
$R^{11}$ für H; $C_{1-8}$-Alkyl, gesättigt oder ungesättigt, verzweigt oder unverzweigt; oder Benzyl steht; vorzugsweise ausgewählt ist aus H; $CH_3$; $C_2H_5$; Propyl; iso-Propyl; iso-Pentyl; oder Benzyl;
$R^{12}$ für $C_{1-8}$-Alkyl, gesättigt oder ungesättigt, verzweigt oder unverzweigt; $C_{3-7}$-Cycloalkyl, gesättigt oder ungesättigt, ggf. mit Phenyl kondensiert, wobei der Phenylring unsubstituiert oder einfach oder zweifach substituiert sein kann mit $CF_3$; Indazolyl; Benzothiadiazolyl, jeweils unsubstituiert oder einfach mit $CH_3$ substituiert; Phenyl, unsubstituiert oder einfach oder zweifach substituiert mit Substituenten unabhängig voneinander ausgewählt aus der Gruppe bestehend aus F; Cl; $OCH_3$; $CH_3$; $CF_3$; O-$CH_2$-O; Benzyl; über $C_{1-4}$-Alkyl verbrücktes Napththyl, Imidazolyl, Indolyl, Pyrimidinyl, Pyridyl, Triazolyl, Furanyl oder Thienyl, jeweils unsubstituiert oder einfach substituiert mit $CH_3$, wobei die Alkylkette jeweils gesättigt oder ungesättigt, verzweigt oder unverzweigt sein kann; über $C_{1-4}$-Alkyl verbrücktes $C_{3-7}$-Cycloalkyl, gesättigt oder ungesättigt; über $C_{1-4}$-Alkyl verbrücktes Piperidinyl, Tetrahydrofuranyl, Morpholinyl oder Diphenylmethyl; über $C_{1-4}$-Alkyl verbrücktes Isoxazolyl, unsubstituiert oder einfach oder zweifach substituiert mit Substituenten unabhängig voneinander ausgewählt aus der Gruppe bestehend aus $CH_3$ oder Phenyl; über $C_{1-4}$-Alkyl verbrücktes Phenyl, unsubstituiert oder einfach oder zweifach substituiert mit Substituenten unabhängig voneinander ausgewählt aus der Gruppe bestehend aus F, Cl, Br, $CH_3$, $CF_3$, $OCH_3$, O-$CH_2$-O, Benzyl, wobei die Alkylkette jeweils gesättigt oder ungesättigt, verzweigt oder unverzweigt sein kann;
oder $R^{11}$ und $R^{12}$ zusammen mit dem sie verbindenden Stickstoffatom als Ringglied ein Piperazinyl bilden, unsubstituiert oder einfach oder zweifach substituiert mit $C_{1-4}$-Alkyl, gesättigt oder ungesättigt, verzweigt oder unverzweigt; C(=O)$CH_3$; C(=O)$C_2H_5$; C(=O)$OCH_3$; C(=O)O$C_2H_5$; C(=O)-Furanyl; Phenyl, unsubstituiert oder einfach oder zweifach substituiert mit Substituenten unabhängig voneinander ausgewählt aus der Gruppe bestehend aus F, Cl, Br, $CH_3$, $CF_3$, $OCH_3$ oder O-$CH_2$-O; Pyrimidinyl, unsubstituiert; Pyridyl, jeweils unsubstituiert oder einfach oder zweifach substituiert mit Substituenten unabhängig voneinander ausgewählt aus der Gruppe bestehend aus F, Cl oder $CH_3$; über $C_{1-4}$-Alkyl verbrücktes Phenyl, unsubstituiert oder einfach, zweifach oder dreifach substituiert mit Substituenten unabhängig voneinander ausgewählt aus der Gruppe bestehend aus F, Cl, Br, $CH_3$, tert-Butyl, $CF_3$, $OCH_3$ oder O-$CH_2$-O, wobei die Alkylkette jeweils gesättigt oder ungesättigt, verzweigt oder unverzweigt sein kann; über $C_{1-4}$-Alkyl verbrücktes $C_{3-7}$-Cycloalkyl, gesättigt oder ungesättigt, ggf. mit Phenyl, unsubstituiert, kondensiert, wobei die Alkylkette jeweils gesättigt oder ungesättigt, verzweigt oder unverzweigt sein kann;
oder $R^{11}$ und $R^{12}$ zusammen mit dem sie verbindenden Stickstoffatom als Ringglied ein Piperidinyl bilden, unsubstituiert oder einfach oder zweifach substituiert mit $C_{1-4}$-Alkyl, gesättigt oder ungesättigt, verzweigt oder unverzweigt; C(=O)$OCH_3$; C(=O)O$C_2H_5$; Phenyl, unsubstituiert oder einfach oder zweifach substituiert mit Substituenten unabhängig voneinander ausgewählt aus der Gruppe bestehend aus F, Cl, $CH_3$, $OCH_3$, oder $CF_3$; über $C_{1-4}$-Alkyl verbrücktes Phenyl, unsubstituiert, wobei die Alkylkette jeweils gesättigt oder ungesättigt, verzweigt oder unverzweigt sein kann; über $C_{1-4}$-Alkyl verbrücktes Pyridyl, unsubstituiert oder einfach oder zweifach substituiert mit Substituenten unabhängig voneinander ausgewählt aus der Gruppe bestehend aus $CH_3$, $OCH_3$ oder $CF_3$, wobei die Alkylkette jeweils gesättigt oder ungesättigt, verzweigt oder unverzweigt sein kann; und/oder das Piperidinyl ggf. mit Cyclohexyl, Phenyl oder Thienyl kondensiert

sein kann, jeweils unsubstituiert oder einfach oder zweifach substituiert mit $CF_3$;
oder $R^{11}$ und $R^{12}$ zusammen mit dem sie verbindenden Stickstoffatom als Ringglied ein Azetidinyl, Aziridinyl, Tetrahydropyridinyl oder Dihydropyrrolyl bilden;
oder $R^{11}$ und $R^{12}$ zusammen mit dem sie verbindenden Stickstoffatom als Ringglied ein Pyrrolidinyl bilden, unsubstituiert oder einfach oder zweifach substituiert mit $C_{1-4}$-Alkyl, gesättigt oder ungesättigt, verzweigt oder unverzweigt; $CH_2OCH_3$; Phenyl, unsubstituiert; Pyridyl, unsubstituiert oder einfach substituiert mit Substituenten unabhängig voneinander ausgewählt aus der Gruppe bestehend aus F, Cl, Br oder $CH_3$; Isothiazolyl; Thiazolyl; über $C_{1-4}$-Alkyl verbrücktes Pyridyl, unsubstituiert oder einfach oder zweifach substituiert mit Substituenten unabhängig voneinander ausgewählt aus der Gruppe bestehend aus $CH_3$, $C_2H_5$, F, Cl, Br, wobei die Alkylkette jeweils gesättigt oder ungesättigt, verzweigt oder unverzweigt sein kann; über $C_{1-4}$-Alkyl verbrücktes Phenyl, unsubstituiert, wobei die Alkylkette jeweils gesättigt oder ungesättigt, verzweigt oder unverzweigt sein kann; und/oder das Pyrrolidinyl ggf. mit Cyclohexyl, Phenyl oder Thienyl kondensiert sein kann, jeweils unsubstituiert oder einfach oder zweifach substituiert mit F; $CH_3$; $CF_3$;
oder $R^{11}$ und $R^{12}$ zusammen mit dem sie verbindenden Stickstoffatom als Ringglied ein Morpholinyl bilden, unsubstituiert oder einfach oder zweifach substituiert mit $CH_3$;
oder $R^{11}$ und $R^{12}$ zusammen mit dem sie verbindenden Stickstoffatom als Ringglied ein Thiomorpholinyl bilden;
einer der Reste $R^{13}$ und $R^{14}$; oder $R^{13}$ und $R^{15}$; oder $R^{14}$ und $R^{15}$ für $CH_3$ steht und der verbleibende Rest H bedeutet.

Ganz besonders bevorzugt sind Verbindungen aus der Gruppe

| | |
|---|---|
| **1** | 4-Oxo-4-(4-(thiophen-2-yl)-6,7-dihydrothieno[3,2-c]pyridin-5(4H)-yl)-N-(3-(trifluormethyl)benzyl) butanamid; |
| **2** | 4-Oxo-4-(4-phenyl-6,7-dihydrothieno[3,2-c]pyridin-5(4H)-yl)-N-(3-(trifluormethyl)benzyl)butanamid; |
| **3** | 4-(4-(3-Fluorphenyl)-6,7-dihydrothieno[3,2-c]pyridin-5(4H)-yl)-4-oxo-N-(3-(trifluormethyl)benzyl) butanamid; |
| **4** | 4-Oxo-4-(4-m-tolyl-6,7-dihydrothieno[3,2-c]pyridin-5(4H)-yl)-N-(3-(trifluormethyl)benzyl)butanamid; |
| **5** | 4-Oxo-4-(4-p-tolyl-6,7-dihydrothieno[3,2-c]pyridin-5(4H)-yl)-N-(3-(trifluormethyl)benzyl)butanamid; |
| **6** | 4-(4-(2-Fluorphenyl)-6,7-dihydrothieno[3,2-c]pyridin-5(4H)-yl)-4-oxo-N-(3-(trifluormethyl)benzyl) butanamid; |
| **7** | 4-(4-(4-Fluorphenyl)-6,7-dihydrothieno[3,2-c]pyridin-5(4H)-yl)-4-oxo-N-(3-(trifluormethyl)benzyl) butanamid; |
| **8** | 4-Oxo-4-(4-o-tolyl-6,7-dihydrothieno[3,2-c]pyridin-5(4H)-yl)-N-(3-(trifluormethyl)benzyl)butanamid; |
| **9** | 4-Oxo-4-(7-phenyl-4,5-dihydrothieno[2,3-c]pyridin-6(7H)-yl)-N-(3-(trifluormethyl)benzyl)butanamid; |
| **10** | 4-(2-Methyl-4-phenyl-6,7-dihydrothieno[3,2-c]pyridin-5(4H)-yl)-4-oxo-N-(3-(trifluormethyl)benzyl) butanamid; |
| **11** | N-(4-Methylbenzyl)-4-oxo-4-(4-phenyl-6,7-dihydrothieno[3,2-c]pyridin-5(4H)-yl)butanamid; |
| **12** | N-Benzyl-4-oxo-4-(4-phenyl-6,7-dihydrothieno[3,2-c]pyridin-5(4H)-yl)butanamid; |
| **13** | 4-Oxo-4-(4-phenyl-6,7-dihydrothieno[3,2-c]pyridin-5(4H)-yl)-N-(4-(trifluormethyl)benzyl)butanamid; |
| **14** | N-(2-Methoxybenzyl)-4-oxo-4-(4-phenyl-6,7-dihydrothieno[3,2-c]pyridin-5(4H)-yl)butanamid; |
| **15** | N-(3-Methoxybenzyl)-4-oxo-4-(4-phenyl-6,7-dihydrothieno[3,2-c]pyridin-5(4H)-yl)butanamid; |
| **16** | N-(4-Methoxybenzyl)-4-oxo-4-(4-phenyl-6,7-dihydrothieno[3,2-c]pyridin-5(4H)-yl)butanamid; |
| **17** | 4-Oxo-4-(4-phenyl-6,7-dihydrothieno[3,2-c]pyridin-5(4H)-yl)-N-(2-(trifluormethyl)benzyl)butanamid; |
| **18** | N-(3-Fluorbenzyl)-4-oxo-4-(4-phenyl-6,7-dihydrothieno[3,2-c]pyridin-5(4H)-yl)butanamid; |
| **19** | N-(3-Methylbenzyl)-4-oxo-4-(4-phenyl-6,7-dihydrothieno[3,2-c]pyridin-5(4H)-yl)butanamid; |
| **20** | 4-(3-Methyl-4-phenyl-6,7-dihydrothieno[3,2-c]pyridin-5(4H)-yl)-4-oxo-N-(3-(trifluormethyl)benzyl) butanamid; |
| **21** | 4-(4-Cyclohexyl-6,7-dihydrothieno[3,2-c]pyridin-5(4H)-yl)-4-oxo-N-(3-(trifluormethyl)benzyl)butanamid; |
| **22** | 4-(4-Isopropyl-6,7-dihydrothieno[3,2-c]pyridin-5(4H)-yl)-4-oxo-N-(3-(trifluormethyl)benzyl)butanamid; |
| **23** | 4-(4-Butyl-6,7-dihydrothieno[3,2-c]pyridin-5(4H)-yl)-4-oxo-N-(3-(trifluormethyl)benzyl)butanamid; |
| **24** | N-Methyl-4-oxo-4-(4-phenyl-6,7-dihydrothieno[3,2-c]pyridin-5(4H)-yl)-N-(3-(trifluormethyl)benzyl) butanamid; |
| **25** | 4-Oxo-N-phenethyl-4-(4-phenyl-6,7-dihydrothieno[3,2-c]pyridin-5(4H)-yl)butanamid; |
| **26** | N-(2-Methylbenzyl)-4-oxo-4-(4-phenyl-6,7-dihydrothieno[3,2-c]pyridin-5(4H)-yl)butanamid; |
| **27** | 4-Oxo-2-phenyl-4-(4-phenyl-6,7-dihydrothieno[3,2-c]pyridin-5(4H)-yl)-N-(3-(trifluormethyl)benzyl) butanamid; |
| **28** | (R)-2-Methyl-4-oxo-4-(4-phenyl-6,7-dihydrothieno[3,2-c]pyridin-5(4H)-yl)-N-(3-(trifluormethyl)benzyl) butanamid; |

(fortgesetzt)

| | |
|---|---|
| **29** | 4-Oxo-3-phenyl-4-(4-phenyl-6,7-dihydrothieno[3,2-c]pyridin-5(4H)-yl)-N-(3-(trifluormethyl)benzyl) butanamid; |
| **30** | 4-(6,7-Dihydrothieno[3,2-c]pyridin-5(4H)-yl)-4-oxo-3-phenyl-N-(3-(trifluormethyl)benzyl)butanamid; |
| **31** | rac. (1S,2R)-2-(4-Phenyl-4,5,6,7-tetrahydrothieno[3,2-c]pyridin-5-carbonyl)-N-(3-(trifluormethyl)benzyl) cyclohexancarboxamid; |
| **32** | rac. (1S)-2-(4-Phenyl-4,5,6,7-tetrahydrothieno[3,2-c]pyridin-5-carbonyl)-N-(3-(trifluormethyl)benzyl) cyclohexancarboxamid; |
| **33** | rac. (1S,2R)-2-(4-Phenyl-4,5,6,7-tetrahydrothieno[3,2-c]pyridin-5-carbonyl)-N-(3-(trifluormethyl)benzyl) cyclopentanecarboxamid; |
| **34** | (R)-3-Methyl-4-oxo-4-(4-phenyl-6,7-dihydrothieno[3,2-c]pyridin-5(4H)-yl)-N-(3-(trifluormethyl)benzyl) butanamid; |
| **35** | (-)-4-Oxo-4-(4-phenyl-6,7-dihydrothieno[3,2-c]pyridin-5(4H)-yl)-N-(3-(trifluormethyl)benzyl)butanamid; |
| **36** | (+)-4-Oxo-4-(4-phenyl-6,7-dihydrothieno[3,2-c]pyridin-5(4H)-yl)-N-(3-(trifluormethyl)benzyl)butanamid; |
| **37** | 4-(6,7-Dihydrothieno[3,2-c]pyridin-5(4H)-yl)-4-oxo-2-phenyl-N-(3-(trifluormethyl)benzyl)butanamid; |
| **38** | N-(3,5-Bis(trifluormethyl)benzyl)-4-oxo-4-(4-phenyl-6,7-dihydrothieno[3,2-c]pyridin-5(4H)-yl)butanamid; |
| **39** | N-(2-Fluor-5-(trifluormethyl)benzyl)-4-oxo-4-(4-phenyl-6,7-dihydrothieno[3,2-c]pyridin-5(4H)-yl) butanamid; |
| **40** | N-(2-Fluor-3-(trifluormethyl)benzyl)-4-oxo-4-(4-phenyl-6,7-dihydrothieno[3,2-c]pyridin-5(4H)-yl) butanamid; |
| **41** | N-(3-Fluor-5-(trifluormethyl)benzyl)-4-oxo-4-(4-phenyl-6,7-dihydrothieno[3,2-c]pyridin-5(4H)-yl) butanamid; |
| **42** | rac. (1S,2R)-2-(4-Phenyl-4,5,6,7-tetrahydrothieno[3,2-c]pyridin-5-carbonyl)-N-(3-(trifluormethyl)benzyl) cyclopropancarboxamid; |
| **43** | N-(3,4-Difluorbenzyl)-4-oxo-4-(4-phenyl-6,7-dihydrothieno[3,2-c]pyridin-5(4H)-yl)butanamid; |
| **44** | 4-Oxo-4-(5-phenyl-4,5-dihydrothieno[2,3-c]pyridin-6(7H)-yl)-N-(3-(trifluormethyl)benzyl)butanamid; |
| **45** | 4-(1-Methyl-4-phenyl-6,7-dihydrothieno[3,4-c]pyridin-5(4H)-yl)-4-oxo-N-(3-(trifluormethyl)benzyl) butanamid; |
| **46** | N-(4-Methoxyphenyl)-4-oxo-4-(4-p-tolyl-6,7-dihydrothieno[3,2-c]pyridin-5(4H)-yl)butanamid; |
| **47** | N-(1-Methyl-1H-indazol-6-yl)-4-oxo-4-(4-p-tolyl-6,7-dihydrothieno[3,2-c]pyridin-5(4H)-yl)butanamid; |
| **48** | N-Benzyl-4-oxo-4-(4-p-tolyl-6,7-dihydrothieno[3,2-c]pyridin-5(4H)-yl)butanamid; |
| **49** | 4-Oxo-N-phenethyl-4-(4-p-tolyl-6,7-dihydrothieno[3,2-c]pyridin-5(4H)-yl)butanamid; |
| **50** | 4-Oxo-N-(pyridin-4-ylmethyl)-4-(4-p-tolyl-6,7-dihydrothieno[3,2-c]pyridin-5(4H)-yl)butanamid; |
| **51** | 4-Oxo-N-(3-phenylpropyl)-4-(4-p-tolyl-6,7-dihydrothieno[3,2-c]pyridin-5(4H)-yl)butanamid; |
| **52** | N-(Benzo[c][1,2,5]thiadiazol-4-yl)-4-oxo-4-(4-m-tolyl-6,7-dihydrothieno[3,2-c]pyridin-5(4H)-yl)butanamid; |
| **53** | N-(1-Methyl-1H-indazol-6-yl)-4-oxo-4-(4-m-tolyl-6,7-dihydrothieno[3,2-c]pyridin-5(4H)-yl)butanamid; |
| **54** | 4-Oxo-N-(pyridin-2-ylmethyl)-4-(4-m-tolyl-6,7-dihydrothieno[3,2-c]pyridin-5(4H)-yl)butanamid; |
| **55** | 1-(4-Methylpiperazin-1-yl)-4-(4-m-tolyl-6,7-dihydrothieno[3,2-c]pyridin-5(4H)-yl)butan-1,4-dion; |
| **56** | 3-Methyl-4-oxo-4-(4-phenyl-6,7-dihydrothieno[3,2-c]pyridin-5(4H)-yl)-N-(pyridin-2-ylmethyl)butanamid; |
| **57** | 4-Oxo-4-(4-phenyl-6,7-dihydrothieno[3,2-c]pyridin-5(4H)-yl)-N-(thiophen-2-ylmethyl)butanamid; |
| **58** | N-(2-Chlorphenyl)-4-(4-(4-fluorphenyl)-6,7-dihydrothieno[3,2-c]pyridin-5(4H)-yl)-4-oxobutanamid; |
| **59** | 4-(4-(4-Chlorphenyl)-6,7-dihydrothieno[3,2-c]pyridin-5(4H)-yl)-N-(furan-2-ylmethyl)-4-oxobutanamid; |
| **60** | 4-(4-(4-Chlorphenyl)-6,7-dihydrothieno[3,2-c]pyridin-5(4H)-yl)-4-oxo-N-propylbutanamid; |
| **61** | N-(2-Chlorbenzyl)-4-oxo-4-(4-m-tolyl-6,7-dihydrothieno[3,2-c]pyridin-5(4H)-yl)butanamid; |
| **62** | N-(2,4-Dichlorbenzyl)-4-(4-(3-fluorphenyl)-6,7-dihydrothieno[3,2-c]pyridin-5(4H)-yl)-4-oxobutanamid; |
| **63** | N-(4-Fluorbenzyl)-4-(4-(3-fluorphenyl)-6,7-dihydrothieno[3,2-c]pyridin-5(4H)-yl)-4-oxobutanamid; |
| **64** | N-(3,4-Dichlorbenzyl)-3-methyl-4-oxo-4-(4-phenyl-6,7-dihydrothieno[3,2-c]pyridin-5(4H)-yl)butanamid; |
| **65** | N-(2,5-Difluorbenzyl)-3-methyl-4-oxo-4-(4-phenyl-6,7-dihydrothieno[3,2-c]pyridin-5(4H)-yl)butanamid; |
| **66** | 3-Methyl-4-oxo-4-(4-phenyl-6,7-dihydrothieno[3,2-c]pyridin-5(4H)-yl)-N-(3-(trifluormethyl)benzyl) butanamid; |
| **67** | 1-(4-(4-Chlorphenyl)-6,7-dihydrothieno[3,2-c]pyridin-5(4H)-yl)-4-morpholinobutan-1,4-dion; |
| **68** | 1-(4-(4-Chlorphenyl)-6,7-dihydrothieno[3,2-c]pyridin-5(4H)-yl)-4-(4-(4-fluorphenyl)piperazin-1-yl)butan-1,4-dion; |

(fortgesetzt)

| | |
|---|---|
| **69** | 2-Methyl-N-(2-(5-methyl-1H-pyrazol-1-yl)ethyl)-4-oxo-4-(4-phenyl-6,7-dihydrothieno[3,2-c]pyridin-5(4H)-yl)butanamid; |
| **70** | N-(Naphthalen-1-ylmethyl)-4-oxo-4-(4-phenyl-6,7-dihydrothieno[3,2-c]pyridin-5(4H)-yl)butanamid; |
| **71** | 4-(4-(4-Fluorphenyl)-6,7-dihydrothieno[3,2-c]pyridin-5(4H)-yl)-N-(4-methylbenzyl)-4-oxobutanamid; |
| **72** | N-(Benzo[d][1,3]dioxol-5-ylmethyl)-4-(4-(4-fluorphenyl)-6,7-dihydrothieno[3,2-c]pyridin-5(4H)-yl)-4-oxobutanamid; |
| **73** | 4-(4-(4-Fluorphenyl)-6,7-dihydrothieno[3,2-c]pyridin-5(4H)-yl)-4-oxo-N-(2-(trifluormethyl)benzyl) butanamid; |
| **74** | 4-(4-(4-Fluorphenyl)-6,7-dihydrothieno[3,2-c]pyridin-5(4H)-yl)-N-(naphthalen-1-ylmethyl)-4-oxobutanamid; |
| **75** | 4-(4-(4-Chlorphenyl)-6,7-dihydrothieno[3,2-c]pyridin-5(4H)-yl)-N-(3-fluorbenzyl)-4-oxobutanamid; |
| **76** | 2-Methyl-N-(1-methyl-1H-indazol-6-yl)-4-oxo-4-(4-phenyl-6,7-dihydrothieno[3,2-c]pyridin-5(4H)-yl) butanamid; |
| **77** | N-(4-Methoxybenzyl)-2-methyl-4-oxo-4-(4-phenyl-6,7-dihydrothieno[3,2-c]pyridin-5(4H)-yl)butanamid; |
| **78** | 2-Methyl-1-(4-methylpiperazin-1-yl)-4-(4-phenyl-6,7-dihydrothieno[3,2-c]pyridin-5(4H)-yl)butan-1,4-dion; |
| **79** | N-(2-(1H-Indol-3-yl)ethyl)-2-methyl-4-oxo-4-(4-phenyl-6,7-dihydrothieno[3,2-c]pyridin-5(4H)-yl) butanamid; |
| **80** | N-(2-Fluorbenzyl)-2-methyl-4-oxo-4-(4-phenyl-6,7-dihydrothieno[3,2-c]pyridin-5(4H)-yl)butanamid; |
| **81** | 2-Methyl-4-oxo-4-(4-phenyl-6,7-dihydrothieno[3,2-c]pyridin-5(4H)-yl)-N-(2-(trifluormethyl)benzyl) butanamid; |
| **82** | 4-Oxo-N-(2-(piperidin-1-yl)ethyl)-4-(4-p-tolyl-6,7-dihydrothieno[3,2-c]pyridin-5(4H)-yl)butanamid; |
| **83** | 4-Oxo-N-((tetrahydrofuran-2-yl)methyl)-4-(4-p-tolyl-6,7-dihydrothieno[3,2-c]pyridin-5(4H)-yl)butanamid; |
| **84** | N-(4-Chlorbenzyl)-4-oxo-4-(4-p-tolyl-6,7-dihydrothieno[3,2-c]pyridin-5(4H)-yl)butanamid; |
| **85** | N-(2,3-Dichlorbenzyl)-4-oxo-4-(4-p-tolyl-6,7-dihydrothieno[3,2-c]pyridin-5(4H)-yl)butanamid; |
| **86** | 4-Oxo-N-(2-(thiophen-2-yl)ethyl)-4-(4-m-tolyl-6,7-dihydrothieno[3,2-c]pyridin-5(4H)-yl)butanamid; |
| **87** | N-(Cyclohexylmethyl)-4-oxo-4-(4-m-tolyl-6,7-dihydrothieno[3,2-c]pyridin-5(4H)-yl)butanamid; |
| **88** | N-(3-Chlorphenethyl)-4-oxo-4-(4-m-tolyl-6,7-dihydrothieno[3,2-c]pyridin-5(4H)-yl)butanamid; |
| **89** | N-(3,3-Diphenylpropyl)-4-oxo-4-(4-m-tolyl-6,7-dihydrothieno[3,2-c]pyridin-5(4H)-yl)butanamid; |
| **90** | 4-(4-(3-Fluorphenyl)-6,7-dihydrothieno[3,2-c]pyridin-5(4H)-yl)-N-(3-morpholinopropyl)-4-oxobutanamid; |
| **91** | 4-(4-(3-Fluorphenyl)-6,7-dihydrothieno[3,2-c]pyridin-5(4H)-yl)-4-oxo-N-(pyridin-3-ylmethyl)butanamid; |
| **92** | 4-(4-(3-Fluorphenyl)-6,7-dihydrothieno[3,2-c]pyridin-5(4H)-yl)-N-(3-methylbenzyl)-4-oxobutanamid; |
| **93** | 4-(4-(3-Fluorphenyl)-6,7-dihydrothieno[3,2-c]pyridin-5(4H)-yl)-N-(4-methylphenethyl)-4-oxobutanamid; |
| **94** | 3-Methyl-4-oxo-4-(4-phenyl-6,7-dihydrothieno[3,2-c]pyridin-5(4H)-yl)-N-(4-phenylbutyl)butanamid; |
| **95** | N-(Biphenyl-4-ylmethyl)-4-oxo-4-(4-m-tolyl-6,7-dihydrothieno[3,2-c]pyridin-5(4H)-yl)butanamid; |
| **96** | 4-(4-(3-Fluorphenyl)-6,7-dihydrothieno[3,2-c]pyridin-5(4H)-yl)-N-(2-methoxybenzyl)-4-oxobutanamid; |
| **97** | N-(4-Chlorphenethyl)-4-(4-(3-fluorphenyl)-6,7-dihydrothieno[3,2-c]pyridin-5(4H)-yl)-4-oxobutanamid; |
| **98** | 4-(4-(3-Fluorphenyl)-6,7-dihydrothieno[3,2-c]pyridin-5(4H)-yl)-N-((5-methyl-3-phenylisoxazol-4-yl) methyl)-4-oxobutanamid; |
| **99** | N-(2,6-Difluorbenzyl)-3-methyl-4-oxo-4-(4-phenyl-6,7-dihydrothieno[3,2-c]pyridin-5(4H)-yl)butanamid; |
| **100** | N-(3,5-Difluorbenzyl)-3-methyl-4-oxo-4-(4-phenyl-6,7-dihydrothieno[3,2-c]pyridin-5(4H)-yl)butanamid; |
| **101** | N-(3-Chlorbenzyl)-3-methyl-4-oxo-4-(4-phenyl-6,7-dihydrothieno[3,2-c]pyridin-5(4H)-yl)butanamid; |
| **102** | N-(3,5-Dimethoxyphenethyl)-3-methyl-4-oxo-4-(4-phenyl-6,7-dihydrothieno[3,2-c]pyridin-5(4H)-yl) butanamid; |
| **103** | N-(3,4-Difluorbenzyl)-4-oxo-4-(4-phenyl-6,7-dihydrothieno[3,2-c]pyridin-5(4H)-yl)butanamid; |
| **104** | N-(2,4-Dichlorphenethyl)-4-oxo-4-(4-phenyl-6,7-dihydrothieno[3,2-c]pyridin-5(4H)-yl)butanamid; |
| **105** | N-(2-(1H-1,2,4-Triazol-1-yl)ethyl)-4-oxo-4-(4-phenyl-6,7-dihydrothieno[3,2-c]pyridin-5(4H)-yl)butanamid; |
| **106** | 4-(4-(4-Fluorphenyl)-6,7-dihydrothieno[3,2-c]pyridin-5(4H)-yl)-N-(2-methylbenzyl)-4-oxobutanamid; |
| **107** | N-(4-Fluorphenethyl)-4-(4-(4-fluorphenyl)-6,7-dihydrothieno[3,2-c]pyridin-5(4H)-yl)-4-oxobutanamid; |
| **108** | 4-(4-(4-Fluorphenyl)-6,7-dihydrothieno[3,2-c]pyridin-5(4H)-yl)-N-((5-methylisoxazol-3-yl)methyl)-4-oxobutanamid; |
| **109** | N-(2-Chlorphenethyl)-4-(4-(4-fluorphenyl)-6,7-dihydrothieno[3,2-c]pyridin-5(4H)-yl)-4-oxobutanamid; |
| **110** | 4-(4-(4-Chlorphenyl)-6,7-dihydrothieno[3,2-c]pyridin-5(4H)-yl)-N-(2-cyclohexenylethyl)-4-oxobutanamid; |

(fortgesetzt)

| | |
|---|---|
| **111** | 4-(4-(4-Chlorphenyl)-6,7-dihydrothieno[3,2-c]pyridin-5(4H)-yl)-N-(3,5-dimethoxybenzyl)-4-oxobutanamid; |
| **112** | 4-(4-(4-Chlorphenyl)-6,7-dihydrothieno[3,2-c]pyridin-5(4H)-yl)-N-(3,4-dichlorphenethyl)-4-oxobutanamid; |
| **113** | 4-(4-(4-Chlorphenyl)-6,7-dihydrothieno[3,2-c]pyridin-5(4H)-yl)-N-(3-fluorphenethyl)-4-oxobutanamid; |
| **114** | 1-(3-Phenylpiperidin-1-yl)-4-(4-p-tolyl-6,7-dihydrothieno[3,2-c]pyridin-5(4H)-yl)butan-1,4-dion; |
| **115** | 1-(4-Benzylpiperazin-1-yl)-4-(4-p-tolyl-6,7-dihydrothieno[3,2-c]pyridin-5(4H)-yl)butan-1,4-dion; |
| **116** | 1-(4-(4-Methoxyphenyl)piperazin-1-yl)-4-(4-p-tolyl-6,7-dihydrothieno[3,2-c]pyridin-5(4H)-yl)butan-1,4-dion; |
| **117** | 1-(2-Benzylpiperidin-1-yl)-4-(4-m-tolyl-6,7-dihydrothieno[3,2-c]pyridin-5(4H)-yl)butan-1,4-dion; |
| **118** | 1-(4-(2-Fluorphenyl)piperazin-1-yl)-4-(4-m-tolyl-6,7-dihydrothieno[3,2-c]pyridin-5(4H)-yl)butan-1,4-dion; |
| **119** | 1-(4-(Cyclohexylmethyl)piperazin-1-yl)-4-(4-m-tolyl-6,7-dihydrothieno[3,2-c]pyridin-5(4H)-yl)butan-1,4-dion; |
| **120** | N-(3-(1H-Imidazol-1-yl)propyl)-4-(4-(3-fluorphenyl)-6,7-dihydrothieno[3,2-c]pyridin-5(4H)-yl)-4-oxobutanamid; |
| **121** | 1-(4-(3-Fluorphenyl)-6,7-dihydrothieno[3,2-c]pyridin-5(4H)-yl)-4-(3-phenylpyrrolidin-1-yl)butan-1,4-dion; |
| **122** | 1-(4-(3-Fluorphenyl)-6,7-dihydrothieno[3,2-c]pyridin-5(4H)-yl)-4-(4-(furan-2-carbonyl)piperazin-1-yl) butan-1,4-dion; |
| **123** | N-(3-(3,4-Dihydroquinolin-1(2H)-yl)propyl)-3-methyl-4-oxo-4-(4-phenyl-6,7-dihydrothieno[3,2-c]pyridin-5 (4H)-yl)butanamid; |
| **124** | 2-Methyl-1-(4-phenyl-6,7-dihydrothieno[3,2-c]pyridin-5(4H)-yl)-4-(piperidin-1-yl)butan-1,4-dion; |
| **125** | 4-(4-(2-Methoxyphenyl)piperazin-1-yl)-2-methyl-1-(4-phenyl-6,7-dihydrothieno[3,2-c]pyridin-5(4H)-yl) butan-1,4-dion; |
| **126** | 4-Oxo-4-(4-phenyl-6,7-dihydrothieno[3,2-c]pyridin-5(4H)-yl)-N-(2-(pyrazin-2-yl)ethyl)butanamid; |
| **127** | N,N-Diethyl-4-oxo-4-(4-phenyl-6,7-dihydrothieno[3,2-c]pyridin-5(4H)-yl)butanamid; |
| **128** | 1-(4-Phenyl-6,7-dihydrothieno[3,2-c]pyridin-5(4H)-yl)-4-(4-(pyridin-2-yl)piperazin-1-yl)butan-1,4-dion; |
| **129** | 1-(4-(4-Fluorphenyl)-6,7-dihydrothieno[3,2-c]pyridin-5(4H)-yl)-4-(4-(3-methoxyphenyl)piperazin-1-yl) butan-1,4-dion; |
| **130** | 1-(4-Isopropylpiperazin-1-yl)-4-(4-p-tolyl-6,7-dihydrothieno[3,2-c]pyridin-5(4H)-yl)butan-1,4-dion; |
| **131** | 1-(4-p-Tolyl-6,7-dihydrothieno[3,2-c]pyridin-5(4H)-yl)-4-(4-(3-(trifluormethyl)phenyl)piperazin-1-yl)butan-1,4-dion; |
| **132** | 1-(4-Phenylpiperazin-1-yl)-4-(4-m-tolyl-6,7-dihydrothieno[3,2-c]pyridin-5(4H)-yl)butan-1,4-dion; |
| **133** | 1-(3,5-Dimethylpiperidin-1-yl)-4-(4-m-tolyl-6,7-dihydrothieno[3,2-c]pyridin-5(4H)-yl)butan-1,4-dion; |
| **134** | 1-(5,6-Dihydropyridin-1 (2H)-yl)-4-(4-(3-fluorphenyl)-6,7-dihydrothieno[3,2-c]pyridin-5(4H)-yl)butan-1,4-dion; |
| **135** | 1-(4-(3-Fluorphenyl)-6,7-dihydrothieno[3,2-c]pyridin-5(4H)-yl)-4-(4-(pyrimidin-2-yl)piperazin-1-yl)butan-1,4-dion; |
| **136** | 1-(4-(3-Fluorphenyl)-6,7-dihydrothieno[3,2-c]pyridin-5(4H)-yl)-4-(3-methylpiperidin-1-yl)butan-1,4-dion; |
| **137** | N-Ethyl-4-(4-(3-fluorphenyl)-6,7-dihydrothieno[3,2-c]pyridin-5(4H)-yl)-4-oxo-N-(pyridin-4-ylmethyl) butanamid; |
| **138** | 4-(4-Acetylpiperazin-1-yl)-2-methyl-1-(4-phenyl-6,7-dihydrothieno[3,2-c]pyridin-5(4H)-yl)butan-1,4-dion; |
| **139** | 4-(2,6-Dimethylmorpholino)-2-methyl-1-(4-phenyl-6,7-dihydrothieno[3,2-c]pyridin-5(4H)-yl)butan-1,4-dion; |
| **140** | 4-(2,5-Dihydro-1H-pyrrol-1-yl)-2-methyl-1-(4-phenyl-6,7-dihydrothieno[3,2-c]pyridin-5(4H)-yl)butan-1,4-dion; |
| **141** | N-(2-Methoxyphenethyl)-4-oxo-4-(4-phenyl-6,7-dihydrothieno[3,2-c]pyridin-5(4H)-yl)butanamid; |
| **142** | 4-(4-(4-Fluorphenyl)-6,7-dihydrothieno[3,2-c]pyridin-5(4H)-yl)-N-isopentyl-4-oxobutanamid; |
| **143** | N-(2,4-Dimethoxybenzyl)-4-(4-(4-fluorphenyl)-6,7-dihydrothieno[3,2-c]pyridin-5(4H)-yl)-4-oxobutanamid; |
| **144** | 4-(4-(4-Chlorphenyl)-6,7-dihydrothieno[3,2-c]pyridin-5(4H)-yl)-N-((5-methylfuran-2-yl)methyl)-4-oxobutanamid; |
| **145** | 4-(4-(4-Chlorphenyl)-6,7-dihydrothieno[3,2-c]pyridin-5(4H)-yl)-4-oxo-N-pentylbutanamid; |
| **146** | 1-(4-Benzylpiperidin-1-yl)-4-(4-(4-chlorphenyl)-6,7-dihydrothieno[3,2-c]pyridin-5(4H)-yl)butan-1,4-dion; |
| **147** | 1-(4-(4-Chlorphenyl)-6,7-dihydrothieno[3,2-c]pyridin-5(4H)-yl)-4-(4-methylpiperidin-1-yl)butan-1,4-dion; |
| **148** | 1-(Azetidin-1-yl)-4-(4-(4-chlorphenyl)-6,7-dihydrothieno[3,2-c]pyridin-5(4H)-yl)butan-1,4-dion; |

(fortgesetzt)

**149** 1-(3,4-Dihydroisoquinolin-2(1H)-yl)-2-methyl-4-(4-phenyl-6,7-dihydrothieno[3,2-c]pyridin-5(4H)-yl)butan-1,4-dion;

**150** 1-(4-(Benzo[d][1,3]dioxol-5-ylmethyl)piperazin-1-yl)-2-methyl-4-(4-phenyl-6,7-dihydrothieno[3,2-c]pyridin-5(4H)-yl)butan-1,4-dion;

**151** N-Benzyl-N-ethyl-4-oxo-4-(4-p-tolyl-6,7-dihydrothieno[3,2-c]pyridin-5(4H)-yl)butanamid;

**152** N-Methyl-4-oxo-N-phenethyl-4-(4-p-tolyl-6,7-dihydrothieno[3,2-c]pyridin-5(4H)-yl)butanamid;

**153** Ethyl 1-(4-oxo-4-(4-p-tolyl-6,7-dihydrothieno[3,2-c]pyridin-5(4H)-yl)butanoyl)piperidin-4-carboxylat;

**154** (E)-1-(4-(3-Phenylprop-2-enyl)-piperazin-1-yl)-4-(4-p-tolyl-6,7-dihydrothieno[3,2-c]pyridin-5(4H)-yl)butan-1,4-dion;

**155** 1-(2-(Thiazol-2-yl)pyrrolidin-1-yl)-4-(4-p-tolyl-6,7-dihydrothieno[3,2-c]pyridin-5(4H)-yl)butan-1,4-dion;

**156** N-Cyclohexyl-N-ethyl-4-oxo-4-(4-m-tolyl-6,7-dihydrothieno[3,2-c]pyridin-5(4H)-yl)butanamid;

**157** N-Ethyl-N-isopropyl-4-oxo-4-(4-m-tolyl-6,7-dihydrothieno[3,2-c]pyridin-5(4H)-yl)butanamid;

**158** 1-(Pyrrolidin-1-yl)-4-(4-m-tolyl-6,7-dihydrothieno[3,2-c]pyridin-5(4H)-yl)butan-1,4-dion;

**159** 1-(4-(Pyridin-4-yl)piperazin-1-yl)-4-(4-m-tolyl-6,7-dihydrothieno[3,2-c]pyridin-5(4H)-yl)butan-1,4-dion;

**160** 1-(2-(Pyridin-2-ylmethyl)pyrrolidin-1-yl)-4-(4-m-tolyl-6,7-dihydrothieno[3,2-c]pyridin-5(4H)-yl)butan-1,4-dion;

**161** Ethyl 1-(4-(4-(4-fluorphenyl)-6,7-dihydrothieno[3,2-c]pyridin-5(4H)-yl)-4-oxobutanoyl)piperidin-3-carboxylat;

**162** 1-(4-(4-Fluorphenyl)-6,7-dihydrothieno[3,2-c]pyridin-5(4H)-yl)-4-(4-(4-(trifluormethyl)phenyl)piperazin-1-yl)butan-1,4-dion;

**163** 1-(2-(5-Brompyridin-3-yl)pyrrolidin-1-yl)-4-(4-(4-fluorphenyl)-6,7-dihydrothieno[3,2-c]pyridin-5(4H)-yl) butan-1,4-dion;

**164** 1-(4-Ethylpiperazin-1-yl)-4-(4-phenyl-6,7-dihydrothieno[3,2-c]pyridin-5(4H)-yl)butan-1,4-dion;

**165** 1-(2-Ethylpiperidin-1-yl)-4-(4-phenyl-6,7-dihydrothieno[3,2-c]pyridin-5(4H)-yl)butan-1,4-dion;

**166** N,N-Dibenzyl-4-oxo-4-(4-phenyl-6,7-dihydrothieno[3,2-c]pyridin-5(4H)-yl)butanamid;

**167** N,N-Diisobutyl-4-oxo-4-(4-phenyl-6,7-dihydrothieno[3,2-c]pyridin-5(4H)-yl)butanamid;

**168** 1-(3,4-Dihydroquinolin-1(2H)-yl)-4-(4-(4-fluorphenyl)-6,7-dihydrothieno[3,2-c]pyridin-5(4H)-yl)butan-1,4-dion;

**169** 1-(4-(3,4-Dichlorbenzyl)piperazin-1-yl)-4-(4-p-tolyl-6,7-dihydrothieno[3,2-c]pyridin-5(4H)-yl)butan-1,4-dion;

**170** 1-(4-p-Tolyl-6,7-dihydrothieno[3,2-c]pyridin-5(4H)-yl)-4-(4-(2,4,6-trimethylbenzyl)piperazin-1-yl)butan-1,4-dion;

**171** 1-(4-(4-Brombenzyl)piperazin-1-yl)-4-(4-m-tolyl-6,7-dihydrothieno[3,2-c]pyridin-5(4H)-yl)butan-1,4-dion;

**172** 1-(4-(4-Chlorbenzyl)piperazin-1-yl)-4-(4-m-tolyl-6,7-dihydrothieno[3,2-c]pyridin-5(4H)-yl)butan-1,4-dion;

**173** 1-(2-((4,6-Dimethylpyridin-2-yl)methyl)pyrrolidin-1-yl)-4-(4-(3-fluorphenyl)-6,7-dihydrothieno[3,2-c]pyridin-5(4H)-yl)butan-1,4-dion;

**174** 1-(4-(3-Fluorphenyl)-6,7-dihydrothieno[3,2-c]pyridin-5(4H)-yl)-4-(2-((6-methylpyridin-2-yl)methyl) pyrrolidin-1-yl)butan-1,4-dion;

**175** 1-(2-((5-Ethylpyridin-2-yl)methyl)pyrrolidin-1-yl)-4-(4-(3-fluorphenyl)-6,7-dihydrothieno[3,2-c]pyridin-5 (4H)-yl)butan-1,4-dion;

**176** 1-(4-(3-Fluorphenyl)-6,7-dihydrothieno[3,2-c]pyridin-5(4H)-yl)-4-(2-(6-methoxypyridin-3-yl)piperidin-1-yl) butan-1,4-dion;

**177** 4-(4-(3-Fluorphenyl)-6,7-dihydrothieno[3,2-c]pyridin-5(4H)-yl)-N-methyl-4-oxo-N-(pyridin-3-ylmethyl) butanamid;

**178** N-(2,2-Diphenylethyl)-3-methyl-4-oxo-4-(4-phenyl-6,7-dihydrothieno[3,2-c]pyridin-5(4H)-yl)butanamid;

**179** N-(Cyclopropylmethyl)-4-oxo-4-(4-phenyl-6,7-dihydrothieno[3,2-c]pyridin-5(4H)-yl)butanamid;

**180** 1-(4-(3-Fluorphenyl)-6,7-dihydrothieno[3,2-c]pyridin-5(4H)-yl)-4-(4-(4-methylbenzyl)piperazin-1-yl)butan-1,4-dion;

**181** 2-Methyl-1-(4-phenyl-6,7-dihydrothieno[3,2-c]pyridin-5(4H)-yl)-4-(2-(pyridin-2-ylmethyl)piperidin-1-yl) butan-1,4-dion;

**182** 4-(4-(3,5-Dichlorpyridin-4-yl)piperazin-1-yl)-2-methyl-1-(4-phenyl-6,7-dihydrothieno[3,2-c]pyridin-5 (4H)-yl)butan-1,4-dion;

(fortgesetzt)

| | |
|---|---|
| **183** | 1-(2-((4,6-Dimethylpyridin-2-yl)methyl)piperidin-1-yl)-4-(4-phenyl-6,7-dihydrothieno[3,2-c]pyridin-5(4H)-yl)butan-1,4-dion; |
| **184** | N-(4-Fluorbenzyl)-N-methyl-4-oxo-4-(4-phenyl-6,7-dihydrothieno[3,2-c]pyridin-5(4H)-yl)butanamid; |
| **185** | 1-(4-(3-Fluorphenyl)-6,7-dihydrothieno[3,2-c]pyridin-5(4H)-yl)-4-(2-(pyridin-2-yl)pyrrolidin-1-yl)butan-1,4-dion; |
| **186** | 4-(4-(4-Methoxybenzyl)piperazin-1-yl)-2-methyl-1-(4-phenyl-6,7-dihydrothieno[3,2-c]pyridin-5(4H)-yl)butan-1,4-dion; |
| **187** | 2-Methyl-1-(4-phenyl-6,7-dihydrothieno[3,2-c]pyridin-5(4H)-yl)-4-(3-(pyridin-3-yl)pyrrolidin-1-yl)butan-1,4-dion; |
| **188** | 1-(4-(2-Fluorbenzyl)piperazin-1-yl)-4-(4-phenyl-6,7-dihydrothieno[3,2-c]pyridin-5(4H)-yl)butan-1,4-dion; |
| **189** | N-Methyl-4-oxo-4-(4-phenyl-6,7-dihydrothieno[3,2-c]pyridin-5(4H)-yl)-N-(2-(pyridin-4-yl)ethyl)butanamid; |
| **190** | N-Butyl-N-ethyl-3-methyl-4-oxo-4-(4-phenyl-6,7-dihydrothieno[3,2-c]pyridin-5(4H)-yl)butanamid; |
| **191** | N,3-Dimethyl-4-oxo-4-(4-phenyl-6,7-dihydrothieno[3,2-c]pyridin-5(4H)-yl)-N-propylbutanamid; |
| **192** | 4-((S)-2-(Methoxymethyl)pyrrolidin-1-yl)-2-methyl-1-(4-phenyl-6,7-dihydrothieno[3,2-c]pyridin-5(4H)-yl)butan-1,4-dion; |
| **193** | 4-(4-(5-Chlor-2-methylphenyl)piperazin-1-yl)-2-methyl-1-(4-phenyl-6,7-dihydrothieno[3,2-c]pyridin-5(4H)-yl)butan-1,4-dion; |
| **194** | N-(Furan-2-ylmethyl)-N-methyl-4-oxo-4-(4-phenyl-6,7-dihydrothieno[3,2-c]pyridin-5(4H)-yl)butanamid; |
| **195** | 1-((4aR,8aS)-Octahydroisoquinolin-2(1H)-yl)-4-(4-phenyl-6,7-dihydrothieno-[3,2-c]pyridin-5(4H)-yl)butan-1,4-dion; |
| **196** | 1-(4-Phenyl-6,7-dihydrothieno[3,2-c]pyridin-5(4H)-yl)-4-thiomorpholinobutan-1,4-dion; |
| **197** | 1-(4-Phenyl-6,7-dihydrothieno[3,2-c]pyridin-5(4H)-yl)-4-(2-(pyridin-3-yl)pyrrolidin-1-yl)butan-1,4-dion; |
| **198** | N-Benzyl-4-(4-(4-fluorphenyl)-6,7-dihydrothieno[3,2-c]pyridin-5(4H)-yl)-N-isopropyl-4-oxobutanamid; |
| **199** | N-Benzyl-4-(4-(4-fluorphenyl)-6,7-dihydrothieno[3,2-c]pyridin-5(4H)-yl)-N-methyl-4-oxobutanamid; |
| **200** | N-(3,4-Dimethoxyphenethyl)-4-(4-(4-fluorphenyl)-6,7-dihydrothieno[3,2-c]pyridin-5(4H)-yl)-N-methyl-4-oxobutanamid; |
| **201** | 1-(4-(4-Fluorphenyl)-6,7-dihydrothieno[3,2-c]pyridin-5(4H)-yl)-4-(4-methyl-2-phenylpiperazin-1-yl)butan-1,4-dion; |
| **202** | 1-(4-(4-Chlorphenyl)-6,7-dihydrothieno[3,2-c]pyridin-5(4H)-yl)-4-(4-phenylpiperidin-1-yl)butan-1,4-dion; |
| **203** | 4-(4-(4-Chlorphenyl)-6,7-dihydrothieno[3,2-c]pyridin-5(4H)-yl)-4-oxo-N-(2-(pyridin-2-yl)ethyl)butanamid; |
| **204** | N-Benzyl-2-methyl-4-oxo-N-phenethyl-4-(4-phenyl-6,7-dihydrothieno[3,2-c]pyridin-5(4H)-yl)butanamid; |
| **205** | 1-(4-(4-Fluorphenyl)-6,7-dihydrothieno[3,2-c]pyridin-5(4H)-yl)-4-(2-((6-methylpyridin-2-yl)methyl)piperidin-1-yl)butan-1,4-dion; |
| **206** | 1-(4-(4-Fluorphenyl)-6,7-dihydrothieno[3,2-c]pyridin-5(4H)-yl)-4-(4-(3-methylbenzyl)piperazin-1-yl)butan-1,4-dion; |
| **207** | 1-(4-(4-Chlorphenyl)-6,7-dihydrothieno[3,2-c]pyridin-5(4H)-yl)-4-(2-(pyridin-4-ylmethyl)piperidin-1-yl)butan-1,4-dion; |
| **208** | 1-(4-(4-Chlorphenyl)-6,7-dihydrothieno[3,2-c]pyridin-5(4H)-yl)-4-(2-((5-ethylpyridin-2-yl)methyl)piperidin-1-yl)butan-1,4-dion; |
| **209** | 1-(4-(4-Chlorphenyl)-6,7-dihydrothieno[3,2-c]pyridin-5(4H)-yl)-4-(2-((3-methylpyridin-2-yl)methyl)piperidin-1-yl)butan-1,4-dion; |
| **210** | 1-(4-(4-Chlorphenyl)-6,7-dihydrothieno[3,2-c]pyridin-5(4H)-yl)-4-(4-(3-chlorphenyl)piperazin-1-yl)butan-1,4-dion; |
| **211** | 1-(4-(4-Chlorphenyl)-6,7-dihydrothieno[3,2-c]pyridin-5(4H)-yl)-4-(4-(2,3-dimethylphenyl)piperazin-1-yl)butan-1,4-dion; |
| **212** | 1-(4-(4-Chlorphenyl)-6,7-dihydrothieno[3,2-c]pyridin-5(4H)-yl)-4-(4-(3,4-dimethylphenyl)piperazin-1-yl)butan-1,4-dion; |
| **213** | 1-(4-(4-Chlorphenyl)-6,7-dihydrothieno[3,2-c]pyridin-5(4H)-yl)-4-(4-(3,4-dichlorphenyl)piperazin-1-yl)butan-1,4-dion; |
| **214** | 1-(4-(4-tert-Butylbenzyl)piperazin-1-yl)-4-(4-(4-chlorphenyl)-6,7-dihydrothieno[3,2-c]pyridin-5(4H)-yl)butan-1,4-dion; |

(fortgesetzt)

| | |
|---|---|
| **215** | 1-[4-(3,5-Dichlor-4-pyridyl)-1-piperazinyl]-2-methyl-4-(4-phenyl-6,7-dihydro-4H-thieno[3,2-c]pyridin-5-yl) butan-1,4-dion; |
| **216** | 1-[4-[(4-Methoxyphenyl)methyl]-1-piperazinyl]-4-[4-(p-tolyl)-6,7-dihydro-4H-thieno[3,2-c]pyridin-5-yl] butan-1,4-dion; |
| **217** | 1-[4-[(2-Fluorphenyl)methyl]-1-piperazinyl]-4-[4-(p-tolyl)-6,7-dihydro-4H-thieno[3,2-c]pyridin-5-yl]butan-1,4-dion; |
| **218** | 1-(4-Methyl-2-phenyl-1-piperazinyl)-4-[4-(p-tolyl)-6,7-dihydro-4H-thieno[3,2-c]pyridin-5-yl]butan-1,4-dion; |
| **219** | 1-(4-Phenyl-1-piperidinyl)-4-[4-(p-tolyl)-6,7-dihydro-4H-thieno[3,2-c]pyridin-5-yl]butan-1,4-dion; |
| **220** | 1-[4-(p-Tolyl)-6,7-dihydro-4H-thieno[3,2-c]pyridin-5-yl]-4-[2-(2-pyridyl)-1-pyrrolidinyl]butan-1,4-dion; |
| **221** | 1-[4-(p-Tolyl)-6,7-dihydro-4H-thieno[3,2-c]pyridin-5-yl]-4-[3-(3-pyridyl)-1-pyrrolidinyl]butan-1,4-dion; |
| **222** | 4-Oxo-4-[4-(p-tolyl)-6,7-dihydro-4H-thieno[3,2-c]pyridin-5-yl]-N-[2-(2-pyridyl)ethyl]butanamid; |
| **223** | 1-[4-[(4-Methoxyphenyl)methyl]-1-piperazinyl]-4-[4-(m-tolyl)-6,7-dihydro-4H-thieno[3,2-c]pyridin-5-yl] butan-1,4-dion; |
| **224** | 1-[4-[(2-Fluorphenyl)methyl]-1-piperazinyl]-4-[4-(m-tolyl)-6,7-dihydro-4H-thieno[3,2-c]pyridin-5-yl]butan-1,4-dion; |
| **225** | 1-(4-Methyl-2-phenyl-1-piperazinyl)-4-[4-(m-tolyl)-6,7-dihydro-4H-thieno[3,2-c]pyridin-5-yl]butan-1,4-dion; |
| **226** | 1-[4-(m-Tolyl)-6,7-dihydro-4H-thieno[3,2-c]pyridin-5-yl]-4-(4-phenyl-1-piperidinyl)butan-1,4-dion; |
| **227** | 1-[4-(m-Tolyl)-6,7-dihydro-4H-thieno[3,2-c]pyridin-5-yl]-4-[2-(2-pyridyl)-1-pyrrolidinyl]butan-1,4-dion; |
| **228** | 1-[4-(m-Tolyl)-6,7-dihydro-4H-thieno[3,2-c]pyridin-5-yl]-4-[3-(3-pyridyl)-1-pyrrolidinyl]butan-1,4-dion; |
| **229** | N-Methyl-4-[4-(m-tolyl)-6,7-dihydro-4H-thieno[3,2-c]pyridin-5-yl]-4-oxo-N-[2-(4-pyridyl)ethyl]butanamid; |
| **230** | 1-[4-(3-Fluorphenyl)-6,7-dihydro-4H-thieno[3,2-c]pyridin-5-yl]-4-[4-[(4-methoxyphenyl)methyl]-1-piperazinyl]butan-1,4-dion; |
| **231** | 1-[4-(3-Fluorphenyl)-6,7-dihydro-4H-thieno[3,2-c]pyridin-5-yl]-4-[4-[(2-fluorphenyl)methyl]-1-piperazinyl] butan-1,4-dion; |
| **232** | 4-[4-(m-Tolyl)-6,7-dihydro-4H-thieno[3,2-c]pyridin-5-yl]-4-oxo-N-(p-tolyl)butanamid; |
| **233** | N-(2,4-Dimethylphenyl)-4-[4-(m-tolyl)-6,7-dihydro-4H-thieno[3,2-c]pyridin-5-yl]-4-oxobutanamid; |
| **234** | 4-[4-(3-Fluorphenyl)-6,7-dihydro-4H-thieno[3,2-c]pyridin-5-yl]-N-(1-methyl-6-indazolyl)-4-oxobutanamid; |
| **235** | 4-[4-(3-Fluorphenyl)-6,7-dihydro-4H-thieno[3,2-c]pyridin-5-yl]-4-oxo-N-(p-tolyl)butanamid; |
| **236** | 4-[4-(3-Fluorphenyl)-6,7-dihydro-4H-thieno[3,2-c]pyridin-5-yl]-4-oxo-N-phenylbutanamid; |
| **237** | N-(2-Chlorphenyl)-4-[4-(3-fluorphenyl)-6,7-dihydro-4H-thieno[3,2-c]pyridin-5-yl]-4-oxobutanamid; |
| **238** | N-(2,4-Dimethylphenyl)-4-[4-(3-fluorphenyl)-6,7-dihydro-4H-thieno[3,2-c]pyridin-5-yl]-4-oxobutanamid; |
| **239** | 4-[4-(4-Chlorphenyl)-6,7-dihydro-4H-thieno[3,2-c]pyridin-5-yl]-N-(1-methyl-6-indazolyl)-4-oxobutanamid; |
| **240** | 4-[4-(4-Chlorphenyl)-6,7-dihydro-4H-thieno[3,2-c]pyridin-5-yl]-4-oxo-N-(p-tolyl)butanamid; |
| **241** | 4-[4-(4-Chlorphenyl)-6,7-dihydro-4H-thieno[3,2-c]pyridin-5-yl]-N-(4-methoxyphenyl)-4-oxobutanamid; |
| **242** | 4-[4-(4-Chlorphenyl)-6,7-dihydro-4H-thieno[3,2-c]pyridin-5-yl]-4-oxo-N-phenylbutanamid; |
| **243** | 4-[4-(4-Chlorphenyl)-6,7-dihydro-4H-thieno[3,2-c]pyridin-5-yl]-N-(2,4-dimethylphenyl)-4-oxobutanamid; |
| **244** | 4-[4-(2,6-Dimethylphenyl)-6,7-dihydro-4H-thieno[3,2-c]pyridin-5-yl]-4-oxo-N-[[3-(trifluormethyl)phenyl] methyl]butanamid; |
| **245** | N-(2-Cyclohexylethyl)-4-oxo-4-(4-phenyl-6,7-dihydro-4H-thieno[3,2-c]pyridin-5-yl)butanamid; |
| **246** | N-(3,3-Dimethylbutyl)-4-oxo-4-(4-phenyl-6,7-dihydro-4H-thieno[3,2-c]pyridin-5-yl)butanamid; |
| **247** | N-(Cyclohexylmethyl)-4-oxo-4-(4-phenyl-6,7-dihydro-4H-thieno[3,2-c]pyridin-5-yl)butanamid; |
| **248** | N-[[3-Methyl-5-(trifluormethoxy)phenyl]methyl]-4-oxo-4-(4-phenyl-6,7-dihydro-4H-thieno[3,2-c]pyridin-5-yl)butanamid; |
| **249** | N-[[4-Methyl-3-(trifluormethyl)phenyl]methyl]-4-oxo-4-(4-phenyl-6,7-dihydro-4H-thieno[3,2-c]pyridin-5-yl) butanamid; |
| **250** | N-[[4-Fluor-3-(trifluormethyl)phenyl]methyl]-4-oxo-4-(4-phenyl-6,7-dihydro-4H-thieno[3,2-c]pyridin-5-yl) butanamid; |
| **251** | 4-[4-(2-Ethylphenyl)-6,7-dihydro-4H-thieno[3,2-c]pyridin-5-yl]-4-oxo-N-[[3-(trifluormethyl)phenyl]methyl] butanamid; |
| **252** | 4-[4-(2-Isopropylphenyl)-6,7-dihydro-4H-thieno[3,2-c]pyridin-5-yl]-4-oxo-N-[[3-(trifluormethyl)phenyl] methyl]butanamid; |
| **253** | N-(3-Cyclohexylpropyl)-4-oxo-4-(4-phenyl-6,7-dihydro-4H-thieno[3,2-c]pyridin-5-yl)butanamid; |

(fortgesetzt)

| | |
|---|---|
| **254** | 4-[4-(3-Methyl-2-thienyl)-6,7-dihydro-4H-thieno[3,2-c]pyridin-5-yl]-4-oxo-N-[[3-(trifluormethyl)phenyl]methyl]butanamid; |
| **255** | (1S,2S)-2-[Oxo-(4-phenyl-6,7-dihydro-4H-thieno[3,2-c]pyridin-5-yl)methyl]-N-[[3-(trifluormethyl)phenyl]methyl]-1-cyclopropancarboxamid; |
| **256** | 4-[4-(o-Tolyl)-6,7-dihydro-4H-thieno[3,2-c]pyridin-5-yl]-4-oxo-N-[[2-(trifluormethyl)phenyl]methyl]butanamid; |
| **257** | 4-[4-(o-Tolyl)-6,7-dihydro-4H-thieno[3,2-c]pyridin-5-yl]-4-oxo-N-[[4-(trifluormethyl)phenyl]methyl]butanamid; |
| **258** | 4-(7-Butyl-5,7-dihydro-4H-thieno[2,3-c]pyridin-6-yl)-4-oxo-N-[[3-(trifluormethyl)phenyl]methyl]butanamid; |
| **259** | 4-[4-(Cyclohexylmethyl)-6,7-dihydro-4H-thieno[3,2-c]pyridin-5-yl]-4-oxo-N-[[3-(trifluormethyl)phenyl]methyl]butanamid; |
| **260** | 4-(4-Cyclopropyl-6,7-dihydro-4H-thieno[3,2-c]pyridin-5-yl)-4-oxo-N-[[3-(trifluormethyl)phenyl]methyl]butanamid; |
| **261** | 4-Oxo-4-(4-phenthyl-6,7-dihydro-4H-thieno[3,2-c]pyridin-5-yl)-N-[[3-(trifluormethyl)phenyl]methyl]butanamid; |
| **262** | 4-[4-(4-Fluor-2-methylphenyl)-6,7-dihydro-4H-thieno[3,2-c]pyridin-5-yl]-4-oxo-N-[[3-(trifluormethyl)phenyl]methyl]butanamid; |
| **263** | 4-(4-Cyclopentyl-6,7-dihydro-4H-thieno[3,2-c]pyridin-5-yl)-4-oxo-N-[[3-(trifluormethyl)phenyl]methyl]butanamid; |
| **264** | N-[[2-Methyl-3-(trifluormethyl)phenyl]methyl]-4-oxo-4-(4-phenyl-6,7-dihydro-4H-thieno[3,2-c]pyridin-5-yl)butanamid; |
| **265** | 4-Oxo-4-[4-(3-phenylpropyl)-6,7-dihydro-4H-thieno[3,2-c]pyridin-5-yl]-N-[[3-(trifluormethyl)phenyl]methyl]butanamid; |
| **266** | N-[[2-Methyl-5-(trifluormethyl)phenyl]methyl]-4-oxo-4-(4-phenyl-6,7-dihydro-4H-thieno[3,2-c]pyridin-5-yl)butanamid; |
| **267** | N-Cyclohexyl-4-oxo-4-(4-phenyl-6,7-dihydro-4H-thieno[3,2-c]pyridin-5-yl)butanamid; |
| **268** | N-(2,2-Dimethylpropyl)-4-oxo-4-(4-phenyl-6,7-dihydro-4H-thieno[3,2-c]pyridin-5-yl)butanamid; |
| **269** | 1-(4-Phenyl-6,7-dihydro-4H-thieno[3,2-c]pyridin-5-yl)-4-[7-(trifluormethyl)-3,4-dihydro-1H-isoquinolin-2-yl]butan-1,4-dion; |
| **270** | 4-Oxo-N-pentyl-4-(4-phenyl-6,7-dihydro-4H-thieno[3,2-c]pyridin-5-yl)butanamid; |
| **271** | 1-(4-Phenyl-6,7-dihydro-4H-thieno[3,2-c]pyridin-5-yl)-4-[5-(trifluormethyl)-3,4-dihydro-1H-isoquinolin-2-yl]butan-1,4-dion; |
| **272** | 4-Oxo-4-(4-phenyl-6,7-dihydro-4H-thieno[3,2-c]pyridin-5-yl)-N-[5-(trifluormethyl)-1-tetralinyl]butanamid; |
| **273** | 4-Oxo-4-(4-phenyl-6,7-dihydro-4H-thieno[3,2-c]pyridin-5-yl)-N-[7-(tritluormethyl)-1-tetralinyl]butanamid; |
| **274** | 4-[7-(o-Tolyl)-5,7-dihydro-4H-thieno[2,3-c]pyridin-6-yl]-4-oxo-N-[[3-(trifluormethyl)phenyl]methyl]butanamid; |
| **275** | 4-(7-Cyclohexyl-5,7-dihydro-4H-thieno[2,3-c]pyridin-6-yl)-4-oxo-N-[[3-(trifluormethyl)phenyl]methyl]butanamid; |

Die erfindungsgemäßen substituierten Tetrahydrothienopyridine sowie jeweils die entsprechenden Säuren, Basen, Salze und Solvate oder eine Verbindung ausgewählt aus der Gruppe bestehend aus

- 1-Morpholino-4-(4-(thiophen-2-yl)-6,7-dihydrothieno[3,2-c]pyridin-5(4H)-yl)butan-1,4-dion,
- 1-(4-Acetylpiperazin-1-yl)-4-(4-(thiophen-2-yl)-6,7-dihydrothieno[3,2-c]pyridin-5(4H)-yl)butan-1,4-dion und
- 1-(3-Phenyl-4,5-dihydropyrazol-1-yl)-4-(4-(thiophen-2-yl)-6,7-dihydrothieno[3,2-c]pyridin-5(4H)-yl)butan-1,4-dion;

eignen sich als pharmazeutische Wirkstoffe in Arzneimitteln.

Ein weiterer Gegenstand der Erfindung ist daher ein Arzneimittel enthaltend wenigstens ein erfindungsgemäßes substituiertes Tetrahydrothienopyridin der allgemeinen Formel (1), worin die Reste $R^1$-$R^{12}$ die oben angegebene Bedeutung haben, einschließlich Verbindungen ausgewählt aus der Gruppe bestehend aus

- 1-Morpholino-4-(4-(thiophen-2-yl)-6,7-dihydrothieno[3,2-c]pyridin-5(4H)-yl)butan-1,4-dion,
- 1-(4-Acetylpiperazin-1-yl)-4-(4-(thiophen-2-yl)-6,7-dihydrothieno[3,2-c]pyridin-5(4H)-yl)butan-1,4-dion und
- 1-(3-Phenyl-4,5-dihydropyrazol-1-yl)-4-(4-(thiophen-2-yl)-6,7-dihydrothieno[3,2-c]pyridin-5(4H)-yl)butan-1,4-dion;

sowie einen oder mehrere pharmazeutisch verträgliche Hilfsstoffe.

Besonders bevorzugt sind Arzneimittel, welche eine oder mehrere Verbindungen enthalten ausgewählt aus der folgenden Gruppe:

| | |
|---|---|
| **2** | 4-Oxo-4-(4-phenyl-6,7-dihydrothieno[3,2-c]pyridin-5(4H)-yl)-N-(3-(trifluormethyl)benzyl)butanamid; |
| **3** | 4-(4-(3-Fluorphenyl)-6,7-dihydrothieno[3,2-c]pyridin-5(4H)-yl)-4-oxo-N-(3-(trifluormethyl)benzyl) butanamid; |
| **4** | 4-Oxo-4-(4-m-tolyl-6,7-dihydrothieno[3,2-c]pyridin-5(4H)-yl)-N-(3-(trifluormethyl)benzyl)butanamid; |
| **5** | 4-Oxo-4-(4-p-tolyl-6,7-dihydrothieno[3,2-c]pyridin-5(4H)-yl)-N-(3-(trifluormethyl)benzyl)butanamid; |
| **6** | 4-(4-(2-Fluorphenyl)-6,7-dihydrothieno[3,2-c]pyridin-5(4H)-yl)-4-oxo-N-(3-(trifluormethyl)benzyl) butanamid; |
| **7** | 4-(4-(4-Fluorphenyl)-6,7-dihydrothieno[3,2-c]pyridin-5(4H)-yl)-4-oxo-N-(3-(trifluormethyl)benzyl) butanamid; |
| **8** | 4-Oxo-4-(4-o-tolyl-6,7-dihydrothieno[3,2-c]pyridin-5(4H)-yl)-N-(3-(trifluormethyl)benzyl)butanamid; |
| **9** | 4-Oxo-4-(7-phenyl-4,5-dihydrothieno[2,3-c]pyridin-6(7H)-yl)-N-(3-(trifluormethyl)benzyl)butanamid; |
| **10** | 4-(2-Methyl-4-phenyl-6,7-dihydrothieno[3,2-c]pyridin-5(4H)-yl)-4-oxo-N-(3-(trifluormethyl)benzyl) butanamid; |
| **13** | 4-Oxo-4-(4-phenyl-6,7-dihydrothieno[3,2-c]pyridin-5(4H)-yl)-N-(4-(trifluormethyl)benzyl)butanamid; |
| **17** | 4-Oxo-4-(4-phenyl-6,7-dihydrothieno[3,2-c]pyridin-5(4H)-yl)-N-(2-(trifluormethyl)benzyl)butanamid; |
| **18** | N-(3-Fluorbenzyl)-4-oxo-4-(4-phenyl-6,7-dihydrothieno[3,2-c]pyridin-5(4H)-yl)butanamid; |
| **20** | 4-(3-Methyl-4-phenyl-6,7-dihydrothieno[3,2-c]pyridin-5(4H)-yl)-4-oxo-N-(3-(trifluormethyl)benzyl) butanamid; |
| **21** | 4-(4-Cyclohexyl-6,7-dihydrothieno[3,2-c]pyridin-5(4H)-yl)-4-oxo-N-(3-(trifluormethyl)benzyl)butanamid; |
| **22** | 4-(4-Isopropyl-6,7-dihydrothieno[3,2-c]pyridin-5(4H)-yl)-4-oxo-N-(3-(trifluormethyl)benzyl)butanamid; |
| **23** | 4-(4-Butyl-6,7-dihydrothieno[3,2-c]pyridin-5(4H)-yl)-4-oxo-N-(3-(trifluormethyl)benzyl)butanamid; |
| **27** | 4-Oxo-2-phenyl-4-(4-phenyl-6,7-dihydrothieno[3,2-c]pyridin-5(4H)-yl)-N-(3-(trifluormethyl)benzyl) butanamid; |
| **28** | (R)-2-Methyl-4-oxo-4-(4-phenyl-6,7-dihydrothieno[3,2-c]pyridin-5(4H)-yl)-N-(3-(trifluormethyl)benzyl) butanamid; |
| **29** | 4-Oxo-3-phenyl-4-(4-phenyl-6,7-dihydrothieno[3,2-c]pyridin-5(4H)-yl)-N-(3-(trifluormethyl)benzyl) butanamid; |
| **30** | 4-(6,7-Dihydrothieno[3,2-c]pyridin-5(4H)-yl)-4-oxo-3-phenyl-N-(3-(trifluormethyl)benzyl)butanamid; |
| **31** | rac. (1S,2R)-2-(4-Phenyl-4,5,6,7-tetrahydrothieno[3,2-c]pyridin-5-carbonyl)-N-(3-(trifluormethyl)benzyl) cyclohexancarboxamid; |
| **32** | rac. (1S)-2-(4-Phenyl-4,5,6,7-tetrahydrothieno[3,2-c]pyridin-5-carbonyl)-N-(3-(trifluormethyl)benzyl) cyclohexancarboxamid; |
| **34** | (R)-3-Methyl-4-oxo-4-(4-phenyl-6,7-dihydrothieno[3,2-c]pyridin-5(4H)-yl)-N-(3-(trifluormethyl)benzyl) butanamid; |
| **35** | (-)-4-Oxo-4-(4-phenyl-6,7-dihydrothieno[3,2-c]pyridin-5(4H)-yl)-N-(3-(trifluormethyl)benzyl)butanamid; |
| **36** | (+)-4-Oxo-4-(4-phenyl-6,7-dihydrothieno[3,2-c]pyridin-5(4H)-yl)-N-(3-(trifluormethyl)benzyl)butanamid; |
| **37** | 4-(6,7-Dihydrothieno[3,2-c]pyridin-5(4H)-yl)-4-oxo-2-phenyl-N-(3-(trifluormethyl)benzyl)butanamid; |
| **40** | N-(2-Fluor-3-(trifluormethyl)benzyl)-4-oxo-4-(4-phenyl-6,7-dihydrothieno[3,2-c]pyridin-5(4H)-yl) butanamid; |
| **41** | N-(3-Fluor-5-(trifluormethyl)benzyl)-4-oxo-4-(4-phenyl-6,7-dihydrothieno[3,2-c]pyridin-5(4H)-yl) butanamid; |
| **45** | 4-(1-Methyl-4-phenyl-6,7-dihydrothieno[3,4-c]pyridin-5(4H)-yl)-4-oxo-N-(3-(trifluormethyl)benzyl) butanamid; |
| **245** | N-(2-Cyclohexylethyl)-4-oxo-4-(4-phenyl-6,7-dihydro-4H-thieno[3,2-c]pyridin-5-yl)butanamid; |
| **247** | N-(Cyclohexylmethyl)-4-oxo-4-(4-phenyl-6,7-dihydro-4H-thieno[3,2-c]pyridin-5-yl)butanamid; |
| **249** | N-[[4-Methyl-3-(trifluormethyl)phenyl]methyl]-4-oxo-4-(4-phenyl-6,7-dihydro-4H-thieno[3,2-c]pyridin-5-yl) butanamid; |
| **250** | N-[[4-Fluor-3-(trifluormethyl)phenyl]methyl]-4-oxo-4-(4-phenyl-6,7-dihydro-4H-thieno[3,2-c]pyridin-5-yl) butanamid; |

(fortgesetzt)

| | |
|---|---|
| **251** | 4-[4-(2-Ethylphenyl)-6,7-dihydro-4H-thieno[3,2-c]pyridin-5-yl]-4-oxo-N-[[3-(trifluormethyl)phenyl]methyl] butanamid; |
| **252** | 4-[4-(2-Isopropylphenyl)-6,7-dihydro-4H-thieno[3,2-c]pyridin-5-yl]-4-oxo-N-[[3-(trifluormethyl)phenyl] methyl]butanamid; |
| **254** | 4-[4-(3-Methyl-2-thienyl)-6,7-dihydro-4H-thieno[3,2-c]pyridin-5-yl]-4-oxo-N-[[3-(trifluormethyl)phenyl] methyl]butanamid; |
| **258** | 4-(7-Butyl-5,7-dihydro-4H-thieno[2,3-c]pyridin-6-yl)-4-oxo-N-[[3-(trifluormethyl)phenyl]methyl]butanamid; |
| **260** | 4-(4-Cyclopropyl-6,7-dihydro-4H-thieno[3,2-c]pyridin-5-yl)-4-oxo-N-[[3-(trifluormethyl)phenyl]methyl] butanamid; |
| **261** | 4-Oxo-4-(4-phenthyl-6,7-dihydro-4H-thieno[3,2-c]pyridin-5-yl)-N-[[3-(trifluormethyl)phenyl]methyl] butanamid; |
| **262** | 4-[4-(4-Fluor-2-methylphenyl)-6,7-dihydro-4H-thieno[3,2-c]pyridin-5-yl]-4-oxo-N-[[3-(trifluormethyl)phenyl] methyl]butanamid; |
| **263** | 4-(4-Cyclopentyl-6,7-dihydro-4H-thieno[3,2-c]pyridin-5-yl)-4-oxo-N-[[3-(trifluormethyl)phenyl]methyl] butanamid; |
| **264** | N-[[2-Methyl-3-(trifluormethyl)phenyl]methyl]-4-oxo-4-(4-phenyl-6,7-dihydro-4H-thieno[3,2-c]pyridin-5-yl) butanamid; |
| **265** | 4-Oxo-4-[4-(3-phenylpropyl)-6,7-dihydro-4H-thieno[3,2-c]pyridin-5-yl]-N-[[3-(trifluormethyl)phenyl]methyl] butanamid; |
| **269** | 1-(4-Phenyl-6,7-dihydro-4H-thieno[3,2-c]pyridin-5-yl)-4-[7-(trifluormethyl)-3,4-dihydro-1H-isoquinolin-2-yl] butan-1,4-dion; |
| **271** | 1-(4-Phenyl-6,7-dihydro-4H-thieno[3,2-c]pyridin-5-yl)-4-[5-(trifluormethyl)-3,4-dihydro-1H-isoquinolin-2-yl] butan-1,4-dion; |
| **274** | 4-[7-(o-Tolyl)-5,7-dihydro-4H-thieno[2,3-c]pyridin-6-yl]-4-oxo-N-[[3-(trifluormethyl)phenyl]methyl] butanamid; |
| **275** | 4-(7-Cyclohexyl-5,7-dihydro-4H-thieno[2,3-c]pyridin-6-yl)-4-oxo-N-[[3-(trifluormethyl)phenyl]methyl] butanamid; |

Die erfindungsgemäßen Arzneimittel enthalten neben mindestens einer erfindungsgemäßen Verbindung ggf. geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen in einem Depot, in gelöster Form oder in einem Pflaster, ggf. unter Zusatz von die Hautpenetration fördernden Mitteln, sind geeignete perkutane Applikationszubereitungen. Oral oder perkutan anwendbare Zubereitungsformen können die erfindungsgemäßen Verbindungen verzögert freisetzen. Die erfindungsgemäßen Verbindungen können auch in parenteralen Langzeitdepotformen wie z.B. Implantaten oder implantierten Pumpen angewendet werden. Prinzipiell können den erfindungsgemäßen Arzneimitteln andere dem Fachmann bekannte weitere Wirkstoffe zugesetzt werden.

Diese erfindungsgemäßen Arzneimittel eignen sich zur Beeinflussung von KCNQ2/3-Kanälen und üben eine agonistische oder antagonistische, insbesondere eine agonistische Wirkung aus.

Bevorzugt eignen sich die erfindungsgemäßen Arzneimittel zur Behandlung von Störungen oder Krankheiten, die zumindest teilweise durch KCNQ2/3-Kanäle vermittelt werden.

Bevorzugt eignet sich das erfindungsgemäße Arzneimittel zur Behandlung von einer oder mehreren Erkrankungen ausgewählt aus der Gruppe bestehend aus Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt eignen sich die erfindungsgemäßen Arzneimittel zur Behandlung von Schmerz, ganz besonders bevorzugt von chronischem Schmerz, neuropathischem Schmerz, inflammatorischem Schmerz und muskulären Schmerz.

Weiterhin eignen sich die erfindungsgemäßen Verbindungen bevorzugt zur Behandlung von Epilepsie.

Ein weiterer Gegenstand der Erfindung ist die Verwendung wenigstens eines erfindungsgemäßen substituierten Tetrahydrothienopyridins einschließlich Verbindungen ausgewählt aus der Gruppe bestehend aus

- 1-Morpholino-4-(4-(thiophen-2-yl)-6,7-dihydrothieno[3,2-c]pyridin-5(4H)-yl)butan-1,4-dion,
- 1-(4-Acetylpiperazin-1-yl)-4-(4-(thiophen-2-yl)-6,7-dihydrothieno[3,2-c]pyridin-5(4H)-yl)butan-1,4-dion und
- 1-(3-Phenyl-4,5-dihydropyrazol-1-yl)-4-(4-(thiophen-2-yl)-6,7-dihydrothieno[3,2-c]pyridin-5(4H)-yl)butan-1,4-dion;

sowie ggf. eines oder mehrerer pharmazeutisch verträglicher Hilfsstoffe zur Herstellung eines Arzneimittels zur Behandlung von Störungen oder Krankheiten, die zumindest teilweise durch KCNQ2/3-Kanäle vermittelt werden.

Bevorzugt ist die Verwendung wenigstens eines erfindungsgemäßen substituierten Tetrahydrothienopyridins sowie ggf. eines oder mehrerer pharmazeutisch verträglicher Hilfsstoffe zur Herstellung eines Arzneimittels zur Behandlung von Schmerz, vorzugsweise von Schmerz ausgewählt aus der Gruppe bestehend aus akutem Schmerz, chronischem Schmerz, neuropathischem Schmerz, muskulärem Schmerz und inflammatorischen Schmerz; Epilepsie, Angstzuständen, Abhängigkeit, Manie, bipolaren Störungen, Migräne, kognitiven Erkrankungen, Dystonie-assoziierten Dyskinesien und/oder Harninkontinenz.

Besonders bevorzugt ist die Verwendung wenigstens eines erfindungsgemäßen substituierten Tetrahydrothienopyridins sowie ggf. eines oder mehrerer pharmazeutisch verträglicher Hilfsstoffe zur Herstellung eines Arzneimittels zur Behandlung von Schmerz, ganz besonders bevorzugt von chronischem Schmerz, neuropathischem Schmerz, inflammatorischem Schmerz und muskulärem Schmerz.

Weiterhin besonders bevorzugt ist die Verwendung wenigstens eines erfindungsgemäßen substituierten Tetrahydrothienopyridins sowie ggf. eines oder mehrerer pharmazeutisch verträglicher Hilfsstoffe zur Herstellung eines Arzneimittels zur Behandlung von Epilepsie.

Die Wirksamkeit gegen Schmerz kann beispielsweise im Bennett- bzw. Chung-Modell gezeigt werden (Bennett, G.J. and Xie, Y.K., A peripheral mononeuropathy in rat that produces disorders of pain sensation like those seen in man, Pain 1988, 33(1), 87-107; Kim, S.H. and Chung, J.M., An experimental model for peripheral neuropathy produced by segmental spinal nerve ligation in the rat, Pain 1992, 50(3), 355-363). Die Wirksamkeit gegen Epilepsie kann beispielsweise im DBA/2 Maus Modell (De Sarro et al., Naunyn-Schmiedeberg's Arch. Pharmacol. 2001, 363, 330-336) nachgewiesen werden.

Bevorzugt weisen die erfindungsgemäßen substituierten Tetrahydrothienopyridine einen $EC_{50}$-Wert von höchstens 10 μM oder höchstens 5 μM auf, bevorzugter höchstens 3 μM oder höchstens 2 μM, noch bevorzugter höchstens 1,5 μM oder höchstens 1 μM, am bevorzugtesten höchstens 0,8 μM oder höchstens 0,6 μM und insbesondere höchstens 0,4 μM oder höchstens 0,2 μM. Methoden zur Bestimmung des $EC_{50}$-Wertes sind dem Fachmann bekannt. Bevorzugt erfolgt die Bestimmung des $EC_{50}$-Wertes fluorimetrisch, besonders bevorzugt wie unter "Pharmakologische Experimente" beschrieben.

Ein weiterer Gegenstand der Erfindung sind Verfahren zur Herstellung der erfindungsgemäßen substituierten Tetrahydrothienopyridine.

Die in den nachstehend beschriebenen Umsetzungen zum Einsatz kommenden Chemikalien und Reaktionskomponenten sind käuflich erhältlich oder können jeweils nach üblichen, dem Fachmann bekannten Methoden hergestellt werden.

## Allgemeine Herstellungsverfahren

**Schema 1:**

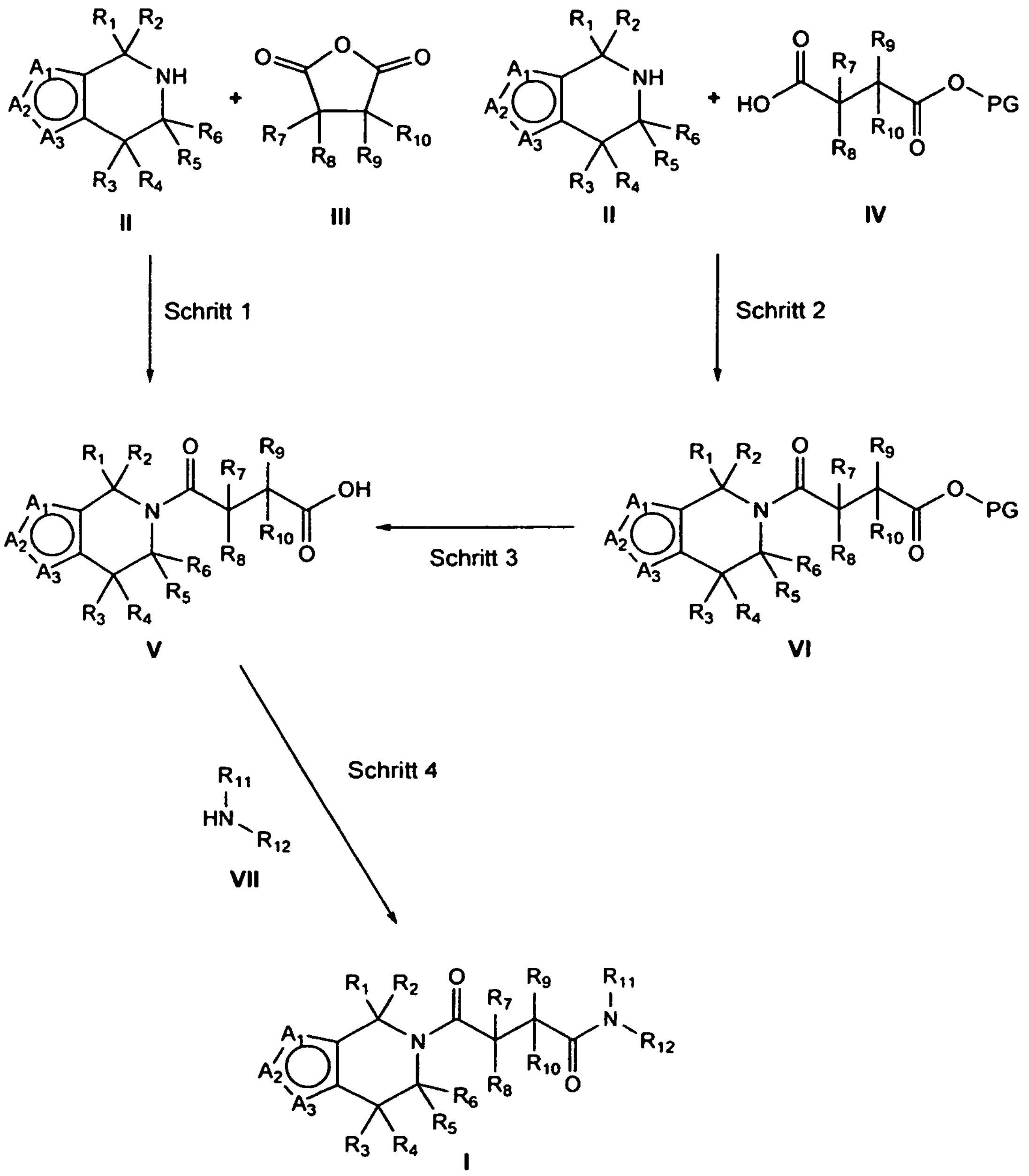


In **Schritt 1** werden Amine der allgemeinen Formel **II** mit Succinanhydriden der allgemeinen Formel **III**, in einem Reaktionsmedium, vorzugsweise ausgewählt aus der Gruppe bestehend aus Aceton, Acetonitril, Chloroform, Dioxan, Dichlormethan, Ethanol, Ethylacetat, Nitrobenzol, Methanol und Tetrahydrofuran, ggf. in Gegenwart einer anorganischen Base, vorzugsweise Kaliumcarbonat oder einer organischen Base, vorzugsweise ausgewählt aus der Gruppe bestehend aus Triethylamin, Pyridin, Dimethylaminopyridin und Diisopropylethylamin vorzugsweise bei Temperaturen von -20°C bis 160°C zu Carbonsäuren der allgemeinen Formel **V** umgesetzt.

In **Schritt 2** werden Carbonsäuren der allgemeinen Formel **IV** worin PG für eine $C_{1-6}$ Alkylgruppe, vorzugsweise Methyl, Ethyl, iso-propyl oder tert.-Butyl steht, mit Aminen der allgemeinen Formel **II** nach den unter Schritt **4** beschriebenen Verfahren zu Verbindungen der allgemeinen Formel **VI** umgesetzt.

In **Schritt 3** werden Carbonsäureester der allgemeinen Formel VI worin PG für eine $C_{1-6}$ Alkylgruppe, vorzugsweise Methyl, Ethyl, iso-propyl oder tert.-Butyl steht, ggf. in einem Reaktionsmedium, vorzugsweise ausgewählt aus der Gruppe bestehend aus Aceton, Acetonitril, Chloroform, Dioxan, Dichlormethan, Ethanol, Methanol, Tetrahydrofuran und Wasser oder in einer Mischung dieser Reaktionsmedien, ggf. in Gegenwart einer anorganischen Base, vorzugsweise LiOH oder NaOH oder ggf. in Gegenwart einer Säure, vorzugsweise Ameisensäure, Salzsäure oder Trifluoressigsäure,

ggf. in Gegenwart von Triethylsilan, Triisopropylsilan oder Ethandiol, vorzugsweise bei Temperaturen von -20°C bis 80°C zu Carbonsäuren der allgemeinen Formel **V** gespalten.

In **Schritt 4** werden Carbonsäuren der allgemeinen Formel V mit Aminen der allgemeinen Formel **VII**, in einem Reaktionsmedium, vorzugsweise ausgewählt aus der Gruppe bestehend aus Diethylether, Tetrahydrofuran, Acetonitril, Methanol, Ethanol, Dimethylformamid und Dichlormethan, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), Diisopropylcarbodiimied (DIC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, Dimethylaminopyridin und Diisopropylethylamin vorzugsweise bei Temperaturen von -70°C bis 150°C zu Verbindungen der allgemeinen Formel I umgesetzt..

**Schema 2:**

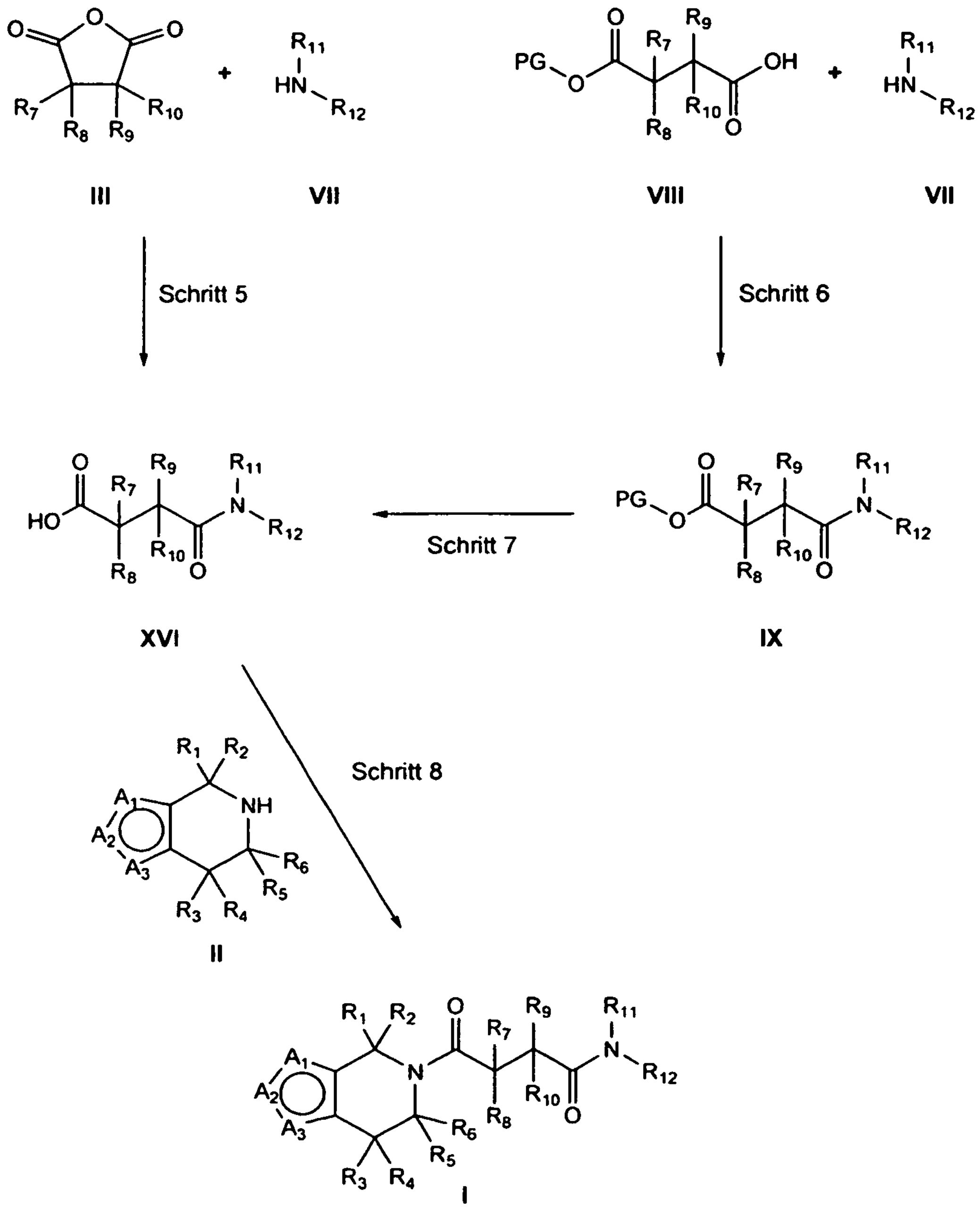


III VII VIII VII

XVI IX

II

I

In **Schritt 5** werden Amine der allgemeinen Formel **VII** mit Succinanhydriden der allgemeinen Formel **III**, nach den unter Schritt 1 beschriebenen Verfahren zu Carbonsäuren der allgemeinen Formel **XVI** umgesetzt.

In **Schritt 6** werden Amine der allgemeinen Formel **VII** mit Carbonsäuren der allgemeinen Formel **VIII**, nach den unter Schritt **4** beschriebenen Verfahren zu Verbindungen der allgemeinen Formel **IX** umgesetzt.

In **Schritt 7** werden Carbonsäureester der allgemeinen Formel **IX** worin PG für eine $C_{1-6}$ Alkylgruppe, vorzugsweise Methyl, Ethyl, iso-propyl oder tert.-Butyl steht, nach den unter Schritt **3** beschriebenen Verfahren zu Carbonsäuren der allgemeinen Formel **XVI** gespalten.

In **Schritt 8** werden Amine der allgemeinen Formel **II** mit Carbonsäuren der allgemeinen Formel **XVI**, nach den unter Schritt **4** beschriebenen Verfahren zu Verbindungen der allgemeinen Formel **I** umgesetzt.

**Schema 3 (Pictet-Spengler Synthese):**

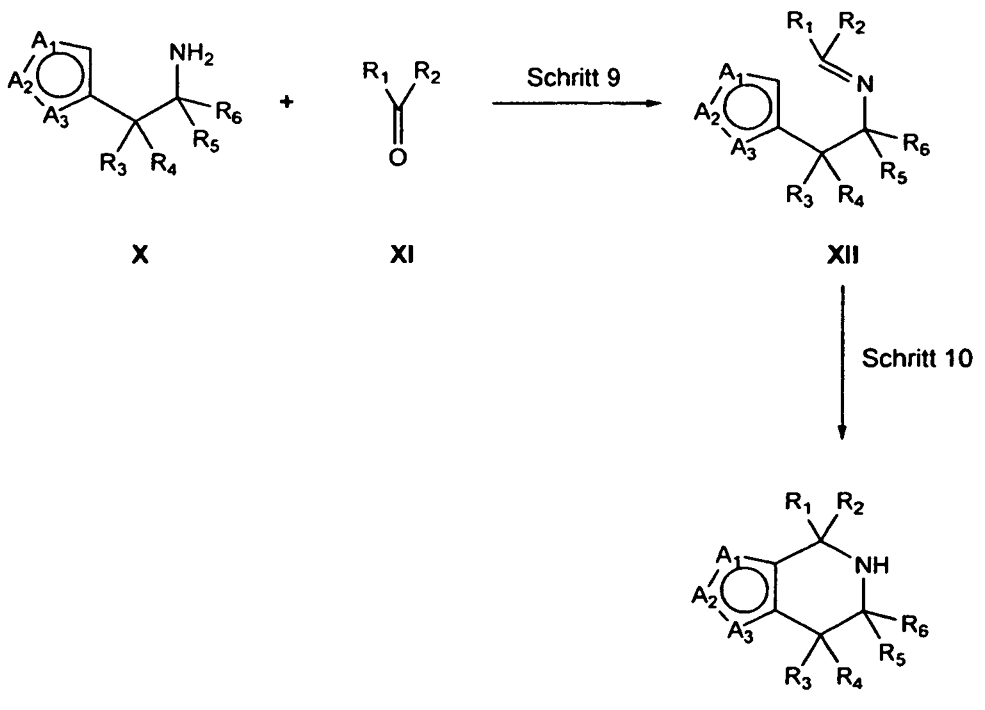


X XI XII

II

In **Schritt 9** werden Amine der allgemeinen Formel **X** mit Ketonen oder Aldehyden ($R^2$=H) der allgemeinen Formel **XI**, in einem Reaktionsmedium, vorzugsweise ausgewählt aus der Gruppe bestehend aus Acetonitril, Chloroform, Dichlormethan, Diethylether, Ethanol, Methanol, Tetrahydrofuran, Toluol und Xylol, ggf. in Gegenwart einer anorganischen Base, vorzugsweise Kaliumcarbonat oder einer organischen Base, vorzugsweise ausgewählt aus der Gruppe bestehend aus Triethylamin, Pyridin, Dimethylaminopyridin und Diisopropylethylamin vorzugsweise bei Temperaturen von 0°C bis 160°C zu Iminen der allgemeinen Formel **XII** umgesetzt.

In **Schritt 10** werden Imine der allgemeinen Formel **XII** ggf. in einem Reaktionsmedium, vorzugsweise ausgewählt aus der Gruppe bestehend aus Benzol, Ethanol, Methanol, Toluol, Wasser und Xylol, unter Zusatz einer Säure, vorzugsweise ausgewählt aus der Gruppe bestehend aus Salzsäure, Trifluoressigsäure oder Trifluormethansulfonsäure, vorzugsweise bei Temperaturen von 0°C bis 160°C zu Verbindungen der allgemeinen Formel 11 zyklisiert.

**Schema 4 (Bischler-Napieralski Synthese):**

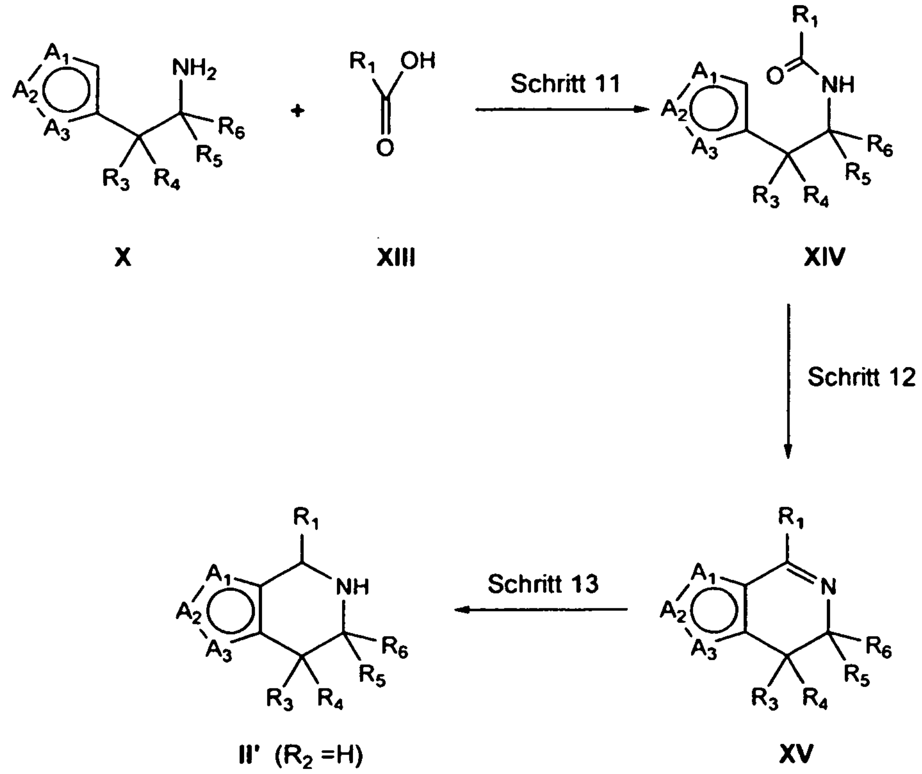


X XIII XIV XV II' ($R_2$ =H)

In **Schritt 11** werden Amine der allgemeinen Formel **X** mit Carbonsäuren der allgemeinen Formel **XIII**, nach den unter Schritt **4** beschriebenen Verfahren zu Amiden der allgemeinen Formel **XIV** umgesetzt.

In **Schritt 12** werden Amide der allgemeinen Formel **XIV** in einem Reaktionsmedium, vorzugsweise ausgewählt aus der Gruppe bestehend Benzol, Chloroform, Toluol oder Xylol in Gegenwart eines geeigneten Zyklisierungsreagens, vorzugsweise Phopsphoryltrichlorid oder Phosphorpentachlorid, ggf. unter Zusatz von Phosphorpentoxid, vorzugsweise bei Temperaturen von 20°C bis 150°C zu Verbindungen der allgemeinen Formel **XV** zyklisiert.

In **Schritt 13** werden Verbindungen der allgemeinen Formel **XIV** in einem Reaktionsmedium, vorzugsweise ausgewählt aus der Gruppe bestehend aus Diethylether, Ethanol, Essigsäure, Methanol, und Tetrahydrofuran in Gegenwart eines geeigneten Reduktionsmittels, vorzugsweise ausgewählt aus der Gruppe bestehend aus Natriumborhydrid, Natriumcyanoborhydrid, Lithiumaluminiumhydrid oder Wasserstoff, ggf. unter Zusatz eines Katalysators, vorzugsweise ausgewählt aus der Gruppe bestehend aus Palladium, Platin, Platinoxid oder Raney-Nickel, ggf. unter Zusatz einer organischen Base ausgewählt aus der Gruppe bestehend aus Ammoniak, Triethylamin und Diisopropylethylamin vorzugsweise bei Temperaturen von -20°C bis 100°C zu Verbindungen der allgemeinen Formel **II**' ($R^2$=H) reduziert.

## Beschreibung der Beispielsynthesen

Abkürzungen

| | |
|---|---|
| Ac | Acetyl |
| AcOH | Essigsäure |
| aq. | wässrig |
| Brine | ges. aq. NaCl-Lsg. |
| CDI | 1,1'-Carbonyldiimidazol |
| d | Tage |
| DCM | Dichlormethan |
| DMAP | 4-(Dimethylamino)-pyridin |
| DIPEA | Diisopropyl-ethyl-amin |
| EDC | N-(3-Dimethylaminopropyl)-N'-ethyl-carbodiimid Hydrochlorid |

| | |
|---|---|
| EE | Ethylacetat |
| Ether | Diethylether |
| Equiv. | Stoffmengenäquivalent |
| ges. | gesättigt |
| h | Stunde(n) |
| HATU | O-(7-Aza-Benzotriazol-1-yl)-N,N,N',N' tetramethyluroniumhexafluorphosphat |
| HOAt | 7-Aza-1-Hydroxy-1H-benzotriazole |
| HOBt | 1-Hydroxy-1H-benzotriazol |
| Lsg. | Lösung |
| m/z | Verhältnis Massung zur Ladung |
| M | molar |
| MeCN | Acetonitril |
| MeOH | Methanol |
| min | Minuten |
| MS | Massenspektrometrie |
| N/A | nicht verfügbar |
| $NEt_3$ | Triethylamin |
| RG | Retigabine |
| RT | Raumtemperatur 23 ± 7 °C |
| SC | Säulenchromatographie auf Kieselgel |
| TFA | Trifluoressigsäure |
| THF | Tetrahydrofuran |
| vv | Volumenverhältnis |

**Synthese von Zwischenprodukten**

**Synthese des Zwischenprodukts TAM01: 2-(2-Methylthiophen-3-yl)ethylamin**

a) Synthese von 2-Methyl-3-(2-nitrovinyl)thiophen

Eine Lösung von 3,78 g (30,0 mmol) 2-Methylthiophen-3-carbaldehyd in Nitromethan (45 ml) wurde mit 0,45 Equiv. $NH_4OAc$ versetzt und 2 h bei 100 °C gerührt. Nach Abkühlen auf RT wurden mit EE verdünnt und mit Wasser und Brine gewaschen. Die organische Phase wurde über $MgSO_4$ getrocknet, filtriert und im Vakuum eingeengt. Nach SC (Hexan / EE 20:1) des Rückstands wurden 4,16 g (24,6 mmol, 82%) 2-Methyl-3-(2-nitrovinyl)thiophen erhalten.

b) Synthese von 2-(2-Methylthiophen-3-yl)ethylamin (TAM01)

Zu einer Lösung von 2,1 Equiv. $LiAlH_4$ in Ether (190 ml) wurde eine Lösung aus 5,08 g (30,0 mmmol) 2-methyl-3-(2-nitrovinyl)thiophen in Ether (150 ml) bei RT getropft. Nach beendeter Zugabe wurde mit einer ges. aq. $Na_2SO_4$-Lsg. gequencht. Anschließend wurde durch Kieselgur filtriert und das Filtrat wurde im Vakuum eingeengt. Nach SC (DCM / MeOH 20:1 + 0.1% $NEt_3$) des Rückstands wurden 2,33 g (16,5 mmol, 55%) Zwischenprodukt TAM01 erhalten.

**Synthese des Zwischenprodukts TAM03: 1-Phenyl-2-(thiophen-3-yl)ethylamin Hydrochlorid**

a) Synthese von N-Methoxy-N-methyl-2-(thiophen-3-yl)acetamid

Zu einer Lösung von 10,0 g (70,3 mmol) 2-(thiophen-3-yl)essigsäure in DCM (200 ml) wurden bei 0 °C 20,2 g (105,5 mmol) EDC, 9,50 g (70,3 mmol) HOBt und 47,8 ml (281,2 mmol) DIPEA gegeben. Nach 20 min Rühren bei 0 °C wurden 3,08 g (84,4 mmol) N,O-Dimethylhydroxylamin Hydrochlorid zugegeben. Anschließend wurde weitere 16 h bei RT gerührt. Dann wurde mit eines ges. aq. $NH_4Cl$-Lsg gequencht und mit DCM extrahiert. Die organische Phase wurde mit Brine gewaschen, über $Na_2SO_4$ getrocknet, filtriert und im Vakuum eingeengt. Nach SC (Hexan / EE 17:3) wurden 9,98 g (53,9 mmol, 77%) N-Methoxy-N-methyl-2-(thiophen-3-yl)acetamid erhalten.

b) Synthese von 1-Phenyl-2-(thiophen-3-yl)ethanon

Zu einer Lösung von 9,9 g (53,4 mmol) N-Methoxy-N-methyl-2-(thiophen-3-yl)acetamid in Ether (100 ml) wurden bei 0 °C, 53,4 ml (53,4 mmol, 1M in THF) Phenylmagnesiumbromidlösung getropft. Anschließend wurde 3 h bei RT gerührt und dann unter Eiskühlung mit einer ges. aq. $NH_4Cl$-Lsg gequencht. Es wurde mit EE extrahiert und die organische

Phase wurde mit Brine gewaschen, über $Na_2SO_4$ getrocknet, filtriert und im Vakuum eingeengt. Das erhaltene Rohprodukt (8,98 g, 44,4 mmol, 83%) 1-Phenyl-2-(thiophen-3-yl)ethanon wurde ohne zusätzliche Aufreinigung weiter umgesetzt.

c) Synthese von 1-Phenyl-2-(thiophen-3-yl)ethanon-oxim

Eine Lösung von 8,98 g (44,4 mmol) 1-Phenyl-2-(thiophen-3-yl)ethanon in EtOH (220 ml) wurde mit 14,56 g (177,6 mmol) Natriumacetat und 6,17 g (88,8 mmol) Hydroxylamin Hydrochlorid versetzt und 4 h auf 85 °C erhitzt. Anschließend wurde durch Celite filtriert und das Filtrat wurde im Vakuum eingeengt. The Rückstand wurde mit EE aufgenommen, über $Na_2SO_4$ getrockent, filtriert und im Vakuum eingeengt. Dabei wurden als Rückstand 7,52 g (34,6 mmol, 78%) 1-Phenyl-2-(thiophen-3-yl)ethanon-oxim erhalten, die ohne zusätzliche Aufreinigung weiter umgesetzt wurden.

d) Synthese von 1-Phenyl-2-(thiophen-3-yl)ethylamin Hydrochlorid (TAM03)

Zu einer Lösung von 7,52 g (34,6 mmol) 1-Phenyl-2-(thiophen-3-yl)ethanon-oxim in EtOH (760 ml) gelöst wurden 3,81 g Raney-Nickel gegeben. Anschließend wurde die Reaktionslösung 3 d unter Wasserstoffatmosphäre bei 4,14 bar und 30 °C gerührt. Danach wurde über Celite filtriert und das Filtrat wurde im Vakuum eingeengt. Der erhaltene Rückstand wurde in 1 M aq. Salzsäure aufgelöst und mit EE gewaschen. Die wässrige Phase wurde unter Eiskühlung mit einer ges. aq. $Na_2CO_3$-Lsg auf pH 8-10 eingestellt und anschließend wurde mit EE extrahiert. Die organische Phase wurde über $Na_2SO_4$ getrocknet, filtriert und im Vakuum eingeengt. Der erhaltene Rückstand wurde in Dioxan (40 ml) gelöst und bei 0 °C (Eisbad) mit einer ges. HCl-Lsg in Dioxan (115 ml) versetzt. Nach 2 h Rühren bei RT wurde im Vakuum eingeengt. Dabei wurden 3,1 g (12,8 mmol, 37%) Zwischenprodukt TAM03 erhalten.

**Synthese des Zwischenprodukts SAMN04: 4-(2-Fluor-phenyl)-4,5,6,7-tetrahydrothieno[3,2-c]pyridin**

Eine Lösung von 9.2 ml (78,6 mmol) 2-Thiophen-2-yl-ethylamin in EtOH (80 ml) wurde mit 4,8 ml (34,6 mmol) $NEt_3$ und 8,2 ml (78,6 mmol) 2-Fluor-benzaldehyd versetzt und 72 h bei 50 °C gerührt. Anschließend wurde im Vakuum eingeengt. Der Rückstand wurde mit TFA (300 ml) aufgenommen und weitere 72 h bei RT gerührt. Nach Einengen im Vakuum wurde der Rückstand mit DCM aufgenommen und es wurde mit einer 2M aq. NaOH-Lsg. gewaschen. Die organische Phase wurde über $MgSO_4$ getrocknet, filtriert und im Vakuum eingeengt. Nach SC (EE / Hexan 3:1) des Rückstands wurden 12,2 g (52,3 mmol, 66%) Zwischenprodukt SAMN04 erhalten.

**Synthese des Zwischenprodukts SAMN08: 2-Methyl-4-phenyl-4,5,6,7-tetrahydrothieno[3,2-c]pyridin**

Zu einer Lösung von 2,0 g (14,1 mmol) 2-(5-Methylthien-2-yl)-ethylamin und 1,42 ml (14,1 mmol) Benzaldehyd in Toluol (75 ml) wurde 4Å Molekularsieb gegeben und das Gemisch wurde 16 h mit einem Wasserabscheider unter Rückfluss erhitzt. Anschließend wurde das Molekularsieb abfiltrirert und mit Toluol nachgewaschen. Das Filtrat wurde im Vakuum eingeengt. Der Rückstand wurde mit TFA (54 ml) aufgenommen und es wurde 6 d bei RT gerührt. Danach wurde im Vakuum eingeengt. Der erhaltene Rückstand wurde mit EE aufgenommen und es wurde mit einer 2M aq. NaOH-Lsg. gewaschen. Die organische Phase wurde über $MgSO_4$ getrocknet, filtriert und im Vakuum eingeengt. Nach SC (EE / Hexan 4:1) des Rückstands wurden 1,98 g (8,6 mmol, 61%) Zwischenprodukt SAMN08 erhalten.

**Synthese des Zwischenprodukts SAMN10: 5-Phenyl-4,5,6,7-tetrahydrothieno[2,3-c]pyridin**

Eine Lösung von 3,1 g (12,8 mmol) des ZwischenproduktsTAM03 in einer Mischung aus Formaldehyd/AcOH (1:1 vv, 230 ml) wurde 1 h unter Rückfluss erhitzt. Anschließend wurde mit Wasser verdünnt und mit flüssigem Ammoniak neutralisiert. Dann wurde mit EE extrahiert und die organische Phase wurde mit Brine gewaschen, über $Na_2SO_4$ getrocknet, filtriert und im Vakuum eingeengt. Nach SC (Hexan / EE 7:3) des Rückstands wurden 1,27 g (5,9 mmol, 46%) Zwischenprodukt SAMN10 erhalten.

**Synthese des Zwischenprodukts SAMN13: 4-(2-Isopropylphenyl)-4,5,6,7-tetrahydrothieno[3,2-c]pyridin**

Zu einer Lösung von 2,5 g (19,7 mmol) 2-Thiophen-2-yl-ethylamin und 2,9 g (19,7 mmol) 2-Isopropyl-benzaldehyd in Toluol (40 ml) wurde 4Å Molekularsieb gegeben und das Gemisch wurde 16 h mit einem Wasserabscheider unter Rückfluss erhitzt. Anschließend wurde das Molekularsieb abfiltirert und mit Toluol nachgewaschen. Das Filtrat wurde im Vakuum eingeengt. Der Rückstand wurde mit TFA (40 ml) aufgenommen und es wurde 72 h bei 90 °C gerührt. Danach wurde im Vakuum eingeengt. Der erhaltene Rückstand wurde mit einer 2M aq. NaOH-Lsg. Versetzt und anschließend wurde mit DCM extrahiert. Die organische Phase wurde über $MgSO_4$ getrocknet, filtriert und im Vakuum

eingeengt. Nach SC (EE / Hexan 1:1) des Rückstands wurden 3,28 g (12,7 mmol, 64%) Zwischenprodukt SAMN13 erhalten.

Synthese des Zwischenprodukts ASP01: 4-Methoxy-2-methyl-4-oxobuttersäure

Eine gerührte Lösung 4-Methoxy-2-methylen-4-oxo-buttersäure (5,0 g, 34,7 mmol) in Ethanol (100 ml) wurde mit $N_2$ für 15 min deoxygeniert, anschließend mit Palladium (10% auf Kohle, 1,85 g, 1,74 mmol) versetzt und noch einmal für 15 min mit $N_2$ deoxygeniert. Der Reaktionsansatz wurde unter $H_2$-Atmosphäre 16 h gerührt und anschließend über Celite filtriert. Das Lösungsmittel wurde am Rotationsverdampfer entfernt. Das erhaltene Rohzwischenprodukt ASP01 wurde ohne zusätzliche Aufreinigung weiter eingesetzt.

**Synthese des Zwischenprodukts ASP02: 4-tert-Butoxy-3-methyl-4-oxobuttersäure**

a) Synthese von 1-tert-Butyl 4-methyl 2-methylsuccinat

Eine Lösung des Zwischenprodukts ASP01 (20,2 g, 138,0 mmol), DMAP (8,44g, 69,0 mmol) und tert.-Butanol (14,45 ml, 152,0 mmol) in DCM (500 ml) wurde auf 0 °C abgekühlt und mit EDC (29,1g, 152,0 mmol) versetzt. Der Reaktionsansatz wurde 16 h bei RT gerührt. Nach beendeter Reaktion wurde mit Brine (500 ml) gewaschen. Die organische Phase wurde anschließend über $Na_2SO_4$ getrocknet, filtriert und bis zur Trockne eingeengt. Das erhaltene Rohprodukt, 1-tert-Butyl 4-methyl 2-methylsuccinat, wurde ohne zusätzliche Aufreinigung weiter eingesetzt.

b) Synthese von 4-tert-Butoxy-3-methyl-4-oxobuttersäure (ASP02)

Zu einer Lösung von (25,0 g, 124,0 mmol) 1-tert-Butyl 4-methyl 2-methylsuccinat in einem Gemisch aus MeOH (85 ml), THF (85 ml) und Wasser (85 ml) wurde $LiOH \cdot H_2O$ (5,19 g, 124,0 mmol) gegeben und der Reaktionsansatz wurde 16 h bei RT gerührt. Nach beendeter Reaktion wurde das Lösungsmittel am Rotationsverdampfer entfernt. Der Rückstand wurde mit aq. HCl (1 M, 130 ml) aufgenommen und zweimal mit DCM (150 ml) extrahiert. Die vereinigten organischen Phasen wurden über $Na_2SO_4$ getrocknet, filtriert und anschließend bis zur Trockne eingeengt. Das erhaltene Rohzwischenprodukt ASP02 wurde ohne zusätzliche Aufreinigung weiter eingesetzt.

**Synthese des Zwischenprodukts SAAS01: 4-Oxo-4-(4-phenyl-6,7-dihydrothieno[3,2-c]pyridin-5(4H)-yl)buttersäure**

Zu einer Suspension von 13,0 g (130,1 mmol) Succinanhydrid in MeCN (80 ml) wurde eine Lösung von 8,0 g (37,2 mmol) 4-Phenyl-4,5,6,7-tetrahydrothieno[3,2-c]pyridin in MeCN (20 ml) gegeben. Anschließend wurde die Reaktionslösung mit 10,3 ml (74,3 mmol) $NEt_3$ versetzt und 72 h bei RT gerührt. Danach wurde im Vakuum eingeengt und der Rückstand wurde mit EE aufgenommen und mit Wasser versetzt. Dann wurde mehrmals mit einer ges. aq. $NaHCO_3$-Lsg. extrahiert. Der vereinten Wasserphasen wurden mit eine 2N Salzsäure auf pH 6-8 eingestellt. Dann wurde mit EE extrahiert. Die organische Phase wurde mehrmals mit Wasser gewaschen, mit Aktivkohle versetzt und 15 min gerührt. Danach wurde über Kieselgur filtriert und das Filtrat wurde über $MgSO_4$ getrocknet, filtriert und im Vakuum eingeengt. Als Rückstand wurden 7,08 g (22,4 mmol, 60%) Zwischenprodukt SAAS01 erhalten.

**Synthese des Zwischenprodukts SAAS02: (3R)-3-Methyl-4-oxo-4-(4-phenyl-6,7-dihydrothieno[3,2-c]pyridin-5(4H)-yl)buttersäure**

a) Synthese von (3R)-3-Methyl-4-oxo-4-(4-phenyl-6,7-dihydrothieno[3,2-c]pyridin-5(4H)-yl)buttersäure methylester

Zu einer Lösung von 945 mg (6,5 mmol) (R)-4-Methoxy-2-methyl-4-oxobuttersäure in THF (50 ml) wurden nacheinander 1,53 g (7,1 mmol) 4-Phenyl-4,5,6,7-tetrahydrothieno[3,2-c]pyridin, 2,46 g (6,5 mmol) HATU und 1,7 ml (12,3 mmol) $NEt_3$ gegeben. Nach 16 h Rühren bei RT wurde die Reaktionslösung mit EE (30 ml) verdünnt und es wurde je zweimal mit einer ges. aq. $NH_4Cl$-Lsg. und einer 1 N aq. $NaHCO_3$-Lsg. gewaschen.

Die organische Phase wurde über $MgSO_4$ getrocknet, filtriert und im Vakuum eingeengt. Nach SC (EE) des Rückstands wurden 1,14 g (3,3 mmol, 51%) (3R)-3-methyl-4-oxo-4-(4-phenyl-6,7-dihydrothieno[3,2-c]pyridin-5(4H)-yl)buttersäure methylester erhalten.

b) Synthese von (3R)-3-Methyl-4-oxo-4-(4-phenyl-6,7-dihydrothieno[3,2-c]pyridin-5(4H)-yl)buttersäure (SAAS02)

Eine Lösung von 1,14 g (3,3 mmol) (3R)-3-methyl-4-oxo-4-(4-phenyl-6,7-dihydrothieno[3,2-c]pyridin-5(4H)-yl)buttersäure methylester in einer Mischung aus THF/MeOH (1:1 w, 50 ml) wurde auf 0 °C abgekühlt (Eisbad) und mit 25 ml einer 1 M aq. LiOH-Lsg. versetzt. Anschließend wurde 10 min gerührt, das Eisbad entfernt und weitere 150 min gerührt. Dann wurde mit EE (100 ml) verdünnt und mit einer 1 M aq. Salzsäure (25 ml) versetzt. Die Phasen wurden getrennt und die wässrige Phase mit EE extrahiert. Die vereinten organischen Phasen wurden über $MgSO_4$ getrocknet, filtriert und im Vakuum eingeengt. Nach SC (EE) des Rückstands wurden 795 mg (2,4 mmol, 73%) des Zwischenprodukts SAAS02 erhalten.

**Synthese des Zwischenprodukts SAAS07: 4-(4-(4-Chlorphenyl)-6,7-dihydrothieno[3,2-c]pyridin-5(4H)-yl)-4-oxobuttersäure**

a) Synthese von 4-(4-(4-Chlorphenyl)-6,7-dihydrothieno[3,2-c]pyridin-5(4H)-yl)-4-oxobuttersäure-methylester

Eine Lösung von 9,82 g (39,3 mmol) Zwischenprodukt SAMN12 in DCM (250 ml) wurde mit 8,2 ml (59,0 mmol) $NEt_3$ versetzt. Anschließend wurde eine Lösung von 6,50 g (43,2 mmol) 4-Chlor-4-oxobuttersäure-methylester in DCM (100 ml) zugetropft. Nach 3 h Rühren bei RT wurde mit einer 0,5 M aq. Salzsäure (250 ml) und mit einer ges. aq. $NaHCO_3$-Lsg. (250 ml) gewaschen. Die organische Phase wurde über $Na_2SO_4$ getrocknet, filtriert und im Vakuum eingeengt. Nach SC mit dem Rückstand (Heptan / EE 3:1) wurden 13,9 g (38,2 mmol, 97%) 4-(4-(4-Chlorphenyl)-6,7-dihydrothieno[3,2-c]pyridin-5(4H)-yl)-4-oxobuttersäure-methylester erhalten.

b) Synthese von 4-(4-(4-Chlorphenyl)-6,7-dihydrothieno[3,2-c]pyridin-5(4H)-yl)-4-oxobuttersäure (SAAS07)

Eine Lösung von 11,0 g (30,3 mmol) 4-(4-(4-Chlorphenyl)-6,7-dihydrothieno[3,2-c]pyridin-5(4H)-yl)-4-oxo-buttersäure-methylester in einer Mischung aus THF/MeOH (1:1 vv, 200 ml) wurde mit einer 6M aq. NaOH-Lsg (30 ml) versetzt und anschließend 2 h bei RT gerührt. Danach wurden die organischen Lösungsmittel im Vakuum größtenteils entfernt. Anschließend wurde mit einer 6M aq. Salzsäure neutralisiert. Dann wurde mit DCM (2 x 200 ml) extrahiert. Die vereinten organischen Phasen wurden über $Na_2SO_4$ getrocknet, filtriert und im Vakuum eingeengt. Das erhaltene Rohzwischenprodukts SAAS07 wurde ohne zusätzliche Aufreinigung weiter eingesetzt.

**Synthese des Zwischenprodukts SAAS09: 2-Methyl-4-oxo-4-(4-phenyl-6,7-dihydrothieno[3,2-c]pyridin-5(4H)-yl)buttersäure**

a) Synthese von 2-Methyl-4-oxo-4-(4-phenyl-6,7-dihydrothieno[3,2-c]pyridin-5(4H)-yl)buttersäure-tert.-butylester

Eine Lösung von 6,95 g (32,3 mmol) 4-Phenyl-4,5,6,7-tetrahydrothieno[3,2-c]pyridin, 6,38 g (33,9 mmol) Zwischenprodukt ASP02, 436 mg (3,2 mmol) HOAt und 11 ml (64,6 mmol) DIPEA in DCM (300 ml) wurde mit 9,3 g (48,5 mmol) EDC versetzt und 16 h bei RT gerührt. Anschließend wurde einer 0,5 M aq. Salzsäure (250 ml) und einer ges. aq. $NaHCO_3$-Lsg. (250 ml) gewaschen. Die organische Phase wurde über $Na_2SO_4$ getrocknet, filtriert und im Vakuum eingeengt. Nach SC (Heptan / EE 4:1) des Rückstands wurden 9,67 g (25,1 mmol, 78%) 2-Methyl-4-oxo-4-(4-phenyl-6,7-dihydrothieno[3,2-c]pyridin-5(4H)-yl)buttersäure-tert.-butylester erhalten.

b) Synthese von 2-Methyl-4-oxo-4-(4-phenyl-6,7-dihydrothieno[3,2-c]pyridin-5(4H)-yl)buttersäure (SAA09)

Eine Lösung von 9,64 g (25,0 mmol) 2-Methyl-4-oxo-4-(4-phenyl-6,7-dihydrothieno-[3,2-c]pyridin-5(4H)-yl)buttersäure-tert.-butylester in einer Mischung aus THF/MeOH (1:1 vv, 165 ml) wurde mit einer 6M aq. NaOH-Lsg. (25 ml) versetzt und anschließend 2 h bei RT gerührt. Danach wurden die organischen Lösungsmittel im Vakuum größtenteils entfernt. Anschließend wurde mit einer 6M aq. Salzsäure neutralisiert. Dann wurde mit DCM (2 x 200 ml) extrahiert. Die vereinten organischen Phasen wurden über $Na_2SO_4$ getrocknet, filtriert und im Vakuum eingeengt. Als Rückstand wurden 7,82 g (23,7 mmol, 95%) Zwischenprodukt SAAS09 erhalten, das ohne zusätzliche Aufreinigung weiter eingesetzt wurde.

**Synthese des Zwischenprodukts PAAS01: 4-Oxo-4-(3-(trifluormethyl)benzylamino)buttersäure**

Zu einer Suspension von 7,5 g (85,6 mmol) Succinanhydrid in Ether (450 ml) wurde eine Lösung von 15,0 g (85,6 mmol) (3-(Trifluormethyl)phenyl)methylamin in Ether (40 ml) innerhalb von 30 min getropft. Anschließend wurde 72 h bei RT gerührt. Der erhaltene Niederschlag wurde abfiltriert und getrocknet. Nach SC (EE / MeOH 1:1) des Rückstands wurden 10,4 g (37,8 mmol, 44%) Zwischenprodukt PAAS01 erhalten.

**Synthese des Zwischenproduktsen PAAS03: 4-Oxo-2-phenyl-4-(3-(trifluormethyl)benzylamino)buttersäure und PAAS04: 4-Oxo-3-phenyl-4-(3-(trifluormethyl)benzylamino)buttersäure**

Zu einer Suspension von 5,0 g (28,4 mmol) 3-Phenyldihydrofuran-2,5-dion in Ether (100 ml) wurde eine Lösung von 5,0 g (28,4 mmol) (3-(Trifluormethyl)phenyl)-methylamin in Ether (50 ml) innerhalb von 30 min getropft. Der erhaltene Niederschlag wurde abfiltriert und getrocknet. Nach mehrfacher SC (TBME /Hexan 4:1, dann TBME / EE 1:1, dann EE / MeOH 9:1) des Rückstands wurden 1,27 g (3,6 mmol, 13%) Zwischenprodukt PAAS03 und 372 mg (1,1 mmol, 4%) Zwischenprodukt PAAS04 erhalten.

**Synthese des Zwischenprodukts PAAS05: (R)-3-Methyl-4-oxo-4-(3-(trifluormethyl)benzylamino)-buttersäure**

a) Synthese von (R)-3-Methyl-4-oxo-4-(3-(trifluormethyl)benzylamino)buttersäuremethylester

Zu einer Lösung von 1,0 g (6,8 mmol) (R)-4-Methoxy-2-methyl-4-oxobuttersäure in THF (50 ml) wurden nacheinander 1,32 g (7,5 mmol) 3-(Trifluormethyl)phenyl)methylamin, 2,60 g (6,8 mmol) HATU und 1,8 ml (13,0 mmol) $NEt_3$ gegeben. Nach 16 h Rühren bei RT wurde die Reaktionslösung mit EE (30 ml) verdünnt und es wurde je zweimal mit einer ges. aq. $NH_4Cl$-Lsg. und einer 1 N aq. $NaHCO_3$-Lsg. gewaschen. Die organische Phase wurde über $Na_2SO_4$ getrocknet, filtriert und im Vakuum eingeengt. Nach SC (EE) des Rückstands wurden 1,30 g (4,3 mmol, 63%) (R)-3-Methyl-4-oxo-4-(3-(trifluormethyl)benzylamino)buttersäure-methylester erhalten.

b) Synthese von (R)-3-Methyl-4-oxo-4-(3-(trifluormethyl)benzylamino)buttersäure (PAAS05)

Eine Lösung von 1,29 g (4,3 mmol) (R)-3-Methyl-4-oxo-4-(3-(trifluormethyl)benzyl-amino)buttersäure-methylester in einer Mischung aus THF/MeOH (1:1 vv, 70 ml) wurde auf 0 °C abgekühlt (Eisbad) und mit 35 ml einer 1 M aq. LiOH-Lsg. versetzt. Anschließend wurde 10 min gerührt, das Eisbad entfernt und weitere 120 min gerührt. Dann wurde mit EE (100 ml) verdünnt und mit einer 1M aq. Salzsäure (25 ml) versetzt. Die Phasen wurden getrennt und die wässrige Phase mit EE extrahiert. Die vereinten organischen Phasen wurden über $Na_2SO_4$ getrocknet, filtriert und im Vakuum eingeengt. Nach SC(EE) des Rückstands wurden 185 mg (0,6 mmol, 15%) Zwischenprodukt PAAS05 erhalten.

**Synthese weiterer Zwischenprodukte**

Die Synthese weiterer Zwischenprodukte erfolgte nach den bereits beschriebenen Verfahren. In Tabelle 1 ist angegeben, welche Verbindung nach welchem Verfahren hergestellt wurden. Dem Fachmann ist dabei ersichtlich, welche Edukte jeweils eingesetzt wurden.

**Tabelle 1:**

| Zwischenproduk t | Chemische Bezeichnung | Herstellung analog Zwischenprodukt | Ausbeute [%] |
|---|---|---|---|
| TAM02 | 2-(4-Methylthiophen-2-yl)ethylamin | TAM01 | 21 |
| SAMN01 | 4-(2-Methyl-phenyl)-4,5,6,7-tetrahydrothieno[3,2-c]pyridin | SAMN08 | 35 |
| SAMN02 | 4-(3-Methyl-phenyl)-4,5,6,7-tetrahydrothieno[3,2-c]pyridin | SAMN04 | 28 |
| SAMN03 | 4-(4-Methyl-phenyl)-4,5,6,7-tetrahydrothieno[3,2-c]pyridin | SAMN08 | 28 |
| SAMN05 | 4-(3-Fluor-phenyl)-4,5,6,7-tetrahydrothieno[3,2-c]pyridin | SAMN08 | 82 |
| SAMN06 | 4-(4-Fluor-phenyl)-4,5,6,7-tetrahydrothieno[3,2-c]pyridin | SAMN08 | 22 |
| SAMN07 | 3-Methyl-4-phenyl-4,5,6,7-tetrahydrothieno[3,2-c]pyridin | SAMN08 | 26 |
| SAMN09 | 7-Phenyl-4,5,6,7-tetrahydrothieno [2,3-c]pyridin | SAMN08 | 51 |

(fortgesetzt)

| Zwischenprodukt | Chemische Bezeichnung | Herstellung analog Zwischenprodukt | Ausbeute [%] |
|---|---|---|---|
| SAMN11 | 1-Methyl-4-phenyl-4,5,6,7-tetrahydrothieno[3,4-c]pyridin | SAMN08 | 18 |
| SAMN12 | 4-(4-Chlorophenyl)-4,5,6,7-tetrahydrothieno[3,2-c]pyridin | SAMN04 | N/A |
| SAMN14 | 7-o-Tolyl-4,5,6,7-tetrahydrothieno (2,3-c]pyridin | SAMN13 | N/A |
| SAMN15 | 7-Cyclohexyl-4,5,6,7-tetrahydrothieno[2,3-c]pyridin | SAMN13 | N/A |
| SAMN16 | 7-Butyl-4,5,6,7-tetrahydrothieno [2,3-c]pyridin | SAMN13 | N/A |
| SAMN17 | 4-(2-Ethyl-ylphenyl)-4,5,6,7-tetrahydrothieno[3,2-c]pyridin | SAMN13 | 68 |
| SAMN18 | 4-(2,6-Dimethylphenyl)-4,5,6,7-tetrahydrothieno[3,2-c]pyridin | SAMN13 | 3 |
| SAMN19 | 4-(3-Methylthiophen-2-yl)-4,5,6,7-tetrahydrothieno[3,2-c]pyridin | SAMN13 | 2 |
| SAMN20 | 4-Phenethyl-4,5,6,7-tetrahydrothieno[3,2-c]pyridin | SAMN13 | N/A |
| SAMN21 | 4-(3-Phenylpropyl)-4,5,6,7-tetrahydrothieno[3,2-c]pyridin | SAMN13 | N/A |
| SAMN22 | 4-Cyclopropyl-4,5,6,7-tetrahydrothieno[3,2-c]pyridin | SAMN13 | N/A |
| SAMN23 | 4-Cyclopentyl-4,5,6,7-tetrahydrothieno[3,2-c]pyridin | SAMN13 | N/A |
| SAMN24 | 4-(Cyclohexylmethyl)-4,5,6,7-tetrahydrothieno[3,2-c]pyridin | SAMN13 | N/A |
| SAMN25 | 4-(4-Fluor-2-methylphenyl)-4,5,6,7-tetrahydrothieno[3,2-c]pyridin | SAMN13 | N/A |
| SAAS03 | 4-Oxo-4-(4-m-tolyl-6,7-dihydrothieno[3,2-c]pyridin-5 (4H)-yl)buttersäure | SAAS07 | N/A |
| SAAS04 | 4-Oxo-4-(4-p-tolyl-6,7-dihydrothieno[3,2-c]pyridin-5 (4H)-yl)buttersäure | SAAS07 | N/A |
| SAAS05 | 4-(4-(3-Fluorphenyl)-6,7-dihydrothieno[3,2-c]pyridin-5 (4H)-yl)-4-oxobuttersäure | SAAS07 | N/A |
| SAAS06 | 4-(4-(4-Fluorphenyl)-6,7-dihydrothieno[3,2-c]pyridin-5 (4H)-yl)-4-oxobuttersäure | SAAS07 | N/A |
| SAAS08 | 3-Methyl-4-oxo-4-(4-phenyl-6,7-dihydrothieno[3,2-c]pyridin-5 (4H)-yl)buttersäure | SAAS09 | N/A |

(fortgesetzt)

| Zwischenprodukt | Chemische Bezeichnung | Herstellung analog Zwischenprodukt | Ausbeute [%] |
|---|---|---|---|
| SAAS10 | 4-Oxo-4-(4-o-tolyl-6,7-dihydrothieno[3,2-c]pyridin-5(4H)-yl)buttersäure | SAAS01 | 45 |
| PAAS02 | 4-(3,4-Difluorbenzylamino)-4-oxobuttersäure | PASS01 | 39 |
| PAAS06 | rac. (1R,2S)-2-(3-(Trifluormethyl) benzylcarbamoyl)cyclopropancarbonsäure | PASS01 | 98 |
| PAAS07 | rac. (1R,2S)-2-(3-(Trifluormethyl) benzylcarbamoyl)cyclopentancarbonsäure | PASS01 | 88 |
| PAAS08 | rac. (1R,2S)-2-(3-(Trifluormethyl) benzylcarbamoyl)cyclohexancarbonsäure | PASS01 | 76 |
| PAAS09 | rac. (1S,2S)-2-(3-Trifluoromethyl) benzylcarbamoyl)cyclohexancarbonsäure | PASS01 | 69 |
| PASS10 | rac. (1S,2S)-2-(3-(Trifluormethyl) benzylcarbamoyl)cyclopropancarbonsäure | PASS05 | 76 |

<u>**Synthese der Beispielverbindungen**</u>

**Synthese von Beispielverbindung 2: 4-Oxo-4-(4-phenyl-6,7-dihydrothieno[3,2-c]pyridin-5(4H)-yl)-N-(3-(trifluormethyl)benzyl)butanamid**

Zu einer Lösung von 275 mg (1,0 mmol) Zwischenprodukt PAAS01 in THF (8 ml) wurden nacheinander 236 mg (1,1 mmol) 4-Phenyl-4,5,6,7-tetrahydrothieno[3,2-c]pyridin, 380 mg (1,0 mmol) HATU und 263 μl (1,9 mmol) $NEt_3$ gegeben. Nach 48 h Rühren bei RT wurde die Reaktionslösung mit EE (30 ml) verdünnt und es wurde je zweimal mit einer 2N aq. Salzsäure und einer 1 N aq. $NaHCO_3$-Lsg. gewaschen. Die organische Phase wurde über $MgSO_4$ getrocknet, filtriert und im Vakuum eingeengt. Nach SC (EE) des Rückstands wurden 386 mg (0,8 mmol, 82%) der Beispielverbindung 2 erhalten. MS: m/z 473,1 $[M+H]^+$.

**Synthese von Beispielverbindung 3: 4-(4-(3-Fluorphenyl)-6,7-dihydrothieno[3,2-c]pyridin-5(4H)-yl)-4-oxo-N-(3-(trifluormethyl)benzyl)butanamid**

Zu einer Lösung von 349 mg (1,27 mmol) des Zwischenprodukts PAAS01 in THF (10 ml) wurden nacheinander 325 mg (1,40 mmol) des Zwischenprodukts SAMN05, 482 mg (1,27 mmol) HATU und 334 μl (2,41 mmol) $NEt_3$ gegeben. Nach 48 h Rühren bei RT wurde die Reaktionslösung mit EE (30 ml) verdünnt und es wurde je zweimal mit einer 2N aq. Salzsäure und einer 1 N aq. $NaHCO_3$-Lsg. gewaschen. Die organische Phase wurde über $Na_2SO_4$ getrocknet, filtriert und im Vakuum eingeengt. Als Rückstand wurden 377 mg (0,77 mmol, 61 %) der Beispielverbindung 3 erhalten. MS: m/z 491,1 $[M+H]^+$.

**Synthese von Beispielverbindung 9: 4-Oxo-4-(7-phenyl-4,5-dihydrothieno[2,3-c]pyridin-6(7H)-yl)-N-(3-(trifluormethyl)benzyl)butanamid**

Zu einer Lösung von 300 mg (1,09 mmol) des Zwischenprodukts PAAS01 in THF (8 ml) wurden nacheinander 258 mg (1,20 mmol) des Zwischenprodukts SAMN09, 414 mg (1,09 mmol) HATU und 287 μl (2,07 mmol) $NEt_3$ gegeben. Nach 72 h Rühren bei RT wurde die Reaktionslösung mit EE (30 ml) verdünnt und es wurde je zweimal mit einer 2N aq. Salzsäure und einer 1 N aq. $NaHCO_3$-Lsg. gewaschen. Die organische Phase wurde über $Na_2SO_4$ getrocknet, filtriert und im Vakuum eingeengt. Durch Umkristallisation des Rückstands aus EE wurden 280 mg (0,59 mmol, 54%) der

Beispielverbindung 9 erhalten. MS: m/z 473,1 $[M+H]^+$.

**Synthese von Beispielverbindung 11: N-(4-Methylbenzyl)-4-oxo-4-(4-phenyl-6,7-dihydrothieno[3,2-c]pyridin-5(4H)-yl)butanamid**

Zu einer Lösung von 400 mg (1,27 mmol) des Zwischenprodukts SAAS01 in THF (10 ml) wurden nacheinander 169 mg (1,40 mmol) (4-Methylphenyl)-methylamin, 482 mg (1,27 mmol) HATU und 334 µl (2,41 mmol) $NEt_3$ gegeben. Nach 24 h Rühren bei RT wurde die Reaktionslösung mit EE (30 ml) verdünnt und es wurde je zweimal mit einer 2N aq. Salzsäure und einer 1 N aq. $NaHCO_3$-Lsg. gewaschen. Die organische Phase wurde über $Na_2SO_4$ getrocknet, filtriert und im Vakuum eingeengt. Durch Umkristallisation des Rückstands aus EE wurden 239 mg (0,57 mmol, 45%) der Beispielverbindung 11 erhalten. MS: m/z 419,2 $[M+H]^+$.

**Synthese von Beispielverbindung 12: N-Benzyl-4-oxo-4-(4-phenyl-6,7-dihydrothieno[3,2-c]pyridin-5(4H)-yl)butanamid**

Eine Lösung von 400 mg (1,27 mmol) des Zwischenprodukts SAAS01 und 216 mg (1,33 mmol) CDI in DCM (10 ml) wurde 1 h bei RT gerührt. Anschließend wurde eine Lösung von 138 µl (1,27 mmol) Benzylamin in DCM (10 ml) zugegeben und es wurde weitere 16 h bei RT gerührt. Dann wurde die Reaktionslösung je dreimal mit einer ges. aq. $NH_4Cl$-Lsg. und einer ges. aq. $NaHCO_3$-Lsg gewaschen. Die organische Phase wurde über $MgSO_4$ getrocknet, filtriert und im Vakuum eingeengt. Durch Umkristallisation des Rückstands aus EE wurden 244 mg (0,60 mmol, 47%) der Beispielverbindung 12 erhalten. MS: m/z 405,2 $[M+H]^+$.

**Synthese von Beispielverbindung 24: N-Methyl-4-oxo-4-(4-phenyl-6,7-dihydrothieno[3,2-c]pyridin-5(4H)-yl)-N-(3-(trifluormethyl)benzyl)butanamid**

Eine Lösung von 400 mg (1,27 mmol) des Zwischenprodukts SAAS01 und 216 mg (1,33 mmol) CDI in THF (13 ml) wurde 1 h bei RT gerührt. Anschließend wurde eine Lösung von 219 µl (1,27 mmol) N-Methyl-1-(3-(trifluormethyl)phenyl)methylamin in THF (13 ml) zugegeben und es wurde weitere 16 h bei RT gerührt. Dann wurde die Reaktionslösung je dreimal mit einer ges. aq. $NH_4Cl$-Lsg. und einer ges. aq. $NaHCO_3$-Lsg gewaschen. Die organische Phase wurde über $MgSO_4$ getrocknet, filtriert und im Vakuum eingeengt. Nach SC (EE) des Rückstands wurden 416 mg (0,85 mmol, 67%) der Beispielverbindung 24 erhalten. MS: m/z 482,2 $[M+H]^+$.

**Synthese von Beispielverbindung 34: (R)-3-Methyl-4-oxo-4-(4-phenyl-6,7-dihydrothieno[3,2-c]pyridin-5(4H)-yl)-N-(3-(trifluormethyl)benzyl)butanamid**

Zu einer Lösung von 515 mg (1,56 mmol) Zwischenprodukt SAAS02 in THF (12 ml) wurden nacheinander 274 mg (1,56 mmol) (3-(Trifluormethyl)phenyl)methylamin, 594 mg (1,56 mmol) HATU und 433 µl (3,13 mmol) $NEt_3$ gegeben. Nach 72 h Rühren bei RT wurde die Reaktionslösung mit EE verdünnt und je dreimal mit einer ges. aq. $NH_4Cl$-Lsg. und einer ges. aq. $NaHCO_3$-Lsg gewaschen. Die organische Phase wurde über $MgSO_4$ getrocknet, filtriert und im Vakuum eingeengt. Nach SC(EE) des Rückstands wurden 550 mg (1,13 mmol, 72%) der Beispielverbindung 34 erhalten. MS: m/z 487,2 $[M+H]^+$.

**Synthese der Beispielverbindung 35: (-)-4-Oxo-4-(4-phenyl-6,7-dihydrothieno[3,2-c]pyridin-5(4H)-yl)-N-(3-(trifluormethyl)benzyl)butanamid und der Beispielverbindung 36: (+)-4-Oxo-4-(4-phenyl-6,7-dihydrothieno[3,2-c]pyridin-5(4H)-yl)-N-(3-(trifluormethyl)benzyl)butanamid**

210 mg racemisches 4-Oxo-4-(4-phenyl-6,7-dihydrothieno[3,2-c]pyridin-5(4H)-yl)-N-(3-(trifluormethyl)benzyl)-butanamid (Synthese siehe Beispiel 2) wurden mittels chiraler HPLC (Säule: Chiralpak AD-H, 250 x 4,6 mm, Eluent: Ethanol + 0,1% Diethylamin, Flussrate 1ml/min) in die beiden (R)- und (S)-Enantiomere aufgetrennt. Dabei wurden 92 mg Beispielverbindung 35, Retentionszeit 14,24 min, Drehwert: $[\alpha]_D^{25}$ . -189,9° (MeOH, c=1,00), MS: m/z 473,1 $[M+H]^+$ und 90 mg Beispielverbindung 36, Retentionszeit 21,28 min, Drehwert: $[\alpha]_D^{25}$ . +194,3° (MeOH, c=1,00), MS: m/z 473,1 $[M+H]^+$ erhalten.

**Synthese von Beispielverbindung 44: 4-Oxo-4-(5-phenyl-4,5-dihydrothieno[2,3-c]pyridin-6(7H)-yl)-N-(3-(trifluormethyl)benzyl)butanamid**

Eine Lösung von 1,62 g (5,9 mmol) Zwischenprodukt PAAS01 in DCM (30 ml) wurde bei 0 °C mit 1,71 g (8,9 mmol) EDC, 797 mg (5,9 mmol) HOBt und 4,0 ml (23,6 mmol) DIPEA versetzt und anschließend 20 min bei 0 °C gerührt. Bei dieser Temperatur wurden 1,27 g (5,9 mmol) Zwischenprodukt SAMN10 zugegeben und anschließend wurde 16 h bei RT gerührt. Danach wurde mit DCM verdünnt und nacheinander mit einer ges. aq. $NH_4Cl$-Lsg, Brine, einer ges. aq. $Na_2CO_3$-Lsg und Brine gewaschen. Die organische Phase wurde über $Na_2SO_4$ getrocknet, filtriert und im Vakuum eingeengt. Nach SC (Hexane / EE 4:1) des Rückstands wurden 2,28 g (4,8 mmol, 82%) Beispielverbindung 44 erhalten. MS: m/z 473,1 $[M+H]^+$.

**Automatisierte Synthese der Beispielverbindungen 46 bis 243:**

In einem Gewindeglas mit Magnetrührstäbchen und Septumkappe wurde eine 0,05 M Lösung der jeweils eingesetzten Carbonsäure (100 μmol) in DCM (2 ml), mit einer 0,105 M Lösung von CDI (105 μmol) in DCM (1 ml) versetzt. Nach 1 h Rühren bei RT wurde eine 0,10 M Lösung des jeweils eingesetzten Amins (100 μmol) in DCM (1ml) hinzu pipettiert. Anschließend wurde weitere 16 h bei RT gerührt. Dann wurde Wasser (3 ml) zugesetzt und die Phasen wurden 30 min im Spin-Reaktor durchmischt. Das Magnetrührstäbchen wurde abgetrennt und das Gewindeglas wurde mit DCM (2 x 1,25 ml) nachgespült. Die Phasen wurden durch Abnahme der wässrigen Phase aus dem Quellgefäß getrennt (Allex-System der Firma Mettler-Toledo). Anschließend wurde nochmals Wasser (3 ml) zugesetzt, durchmischt und die Phasen wie beschrieben getrennt. Dieser Vorgang wurde mit Brine (2,5 ml) wiederholt. Dann wurde die organische Phase in ein Reagenzglas überführt und im Vakuum eingeengt (Evaporator der Firma Genevac). Der erhaltene Rückstand wurde mittels HPLC aufgereinigt.

**Synthese weiterer Beispielverbindungen**

Die Synthese weiterer Beispielverbindungen erfolgte nach den bereits beschriebenen Verfahren. In Tabelle 2 ist angegeben, welche Verbindung nach welchem Verfahren hergestellt wurden. Dem Fachmann ist dabei ersichtlich, welche Edukte jeweils eingesetzt wurden.

**Tabelle 2**

| Beispielverbindung | Chemische Bezeichnung | Herstellung analog Beispiel | Ausbeute [%] | Ms m/z $[M+H]^+$ |
|---|---|---|---|---|
| **1** | 4-Oxo-4-(4-(thiophen-2-yl)-6,7-dihydrothieno[3,2-c]pyridin-5 (4H)-yl)-N-(3-(trifluormethyl) benzyl)butanamid | 2 | 77 | 479,1 |
| **4** | 4-Oxo-4-(4-m-tolyl-6,7-dihydrothieno[3,2-c]pyridin-5 (4H)-yl)-N-(3-(trifluormethyl) benzyl)butanamid | 9 | 67 | 487,2 |
| **5** | 4-Oxo-4-(4-p-tolyl-6,7-dihydrothieno[3,2-c]pyridin-5 (4H)-yl)-N-(3-(trifluormethyl) benzyl)butanamid | 9 | 61 | 487,2 |
| **6** | 4-(4-(2-Fluorphenyl)-6,7-dihydrothieno[3,2-c]pyridin-5 (4H)-yl)-4-oxo-N-(3-(trifluormethyl)benzyl) butanamid | 3 | 59 | 491,1 |
| **7** | 4-(4-(4-Fluorphenyl)-6,7-dihydrothieno[3,2-c]pyridin-5 (4H)-yl)-4-oxo-N-(3-(trifluormethyl)benzyl) butanamid | 9 | 65 | 491,1 |

(fortgesetzt)

| Beispielverbindung | Chemische Bezeichnung | Herstellung analog Beispiel | Ausbeute [%] | Ms m/z $[M+H]^+$ |
|---|---|---|---|---|
| **8** | 4-Oxo-4-(4-o-tolyl-6,7-dihydrothieno[3,2-c]pyridin-5(4H)-yl)-N-(3-(trifluormethyl)benzyl)butanamid | 9 | 46 | 487,2 |
| **10** | 4-(2-Methyl-4-phenyl-6,7-dihydrothieno[3,2-c]pyridin-5(4H)-yl)-4-oxo-N-(3-(trifluormethyl)benzyl)butanamid | 9 | 60 | 487,2 |
| **13** | 4-Oxo-4-(4-phenyl-6,7-dihydrothieno[3,2-c]pyridin-5(4H)-yl)-N-(4-(trifluormethyl)benzyl)butanamid | 12 | 56 | 473,1 |
| **14** | N-(2-Methoxybenzyl)-4-oxo-4-(4-phenyl-6,7-dihydrothieno[3,2-c]pyridin-5(4H)-yl)butanamid | 12 | 61 | 435,2 |
| **15** | N-(3-Methoxybenzyl)-4-oxo-4-(4-phenyl-6,7-dihydrothieno[3,2-c]pyridin-5(4H)-yl)butanamid | 12 | 52 | 435,2 |
| **16** | N-(4-Methoxybenzyl)-4-oxo-4-(4-phenyl-6,7-dihydrothieno[3,2-c]pyridin-5(4H)-yl)butanamid | 12 | 82 | 435,2 |
| **17** | 4-Oxo-4-(4-phenyl-6,7-dihydrothieno[3,2-c]pyridin-5(4H)-yl)-N-(2-(trifluormethyl)benzyl)butanamid | 12 | 33 | 473,1 |
| **18** | N-(3-Fluorbenzyl)-4-oxo-4-(4-phenyl-6,7-dihydrothieno[3,2-c]pyridin-5(4H)-yl)butanamid | 12 | 53 | 423,1 |
| **19** | N-(3-Methylbenzyl)-4-oxo-4-(4-phenyl-6,7-dihydrothieno[3,2-c]pyridin-5(4H)-yl)butanamid | 12 | 49 | 419,2 |
| **20** | 4-(3-Methyl-4-phenyl-6,7-dihydrothieno[3,2-c]pyridin-5(4H)-yl)-4-oxo-N-(3-(trifluormethyl)benzyl)butanamid | 9 | 23 | 487,2 |
| **21** | 4-(4-Cyclohexyl-6,7-dihydrothieno[3,2-c]pyridin-5(4H)-yl)-4-oxo-N-(3-(trifluormethyl)benzyl)butanamid | 9 | 42 | 479,2 |

(fortgesetzt)

| Beispielverbindung | Chemische Bezeichnung | Herstellung analog Beispiel | Ausbeute [%] | Ms m/z [M+H]+ |
|---|---|---|---|---|
| **22** | 4-(4-Isopropyl-6,7-dihydrothieno[3,2-c]pyridin-5 (4H)-yl)-4-oxo-N-(3-(trifluormethyl)benzyl) butanamid | 9 | 58 | 439,2 |
| **23** | 4-(4-Butyl-6,7-dihydrothieno [3,2-c]pyridin-5(4H)-yl)-4-oxo-N-(3-(trifluormethyl)benzyl) butanamid | 2 | 59 | 453,2 |
| **25** | 4-Oxo-N-phenethyl-4-(4-phenyl-6,7-dihydrothieno[3,2-c]pyridin-5(4H)-yl)butanamid | 12 | 59 | 419,2 |
| **26** | N-(2-Methylbenzyl)-4-oxo-4-(4-phenyl-6,7-dihydrothieno[3,2-c] pyridin-5(4H)-yl)butanamid | 12 | 57 | 419,2 |
| **27** | 4-Oxo-2-phenyl-4-(4-phenyl-6,7-4-Oxo-2-phenyl-4-(4-phenyl-6,7-dihydrothieno[3,2-c] pyridin-5 (4H)-yl)-N-(3-(trifluormethyl) benzyl)butanamid | 9 | 55 | 549,2 |
| **28** | (R)-2-Methyl-4-oxo-4-(4-phenyl-6,7-dihydrothieno[3,2-c]pyridin-5(4H)-yl)-N-(3-(trifluormethyl) benzyl)butanamid | 2 | 88 | 487,2 |
| **29** | 4-Oxo-3-phenyl-4-(4-phenyl-6,7-dihydrothieno[3,2-c]pyridin-5(4H)-yl)-N-(3-(trifluormethyl) benzyl)butanamid | 2 | 68 | 549,2 |
| **30** | 4-(6,7-Dihydrothieno[3,2-c] pyridin-5(4H)-yl)-4-oxo-3-phenyl-N-(3-(trifluormethyl) benzyl)butanamid | 2 | 65 | 473,1 |
| **31** | rac. (1S,2R)-2-(4-Phenyl-4,5,6,7-tetrahydrothieno[3,2-c] pyridin-5-carbonyl)-N-(3-(trifluormethyl) benzyl)cyclohexancarboxamid | 2 | 61 | 527,2 |
| **32** | rac. (1S)-2-(4-Phenyl-4,5,6,7-rac. (1S)-2-(4-Phenyl-4,5,6,7-tetrahydrothieno[3,2-c]pyridin-5-carbonyl)-N-(3-(trifluormethyl) benzyl)cyclohexancarboxamid | 2 | 66 | 527,2 |

(fortgesetzt)

| Beispielverbindung | Chemische Bezeichnung | Herstellung analog Beispiel | Ausbeute [%] | Ms m/z [M+H]$^+$ |
|---|---|---|---|---|
| **33** | rac. (1S,2R)-2-(4-Phenyl-4,5,6,7-rac. (1S,2R)-2-(4-Phenyl-4,5,6,7-tetrahydrothieno [3,2-c]pyridin-5-carbonyl)-N-(3-(trifluormethyl) benzyl) cyclopentanecarboxamid | 2 | 21 | 513,2 |
| **37** | 4-(6,7-Dihydrothieno[3,2-c] pyridin-5(4H)-yl)-4-oxo-2-phenyl-N-(3-(trifluormethyl) benzyl)butanamid | 2 | 69 | 473,1 |
| **38** | N-(3,5-Bis(trifluormethyl) benzyl)-4-oxo-4-(4-phenyl-6,7-dihydrothieno[3,2-c]pyridin-5 (4H)-yl)butanamid | 34 | 89 | 541,1 |
| **39** | N-(2-Fluor-5-(trifluormethyl) benzyl)-4-oxo-4-(4-phenyl-6,7-dihydrothieno[3,2-c]pyridin-5 (4H)-yl)butanamid | 11 | 63 | 491,1 |
| **40** | N-(2-Fluor-3-(trifluormethyl) benzyl)-4-oxo-4-(4-phenyl-6,7-dihydrothieno[3,2-c]pyridin-5 (4H)-yl)butanamid | 34 | 38 | 491,1 |
| **41** | N-(3-Fluor-5-(trifluormethyl) benzyl)-4-oxo-4-(4-phenyl-6,7-dihydrothieno[3,2-c]pyridin-5 (4H)-yl)butanamid | 34 | 81 | 491,1 |
| **42** | rac. (1S,2R)-2-(4-Phenyl-4,5,6,7-tetrahydrothieno[3,2-c] pyridin-5-carbonyl)-N-(3-(trifluormethyl) benzyl)cyclopropancarboxamid | 9 | 54 | 485,1 |
| **43** | N-(3,4-Difluorbenzyl)-4-oxo-4-(4-phenyl-6,7-dihydrothieno [3,2-c]pyridin-5(4H)-yl) butanamid | 9 | 75 | 441,1 |
| **45** | 4-(1-Methyl-4-phenyl-6,7-dihydrothieno[3,4-c]pyridin-5 (4H)-yl)-4-oxo-N-(3-(trifluormethyl)benzyl) butanamid | 44 | 42 | 487,2 |
| **Beispiel-verbindung** | **Chemische Bezeichnung** | **Herstellung analog Beispiel** | **Ausbeute [%]** | **MS m/z [M+H]$^+$** |
| **46** | N-(4-Methoxyphenyl)-4-oxo-4-(4-p-tolyl-6,7-dihydrothieno [3,2-c]pyridin-5(4H)-yl) butanamid | 46-243 | N/A | 435,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **47** | N-(1-Methyl-1H-indazol-6-yl)-4-oxo-4-(4-p-tolyl-6,7-dihydrothieno[3,2-c]pyridin-5(4H)-yl)butanamid | 46-243 | N/A | 459,2 |
| **48** | N-Benzyl-4-oxo-4-(4-p-tolyl-6,7-dihydrothieno[3,2-c]pyridin-5(4H)-yl)butanamid | 46-243 | N/A | 419,2 |
| **49** | 4-Oxo-N-phenethyl-4-(4-p-tolyl-6,7-dihydrothieno[3,2-c]pyridin-5(4H)-yl)butanamid | 46-243 | N/A | 433,2 |
| **50** | 4-Oxo-N-(pyridin-4-ylmethyl)-4-(4-p-tolyl-6,7-dihydrothieno[3,2-c]pyridin-5(4H)-yl)butanamid | 46-243 | N/A | 420,2 |
| **51** | 4-Oxo-N-(3-phenylpropyl)-4-(4-p-tolyl-6,7-dihydrothieno[3,2-c]pyridin-5(4H)-yl)butanamid | 46-243 | N/A | 447,2 |
| **52** | N-(Benzo[c][1,2,5]thiadiazol-4-yl)-4-oxo-4-(4-m-tolyl-6,7-dihydrothieno[3,2-c]pyridin-5(4H)-yl)butanamid | 46-243 | N/A | 463,1 |
| **53** | N-(1-Methyl-1H-indazol-6-yl)-4-oxo-4-(4-m-tolyl-6,7-dihydrothieno[3,2-c]pyridin-5(4H)-yl)butanamid | 46-243 | N/A | 459,2 |
| **54** | 4-Oxo-N-(pyridin-2-ylmethyl)-4-(4-m-tolyl-6,7-dihydrothieno[3,2-c]pyridin-5(4H)-yl)butanamid | 46-243 | N/A | 420,2 |
| **55** | 1-(4-Methylpiperazin-1-yl)-4-(4-m-tolyl-6,7-dihydrothieno[3,2-c]pyridin-5(4H)-yl)butan-1,4-dion | 46-243 | N/A | 412,2 |
| **56** | 3-Methyl-4-oxo-4-(4-phenyl-6,7-dihydrothieno[3,2-c]pyridin-5(4H)-yl)-N-(pyridin-2-ylmethyl)butanamid | 46-243 | N/A | 420,2 |
| **57** | 4-Oxo-4-(4-phenyl-6,7-dihydrothieno[3,2-c]pyridin-5(4H)-yl)-N-(thiophen-2-ylmethyl)butanamid | 46-243 | N/A | 411,1 |
| **58** | N-(2-Chlorphenyl)-4-(4-(4-fluorphenyl)-6,7-dihydrothieno[3,2-c]pyridin-5(4H)-yl)-4-oxobutanamid | 46-243 | N/A | 443,1 |
| **59** | 4-(4-(4-Chlorphenyl)-6,7-dihydrothieno[3,2-c]pyridin-5(4H)-yl)-N-(furan-2-ylmethyl)-4-oxobutanamid | 46-243 | N/A | 429,1 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **60** | 4-(4-(4-Chlorphenyl)-6,7-dihydrothieno[3,2-c]pyridin-5(4H)-yl)-4-oxo-N-propylbutanamid | 46-243 | N/A | 391,1 |
| **61** | N-(2-Chlorbenzyl)-4-oxo-4-(4-m-tolyl-6,7-dihydrothieno[3,2-c]pyridin-5(4H)-yl)butanamid | 46-243 | N/A | 453,1 |
| **62** | N-(2,4-Dichlorbenzyl)-4-(4-(3-fluorphenyl)-6,7-dihydrothieno[3,2-c]pyridin-5(4H)-yl)-4-oxobutanamid | 46-243 | N/A | 491,1 |
| **63** | N-(4-Fluorbenzyl)-4-(4-(3-fluorphenyl)-6,7-dihydrothieno[3,2-c]pyridin-5(4H)-yl)-4-oxobutanamid | 46-243 | N/A | 441,1 |
| **64** | N-(3,4-Dichlorbenzyl)-3-methyl-4-oxo-4-(4-phenyl-6,7-dihydrothieno[3,2-c]pyridin-5(4H)-yl)butanamid | 46-243 | N/A | 487,1 |
| **65** | N-(2,5-Difluorbenzyl)-3-methyl-4-oxo-4-(4-phenyl-6,7-dihydrothieno[3,2-c]pyridin-5(4H)-yl)butanamid | 46-243 | N/A | 455,2 |
| **66** | 3-Methyl-4-oxo-4-(4-phenyl-6,7-dihydrothieno[3,2-c]pyridin-5(4H)-yl)-N-(3-(trifluormethyl)benzyl)butanamid | 46-243 | N/A | 487,2 |
| **67** | 1-(4-(4-Chlorphenyl)-6,7-dihydrothieno[3,2-c]pyridin-5(4H)-yl)-4-morpholinobutan-1,4-dion | 46-243 | N/A | 419,1 |
| **68** | 1-(4-(4-Chlorphenyl)-6,7-dihydrothieno[3,2-c]pyridin-5(4H)-yl)-4-(4-(4-fluorphenyl)piperazin-1-yl)butan-1,4-dion | 46-243 | N/A | 512,1 |
| **69** | 2-Methyl-N-(2-(5-methyl-1H-pyrazol-1-yl)ethyl)-4-oxo-4-(4-phenyl-6,7-dihydrothieno[3,2-c]pyridin-5(4H)-yl)butanamid | 46-243 | N/A | 437,2 |
| **70** | N-(Naphthalen-1-ylmethyl)-4-oxo-4-(4-phenyl-6,7-dihydrothieno[3,2-c]pyridin-5(4H)-yl)butanamid | 46-243 | N/A | 455,2 |
| **71** | 4-(4-(4-Fluorphenyl)-6,7-dihydrothieno[3,2-c]pyridin-5(4H)-yl)-N-(4-methylbenzyl)-4-oxobutanamid | 46-243 | N/A | 437,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **72** | N-(Benzo[d][1,3]dioxol-5-ylmethyl)-4-(4-(4-fluorphenyl)-6,7-dihydrothieno[3,2-c]pyridin-5(4H)-yl)-4-oxobutanamid | 46-243 | N/A | 467,1 |
| **73** | 4-(4-(4-Fluorphenyl)-6,7-dihydrothieno[3,2-c]pyridin-5 (4H)-yl)-4-oxo-N-(2-(trifluormethyl)benzyl) butanamid | 46-243 | N/A | 491,1 |
| **74** | 4-(4-(4-Fluorphenyl)-6,7-dihydrothieno[3,2-c]pyridin-5 (4H)-yl)-N-(naphthalen-1-ylmethyl)-4-oxobutanamid | 46-243 | N/A | 473,2 |
| **75** | 4-(4-(4-Chlorphenyl)-6,7-dihydrothieno[3,2-c]pyridin-5 (4H)-yl)-N-(3-fluorbenzyl)-4-oxobutanamid | 46-243 | N/A | 457,1 |
| **76** | 2-Methyl-N-(1-methyl-1H-indazol-6-yl)-4-oxo-4-(4-phenyl-6,7-dihydrothieno[3,2-c]pyridin-5(4H)-yl)butanamid | 46-243 | N/A | 459,2 |
| **77** | N-(4-Methoxybenzyl)-2-methyl-4-oxo-4-(4-phenyl-6,7-dihydrothieno[3,2-c]pyridin-5 (4H)-yl)butanamid | 46-243 | N/A | 449,2 |
| **78** | 2-Methyl-1-(4-methylpiperazin-1-yl)-4-(4-phenyl-6,7-dihydrothieno[3,2-c]pyridin-5 (4H)-yl)butan-1,4-dion | 46-243 | N/A | 412,2 |
| **79** | N-(2-(1H-Indol-3-yl)ethyl)-2-methyl-4-oxo-4-(4-phenyl-6,7-dihydrothieno[3,2-c]pyridin-5 (4H)-yl)butanamid | 46-243 | N/A | 472,2 |
| **80** | N-(2-Fluorbenzyl)-2-methyl-4-oxo-4-(4-phenyl-6,7-dihydrothieno[3,2-c]pyridin-5 (4H)-yl)butanamid | 46-243 | N/A | 437,2 |
| **81** | 2-Methyl-4-oxo-4-(4-phenyl-6,7-dihydrothieno[3,2-c]pyridin-5 (4H)-yl)-N-(2-(trifluormethyl) benzyl)butanamid | 46-243 | N/A | 487,2 |
| **82** | 4-Oxo-N-(2-(piperidin-1-yl) ethyl)-4-(4-p-tolyl-6,7-dihydrothieno[3,2-c]pyridin-5 (4H)-yl)butanamid | 46-243 | N/A | 440,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **83** | 4-Oxo-N-((tetrahydrofuran-2-yl) methyl)-4-(4-p-tolyl-6,7-dihydrothieno[3,2-c]pyridin-5 (4H)-yl)butanamid | 46-243 | N/A | 413,2 |
| **84** | N-(4-Chlorbenzyl)-4-oxo-4-(4-p-tolyl-6,7-dihydrothieno[3,2-c] pyridin-5(4H)-yl)butanamid | 46-243 | N/A | 453,1 |
| **85** | N-(2,3-Dichlorbenzyl)-4-oxo-4-(4-p-tolyl-6,7-dihydrothieno [3,2-c]pyridin-5(4H)-yl) butanamid | 46-243 | N/A | 487,1 |
| **86** | 4-Oxo-N-(2-(thiophen-2-yl) ethyl)-4-(4-m-tolyl-6,7-dihydrothieno[3,2-c]pyridin-5 (4H)-yl)butanamid | 46-243 | N/A | 439,1 |
| **87** | N-(Cyclohexylmethyl)-4-oxo-4-(4-m-tolyl-6,7-dihydrothieno [3,2-c]pyridin-5(4H)-yl) butanamid | 46-243 | N/A | 425,2 |
| **88** | N-(3-Chlorphenethyl)-4-oxo-4-(4-m-tolyl-6,7-dihydrothieno [3,2.c]pyridin-5(4H)-yl) butanamid | 46-243 | N/A | 467,1 |
| **89** | N-(3,3-Diphenylpropyl)-4-oxo-4-(4-m-tolyl-6,7-dihydrothieno [3,2-c]pyridin-5(4H)-yl) butanamid | 46-243 | N/A | 523,2 |
| **90** | 4-(4-(3-Fluorphenyl)-6,7-dihydrothieno[3,2-c]pyridin-5 (4H)-yl)-N-(3-morpholinopropyl)-4-oxobutanamid | 46-243 | N/A | 460,2 |
| **91** | 4-(4-(3-Fluorphenyl)-6,7-dihydrothieno[3,2-c]pyridin-5 (4H)-yl)-4-oxo-N-(pyridin-3-ylmethyl)butanamid | 46-243 | N/A | 424,1 |
| **92** | 4-(4-(3-Fluorphenyl)-6,7-dihydrothieno[3,2-c]pyridin-5 (4H)-yl)-N-(3-methylbenzyl)-4-oxobutanamid | 46-243 | N/A | 437,2 |
| **93** | 4-(4-(3-Fluorphenyl)-6,7-dihydrothieno[3,2-c]pyridin-5 (4H)-yl)-N-(4-methylphenethyl)-4-oxobutanamid | 46-243 | N/A | 451,2 |
| **94** | 3-Methyl-4-oxo-4-(4-phenyl-6,7-dihydrothieno[3,2-c]pyridin-5 (4H)-yl)-N-(4-phenylbutyl) butanamid | 46-243 | N/A | 461,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **95** | N-(Biphenyl-4-ylmethyl)-4-oxo-4-(4-m-totyl-6,7-dihydrothieno [3,2-c]pyridin-5(4H)-yl) butanamid | 46-243 | N/A | 495,2 |
| **96** | 4-(4-(3-Fluorphenyl)-6,7-dihydrothieno[3,2-c]pyridin-5 (4H)-yl)-N-(2-methoxybenzyl)-4-oxobutanamid | 46-243 | N/A | 453,2 |
| **97** | N-(4-Chlorphenethyl)-4-(4-(3-fluorphenyl)-6,7-dihydrothieno [3,2-c]pyridin-5(4H)-yl)-4-oxobutanamid | 46-243 | N/A | 471,1 |
| **98** | 4-(4-(3-Fluorphenyl)-6,7-dihydrothieno[3,2-c]pyridin-5 (4H)-yl)-N-((5-methyl-3-phenylisoxazol-4-yl)methyl)-4-oxobutanamid | 46-243 | N/A | 504,2 |
| **99** | N-(2,6-Difluorbenzyl)-3-methyl-4-oxo-4-(4-phenyl-6,7-dihydrothieno[3,2-c]pyridin-5 (4H)-yl)butanamid | 46-243 | N/A | 455,2 |
| **100** | N-(3,5-Difluorbenzyl)-3-methyl-4-oxo-4-(4-phenyl-6,7-dihydrothieno[3,2-c]pyridin-5 (4H)-yl)butanamid | 46-243 | N/A | 455,2 |
| **101** | N-(3-Chlorbenzyl)-3-methyl-4-oxo-4-(4-phenyl-6,7-dihydrothieno[3,2-c]pyridin-5 (4H)-yl)butanamid | 46-243 | N/A | 453,1 |
| **102** | N-(3,5-Dimethoxyphenethyl)-3-methyl-4-oxo-4-(4-phenyl-6,7-dihydrothieno[3,2.c]pyridin-5 (4H)-yl)butanamid | 46-243 | N/A | 493,2 |
| **103** | N-(3,4-Difluorbenzyl)-4-oxo-4-(4-phenyl-6,7-dihydrothieno [3,2-c]pyridin-5(4H)-yl) butanamid | 46-243 | N/A | 441,1 |
| **104** | N-(2,4-Dichlorphenethyl)-4-oxo-4-(4-phenyl-6,7-dihydrothieno [3,2-c]pyridin-5(4H)-yl) butanamid | 46-243 | N/A | 487,1 |
| **105** | N-(2-(1H-1,2,4-Triazol-1-yl) ethyl)-4-oxo-4-(4-phenyl-6,7-dihydrothieno[3,2-c]pyridin-5 (4H)-yl)butanamid | 46-243 | N/A | 410,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **106** | 4-(4-(4-Fluorphenyl)-6,7-dihydrothieno[3,2-c]pyridin-5 (4H)-yl)-N-(2-methylbenzyl)-4-oxobutanamid | 46-243 | N/A | 437,2 |
| **107** | N-(4-Fluorphenethyl)-4-(4-(4-fluorphenyl)-6,7-dihydrothieno [3,2-c]pyridin-5(4H)-yl)-4-oxobutanamid | 46-243 | N/A | 455,2 |
| **108** | 4-(4-(4-Fluorphenyl)-6,7-dihydrothieno[3,2-c]pyridin-5 (4H)-yl)-N-((5-methylisoxazol-3-yl)methyl)-4-oxobutanamid | 46-243 | N/A | 428,1 |
| **109** | N-(2-Chlorphenethyl)-4-(4-(4-fluorphenyl)-6,7-dihydrothieno [3,2-c]pyridin-5(4H)-yl)-4-oxobutanamid | 46-243 | N/A | 471,1 |
| **110** | 4-(4-(4-Chlorphenyl)-6,7-dihydrothieno[3,2-c]pyridin-5 (4H)-yl)-N-(2-cyclohexenylethyl)-4-oxobutanamid | 46-243 | N/A | 457,2 |
| **111** | 4-(4-(4-Chlorphenyl)-6,7-dihydrothieno[3,2-c]pyridin-5 (4H)-yl)-N-(3,5-dimethoxybenzyl)-4-oxobutanamid | 46-243 | N/A | 499,1 |
| **112** | 4-(4-(4-Chlorphenyl)-6,7-dihydrothieno[3,2-c]pyridin-5 (4H)-yl)-N-(3,4-dichlorphenethyl)-4-oxobutanamid | 46-243 | N/A | 521,1 |
| **113** | 4-(4-(4-Chlorphenyl)-6,7-dihydrothieno[3,2-c]pyridin-5 (4H)-yl)-N-(3-fluorphenethyl)-4-oxobutanamid | 46-243 | N/A | 471,1 |
| **114** | 1-(3-Phenylpiperidin-1-yl)-4-(4-p-tolyl-6,7-dihydrothieno[3,2-c] pyridin-5(4H)-yl)butan-1,4-dion | 46-243 | N/A | 473,2 |
| **115** | 1-(4-Benzylpiperazin-1-yl)-4-(4-p-tolyl-6,7-dihydrothieno[3,2-c] pyridin-5(4H)-yl)butan-1,4-dion | 46-243 | N/A | 488,2 |
| **116** | 1-(4-(4-Methoxyphenyl) piperazin-1-yl)-4-(4-p-tolyl-6,7-dihydrothieno[3,2-c]pyridin-5 (4H)-yl)butan-1,4-dion | 46-243 | N/A | 504,2 |
| **117** | 1-(2-Benzylpiperidin-1-yl)-4-(4-m-tolyl-6,7-dihydrothieno[3,2-c] pyridin-5(4H)-yl)butan-1,4-dion | 46-243 | N/A | 487,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **118** | 1-(4-(2-Fluorphenyl)piperazin-1-yl)-4-(4-m-tolyl-6,7-dihydrothieno[3,2-c]pyridin-5(4H)-yl)butan-1,4-dion | 46-243 | N/A | 492,2 |
| **119** | 1-(4-(Cyclohexylmethyl) piperazin-1-yl)-4-(4-m-tolyl-6,7-dihydrothieno[3,2-c]pyridin-5(4H)-yl)butan-1,4-dion | 46-243 | N/A | 494,3 |
| **120** | N-(3-(1H-Imidazol-1-yl)propyl)-4-(4-(3-fluorphenyl)-6,7-dihydrothieno[3,2-c]pyridin-5(4H)-yl)-4-oxobutanamid | 46-243 | N/A | 441,2 |
| **121** | 1-(4-(3-Fluorphenyl)-6,7-dihydrothieno[3,2-c]pyridin-5(4H)-yl)-4-(3-phenylpyrrolidin-1-yl)butan-1,4-dion | 46-243 | N/A | 463,2 |
| **122** | 1-(4-(3-Fluorphenyl)-6,7-dihydrothieno[3,2-c]pyridin-5(4H)-yl)-4-(4-(furan-2-carbonyl) piperazin-1-yl)butan-1,4-dion | 46-243 | N/A | 496,2 |
| **123** | N-(3-(3,4-Dihydroquinolin-1(2H)-yl)propyl)-3-methyl-4-oxo-4-(4-phenyl-6,7-dihydrothieno[3,2-c]pyridin-5(4H)-yl) butanamid | 46-243 | N/A | 502,2 |
| **124** | 2-Methyl-1-(4-phenyl-6,7-dihydrothieno[3,2-c]pyridin-5(4H)-yl)-4-(piperidin-1-yl)butan-1,4-dion | 46-243 | N/A | 397,2 |
| **125** | 4-(4-(2-Methoxyphenyl) piperazin-1-yl)-2-methyl-1-(4-phenyl-6,7-dihydrothieno[3,2-c] pyridin-5(4H)-yl)butan-1,4-dion | 46-243 | N/A | 504,2 |
| **126** | 4-Oxo-4-(4-phenyl-6,7-dihydrothieno[3,2-c]pyridin-5(4H)-yl)-N-(2-(pyrazin-2-yl)ethyl) butanamid | 46-243 | N/A | 421,2 |
| **127** | N,N-Diethyl-4-oxo-4-(4-phenyl-6,7-dihydrothieno[3,2-c]pyridin-5(4H)-yl)butanamid | 46-243 | N/A | 371,2 |
| **128** | 1-(4-Phenyl-6,7-dihydrothieno[3,2-c]pyridin-5(4H)-yl)-4-(4-(pyridin-2-yl)piperazin-1-yl) butan-1,4-dion | 46-243 | N/A | 461,2 |
| **129** | 1-(4-(4-Fluorphenyl)-6,7-dihydrothieno[3,2-c]pyridin-5(4H)-yl)-4-(4-(3-methoxyphenyl) piperazin-1-yl)butan-1,4-dion | 46-243 | N/A | 508,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **130** | 1-(4-Isopropylpiperazin-1-yl)-4-(4-p-tolyl-6,7-dihydrothieno [3,2-c]pyridin-5(4H)-yl)butan-1,4-dion | 46-243 | N/A | 440,2 |
| **131** | 1-(4-p-Tolyl-6,7-dihydrothieno [3,2-c]pyridin-5(4H)-yl)-4-(4-(3-(trifluormethyl)phenyl) piperazin-1-yl)butan-1,4-dion | 46-243 | N/A | 542,2 |
| **132** | 1-(4-Phenylpiperazin-1-yl)-4-(4-m-tolyl-6,7, dihydrothieno[3,2-c] pyridin-5(4H)-yl)butan-1,4-dion | 46-243 | N/A | 474,2 |
| **133** | 1-(3,5-Dimethylpiperidin-1-yl)-4-(4-m-tolyl-6,7-dihydrothieno [3,2-c]pyridin-5(4H)-yl)butan-1,4-dion | 46-243 | N/A | 425,2 |
| **134** | 1-(5,6-Dihydropyridin-1(2H)-yl)-4-(4-(3-fluorphenyl)-6,7-dihydrothieno[3,2-c]pyridin-5 (4H)-yl)butan-1,4-dion | 46-243 | N/A | 399,1 |
| **135** | 1-(4-(3-Fluorphenyl)-6,7-dihydrothieno[3,2-c)pyridin-5 (4H)-yl)-4-(4-(pyrimidin-2-yl) piperazin-1-yl)butan-1,4-dion | 46-243 | N/A | 480,2 |
| **136** | 1-(4-(3-Fluorphenyl)-6,7-dihydrothieno[3,2-c]pyridin-5 (4H)-yl)-4-(3-methylpiperidin-1-yl)butan-1,4-dion | 46-243 | N/A | 415,2 |
| **137** | N-Ethyl-4-(4-(3-fluorphenyl)-6,7-dihydrothieno[3,2-c]pyridin-5(4H)-yl)-4-oxo-N-(pyridin-4-ylmethyl)butanamid | 46-243 | N/A | 452,2 |
| **138** | 4-(4-Acetylpiperazin-1-yl)-2-methyl-1-(4-phenyl-6,7-dihydrothieno[3,2-c]pyridin-5 (4H)-yl)butan-1,4-dion | 46-243 | N/A | 440,2 |
| **139** | 4-(2,6-Dimethylmorpholino)-2-methyl-1-(4-phenyl-6,7-dihydrothieno[3,2-c]pyridin-5 (4H)-yl)butan-1,4-dion | 46-243 | N/A | 427,2 |
| **140** | 4-(2,5-Dihydro-1H-pyrrol-1-yl)-2-methyl-1-(4-phenyl-6,7-dihydrothieno[3,2-c]pyridin-5 (4H)-yl)butan-1,4-dion | 46-243 | N/A | 381,2 |
| **141** | N-(2-Methoxyphenethyl)-d-oxo-4-(4-phenyl-6,7-dihydrothieno [3,2-c]pyridin-5(4H)-yl) butanamid | 46-243 | N/A | 449,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **142** | 4-(4-(4-Fluorphenyl)-6,7-dihydrothieno[3,2-c]pyridin-5 (4H)-yl)-N-isopentyl-4-oxobutanamid | 46-243 | N/A | 403,2 |
| **143** | N-(2,4-Dimethoxybenzyl)-4-(4-(4-fluorphenyl)-6,7-dihydrothieno[3,2-c]pyridin-5 (4H)-yl)-4-oxobutanamid | 46-243 | N/A | 483,2 |
| **144** | 4-(4-(4-Chlorphenyl)-6,7-dihydrothieno[3,2-c]pyridin-5 (4H)-yl)-N-((5-methylfuran-2-yl) methyl)-4-oxobutanamid | 46-243 | N/A | 443,1 |
| **145** | 4-(4-(4-Chlorphenyl)-6,7-dihydrothieno[3,2-cjpyridin-5 (4H)-yl)-4-oxo-N-pentylbutanamid | 46-243 | N/A | 419,1 |
| **146** | 1-(4-Benzylpiperidin-1-yl)-4-(4-(4-chlorphenyl)-6,7-dihydrothieno[3,2-c]pyridin-5 (4H)-yl)butan-1,4-dion | 46-243 | N/A | 507,2 |
| **147** | 1-(4-(4-Chlorphenyl)-6,7-dihydrothieno[3,2-c]pyridin-5 (4H)-yl)-4-(4-methylpiperidin-1-yl)butan-1,4-dion | 46-243 | N/A | 431,1 |
| **148** | 1-(Azetidin-1-yl)-4-(4-(4-chlorphenyl)-6,7-dihydrothieno [3,2-c]pyridin-5(4H)-yl)butan-1,4-dion | 46-243 | N/A | 389,1 |
| **149** | 1-(3,4-Dihydroisoquinolin-2 (1H)-yl)-2-methyl-4-(4-phenyl-6,7-dihydrothieno[3,2-c]pyridin-5(4H)-yl)butan-1,4-dion | 46-243 | N/A | 445,2 |
| **150** | 1-(4-(Benzo[d][1,3]dioxol-5-ylmethyl)piperazin-1-yl)-2-methyl-4-(4-phenyl-6,7-dihydrothieno[3,2-c]pyridin-5 (4H)-yl)butan-1,4-dion | 46-243 | N/A | 532,2 |
| **151** | N-Benzyl-N-ethyl-4-oxo-4-(4-p-tolyl-6,7-dihydrothieno[3,2-c] pyridin-5(4H)-yl)butanamid | 46-243 | N/A | 447,2 |
| **152** | N-Methyl-4-oxo-N-phenethyl-4-(4-p-tolyl-6,7-dihydrothieno [3,2-c]pyridin-5(4H)-yl) butanamid | 46-243 | N/A | 447,2 |
| **153** | Ethyl 1-(4-oxo-4-(4-p-tolyl-6,7-dihydrothieno[3,2-c]pyridin-5 (4H)-yl)butanoyl)piperidin-4-carboxylat | 46-243 | N/A | 469,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **154** | (E)-1-(4-(3-Phenylprop-2-enyl)-piperazin-1-yl)-4-(4-p-tolyl-6,7-dihydrothieno[3,2-c] pyridin-5(4H)-yl)butan-1,4-dion | 46-243 | N/A | 514,2 |
| **155** | 1-(2-(Thiazol-2-yl)pyrrolidin-1-yl)-4-(4-p-tolyl-6,7-dihydrothieno[3,2-c]pyridin-5 (4H)-yl)butan-1,4-dion | 46-243 | N/A | 466,2 |
| **156** | N-Cyclohexyl-N-ethyl-4-oxo-4-(4-m-tolyl-6,7-dihydrothieno [3,2-c]pyridin-5(4H)-yl) butanamid | 46-243 | N/A | 439,2 |
| **157** | N-Ethyl-N-isopropyl-4-oxo-4-(4-m-tolyl-6,7-dihydrothieno[3,2-c] pyridin-5(4H)-yl)butanamid | 46-243 | N/A | 399,2 |
| **158** | 1-(Pyrrolidin-1-yl)-4-(4-m-tolyl-6,7-dihydrothieno[3,2-c]pyridin-5(4H)-yl)butan-1,4-dion | 46-243 | N/A | 383,2 |
| **159** | 1-(4-(Pyridin-4-yl)piperazin-1-yl)-4-(4-m-tolyl-6,7-dihydrothieno[3,2-c]pyridin-5 (4H)-yl)butan-1,4-dion | 46-243 | N/A | 475,2 |
| **160** | 1-(2-(Pyridin-2-ylmethyl) pyrrolidin-1-yl)-4-(4-m-tolyl-6,7-dihydrothieno[3,2-c]pyridin-5 (4H)-yl)butan-1,4-dion | 46-243 | N/A | 474,2 |
| **161** | Ethyl 1-(4-(4-(4-fluorphenyl)-6,7-dihydrothieno[3,2-c]pyridin-5 (4H)-yl)-4-oxobutanoyl) piperidin-3-carboxylat | 46-243 | N/A | 473,2 |
| **162** | 1-(4-(4-Fluorphenyl)-6,7-dihydrothieno[3,2-c]pyridin-5 (4H)-yl)-4-(4-(4-(trifluormethyl) phenyl)piperazin-1-yl)butan-1,4-dion | 46-243 | N/A | 546,2 |
| **163** | 1-(2-(5-Brompyridin-3-yl) pyrrolidin-1-yl)-4-(4-(4-fluorphenyl)-6,7-dihydrothieno [3,2-c]pyridin-5(4H)-yl)butan-1,4-dion | 46-243 | N/A | 542,1 |
| **164** | 1-(4-Ethylpiperazin-1-yl)-4-(4-phenyl-6,7-dihydrothieno[3,2-c] pyridin-5(4H)-yl)butan-1,4-dion | 46-243 | N/A | 412,2 |
| **165** | 1-(2-Ethylpiperidin-1-yl)-4-(4-phenyl-6,7-dihydrothieno[3,2-c] pyridin-5(4H)-yl)butan-1,4-dion | 46-243 | N/A | 411,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **166** | N,N-Dibenzyl-4-oxo-4-(4-phenyl-6,7-dihydrothieno[3,2-c]pyridin-5(4H)-yl)butanamid | 46-243 | N/A | 495,2 |
| **167** | N,N-Diisobutyl-4-oxo-4-(4-phenyl-6,7-dihydrothieno[3,2-c]pyridin-5(4H)-yl)butanamid | 46-243 | N/A | 427,2 |
| **168** | 1-(3,4-Dihydroquinolin-1(2H)-yl)-4-(4-(4-fluorphenyl)-6,7-dihydrothieno[3,2-c]pyridin-5(4H)-yl)butan-1,4-dion | 46-243 | N/A | 449,2 |
| **169** | 1-(4-(3,4-Dichlorbenzyl)piperazin-1-yl)-4-(4-p-tolyl-6,7-dihydrothieno[3,2-c]pyridin-5(4H)-yl)butan-1,4-dion | 46-243 | N/A | 556,2 |
| **170** | 1-(4-p-Tolyl-6,7-dihydrothieno[3,2-c]pyridin-5(4H)-yl)-4-(4-(2,4,6-trimethylbenzyl)piperazin-1-yl)butan-1,4-dion | 46-243 | N/A | 530,3 |
| **171** | 1-(4-(4-Brombenzyl)piperazin-1-yl)-4-(4-m-tolyl-6,7-dihydrothieno[3,2-c]pyridin-5(4H)-yl)butan-1,4-dion | 46-243 | N/A | 566,1 |
| **172** | 1-(4-(4-Chlorbenzyl)piperazin-1-yl)-4-(4-m-tolyl-6,7-dihydrothieno[3,2-c]pyridin-5(4H)-yl)butan-1,4-dion | 46-243 | N/A | 522,2 |
| **173** | 1-(2-((4,6-Dimethylpyridin-2-yl)methyl)pyrrolidin-1-yl)-4-(4-(3-fluorphenyl)-6,7-dihydrothieno[3,2-c]pyridin-5(4H)-yl)butan-1,4-dion | 46-243 | N/A | 506,2 |
| **174** | 1-(4-(3-Fluorphenyl)-6,7-dihydrothieno[3,2-c]pyridin-5(4H)-yl)-4-(2-((6-methylpyridin-2-yl)methyl)pyrrolidin-1-yl)butan-1,4-dion | 46-243 | N/A | 492,2 |
| **175** | 1-(2-((5-Ethy)pyhdin-2-yl)methyl)pyrrolidin-1-yl)-4-(4-(3-fluorphenyl)-6,7-dihydrothieno[3,2-c]pyridin-5(4H)-yl)butan-1,4-dion | 46-243 | N/A | 506,2 |
| **176** | 1-(4-(3-Fluorphenyl)-6,7-dihydrothieno[3,2-c]pyridin-5(4H)-yl)-4-(2-(6-methoxypyridin-3-yl)piperidin-1-yl)butan-1,4-dion | 46-243 | N/A | 508,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **177** | 4-(4-(3-Fluorphenyl)-6,7-dihydrothieno[3,2-c]pyridin-5 (4H)-yl)-N-methyl-4-oxo-N-(pyridin-3-ylmethyl) butanamid | 46-243 | N/A | 438,2 |
| **178** | N-(2,2-Diphenylethyl)-3-methyl-4-oxo-4-(4-phenyl-6,7-dihydrothieno[3,2-c]pyridin-5 (4H)-yl)butanamid | 46-243 | N/A | 509,2 |
| **179** | N-(Cyclopropylmethyl)-4-oxo-4-(4-phenyl-6,7-dihydrothieno [3,2-c]yridin-5(4H)-yl)butanamid | 46-243 | N/A | 369,2 |
| **180** | 1-(4-(3-Fluorphenyl)-6,7-dihydrothieno[3,2-c]pyridin-5 (4H)-yl)-4-(4-(4-methylbenzyl) piperazin-1-yl)butan-1,4-dion | 46-243 | N/A | 506,2 |
| **181** | 2-Methyl-1-(4-phenyl-6,7-dihydrothieno[3,2-c]pyridin-5 (4H)-yl)-4-(2-(pyridin-2-ylmethyl)piperidin-1-yl)butan-1,4-dion | 46-243 | N/A | 488,2 |
| **182** | 4-(4-(3,5-Dichlorpyridin-4-yl) piperazin-1-yl)-2-methyl-1-(4-phenyl-6,7-dihydrothieno[3,2-c] pyridin-5(4H)-yl)butan-1,4-dion | 46-243 | N/A | 543,1 |
| **183** | 1-(2-((4,6-Dimethylpyridin-2-yl) methyl)piperidin-1-yl)-4-(4-phenyl-6,7-dihydrothieno[3,2-c] pyridin-5(4H)-yl)butan-1,4-dion | 46-243 | N/A | 502,2 |
| **184** | N-(4-Fluorbenzyl)-N-methyl-4-oxo-4-(4-phenyl-6,7-dihydrothieno[3,2-c]pyridin-5 (4H)-yl)butanamid | 46-243 | N/A | 437,2 |
| **185** | 1-(4-(3-Fluorphenyl)-6,7-dihydrothieno[3,2-c]pyridin-5 (4H)-yl)-4-(2-(pyridin-2-yl) pyrrolidin-1-yl)butan-1,4-dion | 46-243 | N/A | 464,2 |
| **186** | 4-(4-(4-Methoxybenzyl) piperazin-1-yl)-2-methyl-1-(4-phenyl-6,7-dihydrothieno[3,2-c] pyridin-5(4H)-yl)butan-1,4-dion | 46-243 | N/A | 518,2 |
| **187** | 2-Methyl-1-(4-phenyl-6,7-dihydrothieno[3,2-c]pyridin-5 (4H)-yl)-4-(3-(pyridin-3-yl) pyrrolidin-1-yl)butan-1,4-dion | 46-243 | N/A | 460,2 |

(fortgesetzt)

| Beispiel-verbindung | Chemische Bezeichnung | Herstellung analog Beispiel | Ausbeute [%] | MS m/z [M+H]+ |
|---|---|---|---|---|
| **188** | 1-(4-(2-Fluorbenzyl)piperazin-1-yl)-4-(4-phenyl-6,7-dihydrothieno[3,2-c]pyridin-5(4H)-yl)butan-1,4-dion | 46-243 | N/A | 492,2 |
| **189** | N-Methyl-4-oxo-4-(4-phenyl-6,7-dihydrothieno[3,2-c]pyridin-5(4H)-yl)-N-(2-(pyridin-4-yl)ethyl)butanamid | 46-243 | N/A | 434,2 |
| **190** | N-Butyl-N-ethyl-3-methyl-4-oxo-4-(4-phenyl-6,7-dihydrothieno[3,2-c]pyridin-5(4H)-yl)butanamid | 46-243 | N/A | 413,2 |
| **191** | N,3-Dimethyl-4-oxo-4-(4-phenyl-6,7-dihydrothieno[3,2-c]pyridin-5(4H)-yl)-N-propylbutanamid | 46-243 | N/A | 385,2 |
| **192** | 4-((S)-2-(Methoxymethyl)pyrrolidin-1-yl)-2-methyl-1-(4-phenyl-6,7-dihydrothieno[3,2-c]pyridin-5(4H)-yl)butan-1,4-dion | 46-243 | N/A | 427,2 |
| **193** | 4-(4-(5-Chlor-2-methylphenyl)piperazin-1-yl)-2-methyl-1-(4-phenyl-6,7-dihydrothieno[3,2-c]pyridin-5(4H)-yl)butan-1,4-dion | 46-243 | N/A | 522,2 |
| **194** | N-(Furan-2-ylmethyl)-N-methyl-4-oxo-4-(4-phenyl-6,7-dihydrothieno[3,2-c]pyridin-5(4H)-yl)butanamid | 46-243 | N/A | 409,2 |
| **195** | 1-((4aR, 8aS)-Octahydroisoquinolin-2(1H)-yl)-4-(4-phenyl-6,7-dihydrothieno[3,2-c]pyridin-5(4H)-yl)butan-1,4-dion | 46-243 | N/A | 437,2 |
| **196** | 1-(4-Phenyl-6,7-dihydrothieno[3,2-c]pyridin-5(4H)-yl)-4-thiomorpholinobutan-1,4-dion | 46-243 | N/A | 401,1 |
| **197** | 1-(4-Phenyl-6,7-dihydrothieno[3,2-c]pyridin-5(4H)-yl)-4-(2-(pyridin-3-yl)pyrrolidin-1-yl)butan-1,4-dion | 46-243 | N/A | 446,2 |
| **198** | N-Benzyl-4-(4-(4-fluorphenyl)-6,7-dihydrothieno[3,2-c]pyridin-5(4H)-yl)-N-isopropyl-4-oxobutanamid | 46-243 | N/A | 465,2 |
| **199** | N-Benzyl-4-(4-(4-fluorphenyl)-6,7-dihydrothieno[3,2-c]pyridin-5(4H)-yl)-N-methyl-4-oxobutanamid | 46-243 | N/A | 437,2 |

(fortgesetzt)

| Beispiel-verbindung | Chemische Bezeichnung | Herstellung analog Beispiel | Ausbeute [%] | MS m/z [M+H]$^+$ |
|---|---|---|---|---|
| **200** | N-(3,4-Dimethoxyphenethyl)-4-(4-(4-fluorphenyl)-6,7-dihydrothieno[3,2-c]pyridin-5(4H)-yl)-N-methyl-4-oxobutanamid | 46-243 | N/A | 511,2 |
| **201** | 1-(4-(4-Fluorphenyl)-6,7-dihydrothieno[3,2-c]pyridin-5(4H)-yl)-4-(4-methyl-2-phenylpiperazin-1-yl)butan-1,4-dion | 46-243 | N/A | 492,2 |
| **202** | 1-(4-(4-Chlorphenyl)-6,7-dihydrothieno[3,2-c]pyridin-5(4H)-yl)-4-(4-phenylpiperidin-1-yl)butan-1,4-dion | 46-243 | N/A | 493,2 |
| **203** | 4-(4-(4-Chlorphenyl)-6,7-dihydrothieno(3,2-c]pyridin-5(4H)-yl)-4-oxo-N-(2-(pyridin-2-yl)ethyl)butanamid | 46-243 | N/A | 454,1 |
| **204** | N-Benzyl-2-methyl-4-oxo-N-phenethyl-4-(4-phenyl-6,7-dihydrothieno[3,2-c]pyridin-5(4H)-yl)butanamid | 46-243 | N/A | 523,2 |
| **205** | 1-(4-(4-Fluorphenyl)-6,7-dihydrothieno[3,2-c]pyridin-5(4H)-yl)-4-(2-((6-methylpyridin-2-yl)methyl)piperidin-1-yl)butan-1,4-dion | 46-243 | N/A | 506,2 |
| **206** | 1-(4-(4-Fluorphenyl)-6,7-dihydrothieno[3,2-c]pyridin-5(4H)-yl)-4-(4-(3-methylbenzyl) piperazin-1-yl)butan-1,4-dion | 46-243 | N/A | 506,2 |
| **207** | 1-(4-(4-Chlorphenyl)-6,7-dihydrothieno[3,2-c]pyridin-5(4H)-yl)-4-(2-(pyridin-4-ylmethyl)piperidin-1-yl)butan-1,4-dion | 46-243 | N/A | 508,2 |
| **208** | 1-(4-(4-Chlorphenyl)-6,7-dihydrothieno[3,2-c]pyridin-5(4H)-yl)-4-(2-((5-ethylpyridin-2-yl)methyl)piperidin-1-yl)butan-1,4-dion | 46-243 | N/A | 536,2 |
| **209** | 1-(4-(4-Chlorphenyl)-6,7-dihydrothieno[3,2-c]pyridin-5(4H)-yl)-4-(2-((3-methylpyridin-2-yl)methyl)piperidin-1-yl)butan-1,4-dion | 46-243 | N/A | 522,2 |

(fortgesetzt)

| Beispiel-verbindung | Chemische Bezeichnung | Herstellung analog Beispiel | Ausbeute [%] | MS m/z [M+H]$^+$ |
|---|---|---|---|---|
| **210** | 1-(4-(4-Chlorphenyl)-6,7-dihydrothieno[3,2-c]pyridin-5 (4H)-yl)-4-(4-(3-chlorphenyl) piperazin-1-yl)butan-1,4-dion | 46-243 | N/A | 528,1 |
| **211** | 1-(4-(4-Chlorphenyl)-6,7-dihydrothieno[3,2-c]pyridin-5 (4H)-yl)-4-(4-(2,3-dimethylphenyl)piperazin-1-yl) butan-1,4-dion | 46-243 | N/A | 522,2 |
| **212** | 1-(4-(4-Chlorphenyl)-6,7-dihydrothieno[3,2-c]pyridin-5 (4H)-yl)-4-(4-(3,4-dimethylphenyl)piperazin-1-yl) butan-1,4-dion | 46-243 | N/A | 522,2 |
| **213** | 1-(4-(4-Chlorphenyl)-6,7-dihydrothieno[3,2-1-(4-(4-Chlorphenyl)-6,7-dihydrothieno [3,2-c]pyridin-5(4H)-yl)-4-(4-(3,4-dichlorphenyl) piperazin-1-yl)butan-1,4-dion | 46-243 | N/A | 562,1 |
| **214** | 1-(4-(4-tert-Butylbenzyl) piperazin-1-yl)-4-(4-(4-chlorphenyl)-6,7-dihydrothieno [3,2-c]pyridin. 5(4H)-yl)butan-1,4-dion | 46-243 | N/A | 564,2 |
| **215** | 1-[4-(3,5-Dichlor-4-pyridyl)-1-piperazinyl]-2-methyl-4-(4-phenyl-6,7-dihydro-4H-thieno [3,2-c]pyridin-5-yl)butan-1,4-dion | 46-243 | N/A | 543,1 |
| **216** | 1-[4-[(4-Methoxyphenyl)methyl]-1-piperazinyl]-4-[4-(p-tolyl)-6,7-dihydro-4H-thieno[3,2-c]pyridin-5-yl]butan-1,4-dion | 46-243 | N/A | 518,2 |
| **217** | 1-[4-[(2-Fluorphenyl)methyl]-1-piperazinyl]-4-[4-(p-tolyl)-6,7-dihydro-4H-thieno[3,2-c]pyridin-5-yl]butan-1,4-dion | 46-243 | N/A | 506,2 |
| **218** | 1-(4-Methyl-2-phenyl-1-piperazinyl)-4-[4-(p-tolyl)-6,7-dihydro-4H-thieno(3,2-c)pyridin-5-yl]butan-1,4-dion | 46-243 | N/A | 488,2 |
| **219** | 1-(4-Phenyl-1-piperidinyl)-4-[4-(p-tolyl)-6,7-dihydro-4H-thieno[3,2-c]pyridin-5-yl]butan-1,4-dion | 46-243 | N/A | 473,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **220** | 1-[4-(p-Tolyl)-6,7-dihydro-4H-thieno[3,2-c]pyridin-5-yl]-4-[2-(2-pyridyl)-1-pyrrolidinyl] butan-1,4-dion | 46-243 | N/A | 460,2 |
| **221** | 1-[4-(p-Tolyl)-6,7-dihydro-4H-thieno[3,2-c]pyridin-5-yl]-4-[3-(3-pyridyl)-1-pyrrolidinyl] butan-1,4-dion | 46-243 | N/A | 460,2 |
| **222** | 4-Oxo-4-[4-(p-tolyl)-6,7-dihydro-4H-thieno[3,2-c]pyridin-5-yl]-N-[2-(2-pyridyl)ethyl] butanamid | 46-243 | N/A | 434,2 |
| **223** | 1-[4-[(4-Methoxyphenyl)methyl]-1-piperazinyl]-4-[4-(m-tolyl)-6,7-dihydro-4H-thieno[3,2-c]pyridin-5-yl]butan-1,4-dion | 46-243 | N/A | 518,2 |
| **224** | 1-[4-[(2-Fluorphenyl)methyl]-1-piperazinyl]-4-[4-(m-tolyl)-6,7-dihydro-4H-thieno[3,2-c]pyridin-5-yl]butan-1,4-dion | 46-243 | N/A | 506,2 |
| **225** | 1-(4-Methyl-2-phenyl-1-piperazinyl)-4-[4-(m-tolyl)-6,7-dihydro-4H-thieno[3,2-c]pyridin-5-yl]butan-1,4-dion | 46-243 | N/A | 488,2 |
| **226** | 1-[4-(m-Tolyl)-6,7-dihydro-4H-thieno[3,2-c]pyridin-5-yl)-4-(4-phenyl-1-piperidinyl)butan-1,4-dion | 46-243 | N/A | 473,2 |
| **227** | 1-[4-(m-Tolyl)-6,7-dihydro-4H-thieno[3,2-c]pyridin-5-yl]-4-[2-(2-pyridyl)-1-pyrrolidinyl] butan-1,4-dion | 46-243 | N/A | 460,2 |
| **228** | 1-[4-(m-Tolyl)-6,7-dihydro-4H-thieno[3,2-c]pyridin-5-yl]-4-[3-(3-pyridyl)-1-pyrrolidinyl] butan-1,4-dion | 46-243 | N/A | 460,2 |
| **229** | N-Methyl-4-14-(m-tolyl)-6,7-dihydro-4H-thieno[3,2-c]pyridin-5-yl]-4-oxo-N-[2-(4-pyridyl)ethyl] butanamid | 46-243 | N/A | 448,2 |
| **230** | 1-[4-(3-Fluorphenyl)-6,7-dihydro-4H-thieno[3,2-c]pyridin-5-yl]-4-[4-[(4-methoxyphenyl) methyl]-1-piperazinyl]butan-1,4-dion | 46-243 | N/A | 522,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **231** | 1-[4-(3-Fluorphenyl)-6,7-dihydro-4H-thieno[3,2-c]pyridin-5-yl)-4-[4-[(2-fluorphenyl) methyl]-1-piperazinyl]butan-1,4-dion | 46-243 | N/A | 510,2 |
| **232** | 4-[4-(m-Tolyl)-6,7-dihydro-4H-thieno[3,2-c]pyridin-5-yl]-4-oxo-N-(p-tolyl)butanamid | 46-243 | N/A | 419,2 |
| **233** | N-(2,4-Dimethylphenyl)-4-[4-(m-tolyl)-6,7-dihydro-4H-thieno[3,2-c]pyridin-5-yl]-4-oxobutanamid | 46-243 | N/A | 433,2 |
| **234** | 4-[4-(3-Fluorphenyl)-6,7-dihydro-4H-thieno[3,2-c]pyridin-5-yl]-N-(1-methyl-6-indazolyl)-4-oxobutanamid | 46-243 | N/A | 463,2 |
| **235** | 4-[4-(3-Fluorphenyl)-6,7-dihydro-4H-thieno[3,2-c]pyridin-5-yl]-4-oxo-N-(p-tolyl)butanamid | 46-243 | N/A | 423,1 |
| **236** | 4-[4-(3-Fluorphenyl)-6,7-dihydro-4H-thieno[3,2-c]pyridin-5-yl]-4-oxo-N-phenylbutanamid | 46-243 | N/A | 409,1 |
| **237** | N-(2-Chlorphenyl)-4-[4-(3-fluorphenyl)-6,7-dihydro-4H-thieno[3,2-c]pyridin-5-yl]-4-oxobutanamid | 46-243 | N/A | 443,1 |
| **238** | N-(2,4-Dimethylphenyl)-4-[4-(3-fluorphenyl)-6,7-dihydro-4H-thieno[3,2-c]pyridin-5-yl]-4-oxobutanamid | 46-243 | N/A | 437,2 |
| **239** | 4-[4-(4-Chlorphenyl)-6,7-dihydro-4H-thieno[3,2-c]pyridin-5-yl]-N-(1-methyl-6-indazolyl)-4-oxobutanamid | 46-243 | N/A | 479,1 |
| **240** | 4-[4-(4-Chlorphenyl)-6,7-dihydro-4H-thieno[3,2-c]pyridin-5-yl]-4-oxo-N-(p-tolyl)butanamid | 46-243 | N/A | 439,1 |
| **241** | 4-[4-(4-Chlorphenyl)-6,7-dihydro-4H-thieno[3,2-c]pyridin-5-yl]-N-(4-methoxyphenyl)-4-oxobutanamid | 46-243 | N/A | 455,1 |
| **242** | 4-[4-(4-Chlorphenyl)-6,7-dihydro-4H-thieno[3,2-c]pyridin-5-yl]-4-oxo-N-phenylbutanamid | 46-243 | N/A | 425,1 |
| **243** | 4-[4-(4-Chlorphenyl)-6,7-dihydro-4H-thieno[3,2-c]pyridin-5-yl]-N-(2,4-dimethylphenyl)-4-oxobutanamid | 46-243 | N/A | 453,1 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **244** | 4-[4-(2,6-Dimethylphenyl)-6,7-dihydro-4H-thieno[3,2-c]pyridin-5-yl]-4-oxo-N-[[3-(trifluormethyl) phenyl]methyl]butanamid | 9 | 33 | 501,2 |
| **245** | N-(2-Cyclohexylethyl)-4-oxo-4-(4-phenyl-6,7-dihydro-4H-thieno[3,2-c]pyridin-5-yl) butanamid | 34 | 66 | 425,2 |
| **246** | N-(3,3-Dimethylbutyl)-4-oxo-4-(4-phenyl-6,7-dihydro-4H-thieno[3,2-c]pyridin-5-yl) butanamid | 34 | 50 | 399,2 |
| **247** | N-(Cyclohexylmethyl)-4-oxo-4-(4-phenyl-6,7-dihydro-4H-thieno[3,2-c]pyridin-5-yl) butanamid | 34 | 63 | 411.2 |
| **248** | N-[[3-Methyl-5-(trifluormethoxy) phenyl]methyl]-4-oxo-4-(4-phenyl-6,7-dihydro-4H-thieno [3,2-c]pyridin-5-yl)butanamid | 11 | 71 | 487,2 |
| **249** | N-[[4-Methyl-3-(trifluormethyl) phenyl]methyl]-4-oxo-4-(4-phenyl-6,7-dihydro-4H-thieno [3,2-c]pyridin-5-yl)butanamid | 11 | 51 | 487,2 |
| **250** | N-[[4-Fluor-3-(trifluormethyl) phenyl]methyl]-4-oxo-4-(4-phenyl-6,7-dihydro-4H-thieno [3,2-c]pyridin-5-yl)butanamid | 11 | 65 | 491,1 |
| **251** | 4-[4-(2-Ethylphenyl)-6,7-dihydro-4H-thieno[3,2-c]pyridin-5-yl]-4-oxo-N-[[3-(trifluormethyl) phenyl]methyl]butanamid | 2 | 68 | 501,2 |
| **252** | 4-[4-(2-Isopropylphenyl)-6,7-dihydro-4H-thieno[3,2-c]pyridin-5-yl]-4-oxo-N-[[3-(trifluormethyl) phenyl]methyl]butanamid | 9 | 35 | 515,2 |
| **253** | N-(3-Cyclohexylpropyl)-4-oxo-4-(4-phenyl-6,7-dihydro-4H-thieno[3,2-c]pyridin-5-yl) butanamid | 34 | 77 | 439,2 |
| **254** | 4-[4-(3-Methyl-2-thienyl)-6,7-dihydro-4H-thieno[3,2-c]pyridin-5-yl]-4-oxo-N-[[3-(trifluormethyl) phenyl]methyl]butanamid | 2 | 39 | 493,1 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **255** | (1S,2S)-2-[Oxo-(4-phenyl-6,7-dihydro-4H-thieno[3,2-c]pyridin-5-yl) methyl]-N-[[3-(trifluormethyl) phenyl]methyl]-1-cyclopropancarboxamid | 2 | 89 | 485,1 |
| **256** | 4-[4-(o-Tolyl)-6,7-dihydro-4H-thieno[3,2-c]pyridin-5-yl]-4-oxo-N-[[2-(trifluormethyl)phenyl] methyl]butanamid | 34 | 61 | 487,2 |
| **257** | 4-[4-(o-Tolyl)-6,7-dihydro-4H-thieno[3,2-c]pyridin-5-yl]-4-oxo-N-[[4-(trifluormethyl)phenyl] methyl]butanamid | 34 | 51 | 487,2 |
| **258** | 4-(7-Butyl-5,7-dihydro-4H-thieno[2,3-c]pyridin-6-yl)-4-oxo-N-[[3-(trifluormethyl)phenyl] methyl]butanamid | 2 | | 453,2 |
| **259** | 4-[4-(Cyclohexylmethyl)-6,7-dihydro-4H-thieno[3,2-c]pyridin-5-yl]-4-oxo-N-[[3-(trifluormethyl) phenyl]methyl]butanamid | 2 | | 493,2 |
| **260** | 4-(4-Cyclopropyl-6,7-dihydro-4H-thieno[3,2-c]pyridin-5-yl)-4-oxo-N-[[3-(trifluormethyl)phenyl] methyl]butanamid | 2 | | 437,1 |
| **261** | 4-Oxo-4-(4-phenthyl-6,7-dihydro-4H-thieno[3,2-c]pyridin-5-yl)-N-[[3-(trifluormethyl) phenyl]methyl]butanamid | 2 | | 501,2 |
| **262** | 4-[4-(4-Fluor-2-methylphenyl)-6,7-dihydro-4H-thieno[3,2-c] pyridin-5-yl]-4-oxo-N-[[3-(trifluormethyl)phenyl] methyl]butanamid | 2 | | 505,1 |
| **263** | 4-(4-Cyclopentyl-6,7-dihydro-4H-thieno[3,2-c]pyridin-5-yl)-4-oxo-N-[[3-(trifluormethyl)phenyl] methyl]butanamid | 2 | | 465,2 |
| **264** | N-[[2-Methyl-3-(trifluormethyl) phenyl]methyl]-4-oxo-4-(4-phenyl-6,7-dihydro-4H-thieno [3,2-c]pyridin-5-yl)butanamid | 44 | | 487,2 |
| **265** | 4-Oxo-4-[4-(3-phenylpropyl)-6,7-dihydro-4H-thieno[3,2-c] pyridin-5-yl]-N-[[3-(trifluormethyl)phenyl] methyl]butanamid | 2 | | 515,2 |

(fortgesetzt)

| Beispiel-verbindung | Chemische Bezeichnung | Herstellung analog Beispiel | Ausbeute [%] | MS m/z $[M+H]^+$ |
|---|---|---|---|---|
| **266** | N-[[2-Methyl-5-(trifluormethyl) phenyl]methyl]-4-oxo-4-(4-phenyl-6,7-dihydro-4H-thieno [3,2-c]pyridin-5-yl]butanamid | 44 | | 487,2 |
| **267** | N-Cyclohexyl-4-oxo-4-(4-phenyl-6,7-dihydro-4H-thieno [3,2-c]pyridin-5-yl)butanamid | 44 | | 397,2 |
| **268** | N-(2,2-Dimethylpropyl)-4-oxo-4-(4-phenyl-6,7-dihydro-4H-thieno[3,2-c]pyridin-5-yl) butanamid | 44 | | 385,2 |
| **269** | 1-(4-Phenyl-6,7-dihydro-4H-thieno[3,2-c]pyridin-5-yl)-4-[7-(trifluormethyl)-3,4-dihydro-1H-isoquinolin-2-yl]butan-1,4-dion | 24 | | 499,2 |
| **270** | 4-Oxo-N-pentyl-4-(4-phenyl-6,7-dihydro-4H-thieno[3,2-c] pyridin-5-yl)butanamid | 44 | | 385,2 |
| **271** | 1-(4-Phenyl-6,7-dihydro-4H-thieno[3,2-c]pyridin-5-yl)-4-[5-(trifluormethyl)-3,4-dihydro-1H-isoquinolin-2-yl]butan-1,4-dion | 24 | | 499,2 |
| **272** | 4-Oxo-4-(4-phenyl-6,7-dihydro-4H-thieno[3,2-c]pyridin-5-yl)-N-[5-(trifluormethyl)-1-tetralinyl]butanamid | 44 | | 513,2 |
| **273** | 4-Oxo-4-(4-phenyl-6.7-dihydro-4H-thieno[3,2-c]pyddin-5-yl)-N-[7-(trifluormethyl)-1-tetralinyl]butanamid | 44 | | 513,2 |
| **274** | 4-[7-(o-Tolyl)-5,7-dihydro-4H-thieno[2,3-c]pyridin-6-yl]-4-oxo-N-[[3-(trifluormethyl)phenyl] methyl]butanamid | 2 | | 487,2 |
| **275** | 4-(7-Cyclohexyl-5,7-dihydro-4H-thieno[2,3-c]pyridin-6-yl)-4-oxo-N-[[3-(trifluormethyl)phenyl] methyl]butanamid | 2 | | 479,2 |

**Pharmakologische Experimente**

**Fluoreszenzassay unter Verwendung eines 'voltage sensitive dyes'**

Humane, KCNQ2/3-Kanäle exprimierende CHO-K1-Zellen werden in Zellkulturflaschen (z.B. 80 $cm^2$ TC flasks, Nunc) mit DMEM-high glucose (Sigma Aldrich, D7777) inklusive 10% FCS (PAN Biotech, z.B. 3302-P270521) oder alternativ MEM Alpha Medium (1x, flüssig, Invitrogen, #22571), 10 % Fetal Calf Serum (FCS) (Invitrogen, #10270-106, hitzeinaktiviert) und den notwendigen Selektionsantibiotika bei 37°C, 5 % $CO_2$ und 95 % Luftfeuchtigkeit adhärent

kultiviert.

Vor Aussaat für die Messungen werden die Zellen mit einem 1 x DPBS-Puffer ohne $Ca^{2+}/Mg^{2+}$ (z.B. Invitrogen, #14190-094) gewaschen und mittels Accutase (PAA Laboratories, #L11-007) vom Boden des Kulturgefäßes abgelöst (Inkubation mit Accutase für 15 min bei 37°C). Die Bestimmung der dann vorliegenden Zellzahl wird mit einem CASY™ cell counter (Modell TCC, Schärfe System) durchgeführt, um anschließend je nach Dichteoptimierung für die individuelle Zelllinie 20.000 - 30.000 Zellen/well/100 µl des beschriebenen Nährmediums auf die 96 well-Messplatten des Typs Corning™ CellBIND™ (Flat Clear Bottom Black Polystyrene Microplates, #3340) auszubringen. Danach erfolgt eine einstündige Inkubation bei Raumtemperatur ohne Begasung oder Regelung der Luftfeuchtigkeit, gefolgt von einer 24stündigen Inkubation bei 37°C, 5 % $CO_2$ und 95 % Luftfeuchtigkeit.

Der spannungssensitive Fluoreszenzfarbstoff aus dem Membrane Potential Assay Kit (Red™ Bulk format part R8123 for FLIPR, MDS Analytical Technologies™) wird vorbereitet, indem der Inhalt eines Gefäßes *Membrane Potential Assay Kit Red Component* A in 200 ml Extrazellulären Puffers (ES-Puffer, 120 mM NaCl, 1 mM KCl, 10 mM HEPES, 2 mM $CarI_2$, 2 mM $MgCl_2$, 10 mM Glucose; pH 7,4) gelöst wird. Nach Abnahme des Nährmediums werden die Zellen mit 200 µl ES-Puffer gewaschen, anschließend mit 100 µl der oben angesetzten Farbstofflösung überschichtet und 45 min bei Raumtemperatur unter Lichtverschluss inkubiert.

Die Fluoreszenzmessungen werden mit einem BMG Labtech FLUOstar™-, BMG Labtech NOVOstar™- oder BMG Labtech POLARstar™-Instrument durchgeführt (525 nm Exication, 560 nm Emission, Bottom Read mode). Nach der Farbstoff-Inkubation werden 50 µl der zu testenden Substanzen in den gewünschten Konzentrationen oder 50 µl ES-Puffer zwecks Kontrolle in separate Cavitäten der Messplatte gegeben und 30 min bei Raumtemperatur unter Abschirmung von Licht inkubiert. Anschließend misst man für 5 min die Fluoreszenzintensität des Farbstoffs und ermittelt so zu einem festgelegten und gleichbleibenden Zeitpunkt den Fluoreszenzwert $F_1$ eines jeden wells. Daraufhin erfolgt Zugabe von 15 µl einer 100 mM KCl-Lösung (Endkonzentration 92 mM) in jedes well. Die Veränderung der Fluoreszenz wird anschließend so lange gemessen, bis alle relevanten Messwerte erhalten sind (vornehmlich 5-30 min). Zu einem festgelegten Zeitpunkt nach KCl-Applikation wird ein Fluoreszenzwert $F_2$ ermittelt, in diesem Falle zum Zeitpunkt des Fluoreszenzpeaks.

Zur Berechnung wird die Fluoreszenzintensität $F_2$ mit der Fluoreszenzintensität $F_1$ verglichen und daraus die agonistische Aktivität der Zielverbindung auf den Kaliumkanal ermittelt. $F_2$ und $F_1$ werden hierfür wie folgt verrechnet:

$$\left(\frac{F_2 - F_1}{F_1}\right) \times 100 = \frac{\Delta F}{F}(\%)$$

Um zu ermitteln, ob eine Substanz eine agonistische Aktivität besitzt, kann z.B. $\frac{\Delta F}{F}$ mit $\left(\frac{\Delta F}{F}\right)_K$ von Kontrollzellen verglichen werden. $\left(\frac{\Delta F}{F}\right)_K$ wird ermittelt, indem man dem Reaktionsansatz anstatt der zu testenden Substanz lediglich die Pufferlösung zusetzt, den Wert $F_{1K}$ der Fluoreszenzintensität bestimmt, die Kaliumionen wie oben beschrieben zugibt und einen Wert $F_{2K}$ der Fluoreszenzintensität misst. Dann werden $F_{2K}$ und $F_{1K}$ wie folgt verrechnet:

$$\left(\frac{F_{2K} - F_{1K}}{F_{1K}}\right) \times 100 = \left(\frac{\Delta F}{F}\right)_K (\%)$$

Eine Substanz besitzt eine agonistische Aktivität auf den Kaliumkanal, wenn $\frac{\Delta F}{F}$ größer als $\left(\frac{\Delta F}{F}\right)_K$ ist:

$$\frac{\Delta F}{F} \rangle \left(\frac{\Delta F}{F}\right)_K$$

Unabhängig vom Vergleich von $\frac{\Delta F}{F}$ mit $\left(\frac{\Delta F}{F}\right)_K$ lässt sich auch auf eine agonistische Aktivität einer Zielverbindung schließen, wenn mit steigender Dosierung der Zielverbindung eine Zunahme von $\frac{\Delta F}{F}$ zu beobachten ist.

Kalkulationen von $EC_{50}$- und $IC_{50}$-Werten werden mit Hilfe der Software 'Prism v4.0' (GraphPad Software™) durchgeführt.

**Voltage-clamp Messungen**

Um eine KCNQ2/3 agonistische Wirkung der Substanzen elektrophysiologisch zu konfirmieren, wurden patch-clamp Messungen (Hamill OP, Marty A, Neher E, Sakmann B, Sigworth FJ.: Improved patch-clamp techniques for high-resolution current recording from cells and cell-free membrane patches, Pflugers Arch. 1981 Aug; 391(2):85-100) im voltage-clamp Modus an einer stabil transfizierten hKCNQ2/3 CHO-K1 Zelllinie durchgeführt. Nach Ausbildung des Gigaseals wurden die Zellen zunächst auf ein Haltepotential von -60 mV geklemmt. Im Anschluss wurden depolarisierende Spannungssprünge bis zu einem Potential von +20 mV appliziert (Inkrement: 20 mV, Dauer: 1 Sekunde), um die funktionelle Expression von KCNQ2/3 typischen Strömen zu bestätigen. Die Substanztestung erfolgte bei einem Potential von -40 mV. An jeder Zelle wurde zunächst die durch Retigabin (10 μM) induzierte Stromzunahme bei -40 mV als Positivkontrolle registriert. Nach komplettem Auswaschen des Retigabineffektes (Dauer: 80 s) wurde die Testsubstanz (10 μM) appliziert. Die durch die Testsubstanz induzierte Stromzunahme wurde auf den Retigabineffekt normiert und als relative Efficacy angegeben (s. u.).

**Formalin-Test bei der Ratte**

Der Formalin Test (Dubuisson, D. and Dennis, S.G., 1977, Pain, 4, 161 - 174) stellt ein Modell für den akuten sowie den chronischen Schmerz dar. In den hier vorgestellten Untersuchungen wurde die chronische Schmerzkomponente (II. Phase des Formalintests; Zeitintervall 21 - 27min nach Formalinapplikation) ausgewertet. Durch eine einmalige Formalin-Injektion in die dorsale Seite einer Hinterpfote wird bei freibeweglichen Versuchstieren eine biphasische nozizeptive Reaktion induziert, die durch Beobachtung von drei deutlich voneinander unterscheidbaren Verhaltensmustern erfasst wird.

Formalin wird mit dem Volumen von 50 μl und einer Konzentration von 5 % subkutan in die dorsale Seite der rechten Hinterpfote jedes Tieres appliziert. Das Vehikel und die zu prüfenden Substanzen werden intravenös 5 min, oder oral 30 min, vor der Formalin-Injektion appliziert.

Die spezifischen Verhaltensänderungen, wie Anheben und Schütteln der Pfote, Gewichtsverlagerungen des Tieres sowie Beiß- und Leckreaktionen werden bis 60 min nach Formalinapplikation kontinuierlich beobachtet und registriert. Die Verhaltensänderungen werden unterschiedlich gewichtet (Score 0-3) und eine Pain-Rate (PR) errechnet mit folgender Formel:

$$PR = [(T_0 \times 0) + (T_1 \times 1) + (T_2 \times 2) + (T_3 \times 3) / 180.$$

Dabei entsprechen $T_0$, $T_1$, $T_2$, $T_3$ jeweils der Zeit in Sekunden, in der das Tier die Verhaltensweisen 0, 1, 2, oder 3 zeigte.

Als Tierstamm werden Ratten des Stammes Sprague Dawley (Janvier, Belgien) verwendet. Das Gewicht der Tiere beträgt 180 - 200g; Die Gruppengröße betrug n = 10.

**Pharmakologische Daten**

In Tabelle 3 sind die Ergebnisse aus den zuvor beschriebenen pharmakologischen Modellen zusammengefasst.

Tabelle 3

| Beispielverbindung | Fluorimetrie % Efficacy @ 10 μM (Retigabine = 100%) | Fluorimetrie $EC_{50}$ [nM] | Fluorimetrie $IC_{50}$ [nM] | patch-clamp % Efficacy @ 10μM (Retigabine = 100%) | Formalintest Ratte i.v. Reduzierung des nozizeptiven Verhaltens [% (Dosis [ mg/kg])] |
|---|---|---|---|---|---|
| **1** | 168 | 1690 | | 97 | |
| **2** | 165 | 758 | | 106 | 56% (10,0) |
| **3** | 103 | 357 | | | |
| **4** | 102 | 456 | | | |
| **5** | 61 | >1662 | | | |
| **6** | 133 | 284 | | | |
| **7** | 132 | 348 | | | |
| **8** | 148 | 79 | | | 11% (4,64) |
| **9** | 110 | 407 | | | |
| **10** | 121 | 382 | | | |
| **11** | -18 | | | | |
| **12** | -46 | | | | |
| **13** | 51 | 375 | | | |
| **14** | -54 | | | | |
| **15** | -46 | | | | |
| **16** | 21 | | | | |
| **17** | 74 | 62 | | | 15% (10,0) |
| **18** | 11 | 2181 | | | |
| **19** | -45 | | | | |
| **20** | 54 | 722 | | | |
| **21** | 148 | 324 | | | |
| **22** | 139 | 1219 | | | |
| **23** | 146 | 947 | | | |
| **24** | -56 | | | | |
| **25** | 12 | | | | |
| **26** | -20 | | | | |
| **27** | 119 | >404 | | | |
| **28** | 106 | 540 | | | |
| **29** | -45 | | 133 | | |
| **30** | -101 | | 212 | | |
| **31** | -44 | | 250 | | |
| **32** | 110 | 414 | | | |
| **33** | -6 | | | | |

(fortgesetzt)

| Beispielverbindung | Fluorimetrie % Efficacy @ 10 μM (Retigabine = 100%) | Fluorimetrie $EC_{50}$ [nM] | Fluorimetrie $IC_{50}$ [nM] | patch-clamp % Efficacy @ 10μM (Retigabine = 100%) | Formalintest Ratte i.v. Reduzierung des nozizeptiven Verhaltens [% (Dosis [ mg/kg])] |
|---|---|---|---|---|---|
| **34** | 179 | 628 | | | |
| **35** | | 572 | | | |
| **36** | | 707 | | | |
| **40** | | 156 | | | |
| **41** | | 261 | | | |
| **44** | -21 | | | | |
| **45** | 39 | 311 | | | |
| **46** | 35 | | | | |
| **47** | 37 | | | | |
| **48** | -15 | | | | |
| **49** | 38 | | | | |
| **50** | 10 | | | | |
| **51** | 38 | | | | |
| **52** | 15 | | | | |
| **53** | 28 | | | | |
| **54** | 19 | | | | |
| **245** | | 690 | | | |
| **247** | | 1438 | | | |
| **249** | | 347 | | | |
| **250** | | 517 | | | |
| **251** | | 131 | | | |
| **252** | | 1012 | | | |
| **254** | | 211 | | | |
| **256** | | 90 | | | |
| **257** | | 547 | | | |
| **258** | | 306 | | | |
| **260** | | 108 | | 91 | 46% (4,64) |
| **261** | | 84 | | | |
| **262** | | 166 | | | |
| **263** | | 160 | | | |
| **265** | | 596 | | | |
| **269** | | 519 | | | |
| **271** | | 431 | | | |

(fortgesetzt)

| Beispielverbindung | Fluorimetrie % Efficacy @ 10 μM (Retigabine = 100%) | Fluorimetrie $EC_{50}$ [nM] | Fluorimetrie $IC_{50}$ [nM] | patch-clamp % Efficacy @ 10μM (Retigabine = 100%) | Formalintest Ratte i.v. Reduzierung des nozizeptiven Verhaltens [% (Dosis [ mg/kg])] |
|---|---|---|---|---|---|
| **274** | | 323 | | | |
| **275** | | 962 | | | |

**Vergleichsexperimente**

Die erfindungsgemäßen substituierten Tetrahydrothienopyridine zeichnen sich im Vergleich zu substituierten Tetrahydropyrrolopyrazinen, welche aus WO 2008/046582 bekannt sind, durch eine verbesserte Wirksamkeit *in vitro* bzw. *in vivo* aus, wie die nachfolgenden Vergleichsexperimente verdeutlichen:

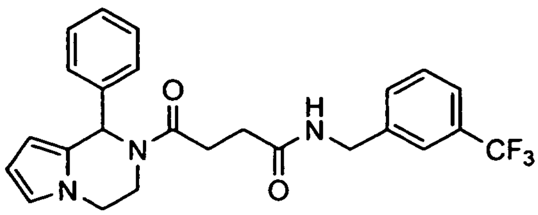

Beispiel Nr. 2 in WO2008/046582

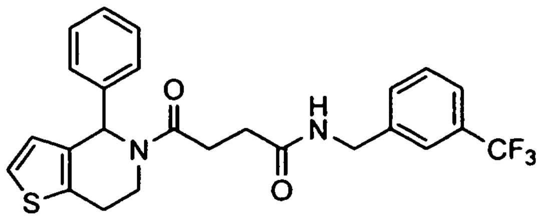

erfindungsgemäße Verbindung **2**

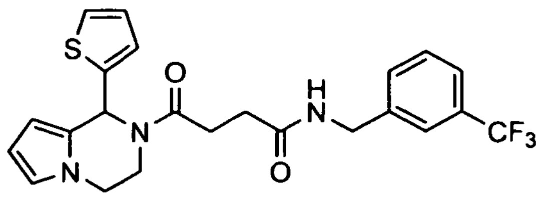

Beispiel Nr. 76 in WO2008/046582

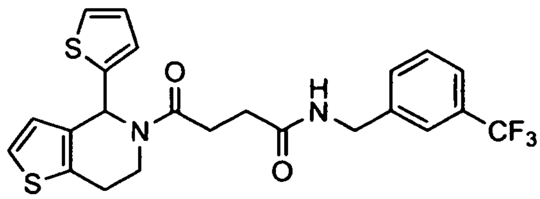

erfindungsgemäße Verbindung **1**

| | Fluorometrie % Effcacy @ 10 μM (Retigabine = 100%) | Fluorometrie $EC_{50}$ [μM] | Formalintest Ratte i.v. @ 10 mg/kg % Reduzierung des nozizeptiven Verhaltens [%] |
|---|---|---|---|
| Beispiel Nr. 2 in WO 2008/046582 | 115 | 2,81 | kein Effekt |
| **2** | 145 | 1,44 | 56 |
| | | | |
| Beispiel Nr. 76 in WO 2008/046582 | 113 | 3,29 | nicht getestet |
| **1** | 165 | 0,76 | nicht getestet |

**Patentansprüche**

**1.** Substituierte Tetrahydrothienopyridine der allgemeinen Formel (1),

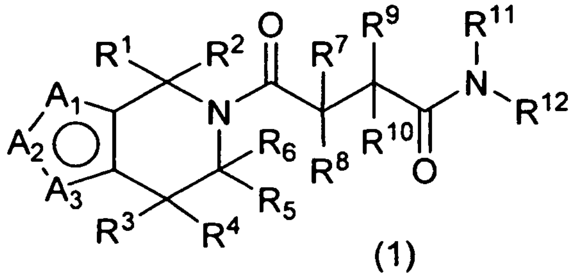

(1)

,

worin

$A_1$ für S, $A_2$ für $CR^{14}$ und $A_3$ für $CR^{15}$ steht; oder
$A_1$ für $CR^{13}$, $A_2$ für S und $A_3$ für $CR^{15}$ steht; oder
$A_1$ für $CR^{13}$, $A_2$ für $CR^{14}$ und $A_3$ für S steht;
$R^0$ für $C_{1-8}$-Alkyl, gesättigt oder ungesättigt, verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über $C_{1-8}$-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; steht;
$R^1$ für H; F; Cl; Br; CN; oder $R^0$ steht;
$R^2$ für H; F; Cl; Br; oder $C_{1-8}$-Alkyl, gesättigt oder ungesättigt, verzweigt oder unverzweigt, unsubstituiert oder einfach oder mehrfach substituiert; steht;
oder $R^1$ und $R^2$ zusammen mit dem sie verbindenden Kohlenstoffatom als Ringglied ein $C_{3-7}$-Cycloalkyl oder Heterocyclyl bilden, jeweils gesättigt oder ungesättigt, unsubstituiert oder einfach oder mehrfach substituiert, jeweils ggf. mit (Hetero-)aryl kondensiert, unsubstituiert oder einfach oder mehrfach substituiert;
$R^3$, $R^4$, $R^5$, $R^6$ unabhängig voneinander für H; F; Cl; Br; $C_{1-8}$-Alkyl, gesättigt oder ungesättigt, verzweigt oder unverzweigt, unsubstituiert oder einfach oder mehrfach substituiert; oder Phenyl, unsubstituiert oder einfach oder mehrfach substituiert stehen;
$R^7$ für H; F; Cl; Br; CN; $R^0$; $OR^0$; O-C(=O)-$R^0$; O-C(=O)-O-$R^0$; O-(C=O)-NH-$R^0$; O-C(=O)-N$(R^0)_2$; NH-$R^0$; N$(R^0)_2$; NH-C(=O)-$R^0$; NH-C(=O)-O-$R^0$; NH-C(=O)-NH-$R^0$; NH-C(=O)-N$(R^0)_2$; $NR^0$-C(=O)-$R^0$; $NR^0$-C(=O)-O-$R^0$; $NR^0$-C(=O)-$NH_2$; $NR^0$-C(=O)-NH-$R^0$; $NR^0$-C(=O)-N$(R^0)_{21}$NH-S$(=O)_2$OH; NH-S$(=O)_2R^0$; NH-S$(=O)_2OR^0$; NH-S$(=O)_2NH_2$; NH-S$(=O)_2NHR^0$; NH-S$(=O)_2$N$(R^0)_2$; $NR^0$-S$(=O)_2$OH; $NR^0$-S$(=O)_2R^0$; $NR^0$-S$(=O)_2OR^0$; $NR^0$-S$(=O)_2NH_2$; $NR^0$-S$(=O)_2NHR^0$; $NR^0$-S$(=O)_2$N$(R^0)_2$; C(=O)-OH; C(=O)-$OR^0$; C(=O)-$NH_2$; C(=O)-NH-$R^0$; C(=O)-N$(R^0)_2$; S$(=O)_2$-OH; S$(=O)_2OR^0$; S$(=O)_2$-$NH_2$; S$(=O)_2$-NH-$R^0$; S$(=O)_2$-N$(R^0)_2$;
$R^8$ für H; F; Cl; Br; CN; oder $C_{1-8}$-Alkyl, gesättigt oder ungesättigt, verzweigt oder unverzweigt, unsubstituiert oder einfach oder mehrfach substituiert; steht;
$R^9$ für H; F; Cl; Br; CN; $C_{1-8}$-Alkyl, gesättigt oder ungesättigt, verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; oder über $C_{2-8}$-Alkyl-verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; O-$C_{1-8}$-Alkyl, gesättigt oder ungesättigt, verzweigt oder unverzweigt, unsubstituiert oder einfach oder mehrfach substituiert; O-$C_{3-7}$-Cycloalkyl oder O-Heterocyclyl, jeweils gesättigt oder ungesättigt, unsubstituiert oder einfach oder mehrfach substituiert; über O-$C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei

die Alkylkette jeweils verzweigt oder unverzweigt, gesättigt oder ungesättigt, unsubstituiert, einfach oder mehrfach substituiert sein kann; oder über O-$C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; O-C(=O)-$R^0$; O-C(=O)-O-$R^0$; O-(C=O)-NH-$R^0$; O-C(=O)-N($R^0$)$_2$; NH-$R^0$; N($R^0$)$_2$; NH-C(=O)-$R^0$, NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$;NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$; N$R^0$-S(=O)$_2$O$R^0$; N$R^0$-S(=O)$_2NH_2$; N$R^0$-S(=O)$_2$NH$R^0$; N$R^0$-S(=O)$_2$N($R^0$)$_2$; C(=O)-OH; C(=O)-O$R^0$; C(=O)-$NH_2$; C(=O)-NH-$R^0$; C(=O)-N($R^0$)$_2$; S(=O)$_2$-OH; S(=O)$_2$O$R^0$; S(=O)$_2$-$NH_2$; S(=O)$_2$-NH-$R^0$; S(=O)$_2$-N($R^0$)$_2$ steht;

$R^{10}$ für H; F; Cl; Br; CN; oder $C_{1-8}$-Alkyl, gesättigt oder ungesättigt, verzweigt oder unverzweigt, unsubstituiert oder einfach oder mehrfach substituiert; steht;

oder $R^7$ und $R^9$ zusammen mit den sie verbindenden Kohlenstoffatomen als Ringgliedern ein $C_{3-7}$-Cycloalkyl oder Heterocyclyl bilden, jeweils gesättigt oder ungesättigt, unsubstituiert oder einfach oder mehrfach substituiert, ggf. mit (Hetero-)aryl kondensiert, unsubstituiert oder einfach oder mehrfach substituiert;

oder $R^7$ und $R^8$; oder $R^9$ und $R^{10}$; zusammen mit den sie verbindenden Kohlenstoffatomen als Ringgliedern ein $C_{3-7}$-Cyclloalkyl oder Heterocyclyl bilden, jeweils gesättigt oder ungesättigt, unsubstituiert oder einfach oder mehrfach substituiert, jeweils ggf. mit (Hetero-)aryl kondensiert, unsubstituiert oder einfach oder mehrfach substituiert;

$R^{11}$ für H; $C_{1-8}$-Alkyl, gesättigt oder ungesättigt, verzweigt oder unverzweigt, unsubstituiert oder einfach oder mehrfach substituiert; oder $C_{3-7}$-Cycloalkyl, gesättigt oder ungesättigt, unsubstituiert oder einfach oder mehrfach substituiert; steht;

$R^{12}$ für $C_{2-16}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann;

oder $R^{11}$ und $R^{12}$ zusammen mit dem sie verbindenden Stickstoffatom als Ringglied ein Heterocyclyl bilden, gesättigt oder ungesättigt, unsubstituiert oder einfach oder mehrfach substituiert, ggf. mit (Hetero-)aryl kondensiert, unsubstituiert oder einfach oder mehrfach substituiert;

$R^{13}$, $R^{14}$ und $R^{15}$ jeweils unabhängig voneinander H; F; Cl; Br; I; NO; $NO_2$; $CF_3$; CN; $R^0$; C(=O)H; C(=O)$R^0$; $CO_2$H: C(=O)O$R^0$; CON$H_2$; C(=O)NH$R^0$; C(=O)N($R^0$)$_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2NH_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$; N$R^0$-S(=O)$_2$O$R^0$; N$R^0$-S(=O)$_2NH_2$; N$R^0$-S(=O)$_2$NH$R^0$; N$R^0$-S(=O)$_2$N($R^0$)$_2$; SH; S$R^0$; S(=O)$R^0$; S(=O)$_2R^0$; S(=O)$_2$OH; S(=O)$_2$O$R^0$; S(=O)$_2NH_2$; S(=O)$_2$NH$R^0$; oder S(=O)$_2$N($R^0$)$_2$ bedeuten;

worin "Alkyl substituiert", "Heterocyclyl substituiert" und "Cycloalkyl substituiert" für die Substitution eines oder mehrerer Wasserstoffatome jeweils unabhängig voneinander durch F; Cl; Br; I; CN; $CF_3$; =O; =NH; =C($NH_2$)$_2$; $NO_2$; $R^0$; C(=O)H; C(=O)$R^0$; $CO_2$H; C(=O)O$R^0$; CON$H_2$; C(=O)NH$R^0$; C(=O)N($R^0$)$_2$; OH; O$R^0$; O-($C_{1-8}$-Alkyl)-O; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2NH_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; NR°-C(=O)-R°; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$; N$R^0$-S(=O)$_2$O$R^0$; N$R^0$-S(=O)$_2NH_2$; N$R^0$-S(=O)$_2$NH$R^0$; N$R^0$-S(=O)$_2$N($R^0$)$_2$; SH; S$R^0$; S(=O)$R^0$; S(=O)$_2R^0$; S(=O)$_2$OH; S(=O)$_2$O$R^0$; S(=O)$_2NH_2$; S(=O)$_2$NH$R^0$; S(=O)$_2$N($R^0$)$_2$; steht;

worin "Aryl substituiert" und "Heteroaryl substituiert" für die Substitution eines oder mehrerer Wasserstoffatome jeweils unabhängig voneinander durch F; Cl; Br; I; NO; $NO_2$; $CF_3$; CN; $R^0$; C(=O)H; C(=O)$R^0$; $CO_2$H; C(=O)O$R^0$; CON$H_2$; C(=O)NH$R^0$; C(=O)N($R^0$)$_2$; OH; O$R^0$; O-($C_{1-8}$-Alkyl-O; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2NH_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$;

$NR^0$-$S(=O)_2OR^0$; $NR^0$-$S(=O)_2NH_2$; $NR^0$-$S(=O)_2NHR^0$; $NR^0$-$S(=O)_2N(R^0)_2$, SH; $SR^0$; $S(=O)R^0$, $S(=O)_2R^0$; $S(=O)_2OH$; $S(=O)_2OR^0$; $S(=O)_2NH_2$; $S(=O)_2NHR^0$; $S(=O)_2N(R^0)_2$; steht;
mit Ausnahme der folgenden Verbindungen:

1-Morpholino-4-(4-(thiophen-2-yl)-6,7-dihydrothieno[3,2-c]pyridin-5(4H)-yl)butan-1,4-dion,
1-(4-Acetylpiperazin-1-yl)-4-(4-(thiophen-2-yl)-6,7-dihydrothieno[3,2-c]pyridin-5(4H)-yl)butan-1,4-dion und
1-(3-Phenyl-4,5-dihydropyrazol-1-yl)-4-(4-(thiophen-2-yl)-6,7-dihydrothieno[3,2-c]pyridin-5(4H)-yl)butan-1,4-dion;

in Form der freien Verbindungen oder Salze physiologisch verträglicher Säuren oder Basen.

2. Tetrahydrothienopyridine nach Anspruch 1, **dadurch gekennzeichnet, dass** $R^{12}$ für $C_{4-16}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; steht.

3. Tetrahydrothienopyridine nach einem der vorstehenden Ansprüche, **dadurch gekennzeichnet, dass**
$R^7$ für H; F; Cl; Br; CN; OH; $NH_2$; $C_{1-8}$-Alkyl, O-$C_{1-8}$-Alkyl, NH-$C_{1-8}$-Alkyl, N($C_{1-8}$-Alkyl)$_2$, jeweils gesättigt oder ungesättigt, verzweigt oder unverzweigt, unsubstituiert oder einfach oder mehrfach substituiert; Phenyl oder Heteroaryl, jeweils unsubstituiert oder einfach oder mehrfach substituiert; über $C_{1-2}$-Alkyl verbrücktes Phenyl oder Heteroaryl, jeweils unsubstituiert oder einfach oder mehrfach substituiert, wobei die Alkylkette jeweils verzweigt oder unverzweigt, gesättigt oder ungesättigt, unsubstituiert oder einfach oder mehrfach substituiert sein kann; steht;
$R^9$ für H; F; Cl; Br; CN; OH; $NH_2$; $C_{1-8}$-Alkyl, O-$C_{1-8}$-Alkyl, NH-$C_{1-8}$-Alkyl, N($C_{1-8}$-Alkyl)$_2$, jeweils gesättigt oder ungesättigt, verzweigt oder unverzweigt, unsubstituiert oder einfach oder mehrfach substituiert; Phenyl oder Heteroaryl, jeweils unsubstituiert oder einfach oder mehrfach substituiert; über $C_2$-Alkyl verbrücktes Phenyl oder Heteroaryl, jeweils unsubstituiert oder einfach oder mehrfach substituiert, wobei die Alkylkette jeweils verzweigt oder unverzweigt, gesättigt oder ungesättigt, unsubstituiert oder einfach oder mehrfach substituiert sein kann; steht;

4. Tetrahydrothienopyridine nach einem der vorstehenden Ansprüche, **dadurch gekennzeichnet, dass**
$R^1$ und $R^2$ jeweils H bedeuten;
oder $R^2$ H bedeutet und $R^1$ ungleich H ist.

5. Tetrahydrothienopyridine nach einem der vorstehenden Ansprüche, welche eine der allgemeinen Formeln (1a), (1b) oder (1c) aufweisen, worin

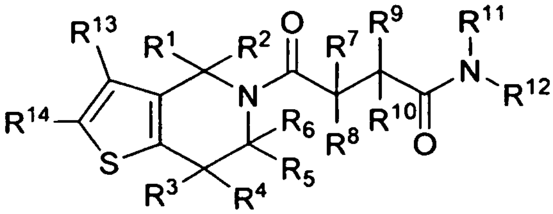

(1a)

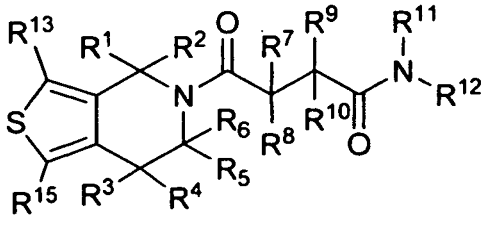

(1b)

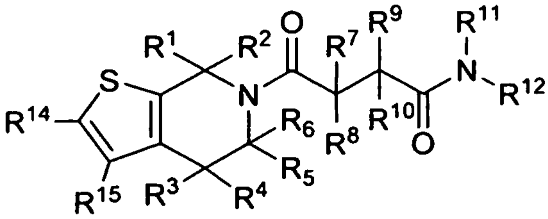

(1c)

$R^{13}$, $R^{14}$ und $R^{15}$ jeweils unabhängig voneinander H; F; Cl; Br; I; NO; $NO_2$; CN; $NH_2$, NH-$C_{1-8}$-Alkyl; N($C_{1-8}$-Alkyl)$_2$; NH-C(=O)$C_{1-8}$-Alkyl; NH-C(=O)-Aryl. NH-C(=O)-Heteroaryl; $C_{1-8}$-Alkyl; $CF_3$; CHO; C(=O)$C_{1-8}$-Alkyl; C(=O)Aryl; C(=O)Heteroaryl; $CO_2H$; C(=O)O-$C_{1-8}$-Alkyl; C(=O)O-Aryl; C(=O)O-Heteroaryl; $CONH_2$; C(=O)NH-$C_{1-8}$-Alkyl: C(=O)N($C_{1-8}$-Alkyl)2; C(=O)NH-Aryl; C(=O)N(Aryl)$_2$; C(=O)NH-Heteroaryl; C(=O)N(Heteroaryl)$_2$; C(=O)N($C_{1-8}$-Alkyl)(Aryl); C(=O)N($C_{1-8}$-Alkyl)(Heteroaryl); C(=O)N(Heteroaryl)(Aryl); OH; O-$C_{1-8}$-Alkyl; $OCF_3$; O-($C_{1-8}$-Alkyl)-O; O-($C_{1-8}$-Alkyl)-O-$C_{1-8}$-Alkyl; O-Benzyl; O-Aryl; O-Heteroaryl; O-C(=O)$C_{1-8}$-Alkyl-, O-C(=O)Aryl; O-C(=O)Heteroaryl; SH; S-$C_{1-8}$-Alkyl; $SCF_3$; S-Benzyl; S-Aryl; S-Heteroaryl; Aryl; Heteroaryl; $C_{3-7}$-Cycloalkyl; Heterocyclyl oder $C_{1-8}$-Alkyl verbrücktes Aryl, Heteroaryl, $C_{3-7}$-Cycloalkyl oder Heterocyclyl.

6. Tetrahydrothienopyridine nach einem der vorstehenden Ansprüche, worin
$R^3$, $R^4$, $R^5$ und $R^6$ unabhängig voneinander für H, $C_{1-8}$-Alkyl, gesättigt oder ungesättigt, verzweigt oder unverzweigt, unsubstituiert oder einfach oder mehrfach substituiert; oder Phenyl, unsubstituiert oder einfach oder mehrfach substituiert; stehen.

7. Tetrahydrothienopyridine nach einem der vorstehenden Ansprüche, worin
$R^8$ für H; oder $C_{1-8}$-Alkyl, gesättigt oder ungesättigt, verzweigt oder unverzweigt, unsubstituiert oder einfach oder mehrfach substituiert; steht;
$R^{10}$ für H; oder $C_{1-8}$-Alkyl, gesättigt oder ungesättigt, verzweigt oder unverzweigt, unsubstituiert oder einfach oder mehrfach substituiert; steht;

8. Tetrahydrothienopyridine nach einem der vorstehenden Ansprüche, worin
$R^{11}$ für H; $C_{1-8}$-Alkyl, gesättigt oder ungesättigt, verzweigt oder unverzweigt, $C_{3-7}$-Cycloalkyl, gesättigt oder ungesättigt; oder Benzyl, unsubstituiert oder einfach oder mehrfach substituiert; steht.

9. Tetrahydrothienopyridine nach einem der vorstehenden Ansprüche, worin
$R^{12}$ für $C_{4-16}$-Alkyl, gesättigt oder ungesättigt, verzweigt oder unverzweigt, unsubstituiert oder einfach oder mehrfach substituiert steht; oder ausgewählt ist aus den folgenden Teilstrukturen A, B oder C,

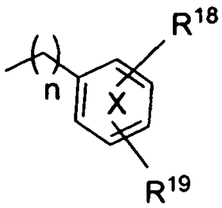

A

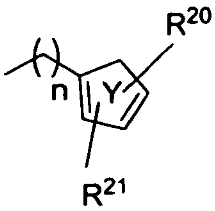

B

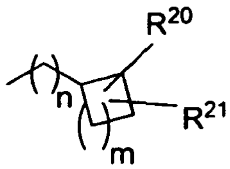

C

, wobei

n = 0, 1, 2, 3, 4, 5, 6, 7 oder 8;
m = 0, 1, 2 oder 3;
der Ring X ein oder zwei N-Atome als Ringglied(er) enthalten kann;
der Ring Y mindestens 1 Heteroatom ausgewählt aus N, O oder S enthält und bis zu 3 Heteroatome unabhängig voneinander ausgewählt aus N, O oder S enthalten kann;
$R^{18}$ und $R^{19}$ unabhängig voneinander H; F; Cl; Br; I; NO; $NO_2$; $CF_3$; CN; $R^0$; C(=O)H; C(=O)$R^0$; $CO_2H$; C(=O)O$R^0$;

$CONH_2$; $C(=O)NHR^0$; $C(=O)N(R^0)_2$; OH; $OR^0$; $O-(C_{1-8}$-Alkyl)-O; $O-C(=O)-R^0$; $O-C(=O)-O-R^0$; $O-(C=O)-NH-R^0$; $O-C(=O)-N(R^0)_2$; $O-S(=O)_2-R^0$; $O-S(=O)_2OH$; $O-S(=O)_2OR^0$; $O-S(=O)_2NH_2$; $O-S(=O)_2NHR^0$; $O-S(=O)_2N(R^0)_2$; $NH_2$; $NH-R^0$; $N(R^0)_2$; $NH-C(=O)-R^0$; $NH-C(=O)-O-R^0$; $NH-C(=O)-NH-R^0$; $NH-C(=O)-N(R^0)_2$; $NR^0-C(=O)-R^0$; $NR^0-C(=O)-O-R^0$; $NR^0-C(=O)-NH_2$; $NR)-C(=O)-NH-R^0$; $NR^0-C(=O)-N(R^0)_2$; $NH-S(=O)_2OH$; $NH-S(=O)_2R^0$; $NH-S(=O)_2OR^0$; $NH-S(=O)_2NH_2$; $NH-S(=O)_2NHR^0$; $NH-S(=O)_2N(R^0)_2$; $NR^0-S(=O)_2OH$; $NR^0-S(=O)_2R^0$; $NR^0-S(=O)_2OR^0$; $NR^0-S(=O)_2NH_2$; $NR^0-S(=O)_2NHR^0$; $NR^0-S(=O)_2N(R^0)_2$; SH; $SR^0$; $S(=O)R^0$; $S(=O)_2R^0$; $S(=O)_2OH$; $S(=O)_2OR^0$; $S(=O)_2NH_2$; $S(=O)_2NHR^0$; oder $S(=O)_2N(R^0)_2$ bedeuten;

oder $R^{18}$ und $R^{19}$ zusammen mit den sie verbindenden Kohlenstoff- oder Stickstoffatomen als Ringgliedern ein an den Phenyl-Ring kondensiertes Aryl oder Heteroaryl, jeweils unsubstituiert oder einfach oder mehrfach substituiert; oder ein an den Phenyl-Ring kondensiertes $C_{3-7}$-Cycloalkyl oder Heterocyclyl, jeweils gesättigt oder ungesättigt, unsubstituiert oder einfach oder mehrfach substituiert; bilden;

$R^{20}$ und $R^{21}$ unabhängig voneinander H oder $C_{1-8}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; bedeuten.

**10.** Tetrahydrothienopyridine nach einem der Ansprüche 1 oder 3 bis 7, worin $R^{11}$ und $R^{12}$ zusammen mit dem sie verbindenden Stickstoffatom als Ringglied einen der folgenden Ringe bilden

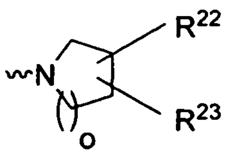 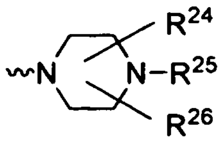 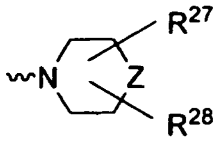 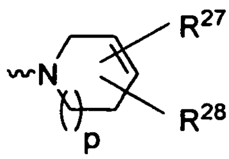 ,

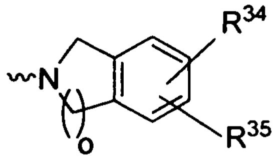 ,

worin

Z = O oder S;
o = 0, 1 oder 2;
p=0 oder 1;

$R^{22}$, $R^{23}$ , $R^{24}$, $R^{25}$ und $R^{26}$ unabhängig voneinander H; $C_{1-8}$-Alkyl, gesättigt oder ungesättigt; verzweigt oder unverzweigt, unsubstituiert oder einfach oder mehrfach substituiert; $C_{3-7}$-Cycloalkyl oder Heterocyclyl, jeweils gesättigt oder ungesättigt, unsubstituiert oder einfach oder mehrfach substituiert; Aryl oder Heteroaryl, jeweils unsubstituiert oder einfach oder mehrfach substituiert; über $C_{1-8}$-Alkyl verbrücktes $C_{3-7}$-Cycloalkyl oder Heterocyclyl, jeweils gesättigt oder ungesättigt, unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann; über $C_{1-8}$-Alkyl verbrücktes Aryl oder Heteroaryl, jeweils unsubstituiert oder einfach oder mehrfach substituiert, wobei die Alkylkette jeweils verzweigt oder unverzweigt, gesättigt oder ungesättigt, unsubstituiert, einfach oder mehrfach substituiert sein kann;

oder $R^{22}$ und $R^{23}$ mit dem oder den sie verbindenden Kohlenstoffatom(en) als Ringglied(ern) ein $C_{3-7}$-Cycloalkyl oder Heterocyclyl bilden, jeweils gesättigt oder ungesättigt, unsubstituiert oder einfach oder mehrfach substituiert, ggf. mit (Hetero-)aryl kondensiert, unsubstituiert oder einfach oder mehrfach substituiert; oder ein ankondensiertes Aryl oder Heteroaryl bilden, unsubstituiert oder einfach oder mehrfach substituiert;

$R^{27}$ und $R^{28}$ unabhängig voneinander H; $C_{1-8}$-Alkyl, gesättigt oder ungesättigt; verzweigt oder unverzweigt, unsubstituiert oder einfach oder mehrfach substituiert, bedeuten;

$R^{34}$ und $R^{35}$ unabhängig voneinander H; F; Cl; Br; I; NO; $NO_2$; CN; $NH_2$; $NH-C_{1-8}$-Alkyl; $N(C_{1-8}$-Alkyl$)_2$; $NH-C(=O)C_{1-8}$-Alkyl; NH-C(=O)-Aryl; NH-C(=O)-Heteroaryl; $C_{1-8}$-Alkyl; $CF_3$; C(=O)H; $C(=O)C_{1-8}$-Alkyl; C(=O)Aryl; C(=O)Heteroaryl; $CO_2H$; $C(=O)O-C_{1-8}$-Alkyl; C(=O)O-Aryl; C(=O)O-Heteroaryl; $CONH_2$; $C(=O)NH-C_{1-8}$-Alkyl; $C(=O)N(C_{1-8}$-Alkyl$)_2$; C(=O)NH-Aryl; $C(=O)N(Aryl)_2$; C(=O)NH-Heteroaryl; C(=O)N(Hete-

roaryl)$_2$; C(=O)N($C_{1-8}$-Alkyl)(Aryl); C(=O)N($C_{1-8}$-Alkyl)(Heteroaryl); C(=O)N(Aryl)(Heteroaryl); OH; O-$C_{1-8}$-Alkyl; O-$C_{1-8}$-Alkyl-OH; $OCF_3$; O-($C_{1-8}$-Alkyl)-O-$C_{1-8}$-Alkyl; O-Benzyl; O-Aryl; O-Heteroaryl; O-C(=O)$C_{1-8}$-Alkyl; O-C(=O)Aryl; O-C(=O)Heteroaryl; SH; S-$C_{1-8}$-Alkyl; $SCF_3$; S-Benzyl; S-Aryl; S-Heteroaryl; Aryl; Heteroaryl; $C_{3-7}$-Cycloalkyl; Heterocyclyl; oder $C_{1-8}$-Alkyl verbrücktes Aryl, Heteroaryl, $C_{3-7}$-Cycloalkyl oder Heterocyclyl, bedeuten;

**11.** Tetrahydrothienopyridine nach Anspruch 1 ausgewählt aus der Gruppe

| | |
|---|---|
| **1** | 4-Oxo-4-(4-(thiophen-2-yl)-6,7-dihydrothieno[3,2-c]pyridin-5(4H)-yl)-N-(3-(trifluormethyl)benzyl) butanamid; |
| **2** | 4-Oxo-4-(4-phenyl-6,7-dihydrothieno[3,2-c]pyridin-5(4H)-yl)-N-(3-(trifluormethyl)benzyl)butanamid; |
| **3** | 4-(4-(3-Fluorphenyl)-6,7-dihydrothieno[3,2-c]pyridin-5(4H)-yl)-4-oxo-N-(3-(trifluormethyl)benzyl) butanamid; |
| **4** | 4-Oxo-4-(4-m-tolyl-6,7-dihydrothieno[3,2-c]pyridin-5(4H)-yl)-N-(3-(trifluormethyl)benzyl)butanamid; |
| **5** | 4-Oxo-4-(4-p-tolyl-6,7-dihydrothieno[3,2-c]pyridin-5(4H)-yl)-N-(3-(trifluormethyl)benzyl)butanamid; |
| **6** | 4-(4-(2-Fluorphenyl)-6,7-dihydrothieno[3,2-c]pyridin-5(4H)-yl)-4-oxo-N-(3-(trifluormethyl)benzyl) butanamid; |
| **7** | 4-(4-(4-Fluorphenyl)-6,7-dihydrothieno[3,2-c]pyridin-5(4H)-yl)-4-oxo-N-(3-(trifluormethyl)benzyl) butanamid; |
| **8** | 4-Oxo-4-(4-o-tolyl-6,7-dihydrothieno[3,2-c]pyridin-5(4H)-yl)-N-(3-(trifluormethyl)benzyl)butanamid; |
| **9** | 4-Oxo-4-(7-phenyl-4,5-dihydrothieno[2,3-c]pyridin-6(7H)-yl)-N-(3-(trifluormethyl)benzyl)butanamid; |
| **10** | 4-(2-Methyl-4-phenyl-6,7-dihydrothieno[3,2-c]pyridin-5(4H)-yl)-4-oxo-N-(3-(trifluormethyl)benzyl) butanamid; |
| **11** | N-(4-Methylbenzyl)-4-oxo-4-(4-phenyl-6,7-dihydrothieno[3,2-c]pyridin-5(4H)-yl)butanamid; |
| **12** | N-Benzyl-4-oxo-4-(4-phenyl-6,7-dihydrothieno[3,2-c]pyridin-5(4H)-yl)butanamid; |
| **13** | 4-Oxo-4-(4-phenyl-6,7-dihydrothieno[3,2-c]pyridin-5(4H)-yl)-N-(4-(trifluormethyl)benzyl)butanamid; |
| **14** | N-(2-Methoxybenzyl)-4-oxo-4-(4-phenyl-6,7-dihydrothieno[3,2-c]pyridin-5(4H)-yl)butanamid; |
| **15** | N-(3-Methoxybenzyl)-4-oxo-4-(4-phenyl-6,7-dihydrothieno[3,2-c]pyridin-5(4H)-yl)butanamid; |
| **16** | N-(4-Methoxybenzyl)-4-oxo-4-(4-phenyl-6,7-dihydrothieno[3,2-c]pyridin-5(4H)-yl)butanamid; |
| **17** | 4-Oxo-4-(4-phenyl-6,7-dihydrothieno[3,2-c]pyridin-5(4H)-yl)-N-(2-(trifluormethyl)benzyl)butanamid; |
| **18** | N-(3-Fluorbenzyl)-4-oxo-4-(4-phenyl-6,7-dihydrothieno[3,2-c]pyridin-5(4H)-yl)butanamid; |
| **19** | N-(3-Methylbenzyl)-4-oxo-4-(4-phenyl-6,7-dihydrothieno[3,2-c]pyridin-5(4H)-yl)butanamid; |
| **20** | 4-(3-Methyl-4-phenyl-6,7-dihydrothieno[3,2-c]pyridin-5(4H)-yl)-4-oxo-N-(3-(trifluormethyl)benzyl) butanamid; |
| **21** | 4-(4-Cyclohexyl-6,7-dihydrothieno[3,2-c]pyridin-5(4H)-yl)-4-oxo-N-(3-(trifluormethyl)benzyl)butanamid; |
| **22** | 4-(4-Isopropyl-6,7-dihydrothieno[3,2-c]pyridin-5(4H)-yl)-4-oxo-N-(3-(trifluormethyl)benzyl)butanamid; |
| **23** | 4-(4-Butyl-6,7-dihydrothieno[3,2-c]pyridin-5(4H)-yl)-4-oxo-N-(3-(trifluormethyl)benzyl)butanamid; |
| **24** | N-Methyl-4-oxo-4-(4-phenyl-6,7-dihydrothieno[3,2-c]pyridin-5(4H)-yl)-N-(3-(trifluormethyl)benzyl) butanamid; |
| **25** | 4-Oxo-N-phenethyl-4-(4-phenyl-6,7-dihydrothieno[3,2-c]pyridin-5(4H)-yl)butanamid; |
| **26** | N-(2-Methylbenzyl)-4-oxo-4-(4-phenyl-6,7-dihydrothieno[3,2-c]pyridin-5(4H)-yl)butanamid; |
| **27** | 4-Oxo-2-phenyl-4-(4-phenyl-6,7-dihydrothieno[3,2-c]pyridin-5(4H)-yl)-N-(3-(trifluormethyl)benzyl) butanamid; |
| **28** | (R)-2-Methyl-4-oxo-4-(4-phenyl-6,7-dihydrothieno[3,2-c]pyridin-5(4H)-yl)-N-(3-(trifluormethyl)benzyl) butanamid; |
| **29** | 4-Oxo-3-phenyl-4-(4-phenyl-6,7-dihydrothieno[3,2-c]pyridin-5(4H)-yl)-N-(3-(trifluormethyl)benzyl) butanamid; |
| **30** | 4-(6,7-Dihydrothieno[3,2-c]pyridin-5(4H)-yl)-4-oxo-3-phenyl-N-(3-(trifluormethyl)benzyl)butanamid; |
| **31** | rac. (1S,2R)-2-(4-Phenyl-4,5,6,7-tetrahydrothieno[3,2-c]pyridin-5-carbonyl)-N-(3-(trifluormethyl)benzyl) cyclohexancarboxamid; |
| **32** | rac. (1S)-2-(4-Phenyl-4,5,6,7-tetrahydrothieno[3,2-c]pyridin-5-carbonyl)-N-(3-(trifluormethyl)benzyl) cyclohexancarboxamid; |
| **33** | rac. (1S,2R)-2-(4-Phenyl-4,5,6,7-tetrahydrothieno[3,2-c]pyridin-5-carbonyl)-N-(3-(trifluormethyl)benzyl) cyclopentanecarboxamid; |

(fortgesetzt)

| | |
|---|---|
| **34** | (R)-3-Methyl-4-oxo-4-(4-phenyl-6,7-dihydrothieno[3,2-c]pyridin-5(4H)-yl)-N-(3-(trifluormethyl)benzyl) butanamid; |
| **35** | (-)-4-Oxo-4-(4-phenyl-6,7-dihydrothieno[3,2-c]pyridin-5(4H)-yl)-N-(3-(trifluormethyl)benzyl)butanamid; |
| **36** | (+)-4-Oxo-4-(4-phenyl-6,7-dihydrothieno[3,2-c]pyridin-5(4H)-yl)-N-(3-(trifluormethyl)benzyl)butanamid; |
| **37** | 4-(6,7-Dihydrothieno[3,2-c]pyridin-5(4H)-yl)-4-oxo-2-phenyl-N-(3-(trifluormethyl)benzyl)butanamid; |
| **38** | N-(3,5-Bis(trifluormethyl)benzyl)-4-oxo-4-(4-phenyl-6,7-dihydrothieno[3,2-c]pyridin-5(4H)-yl)butanamid; |
| **39** | N-(2-Fluor-5-(trifluormethyl)benzyl)-4-oxo-4-(4-phenyl-6,7-dihydrothieno[3,2-c]pyridin-5(4H)-yl) butanamid; |
| **40** | N-(2-Fluor-3-(trifluormethyl)benzyl)-4-oxo-4-(4-phenyl-6,7-dihydrothieno(3,2-c]pyridin-5(4H)-yl) butanamid; |
| **41** | N-(3-Fluor-5-(trifluormethyl)benzyl)-4-oxo-4-(4-phenyl-6,7-dihydrothieno[3,2-c]pyridin-5(4H)-yl) butanamid; |
| **42** | rac. (1S,2R)-2-(4-Phenyl-4,5,6,7-tetrahydrothieno[3,2-c]pyridin-5-carbonyl)-N-(3-(trifluormethyl)benzyl) cyclopropancarboxamid; |
| **43** | N-(3,4-Difluorbenzyl)-4-oxo-4-(4-phenyl-6,7-dihydrothieno[3,2-c]pyridin-5(4H)-yl)butanamid; |
| **44** | 4-Oxo-4-(5-phenyl-4,5-dihydrothieno[2,3-c]pyridin-6(7H)-yl)-N-(3-(trifluormethyl)benzyl)butanamid; |
| **45** | 4-(1-Methyl-4-phenyl-6,7-dihydrothieno[3,4-c]pyridin-5(4H)-yl)-4-oxo-N-(3-(trifluormethyl)benzyl) butanamid; |
| **46** | N-(4-Methoxyphenyl)-4-oxo-4-(4-p-tolyl-6,7-dihydrothieno[3,2-c]pyridin-5(4H)-yl)butanamid; |
| **47** | N-(1-Methyl-1H-indazol-6-yl)-4-oxo-4-(4-p-tolyl-6,7-dihydrothieno[3,2-c]pyridin-5(4H)-yl)butanamid; |
| **48** | N-Benzyl-4-oxo-4-(4-p-tolyl-6,7-dihydrothieno[3,2-c]pyridin-5(4H)-yl)butanamid; |
| **49** | 4-Oxo-N-phenethyl-4-(4-p-tolyl-6,7-dihydrothieno[3,2-c]pyridin-5(4H)-yl)butanamid; |
| **50** | 4-Oxo-N-(pyridin-4-ylmethyl)-4-(4-p-tolyl-6,7-dihydrothieno[3,2-c]pyridin-5(4H)-yl)butanamid; |
| **51** | 4-Oxo-N-(3-phenylpropyl)-4-(4-p-tolyl-6,7-dihydrothieno[3,2-c]pyridin-5(4H)-yl)butanamid; |
| **52** | N-(Benzo[c][1,2,5]thiadiazol-4-yl)-4-oxo-4-(4-m-tolyl-6,7-dihydrothieno[3,2-c]pyridin-5(4H)-yl)butanamid; |
| **53** | N-(1-Methyl-1H-indazol-6-yl)-4-oxo-4-(4-m-tolyl-6,7-dihydrothieno[3,2-c]pyridin-5(4H)-yl)butanamid; |
| **54** | 4-Oxo-N-(pyridin-2-ylmethyl)-4-(4-m-tolyl-6,7-dihydrothieno[3,2-c]pyridin-5(4H)-yl)butanamid; |
| **55** | 1-(4-Methylpiperazin-1-yl)-4-(4-m-tolyl-6,7-dihydrothieno[3,2-c]pyridin-5(4H)-yl)butan-1,4-dion; |
| **56** | 3-Methyl-4-oxo-4-(4-phenyl-6,7-dihydrothieno[3,2-c]pyridin-5(4H)-yl)-N-(pyridin-2-ylmethyl)butanamid; |
| **57** | 4-Oxo-4-(4-phenyl-6,7-dihydrothieno[3,2-c]pyridin-5(4H)-yl)-N-(thiophen-2-ylmethyl)butanamid; |
| **58** | N-(2-Chlorphenyl)-4-(4-(4-fluorphenyl)-6,7-dihydrothieno[3,2-c]pyridin-5(4H)-yl)-4-oxobutanamid; |
| **59** | 4-(4-(4-Chlorphenyl)-6,7-dihydrothieno[3,2-c]pyridin-5(4H)-yl)-N-(furan-2-ylmethyl)-4-oxobutanamid; |
| **60** | 4-(4-(4-Chlorphenyl)-6,7-dihydrothieno[3,2-c)pyridin-5(4H)-yl)-4-oxo-N-propylbutanamid; |
| **61** | N-(2-Chlorbenzyl)-4-oxo-4-(4-m-tolyl-6,7-dihydrothieno[3,2-c]pyridin-5(4H)-yl)butanamid; |
| **62** | N-(2,4-Dichlorbenzyl)-4-(4-(3-fluorphenyl)-6,7-dihydrothieno[3,2-c]pyridin-5(4H)-yl)-4-oxobutanamid; |
| **63** | N-(4-Fluorbenzyl)-4-(4-(3-fluorphenyl)-6,7-dihydrothieno[3,2-c]pyridin-5(4H)-yl)-4-oxobutanamid; |
| **64** | N-(3,4-Dichlorbenzyl)-3-methyl-4-oxo-4-(4-phenyl-6,7-dihydrothieno[3,2-c]pyridin-5(4H)-yl)butanamid; |
| **65** | N-(2,5-Difluorbenzyl)-3-methyl-4-oxo-4-(4-phenyl-6,7-dihydrothieno[3,2-c]pyridin-5(4H)-yl)butanamid; |
| **66** | 3-Methyl-4-oxo-4-(4-phenyl-6,7-dihydrothieno[3,2-c]pyridin-5(4H)-yl)-N-(3-(trifluormethyl)benzyl) butanamid; |
| **67** | 1-(4-(4-Chlorphenyl)-6, 7-d ihydrothieno[3,2-c]pyridin-5(4H)-yl)-4-morpholinobutan-1,4-dion; |
| **68** | 1-(4-(4-Chlorphenyl)-6, 7-d ihydrothieno[3,2-c]pyridin-5(4H)-yl)-4-(4-(4-fluorphenyl)piperazin-1-yl)butan-1,4-dion; |
| **69** | 2-Methyl-N-(2-(5-methyl-1H-pyrazol-1-yl)ethyl)-4-oxo-4-(4-phenyl-6,7-dihydrothieno[3,2-c]pyridin-5 (4H)-yl)butanamid; |
| **70** | N-(Naphthalen-1-ylmethyl)-4-oxo-4-(4-phenyl-6,7-dihydrothieno[3,2-c]pyridin-5(4H)-yl)butanamid; |
| **71** | 4-(4-(4-Fluorphenyl)-6,7-dihydrothieno[3,2-c]pyridin-5(4H)-yl)-N-(4-methylbenzyl)-4-oxobutanamid; |
| **72** | N-(Benzo[d][1,3]dioxol-5-ylmethyl)-4-(4-(4-fluorphenyl)-6,7-dihydrothieno[3,2-c]pyridin-5(4H)-yl)-4-oxobutanamid; |
| **73** | 4-(4-(4-Fluorphenyl)-6,7-dihydrothieno[3,2-c]pyridin-5(4H)-yl)-4-oxo-N-(2-(trifluormethyl)benzyl) butanamid; |

(fortgesetzt)

| | |
|---|---|
| **74** | 4-(4-(4-Fluorphenyl)-6,7-dihydrothieno[3,2-c]pyridin-5(4H)-yl)-N-(naphthalen-1-ylmethyl)-4-oxobutanamid; |
| **75** | 4-(4-(4-Chlorphenyl)-6,7-dihydrothieno[3,2-c]pyridin-5(4H)-yl)-N-(3-fluorbenzyl)-4-oxobutanamid; |
| **76** | 2-Methyl-N-(1-methyl-1H-indazol-6-yl)-4-oxo-4-(4-phenyl-6,7-dihydrothieno[3,2-c]pyridin-5(4H)-yl) butanamid; |
| **77** | N-(4-Methoxybenzyl)-2-methyl-4-oxo-4-(4-phenyl-6,7-dihydrothieno[3,2-c]pyridin-5(4H)-yl)butanamid; |
| **78** | 2-Methyl-1-(4-methylpiperazin-1-yl)-4-(4-phenyl-6,7-dihydrothieno[3,2-c]pyridin-5(4H)-yl)butan-1,4-dion; |
| **79** | N-(2-(1H-Indol-3-yl)ethyl)-2-methyl-4-oxo-4-(4-phenyl-6,7-dihydrothieno[3,2-c]pyridin-5(4H)-yl) butanamid; |
| **80** | N-(2-Fluorbenzyl)-2-methyl-4-oxo-4-(4-phenyl-6,7-dihydrothieno[3,2-c]pyridin-5(4H)-yl)butanamid; |
| **81** | 2-Methyl-4-oxo-4-(4-phenyl-6,7-dihydrothieno[3,2-c]pyridin-5(4H)-yl)-N-(2-(trifluormethyl)benzyl) butanamid; |
| **82** | 4-Oxo-N-(2-(piperidin-1-yl)ethyl)-4-(4-p-tolyl-6,7-dihydrothieno[3,2-c]pyridin-5(4H)-yl)butanamid; |
| **83** | 4-Oxo-N-((tetrahydrofuran-2-yl)methyl)-4-(4-p-tolyl-6,7-dihydrothieno[3,2-c]pyridin-5(4H)-yl)butanamid; |
| **84** | N-(4-Chlorbenzyl)-4-oxo-4-(4-p-tolyl-6,7-dihydrothieno[3,2-c]pyridin-5(4H)-yl)butanamid; |
| **85** | N-(2,3-Dichlorbenzyl)-4-oxo-4-(4-p-tolyl-6,7-dihydrothieno[3,2-c]pyridin-5(4H)-yl)butanamid; |
| **86** | 4-Oxo-N-(2-(thiophen-2-yl)ethyl)-4-(4-m-tolyl-6,7-dihydrothieno[3,2-c]pyridin-5(4H)-yl)butanamid; |
| **87** | N-(Cyclohexylmethyl)-4-oxo-4-(4-m-tolyl-6,7-dihydrothieno[3,2-c]pyridin-5(4H)-yl)butanamid; |
| **88** | N-(3-Chtorphenethyl)-4-oxo-4-(4-m-tolyl-6,7-dihydrothieno[3,2-c]pyridin-5(4H)-yl)butanamid; |
| **89** | N-(3,3-Diphenylpropyl)-4-oxo-4-(4-m-tolyl-6,7-dihydrothieno[3,2-c]pyridin-5(4H)-yl)butanamid; |
| **90** | 4-(4-(3-Fluorphenyl)-6,7-dihydrothieno[3,2-c]pyridin-5(4H)-yl)-N-(3-morpholinopropyl)-4-oxobutanamid; |
| **91** | 4-(4-(3-Fluorphenyl)-6,7-dihydrothieno[3,2-c]pyridin-5(4H)-yl)-4-oxo-N-(pyridin-3-ylmethyl)butanamid; |
| **92** | 4-(4-(3-Fluorphenyl)-6,7-dihydrothieno[3,2-c]pyridin-5(4H)-yl)-N-(3-methylbenzyl)-4-oxobutanamid; |
| **93** | 4-(4-(3-Fluorphenyl)-6,7-dihydrothieno[3,2-c]pyridin-5(4H)-yl)-N-(4-methylphenethyl)-4-oxobutanamid; |
| **94** | 3-Methyl-4-oxo-4-(4-phenyl-6,7-dihydrothieno[3,2-c]pyridin-5(4H)-yl)-N-(4-phenylbutyl)butanamid; |
| **95** | N-(Biphenyl-4-ylmethyl)-4-oxo-4-(4-m-tolyl-6,7-dihydrothieno[3,2-c]pyridin-5(4H)-yl)butanamid; |
| **96** | 4-(4-(3-Fluorphenyl)-6,7-dihydrothieno[3,2-c]pyridin-5(4H)-yl)-N-(2-methoxybenzyl)-4-oxobutanamid; |
| **97** | N-(4-Chlorphenethyl)-4-(4-(3-fluorphenyl)-6,7-dihydrothieno[3,2-c]pyridin-5(4H)-yl)-4-oxobutanamid; |
| **98** | 4-(4-(3-Fluorphenyl)-6,7-dihydrothieno[3,2-c]pyridin-5(4H)-yl)-N-((5-methyl-3-phenylisoxazol-4-yl) methyl)-4-oxobutanamid; |
| **99** | N-(2,6-Difluorbenzyl)-3-methyl-4-oxo-4-(4-phenyl-6,7-dihydrothieno[3,2-c]pyridin-5(4H)-yl)butanamid; |
| **100** | N-(3,5-Difluorbenzyl)-3-methyl-4-oxo-4-(4-phenyl-6,7-dihydrothieno[3,2-c]pyridin-5(4H)-yl)butanamid; |
| **101** | N-(3-Chlorbenzyl)-3-methyl-4-oxo-4-(4-phenyl-6,7-dihydrothieno[3,2-c]pyridin-5(4H)-yl)butanamid; |
| **102** | N-(3,5-Dimethoxyphenethyl)-3-methyl-4-oxo-4-(4-phenyl-6,7-dihydrothieno[3,2-c]pyridin-5(4H)-yl) butanamid; |
| **103** | N-(3,4-Difluorbenzyl)-4-oxo-4-(4-phenyl-6,7-dihydrothieno[3,2-c]pyridin-5(4H)-yl)butanamid; |
| **104** | N-(2,4-Dichlorphenethyl)-4-oxo-4-(4-phenyl-6,7-dihydrothieno[3,2-c]pyridin-5(4H)-yl)butanamid; |
| **105** | N-(2-(1H-1,2,4-Triazol-1-yl)ethyl)-4-oxo-4-(4-phenyl-6,7-dihydrothieno[3,2-c]pyridin-5(4H)-yl)butanamid; |
| **106** | 4-(4-(4-Fluorphenyl)-6,7-dihydrothieno[3,2-c]pyridin-5(4H)-yl)-N-(2-methylbenzyl)-4-oxobutanamid; |
| **107** | N-(4-Fluorphenethyl)-4-(4-(4-fluorphenyl)-6,7-dihydrothieno[3,2-c]pyridin-5(4H)-yl)-4-oxobutanamid; |
| **108** | 4-(4-(4-Fluorphenyl)-6,7-dihydrothieno[3,2-c]pyridin-5(4H)-yl)-N-((5-methylisoxazol-3-yl)methyl)-4-oxobutanamid; |
| **109** | N-(2-Chlorphenethyl)-4-(4-(4-fluorphenyl)-6,7-dihydrothieno[3,2-c]pyridin-5(4H)-yl)-4-oxobutanamid; |
| **110** | 4-(4-(4-Chlorphenyl)-6,7-dihydrothieno[3,2-c]pyridin-5(4H)-yl)-N-(2-cyclohexenylethyl)-4-oxobutanamid; |
| **111** | 4-(4-(4-Chlorphenyl)-6,7-dihydrothieno[3,2-c]pyridin-5(4H)-yl)-N-(3,5-dimethoxybenzyl)-4-oxobutanamid; |
| **112** | 4-(4-(4-Chlorphenyl)-6,7-dihydrothieno[3,2-c]pyridin-5(4H)-yl)-N-(3,4-dichlorphenethyl)-4-oxobutanamid; |
| **113** | 4-(4-(4-Chlorphenyl)-6,7-dihydrothieno[3,2-c]pyridin-5(4H)-yl)-N-(3-fluorphenethyl)-4-oxobutanamid; |
| **114** | 1-(3-Phenylpiperidin-1-yl)-4-(4-p-tolyl-6,7-dihydrothieno[3,2-c]pyridin-5(4H)-yl)butan-1,4-dion; |
| **115** | 1-(4-Benzylpiperazin-1-yl)-4-(4-p-tolyl-6,7-dihydrothieno[3,2-c]pyridin-5(4H)-yl)butan-1,4-dion; |
| **116** | 1-(4-(4-Methoxyphenyl)piperazin-1-yl)-4-(4-p-tolyl-6,7-dihydrothieno[3,2-c]pyridin-5(4H)-yl)butan-1,4-dion; |

(fortgesetzt)

| | |
|---|---|
| **117** | 1-(2-Benzylpiperidin-1-yl)-4-(4-m-tolyl-6,7-dihydrothieno[3,2-c]pyridin-5(4H)-yl)butan-1,4-dion; |
| **118** | 1-(4-(2-Fluorphenyl)piperazin-1-yl)-4-(4-m-tolyl-6,7-dihydrothieno[3,2-c]pyridin-5(4H)-yl)butan-1,4-dion; |
| **119** | 1-(4-(Cyclohexylmethyl)piperazin-1-yl)-4-(4-m-tolyl-6,7-dihydrothieno[3,2-c]pyridin-5(4H)-yl)butan-1,4-dion; |
| **120** | N-(3-(1H-Imidazol-1-yl)propyl)-4-(4-(3-fluorphenyl)-6,7-dihydrothieno[3,2-c]pyridin-5(4H)-yl)-4-oxobutanamid; |
| **121** | 1-(4-(3-Fluorphenyl)-6,7-dihydrothieno[3,2-c]pyridin-5(4H)-yl)-4-(3-phenylpyrrolidin-1-yl)butan-1,4-dion; |
| **122** | 1-(4-(3-Fluorphenyl)-6,7-dihydrothieno[3,2-c]pyridin-5(4H)-yl)-4-(4-(furan-2-carbonyl)piperazin-1-yl) butan-1,4-dion; |
| **123** | N-(3-(3,4-Dihydroquinolin-1(2H)-yl)propyl)-3-methyl-4-oxo-4-(4-phenyl-6,7-dihydrothieno[3,2-c]pyridin-5(4H)-yl)butanamid; |
| **124** | 2-Methyl-1-(4-phenyl-6,7-dihydrothieno[3,2-c]pyridin-5(4H)-yl)-4-(piperidin-1-yl)butan-1,4-dion; |
| **125** | 4-(4-(2-Methoxyphenyl)piperazin-1-yl)-2-methyl-1-(4-phenyl-6,7-dihydrothieno[3,2-c]pyridin-5(4H)-yl) butan-1,4-dion; |
| **126** | 4-Oxo-4-(4-phenyl-6,7-dihydrothieno[3,2-c]pyridin-5(4H)-yl)-N-(2-(pyrazin-2-yl)ethyl)butanamid; |
| **127** | N,N-Diethyl-4-oxo-4-(4-phenyl-6,7-dihydrothieno[3,2-c]pyridin-5(4H)-yl)butanamid; |
| **128** | 1-(4-Phenyl-6,7-dihydrothieno[3,2-c]pyridin-5(4H)-yl)-4-(4-(pyridin-2-yl)piperazin-1-yl)butan-1,4-dion; |
| **129** | 1-(4-(4-Fluorphenyl)-6,7-dihydrothieno[3,2-c]pyridin-5(4H)-yl)-4-(4-(3-methoxyphenyl)piperazin-1-yl) butan-1,4-dion; |
| **130** | 1-(4-Isopropylpiperazin-1-yl)-4-(4-p-tolyl-6,7-dihydrothieno[3,2-c]pyridin-5(4H)-yl)butan-1,4-dion; |
| **131** | 1-(4-p-Tolyl-6,7-dihydrothieno[3,2-c]pyridin-5(4H)-yl)-4-(4-(3-(trifluormethyl)phenyl)piperazin-1-yl)butan-1,4-dion; |
| **132** | 1-(4-Phenylpiperazin-1-yl)-4-(4-m-tolyl-6,7-dihydrothieno[3,2-c]pyridin-5(4H)-yl)butan-1,4-dion; |
| **133** | 1-(3,5-Dimethylpiperidin-1-yl)-4-(4-m-tolyl-6,7-dihydrothieno[3,2-c]pyridin-5(4H)-yl)butan-1,4-dion; |
| **134** | 1-(5,6-Dihydropyridin-1 (2H)-yl)-4-(4-(3-fluorphenyl)-6,7-dihydrothieno[3,2-c]pyridin-5(4H)-yl)butan-1,4-dion; |
| **135** | 1-(4-(3-Fluorphenyl)-6,7-dihydrothieno[3,2-c]pyridin-5(4H)-yl)-4-(4-(pyrimidin-2-yl)piperazin-1-yl)butan-1,4-dion; |
| **136** | 1-(4-(3-Fluorphenyl)-6,7-dihydrothieno[3,2-c]pyridin-5(4H)-yl)-4-(3-methylpiperidin-1-yl)butan-1,4-dion; |
| **137** | N-Ethyl-4-(4-(3-fluorphenyl)-6,7-dihydrothieno[3,2-c]pyridin-5(4H)-yl)-4-oxo-N-(pyridin-4-ylmethyl) butanamid; |
| **138** | 4-(4-Acetylpiperazin-1-yl)-2-methyl-1-(4-phenyl-6,7-dihydrothieno[3,2-c]pyridin-5(4H)-yl)butan-1,4-dion; |
| **139** | 4-(2,6-Dimethylmorpholino)-2-methyl-1-(4-phenyl-6,7-dihydrothieno[3,2-c]pyridin-5(4H)-yl)butan-1,4-dion; |
| **140** | 4-(2,5-Dihydro-1H-pyrrol-1-yl)-2-methyl-1-(4-phenyl-6,7-dihydrothieno[3,2-c]pyridin-5(4H)-yl)butan-1,4-dion; |
| **141** | N-(2-Methoxyphenethyl)-4-oxo-4-(4-phenyl-6,7-dihydrothieno[3,2-c]pyridin-5(4H)-yl)butanamid; |
| **142** | 4-(4-(4-Fluorphenyl)-6,7-dihydrothieno[3,2-c]pyridin-5(4H)-yl)-N-isopentyl-4-oxobutanamid; |
| **143** | N-(2,4-Dimethoxybenzyl)-4-(4-(4-fluorphenyl)-6,7-dihydrothieno[3,2-c]pyridin-5(4H)-yl)-4-oxobutanamid; |
| **144** | 4-(4-(4-Chlorphenyl)-6,7-dihydrothieno[3,2-c]pyridin-5(4H)-yl)-N-((5-methylfuran-2-yl)methyl)-4-oxobutanamid; |
| **145** | 4-(4-(4-Chlorphenyl)-6,7-dihydrothieno[3,2-c]pyridin-5(4H)-yl)-4-oxo-N-pentylbutanamid; |
| **146** | 1-(4-Benzylpiperidin-1-yl)-4-(4-(4-chlorphenyl)-6,7-dihydrothieno[3,2-c]pyridin-5(4H)-yl)butan-1,4-dion; |
| **147** | 1-(4-(4-Chlorphenyl)-6,7-dihydrothieno[3,2-c]pyridin-5(4H)-yl)-4-(4-methylpiperidin-1-yl)butan-1,4-dion; |
| **148** | 1-(Azetidin-1-yl)-4-(4-(4-chlorphenyl)-6,7-dihydrothieno[3,2-c]pyridin-5(4H)-yl)butan-1,4-dion; |
| **149** | 1-(3,4-Dihydroisoquinolin-2(1H)-yl)-2-methyl-4-(4-phenyl-6,7-dihydrothieno[3,2-c]pyridin-5(4H)-yl)butan-1,4-dion; |
| **150** | 1-(4-(Benzo[d][1,3]dioxol-5-ylmethyl)piperazin-1-yl)-2-methyl-4-(4-phenyl-6,7-dihydrothieno[3,2-c]pyridin-5(4H)-yl)butan-1,4-dion; |
| **151** | N-Benzyl-N-ethyl-4-oxo-4-(4-p-tolyl-6,7-dihydrothieno[3,2-c]pyridin-5(4H)-yl)butanamid; |
| **152** | N-Methyl-4-oxo-N-phenethyl-4-(4-p-tolyl-6,7-dihydrothieno[3,2-c]pyridin-5(4H)-yl)butanamid; |
| **153** | Ethyl 1-(4-oxo-4-(4-p-tolyl-6,7-dihydrothieno[3,2-c]pyridin-5(4H)-yl)butanoyl)piperidin-4-carboxylat; |

(fortgesetzt)

| | |
|---|---|
| **154** | (E)-1-(4-(3-Phenylprop-2-enyl)-piperazin-1-yl)-4-(4-p-tolyl-6,7-dihydrothieno[3,2-c]pyridin-5(4H)-yl)butan-1,4-dion; |
| **155** | 1-(2-(Thiazol-2-yl)pyrrolidin-1-yl)-4-(4-p-tolyl-6,7-dihydrothieno[3,2-c]pyridin-5(4H)-yl)butan-1,4-dion; |
| **156** | N-Cyclohexyl-N-ethyl-4-oxo-4-(4-m-tolyl-6,7-dihydrothieno[3,2-c]pyridin-5(4H)-yl)butanamid; |
| **157** | N-Ethyl-N-isopropyl-4-oxo-4-(4-m-tolyl-6,7-dihydrothieno[3,2-c]pyridin-5(4H)-yl)butanamid; |
| **158** | 1-(Pyrrolidin-1-yl)-4-(4-m-tolyl-6,7-dihydrothieno[3,2-c]pyridin-5(4H)-yl)butan-1,4-dion; |
| **159** | 1-(4-(Pyrid in-4-yl)piperazin-1-yl)-4-(4-m-tolyl-6, 7-d ihydrothieno[3, 2-c]pyridin-5(4H)-yl)butan-1,4-dion; |
| **160** | 1-(2-(Pyridin-2-ylmethyl)pyrrolidin-1-yl)-4-(4-m-tolyl-6,7-dihydrothieno[3,2-c]pyridin-5(4H)-yl)butan-1,4-dion; |
| **161** | Ethyl 1-(4-(4-(4-fluorphenyl)-6,7-dihydrothieno[3,2-c]pyridin-5(4H)-yl)-4-oxobutanoyl)piperidin-3-carboxylat; |
| **162** | 1-(4-(4-Fluorphenyl)-6,7-dihydrothieno[3,2-c]pyridin-5(4H)-yl)-4-(4-(4-(trifluormethyl)phenyl)piperazin-1-yl)butan-1,4-dion; |
| **163** | 1-(2-(5-Brompyridin-3-yl)pyrrolidin-1-yl)-4-(4-(4-fluorphenyl)-6,7-dihydrothieno[3,2-c]pyridin-5(4H)-yl) butan-1,4-dion; |
| **164** | 1-(4-Ethylpiperazin-1-yl)-4-(4-phenyl-6,7-dihydrothieno[3,2-c]pyridin-5(4H)-yl)butan-1,4-dion; |
| **165** | 1-(2-Ethylpiperidin-1-yl)-4-(4-phenyl-6,7-dihydrothieno[3,2-c]pyridin-5(4H)-yl)butan-1,4-dion; |
| **166** | N,N-Dibenzyl-4-oxo-4-(4-phenyl-6,7-dihydrothieno[3,2-c]pyridin-5(4H)-yl)butanamid; |
| **167** | N,N-Diisobutyl-4-oxo-4-(4-phenyl-6,7-dihydrothieno[3,2-c]pyridin-5(4H)-yl)butanamid; |
| **168** | 1-(3,4-Dihydroquinolin-1 (2H)-yl)-4-(4-(4-fluorphenyl)-6,7-dihydrothieno[3,2-c]pyridin-5(4H)-yl)butan-1,4-dion; |
| **169** | 1-(4-(3,4-Dichlorbenzyl)piperazin-1-yl)-4-(4-p-tolyl-6,7-dihydrothieno[3,2-c]pyridin-5(4H)-yl)butan-1,4-dion; |
| **170** | 1-(4-p-Tolyl-6,7-dihydrothieno[3,2-c]pyridin-5(4H)-yl)-4-(4-(2,4,6-trimethylbenzyl)piperazin-1-yl)butan-1,4-dion; |
| **171** | 1-(4-(4-Brombenzyl)piperazin-1-yl)-4-(4-m-tolyl-6,7-dihydrothieno[3,2-c]pyridin-5(4H)-yl)butan-1,4-dion; |
| **172** | 1-(4-(4-Chlorbenzyl)piperazin-1-yl)-4-(4-m-tolyl-6,7-dihydrothieno[3,2-c]pyridin-5(4H)-yl)butan-1,4-dion; |
| **173** | 1-(2-((4,6-Dimethylpyridin-2-yl)methyl)pyrrolidin-1-yl)-4-(4-(3-fluorphenyl)-6,7-dihydrothieno[3,2-c]pyridin-5(4H)-yl)butan-1,4-dion; |
| **174** | 1-(4-(3-Fluorphenyl)-6,7-dihydrothieno[3,2-c]pyridin-5(4H)-yl)-4-(2-((6-methylpyridin-2-yl)methyl) pyrrolidin-1-yl)butan-1,4-dion; |
| **175** | 1-(2-((5-Ethylpyridin-2-yl)methyl)pyrrolidin-1-yl)-4-(4-(3-fluorphenyl)-6,7-dihydrothieno[3,2-c]pyridin-5 (4H)-yl)butan-1,4-dion; |
| **176** | 1-(4-(3-Fluorphenyl)-6,7-dihydrothieno[3,2-c]pyridin-5(4H)-yl)-4-(2-(6-methoxypyridin-3-yl)piperidin-1-yl) butan-1,4-dion; |
| **177** | 4-(4-(3-Fluorphenyl)-6,7-dihydrothieno[3,2-c]pyridin-5(4H)-yl)-N-methyl-4-oxo-N-(pyridin-3-ylmethyl) butanamid; |
| **178** | N-(2,2-Diphenylethyl)-3-methyl-4-oxo-4-(4-phenyl-6,7-dihydrothieno[3,2-c]pyridin-5(4H)-yl)butanamid; |
| **179** | N-(Cyclopropylmethyl)-4-oxo-4-(4-phenyl-6,7-dihydrothieno[3,2-c]pyridin-5(4H)-yl)butanamid; |
| **180** | 1-(4-(3-Fluorphenyl)-6,7-dihydrothieno[3,2-c]pyridin-5(4H)-yl)-4-(4-(4-methylbenzyl)piperazin-1-yl)butan-1,4-dion; |
| **181** | 2-Methyl-1-(4-phenyl-6,7-dihydrothieno[3,2-c]pyridin-5(4H)-yl)-4-(2-(pyridin-2-ylmethyl)piperidin-1-yl) butan-1,4-dion; |
| **182** | 4-(4-(3,5-Dichlorpyridin-4-yl)piperazin-1-yl)-2-methyl-1-(4-phenyl-6,7-dihydrothieno[3,2-c]pyridin-5 (4H)-yl)butan-1,4-dion; |
| **183** | 1-(2-((4,6-Dimethylpyridin-2-yl)methyl)piperidin-1-yl)-4-(4-phenyl-6,7-dihydrothieno[3,2-c]pyridin-5 (4H)-yl)butan-1,4-dion; |
| **184** | N-(4-Fluorbenzyl)-N-methyl-4-oxo-4-(4-phenyl-6,7-dihydrothieno[3,2-c]pyridin-5(4H)-yl)butanamid; |
| **185** | 1-(4-(3-Fluorphenyl)-6,7-dihydrothieno[3,2-c]pyridin-5(4H)-yl)-4-(2-(pyridin-2-yl)pyrrolidin-1-yl)butan-1,4-dion; |
| **186** | 4-(4-(4-Methoxybenzyl)piperazin-1-yl)-2-methyl-1-(4-phenyl-6,7-dihydrothieno[3,2-c]pyridin-5(4H)-yl) butan-1,4-dion; |

(fortgesetzt)

| | |
|---|---|
| **187** | 2-Methyl-1-(4-phenyl-6,7-dihydrothieno[3,2-c]pyridin-5(4H)-yl)-4-(3-(pyridin-3-yl)pyrrolidin-1-yl)butan-1,4-dion; |
| **188** | 1-(4-(2-Fluorbenzyl)piperazin-1-yl)-4-(4-phenyl-6,7-dihydrothieno[3,2-c]pyridin-5(4H)-yl)butan-1,4-dion; |
| **189** | N-Methyl-4-oxo-4-(4-phenyl-6,7-dihydrothieno[3,2-c]pyridin-5(4H)-yl)-N-(2-(pyridin-4-yl)ethyl)butanamid; |
| **190** | N-Butyl-N-ethyl-3-methyl-4-oxo-4-(4-phenyl-6,7-dihydrothieno[3,2-c]pyridin-5(4H)-yl)butanamid; |
| **191** | N,3-Dimethyl-4-oxo-4-(4-phenyl-6,7-dihydrothieno[3,2-c]pyridin-5(4H)-yl)-N-propylbutanamid; |
| **192** | 4-((S)-2-(Methoxymethyl)pyrrolidin-1-yl)-2-methyl-1-(4-phenyl-6,7-dihydrothieno[3,2-c]pyridin-5(4H)-yl) butan-1,4-dion; |
| **193** | 4-(4-(5-Chlor-2-methylphenyl)piperazin-1-yl)-2-methyl-1-(4-phenyl-6,7-dihydrothieno[3,2-c]pyridin-5 (4H)-yl)butan-1,4-dion; |
| **194** | N-(Furan-2-ylmethyl)-N-methyl-4-oxo-4-(4-phenyl-6,7-dihydrothieno[3,2-c]pyridin-5(4H)-yl)butanamid; |
| **195** | 1-((4aR,8aS)-Octahydroisoquinolin-2(1H)-yl)-4-(4-phenyl-6,7-dihydrothieno-[3,2-c]pyridin-5(4H)-yl)butan-1,4-dion; |
| **196** | 1-(4-Phenyl-6,7-dihydrothieno[3,2-c]pyridin-5(4H)-yl)-4-thiomorpholinobutan-1,4-dion; |
| **197** | 1-(4-Phenyl-6,7-dihydrothieno[3,2-c]pyridin-5(4H)-yl)-4-(2-(pyridin-3-yl)pyrrolidin-1-yl)butan-1,4-dion; |
| **198** | N-Benzyl-4-(4-(4-fluorphenyl)-6,7-dihydrothieno[3,2-c]pyridin-5(4H)-yl)-N-isopropyl-4-oxobutanamid; |
| **199** | N-Benzyl-4-(4-(4-fluorphenyl)-6,7-dihydrothieno[3,2-c]pyridin-5(4H)-yl)-N-methyl-4-oxobutanamid; |
| **200** | N-(3,4-Dimethoxyphenethyl)-4-(4-(4-fluorphenyl)-6,7-dihydrothieno[3,2-c]pyridin-5(4H)-yl)-N-methyl-4-oxobutanamid; |
| **201** | 1-(4-(4-Fluorphenyl)-6,7-dihydrothieno[3,2-c]pyridin-5(4H)-yl)-4-(4-methyl-2-phenylpiperazin-1-yl)butan-1,4-dion; |
| **202** | 1-(4-(4-Chlorphenyl)-6,7-dihydrothieno[3,2-c]pyridin-5(4H)-yl)-4-(4-phenylpiperidin-1-yl)butan-1,4-dion; |
| **203** | 4-(4-(4-Chlorphenyl)-6,7-dihydrothieno[3,2-c]pyridin-5(4H)-yl)-4-oxo-N-(2-(pyridin-2-yl)ethyl)butanamid; |
| **204** | N-Benzyl-2-methyl-4-oxo-N-phenethyl-4-(4-phenyl-6,7-dihydrothieno[3,2-c]pyridin-5(4H)-yl)butanamid; |
| **205** | 1-(4-(4-Fluorphenyl)-6,7-dihydrothieno[3,2-c]pyridin-5(4H)-yl)-4-(2-((6-methylpyridin-2-yl)methyl) piperidin-1-yl)butan-1,4-dion; |
| **206** | 1-(4-(4-Fluorphenyl)-6,7-dihydrothieno[3,2-c]pyridin-5(4H)-yl)-4-(4-(3-methylbenzyl)piperazin-1-yl)butan-1,4-dion; |
| **207** | 1-(4-(4-Chlorphenyl)-6,7-dihydrothieno[3,2-c]pyridin-5(4H)-yl)-4-(2-(pyridin-4-ylmethyl)piperidin-1-yl) butan-1,4-dion; |
| **208** | 1-(4-(4-Chlorphenyl)-6,7-dihydrothieno[3,2-c]pyridin-5(4H)-yl)-4-(2-((5-ethylpyridin-2-yl)methyl)piperidin-1-yl)butan-1,4-dion; |
| **209** | 1-(4-(4-Chlorphenyl)-6,7-dihydrothieno[3,2-c]pyridin-5(4H)-yl)-4-(2-((3-methylpyridin-2-yl)methyl) piperidin-1-yl)butan-1,4-dion; |
| **210** | 1-(4-(4-Chlorphenyl)-6,7-dihydrothieno[3,2-c]pyridin-5(4H)-yl)-4-(4-(3-chlorphenyl)piperazin-1-yl)butan-1,4-dion; |
| **211** | 1-(4-(4-Chlorphenyl)-6,7-dihydrothieno[3,2-c]pyridin-5(4H)-yl)-4-(4-(2,3-dimethylphenyl)piperazin-1-yl) butan-1,4-dion; |
| **212** | 1-(4-(4-Chlorphenyl)-6,7-dihydrothieno[3,2-c]pyridin-5(4H)-yl)-4-(4-(3,4-dimethylphenyl)piperazin-1-yl) butan-1,4-dion; |
| **213** | 1-(4-(4-Chlorphenyl)-6,7-dihydrothieno[3,2-c]pyridin-5(4H)-yl)-4-(4-(3,4-dichlorphenyl)piperazin-1-yl) butan-1,4-dion; |
| **214** | 1-(4-(4-tert-Butylbenzyl)piperazin-1-yl)-4-(4-(4-chlorphenyl)-6,7-dihydrothieno[3,2-c]pyridin-5(4H)-yl) butan-1,4-dion; |
| **215** | 1-[4-(3,5-Dichlor-4-pyridyl)-1-piperazinyl]-2-methyl-4-(4-phenyl-6,7-dihydro-4H-thieno[3,2-c]pyridin-5-yl) butan-1,4-dion; |
| **216** | 1-[4-[(4-Methoxyphenyl)methyl]-1-piperazinyl]-4-[4-(p-tolyl)-6,7-dihydro-4H-thieno[3,2-c]pyridin-5-yl] butan-1,4-dion; |
| **217** | 1-[4-[(2-Fluorphenyl)methyl]-1-piperazinyl]-4-[4-(p-tolyl)-6,7-dihydro-4H-thieno[3,2-c]pyridin-5-yl]butan-1,4-dion; |
| **218** | 1-(4-Methyl-2-phenyl-1-piperazinyl)-4-[4-(p-tolyl)-6,7-dihydro-4H-thieno[3,2-c]pyridin-5-yl]butan-1,4-dion; |
| **219** | 1-(4-Phenyl-1-piperidinyl)-4-[4-(p-tolyl)-6,7-dihydro-4H-thieno[3,2-c]pyridin-5-yl]butan-1,4-dion; |

(fortgesetzt)

| | |
|---|---|
| **220** | 1-[4-(p-Tolyl)-6,7-dihydro-4H-thieno[3,2-c]pyridin-5-yl]-4-[2-(2-pyridyl)-1-pyrrolidinyl]butan-1,4-dion; |
| **221** | 1-[4-(p-Tolyl)-6,7-dihydro-4H-thieno[3,2-c]pyridin-5-yl]-4-[3-(3-pyridyl)-1-pyrrolidinyl]butan-1,4-dion; |
| **222** | 4-Oxo-4-[4-(p-tolyl)-6,7-dihydro-4H-thieno[3,2-c]pyridin-5-yl]-N-[2-(2-pyridyl)ethyl]butanamid; |
| **223** | 1-[4-[(4-Methoxyphenyl)methyl]-1-piperazinyl]-4-[4-(m-tolyl)-6,7-dihydro-4H-thieno[3,2-c]pyridin-5-yl] butan-1,4-dion; |
| **224** | 1-[4-[(2-Fluorphenyl)methyl]-1-piperazinyl]-4-[4-(m-tolyl)-6,7-dihydro-4H-thieno[3,2-c]pyridin-5-yl]butan-1,4-dion; |
| **225** | 1-(4-Methyl-2-phenyl-1-piperazinyl)-4-[4-(m-tolyl)-6,7-dihydro-4H-thieno[3,2-c]pyridin-5-yl]butan-1,4-dion; |
| **226** | 1-[4-(m-Tolyl)-6,7-dihydro-4H-thieno[3,2-c]pyridin-5-yl]-4-(4-phenyl-1-piperidinyl)butan-1,4-dion; |
| **227** | 1-[4-(m-Tolyl)-6,7-dihydro-4H-thieno[3,2-c]pyridin-5-yl]-4-[2-(2-pyridyl)-1-pyrrolidinyl]butan-1,4-dion; |
| **228** | 1-[4-(m-Tolyl)-6,7-dihydro-4H-thieno[3,2-c]pyridin-5-yl]-4-[3-(3-pyridyl)-1-pyrrolidinyl]butan-1,4-dion; |
| **229** | N-Methyl-4-[4-(m-tolyl)-6,7-dihydro-4H-thieno[3,2-c]pyridin-5-yl]-4-oxo-N-[2-(4-pyridyl)ethyl]butanamid; |
| **230** | 1-[4-(3-Fluorphenyl)-6,7-dihydro-4H-thieno[3,2-c]pyridin-5-yl]-4-[4-[(4-methoxyphenyl)methyl]-1-piperazinyl]butan-1,4-dion; |
| **231** | 1-[4-(3-Fluorphenyl)-6,7-dihydro-4H-thieno[3,2-c]pyridin-5-yl]-4-[4-[(2-fluorphenyl)methyl]-1-piperazinyl] butan-1,4-dion; |
| **232** | 4-[4-(m-Tolyl)-6,7-dihydro-4H-thieno[3,2-c]pyridin-5-yl]-4-oxo-N-(p-tolyl)butanamid; |
| **233** | N-(2,4-Dimethylphenyl)-4-[4-(m-tolyl)-6,7-dihydro-4H-thieno[3,2-c]pyridin-5-yl]-4-oxobutanamid; |
| **234** | 4-[4-(3-Fluorphenyl)-6,7-dihydro-4H-thieno[3,2-c]pyridin-5-yl]-N-(1-methyl-6-indazolyl)-4-oxobutanamid; |
| **235** | 4-[4-(3-Fluorphenyl)-6,7-dihydro-4H-thieno[3,2-c]pyridin-5-yl]-4-oxo-N-(p-tolyl)butanamid; |
| **236** | 4-[4-(3-Fluorphenyl)-6,7-dihydro-4H-thieno[3,2-c]pyridin-5-yl]-4-oxo-N-phenylbutanamid; |
| **237** | N-(2-Chlorphenyl)-4-[4-(3-fluorphenyl)-6,7-dihydro-4H-thieno[3,2-c]pyridin-5-yl]-4-oxobutanamid; |
| **238** | N-(2,4-Dimethylphenyl)-4-[4-(3-fluorphenyl)-6,7-dihydro-4H-thieno[3,2-c]pyridin-5-yl]-4-oxobutanamid; |
| **239** | 4-[4-(4-Chlorphenyl)-6,7-dihydro-4H-thieno[3,2-c]pyridin-5-yl]-N-(1-methyl-6-indazolyl)-4-oxobutanamid; |
| **240** | 4-[4-(4-Chlorphenyl)-6,7-dihydro-4H-thieno[3,2-c]pyridin-5-yl]-4-oxo-N-(p-tolyl)butanamid; |
| **241** | 4-[4-(4-Chlorphenyl)-6,7-dihydro-4H-thieno[3,2-c]pyridin-5-yl]-N-(4-methoxyphenyl)-4-oxobutanamid; |
| **242** | 4-[4-(4-Chlorphenyl)-6,7-dihydro-4H-thieno[3,2-c]pyridin-5-yl]-4-oxo-N-phenylbutanamid; |
| **243** | 4-[4-(4-Chlorphenyl)-6,7-dihydro-4H-thieno[3,2-c]pyridin-5-yl]-N-(2,4-dimethylphenyl)-4-oxobutanamid; |
| **244** | 4-[4-(2,6-Dimethylphenyl)-6,7-dihydro-4H-thieno[3,2-c]pyridin-5-yl]-4-oxo-N-[[3-(trifluormethyl)phenyl] methyl]butanamid; |
| **245** | N-(2-Cyclohexylethyl)-4-oxo-4-(4-phenyl-6,7-dihydro-4H-thieno[3,2-c]pyridin-5-yl)butanamid; |
| **246** | N-(3,3-Dimethylbutyl)-4-oxo-4-(4-phenyl-6,7-dihydro-4H-thieno[3,2-c]pyridin-5-yl)butanamid; |
| **247** | N-(Cyclohexylmethyl)-4-oxo-4-(4-phenyl-6,7-dihydro-4H-thieno[3,2-c]pyridin-5-yl)butanamid; |
| **248** | N-[[3-Methyl-5-(trifluormethoxy)phenyl]methyl]-4-oxo-4-(4-phenyl-6,7-dihydro-4H-thieno[3,2-c]pyridin-5-yl)butanamid; |
| **249** | N-[[4-Methyl-3-(trifluormethyl)phenyl]methyl]-4-oxo-4-(4-phenyl-6,7-dihydro-4H-thieno[3,2-c]pyridin-5-yl) butanamid; |
| **250** | N-[[4-Fluor-3-(trifluormethyl)phenyl]methyl]-4-oxo-4-(4-phenyl-6,7-dihydro-4H-thieno[3,2-c]pyridin-5-yl) butanamid; |
| **251** | 4-[4-(2-Ethylphenyl)-6,7-dihydro-4H-thieno[3,2-c]pyridin-5-yl]-4-oxo-N-[[3-(trifluormethyl)phenyl]methyl] butanamid; |
| **252** | 4-[4-(2-Isopropylphenyl)-6,7-dihydro-4H-thieno[3,2-c]pyridin-5-yl]-4-oxo-N-[[3-(trifluormethyl)phenyl] methyl]butanamid; |
| **253** | N-(3-Cyclohexylpropyl)-4-oxo-4-(4-phenyl-6,7-dihydro-4H-thieno[3,2-c]pyridin-5-yl)butanamid; |
| **254** | 4-[4-(3-Methyl-2-thienyl)-6,7-dihydro-4H-thieno[3,2-c]pyridin-5-yl]-4-oxo-N-[[3-(trifluormethyl)phenyl] methyl]butanamid; |
| **255** | (1S,2S)-2-[Oxo-(4-phenyl-6,7-dihydro-4H-thieno[3,2-c]pyridin-5-yl)methyl]-N-[[3-(trifluormethyl)phenyl] methyl]-1-cyclopropancarboxamid; |
| **256** | 4-[4-(o-Tolyl)-6,7-dihydro-4H-thieno[3,2-c]pyridin-5-yl]-4-oxo-N-[[2-(trifluormethyl)phenyl]methyl] butanamid; |
| **257** | 4-[4-(o-Tolyl)-6,7-dihydro-4H-thieno[3,2-c]pyridin-5-yl]-4-oxo-N-[[4-(trifluormethyl)phenyl]methyl] butanamid; |

(fortgesetzt)

| | |
|---|---|
| **258** | 4-(7-Butyl-5,7-dihydro-4H-thieno[2,3-c]pyridin-6-yl)-4-oxo-N-[[3-(trifluormethyl)phenyl]methyl]butanamid; |
| **259** | 4-[4-(Cyclohexylmethyl)-6,7-dihydro-4H-thieno[3,2-c]pyridin-5-yl]-4-oxo-N-[[3-(trifluormethyl)phenyl] methyl]butanamid; |
| **260** | 4-(4-Cyclopropyl-6,7-dihydro-4H-thieno[3,2-c]pyridin-5-yl)-4-oxo-N-[[3-(trifluormethyl)phenyl]methyl] butanamid; |
| **261** | 4-Oxo-4-(4-phenthyl-6,7-dihydro-4H-thieno[3,2-c]pyridin-5-yl)-N-[[3-(trifluormethyl)phenyl]methyl] butanamid; |
| **262** | 4-[4-(4-Fluor-2-methylphenyl)-6,7-dihydro-4H-thieno[3,2-c]pyridin-5-yl]-4-oxo-N-[[3-(trifluormethyl)phenyl] methyl]butanamid; |
| **263** | 4-(4-Cyclopentyl-6,7-dihydro-4H-thieno[3,2-c]pyridin-5-yl)-4-oxo-N-[[3-(trifluormethyl)phenyl]methyl] butanamid; |
| **264** | N-[[2-Methyl-3-(trifluormethyl)phenyl]methyl]-4-oxo-4-(4-phenyl-6,7-dihydro-4H-thieno[3,2-c]pyridin-5-yl) butanamid; |
| **265** | 4-Oxo-4-[4-(3-phenylpropyl)-6,7-dihydro-4H-thieno[3,2-c]pyridin-5-yl]-N-[[3-(trifluormethyl)phenyl]methyl] butanamid; |
| **266** | N-[[2-Methyl-5-(trifluormethyl)phenyl]methyl]-4-oxo-4-(4-phenyl-6,7-dihydro-4H-thieno[3,2-c]pyridin-5-yl) butanamid; |
| **267** | N-Cyclohexyl-4-oxo-4-(4-phenyl-6,7-dihydro-4H-thieno[3,2-c]pyridin-5-yl)butanamid; |
| **268** | N-(2,2-Dimethylpropyl)-4-oxo-4-(4-phenyl-6,7-dihydro-4H-thieno[3,2-c]pyridin-5-yl)butanamid; |
| **269** | 1-(4-Phenyl-6,7-dihydro-4H-thieno[3,2-c]pyridin-5-yl)-4-[7-(trifluormethyl)-3,4-dihydro-1H-isoquinolin-2-yl] butan-1,4-dion; |
| **270** | 4-Oxo-N-pentyl-4-(4-phenyl-6,7-dihydro-4H-thieno[3,2-c]pyridin-5-yl)butanamid; |
| **271** | 1-(4-Phenyl-6,7-dihydro-4H-thieno[3,2-c]pyridin-5-yl)-4-[5-(trifluormethyl)-3,4-dihydro-1H-isoquinolin-2-yl] butan-1,4-dion; |
| **272** | 4-Oxo-4-(4-phenyl-6,7-dihydro-4H-thieno[3,2-c]pyridin-5-yl)-N-[5-(trifluormethyl)-1-tetralinyl]butanamid; |
| **273** | 4-Oxo-4-(4-phenyl-6,7-dihydro-4H-thieno[3,2-c]pyridin-5-yl)-N-[7-(trifluormethyl)-1-tetralinyl]butanamid; |
| **274** | 4-[7-(o-Tolyl)-5,7-dihydro-4H-thieno[2,3-c]pyridin-6-yl]-4-oxo-N-[[3-(trifluormethyl)phenyl]methyl] butanamid; |
| **275** | 4-(7-Cyclohexyl-5,7-dihydro-4H-thieno[2,3-c]pyridin-6-yl)-4-oxo-N-[[3-(trifluormethyl)phenyl]methyl] butanamid; |

oder deren physiologisch verträgliche Salze.

**12.** Arzneimittel enthaltend wenigstens eine Verbindung nach einem der Ansprüche 1 bis 11 oder eine Verbindung ausgewählt aus der Gruppe bestehend aus

- 1-Morpholino-4-(4-(thiophen-2-yl)-6,7-dihydrothieno[3,2-c]pyridin-5(4H)-yl)butan-1,4-dion,
- 1-(4-Acetylpiperazin-1-yl)-4-(4-(thiophen-2-yl)-6,7-dihydrothieno[3,2-c]pyridin-5(4H)-yl)butan-1,4-dion und
- 1-(3-Phenyl-4,5-dihydropyrazol-1-yl)-4-(4-(thiophen-2-yl)-6,7-dihydrothieno[3,2-c]pyridin-5(4H)-yl)butan-1,4-dion;

in Form eines einzelnen Stereoisomers oder deren Mischung, der freien Verbindungen und/oder ihrer physiologisch verträglichen Salze, sowie geeignete Zusatz- und/ oder Hilfsstoffe und/oder gegebenenfalls weiterer Wirkstoffe.

**13.** Verwendung einer Verbindung nach einem der Ansprüche 1 bis 11 oder eine Verbindung ausgewählt aus der Gruppe bestehend aus

- 1-Morpholino-4-(4-(thiophen-2-yl)-6,7-dihydrothieno[3,2-c]pyridin-5(4H)-yl)butan-1,4-dion,
- 1-(4-Acetylpiperazin-1-yl)-4-(4-(thiophen-2-yl)-6,7-dihydrothieno[3,2-c]pyridin-5(4H)-yl)butan-1,4-dion und
- 1-(3-Phenyl-4,5-dihydropyrazol-1-yl)-4-(4-(thiophen-2-yl)-6,7-dihydrothieno[3,2-c]pyridin-5(4H)-yl)butan-1,4-dion;

in Form eines einzelnen Stereoisomers oder deren Mischung, der freien Verbindung und/oder ihrer physiologisch verträglichen Salze, zur Herstellung eines Arzneimittels zur Behandlung von Schmerz, Epilepsie, Angstzuständen,

Abhängigkeit, Manie, bipolaren Störungen, Migräne, kognitiven Erkrankungen, Dystonie-assoziierten Dyskinesien und/oder Harninkontinenz.

## Claims

**1.** Substituted tetrahydrothienopyridines of the general formula (1)

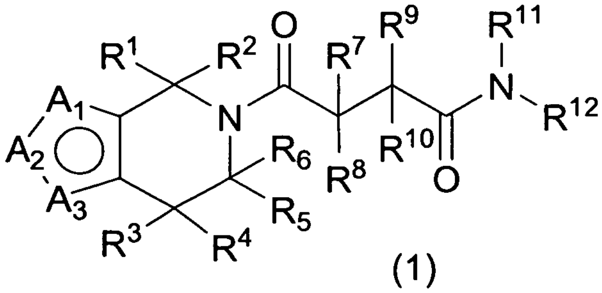

(1)

wherein

$A_1$ represents S, $A_2$ represents $CR^{14}$ and $A_3$ represents $CR^{15}$; or
$A_1$ represents $CR^{13}$, $A_2$ represents S and $A_3$ represents $CR^{15}$; or
$A_1$ represents $CR^{13}$, $A_2$ represents $CR^{14}$ and $A_3$ represents S;
$R^0$ represents $C_{1-8}$-alkyl, saturated or unsaturated, branched or unbranched, unsubstituted or mono- or poly-substituted; $C_{3-7}$-cycloalkyl or heterocyclyl, in each case saturated or unsaturated, unsubstituted or mono- or poly-substituted; aryl or heteroaryl, in each case unsubstituted or mono- or poly-substituted; $C_{1-8}$-alkyl-bridged $C_{3-7}$-cycloalkyl or heterocyclyl, in each case saturated or unsaturated, unsubstituted or mono- or poly-substituted, wherein the alkyl chain in each case can be branched or unbranched, saturated or unsaturated, unsubstituted, mono- or poly-substituted; or $C_{1-8}$-alkyl-bridged aryl or heteroaryl, in each case unsubstituted or mono- or poly-substituted, wherein the alkyl chain in each case can be branched or unbranched, saturated or unsaturated, unsubstituted, mono- or poly-substituted;
$R^1$ represents H; F; Cl; Br; CN; or $R^0$;
$R^2$ represents H; F; Cl; Br; or $C_{1-8}$-alkyl, saturated or unsaturated, branched or unbranched, unsubstituted or mono- or poly-substituted;
or $R^1$ and $R^2$, together with the carbon atom joining them as ring member, form a $C_{3-7}$-cycloalkyl or heterocyclyl, in each case saturated or unsaturated, unsubstituted or mono- or poly-substituted, in each case optionally fused with (hetero)aryl, unsubstituted or mono- or poly-substituted;
$R^3$, $R^4$, $R^5$, $R^6$ independently of one another represent H; F; Cl; Br; $C_{1-8}$-alkyl, saturated or unsaturated, branched or unbranched, unsubstituted or mono- or poly-substituted; or phenyl, unsubstituted or mono- or poly-substituted;
$R^7$ represents H; F; Cl; Br; CN; $R^0$; $OR^0$; O-C(=O)-$R^0$; O-C(=O)-O-$R^0$; O-(C=O)-NH-$R^0$; O-C(=O)-N($R^0$)$_2$; NH-$R^0$; N($R^0$)$_2$; NH-C(=O)-$R^0$; NH-C(=O)-O$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2$$R^0$; NH-S(=O)$_2$O$R^0$; NH-S(=O)$_2$$NH_2$; NH-S(=O)$_2$NH$R^0$; NH-S(=O)$_2$N($R^0$)$_2$; N$R^0$-S(=O)$_2$OH; N$R^0$-S(=O)$_2$$R^0$; N$R^0$-S(=O)$_2$O$R^0$; N$R^0$-S(=O)$_2$$NH_2$; N$R^0$-S(=O)$_2$NH$R^0$; N$R^0$-S(=O)$_2$N($R^0$)$_2$; C(=O)-OH; C(=O)-O$R^0$; C(=O)-$NH_2$; C(=O)-NH-$R^0$; C(=O)-N($R^0$)$_2$; S(=O)$_2$-OH; S(=O)$_2$O$R^0$; S(=O)$_2$-$NH_2$; S(=O)$_2$-NH-$R^0$; S(=O)$_2$-N($R^0$)$_2$;
$R^8$ represents H; F; Cl; Br; CN; or $C_{1-8}$-alkyl, saturated or unsaturated, branched or unbranched, unsubstituted or mono- or poly-substituted;
$R^9$ represents H; F; Cl; Br; CN; $C_{1-8}$-alkyl, saturated or unsaturated, branched or unbranched, unsubstituted or mono- or poly-substituted; $C_{3-7}$-cycloalkyl or heterocyclyl, in each case saturated or unsaturated, unsubstituted or mono- or poly-substituted; aryl or heteroaryl, in each case unsubstituted or mono- or poly-substituted; $C_{1-8}$-alkyl-bridged $C_{3-7}$-cycloalkyl or heterocyclyl, in each case saturated or unsaturated, unsubstituted or mono- or poly-substituted, wherein the alkyl chain in each case can be branched or unbranched, saturated or unsaturated, unsubstituted, mono- or poly-substituted; or $C_{2-8}$-alkyl-bridged aryl or heteroaryl, in each case unsubstituted or mono- or poly-substituted, wherein the alkyl chain in each case can be branched or unbranched, saturated or unsaturated, unsubstituted, mono- or poly-substituted; O-$C_{1-8}$-alkyl, saturated or unsaturated, branched or unbranched, unsubstituted or mono- or poly-substituted; O-$C_{3-7}$-cycloalkyl or O-heterocyclyl, in each case saturated or unsaturated, unsubstituted or mono- or poly-substituted; O-$C_{1-8}$-alkyl-bridged $C_{3-7}$-cy-

cloalkyl or heterocyclyl, in each case saturated or unsaturated, unsubstituted or mono- or poly-substituted, wherein the alkyl chain in each case can be branched or unbranched, saturated or unsaturated, unsubstituted, mono- or poly-substituted; or O-$C_{1-8}$-alkyl-bridged aryl or heteroaryl, in each case unsubstituted or mono- or poly-substituted, wherein the alkyl chain in each case can be branched or unbranched, saturated or unsaturated, unsubstituted, mono- or poly-substituted; O-C(=O)-$R^0$; O-C(=O)-O-$R^0$; O-(C=O)-NH-$R^0$; O-C(=O)-N($R^0$)$_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$; N$R^0$-S(=O)$_2$O$R^0$; N$R^0$-S(=O)$_2NH_2$; N$R^0$-S(=O)$_2$NH$R^0$; N$R^0$-S(=O)$_2$N($R^0$)$_2$; C(=O)-OH; C(=O)-O$R^0$; C(=O)-$NH_2$; C(=O)-NH-$R^0$; C(=O)-N($R^0$)$_2$; S(=O)$_2$-OH; S(=O)$_2$O$R^0$; S(=O)$_2$-$NH_2$; S(=O)$_2$-NH-$R^0$; S(=O)$_2$-N($R^0$)$_2$;

$R^{10}$ represents H; F; Cl; Br; CN; or $C_{1-8}$-alkyl, saturated or unsaturated, branched or unbranched, unsubstituted or mono- or poly-substituted;

or $R^7$ and $R^9$, together with the carbon atoms joining them as ring members, form a $C_{3-7}$-cydoalkyl or heterocyclyl, in each case saturated or unsaturated, unsubstituted or mono- or poly-substituted, optionally fused with (hetero)aryl, unsubstituted or mono- or poly-substituted;

or $R^7$ and $R^8$; or $R^9$ and $R^{10}$; together with the carbon atoms joining them as ring members, form a $C_{3-7}$-cycloalkyl or heterocyclyl, in each case saturated or unsaturated, unsubstituted or mono- or poly-substituted, in each case optionally fused with (hetero)aryl, unsubstituted or mono- or poly-substituted;

$R^{11}$ represents H; $C_{1-8}$-alkyl, saturated or unsaturated, branched or unbranched, unsubstituted or mono- or poly-substituted; or $C_{3-7}$-cycloalkyl, saturated or unsaturated, unsubstituted or mono- or poly-substituted;

$R^{12}$ represents $C_{2-16}$-alkyl, saturated or unsaturated; branched or unbranched, unsubstituted or mono- or poly-substituted; $C_{3-7}$-cycloalkyl or heterocyclyl, in each case saturated or unsaturated, unsubstituted or mono- or poly-substituted; aryl or heteroaryl, unsubstituted or mono- or poly-substituted; $C_{1-8}$-alkyl-bridged $C_{3-7}$-cycloalkyl or heterocyclyl, in each case saturated or unsaturated, unsubstituted or mono- or poly-substituted, wherein the alkyl chain in each case can be branched or unbranched, saturated or unsaturated, unsubstituted, mono- or poly-substituted; $C_{1-8}$-alkyl-bridged aryl or heteroaryl, in each case unsubstituted or mono- or poly-substituted; wherein the alkyl chain in each case can be branched or unbranched, saturated or unsaturated, unsubstituted, mono- or poly-substituted;

or $R^{11}$ and $R^{12}$, together with the nitrogen atom joining them as ring member, form a heterocyclyl, saturated or unsaturated, unsubstituted or mono- or poly-substituted, optionally fused with (hetero)aryl, unsubstituted or mono- or poly-substituted;

$R^{13}$, $R^{14}$ and $R^{15}$ each independently of the others denotes H; F; Cl; Br; I; NO; $NO_2$; $CF_3$; CN; $R^0$; C(=O)H; C(=O)$R^0$; $CO_2H$; C(=O)O$R^0$; CON$H_2$; C(=O)NH$R^0$; C(=O)N($R^0$)$_2$; OH; O$R^0$; 0-($C_{1-8}$-alkyl)-O; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2NH_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$; N$R^0$-S(=O)$_2$O$R^0$; N$R^0$-S(=O)$_2NH_2$; N$R^0$-S(=O)$_2$NH$R^O$; N$R^0$-S(=O)$_2$N($R^0$)$_2$; SH; S$R^0$; S(=O)$R^0$; S(=O)$_2R^0$; S(=O)$_2$OH; S(=O)$_2$O$R^0$; S(=O)$_2NH_2$; S(=O)$_2$NH$R^0$; or S(=O)$_2$N($R^0$)$_2$;

wherein "alkyl substituted", "heterocyclyl substituted" and "cycloalkyl substituted" denote the substitution of one or more hydrogen atoms, in each case independently of one another, by F; Cl; Br; I; CN; $CF_3$; =O; =NH; =C($NH_2$)$_2$; $NO_2$; $R^0$; C(=O)H; C(=O)$R^0$; $CO_2H$; C(=O)O$R^0$; CON$H_2$; C(=O)NH$R^0$; C(=O)N($R^0$)$_2$; OH; O$R^0$; 0-($C_{1-8}$-alkyl)-O; O-C(=O)-$R^0$; O-C(=O)-O-$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2NH_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$; N$R^0$-S(=O)$_2$O$R^0$; N$R^0$-S(=O)$_2NH_2$; N$R^0$-S(=O)$_2$NH$R^0$; N$R^0$-S(=O)$_2$N($R^0$)$_2$; SH; S$R^0$; S(=O)$R^0$; S(=O)$_2R^0$; S(=O)$_2$OH; S(=O)$_2$O$R^0$; S(=O)$_2NH_2$; S(=O)$_2$NH$R^0$; S(=O)$_2$N($R^0$)$_2$;

wherein "aryl substituted" and "heteroaryl substituted" denote the substitution of one or more hydrogen atoms, in each case independently of one another, by F; Cl; Br; I; NO; $NO_2$; $CF_3$; CN; $R^0$; C(=O)H; C(=O)$R^0$; $CO_2H$; C(=O)O$R^0$; CON$H_2$; C(=O)NH$R^0$; C(=O)N($R^0$)$_2$; OH; O$R^0$; O-($C_{1-8}$-alkyl)-O; O-C(=O)-$R^0$; O-C(=O)-O$R^0$; O-(C=O)-NH-$R^0$; O-C(=O)-N($R^0$)$_2$; O-S(=O)$_2$-$R^0$; O-S(=O)$_2$OH; O-S(=O)$_2$O$R^0$; O-S(=O)$_2NH_2$; O-S(=O)$_2$NH$R^0$; O-S(=O)$_2$N($R^0$)$_2$; $NH_2$; NH-$R^0$; N($R^0$)$_2$; NH-C(=O)-$R^0$; NH-C(=O)-O-$R^0$; NH-C(=O)-NH-$R^0$; NH-C(=O)-N($R^0$)$_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N($R^0$)$_2$; NH-S(=O)$_2$OH; NH-S(=O)$_2R^0$; NH-S(=O)$_2$O$R^0$; NH-S(=O)$_2NH_2$; NH-S(=O)$_2$NH$R^0$; NH-S(=O)$_2$N($R^0$)$_2$; N$R^0$-S(=O)$_2$OH; N$R^0$-S(=O)$_2R^0$; N$R^0$-S(=O)$_2$O$R^0$; N$R^0$-S(=O)$_2NH_2$; N$R^0$-S(=O)$_2$NH$R^0$; N$R^0$-S(=O)$_2$N($R^0$)$_2$; SH; S$R^0$; S(=O)$R^0$; S(=O)$_2R^0$; S(=O)$_2$OH; S(=O)$_2$O$R^0$; S(=O)$_2NH_2$; S(=O)$_2$NH$R^0$; S(=O)$_2$N($R^0$)$_2$;

with the exception of the following compounds:

1-morpholino-4-(4-(thiophen-2-yl)-6,7-dihydrothieno[3,2-c]pyridin-5(4H)-yl)-butane-1,4-dione,
1-(4-acetylpiperazin-1-yl)-4-(4-(thiophen-2-yl)-6, 7-dihydrothieno[3,2-c]pyridin-5(4H)-yl)butane-1,4-dione and
1-(3-phenyl-4,5-dihydropyrazol-1-yl)-4-(4-(thiophen-2-yl)-6,7-dihydrothieno-[3,2-c]pyridin-5(4H)-yl)butane-1,4-dione;

in the form of the free compounds or of salts of physiologically acceptable acids or bases.

2. Tetrahydrothienopyridines according to claim 1, **characterised in that**
$R^{12}$ represents $C_{4-16}$-alkyl, saturated or unsaturated; branched or unbranched, unsubstituted or mono- or poly-substituted; $C_{3-7}$-cycloalkyl or heterocyclyl, in each case saturated or unsaturated, unsubstituted or mono- or poly-substituted; aryl or heteroaryl, unsubstituted or mono- or poly-substituted; $C_{1-8}$-alkyl-bridged $C_{3-7}$-cycloalkyl or heterocyclyl, in each case saturated or unsaturated, unsubstituted or mono- or poly-substituted, wherein the alkyl chain in each case can be branched or unbranched, saturated or unsaturated, unsubstituted, mono- or poly-substituted; $C_{1-8}$-alkyl-bridged aryl or heteroaryl, in each case unsubstituted or mono- or poly-substituted, wherein the alkyl chain in each case can be branched or unbranched, saturated or unsaturated, unsubstituted, mono- or poly-substituted.

3. Tetrahydrothienopyridines according to any one of the preceding claims, **characterised in that**
$R^7$ represents H; F; Cl; Br; CN; OH; $NH_2$; $C_{1-8}$-alkyl, O-$C_{1-8}$-alkyl, NH-$C_{1-8}$-alkyl, N($C_{1-8}$-alkyl)$_2$, in each case saturated or unsaturated, branched or unbranched, unsubstituted or mono- or poly-substituted; phenyl or heteroaryl, in each case unsubstituted or mono- or poly-substituted; $C_{1-2}$-alkyl-bridged phenyl or heteroaryl, in each case unsubstituted or mono- or poly-substituted, wherein the alkyl chain in each case can be branched or unbranched, saturated or unsaturated, unsubstituted or mono- or poly-substituted;
$R^9$ represents H; F; Cl; Br; CN; OH; $NH_2$; $C_{1-8}$-alkyl, O-$C_{1-8}$-alkyl, NH-$C_{1-8}$-alkyl, N($C_{1-8}$-alkyl)$_2$, in each case saturated or unsaturated, branched or unbranched, unsubstituted or mono- or poly-substituted; phenyl or heteroaryl, in each case unsubstituted or mono- or poly-substituted; $C_2$-alkyl-bridged phenyl or heteroaryl, in each case unsubstituted or mono- or poly-substituted, wherein the alkyl chain in each case can be branched or unbranched, saturated or unsaturated, unsubstituted or mono- or poly-substituted.

4. Tetrahydrothienopyridines according to any one of the preceding claims, **characterised in that**
$R^1$ and $R^2$ each denotes H;
or $R^2$ denotes H and $R^1$ is not H.

5. Tetrahydrothienopyridines according to any one of the preceding claims having one of the general formulae (1 a), (1 b) or (1 c)

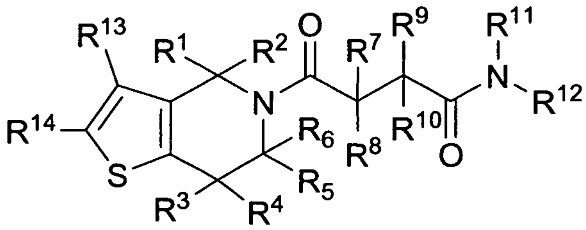

(1a)

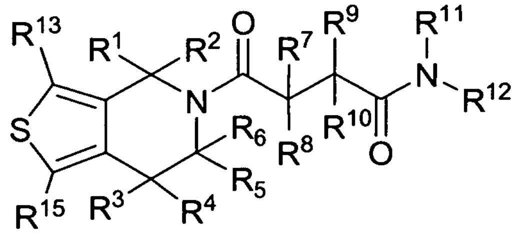

(1b)

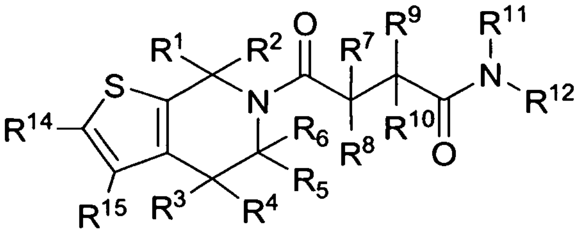

(1c)

wherein $R^{13}$, $R^{14}$ and $R^{15}$ each independently of the others denotes H; F; Cl; Br; I; NO; $NO_2$; CN; $NH_2$ ; NH-$C_{1-8}$-alkyl; N($C_{1-8}$-alkyl)$_2$; NH-C(=O)$C_{1-8}$-alkyl; NH-C(=O)-aryl; NH-C(=O)-heteroaryl; $C_{1-8}$-alkyl; $CF_3$; CHO; C(=O)$C_{1-8}$-alkyl; C(=O)aryl; C(=O)heteroaryl; $CO_2H$; C(=O)O-$C_{1-8}$-alkyl; C(=O)O-aryl; C(=O)O-heteroaryl; $CONH_2$; C(=O)NH-$C_{1-8}$-alkyl; C(=O)N($C_{1-8}$-alkyl)$_2$; C(=O)NH-aryl; C(=O)N(aryl)$_2$; C(=O)NH-heteroaryl; C(=O)N(heteroaryl)$_2$; C(=O)N($C_{1-8}$-alkyl)(aryl); C(=O)N($C_{1-8}$-alkyl)(heteroaryl); C(=O)N(heteroaryl)(aryl); OH; O-$C_{1-8}$-alkyl; $OCF_3$; O-($C_{1-8}$-alkyl)-O; O-($C_{1-8}$-alkyl)-O-$C_{1-8}$-alkyl; O-benzyl; O-aryl; O-heteroaryl; O-C(=O)$C_{1-8}$-alkyl; O-C(=O)aryl; O-C(=O)heteroaryl; SH; S-$C_{1-8}$-alkyl; $SCF_3$; S-benzyl; S-aryl; S-heteroaryl; aryl; heteroaryl; $C_{3-7}$-cycloalkyl; heterocyclyl or $C_{1-8}$-alkyl-bridged aryl, heteroaryl, $C_{3-7}$-cycloalkyl or heterocyclyl.

**6.** Tetrahydrothienopyridines according to any one of the preceding claims, wherein
$R^3$, $R^4$, $R^5$ and $R^6$ independently of one another represent H, $C_{1-8}$-alkyl, saturated or unsaturated, branched or unbranched, unsubstituted or mono- or poly-substituted; or phenyl, unsubstituted or mono- or poly-substituted.

**7.** Tetrahydrothienopyridines according to any one of the preceding claims, wherein
$R^8$ represents H; or $C_{1-8}$-alkyl, saturated or unsaturated, branched or unbranched, unsubstituted or mono- or poly-substituted;
$R^{10}$ represents H; or $C_{1-8}$-alkyl, saturated or unsaturated, branched or unbranched, unsubstituted or mono- or poly-substituted.

**8.** Tetrahydrothienopyridines according to any one of the preceding claims, wherein
$R^{11}$ represents H; $C_{1-8}$-alkyl, saturated or unsaturated, branched or unbranched, $C_{3-7}$-cycloalkyl, saturated or unsaturated; or benzyl, unsubstituted or mono- or poly-substituted.

**9.** Tetrahydrothienopyridines according to any one of the preceding claims, wherein
$R^{12}$ represents $C_{4-16}$-alkyl, saturated or unsaturated, branched or unbranched, unsubstituted or mono- or poly-substituted; or is selected from the following partial structures A, B and C

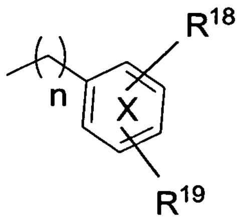
A

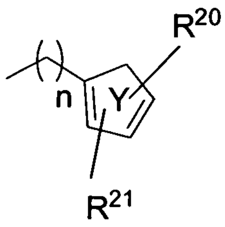
B

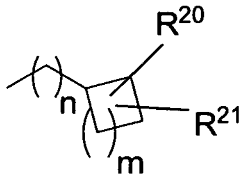
C

wherein
n = 0, 1, 2, 3, 4, 5, 6, 7 or 8;
m = 0, 1, 2 or 3;
the ring X can contain one or two N atoms as ring member(s);
the ring Y contains at least 1 heteroatom selected from N, O and S and can contain up to 3 heteroatoms selected independently of one another from N, O and S;
$R^{18}$ and $R^{19}$ independently of one another denote H; F; Cl; Br; I; NO; $NO_2$; $CF_3$; CN; $R^0$; C(=O)H; C(=O)$R^0$; $CO_2H$; C(=O)O$R^0$; $CONH_2$; C(=O)NH$R^0$; C(=O)N$(R^0)_2$; OH; O$R^0$; O-($C_{1-8}$-alkyl)-O; O-C(=O)-$R^0$; O-C(=O)-O-$R^0$; O-(C=O)-NH-$R^0$; O-C(=O)-N$(R^0)_2$; O-S$(=O)_2$-$R^0$; O-S$(=O)_2$OH; O-S$(=O)_2$O$R^0$; O-S$(=O)_2NH_2$; O-S$(=O)_2$NH$R^0$; O-S$(=O)_2$N$(R^0)_2$; $NH_2$; NH-$R^0$; N$(R^0)_2$; NH-C(=O)-$R^0$; NH-C(=O)-O-$R^0$; NH-C(=O)-NH-$R^0$; NH-C(=O)-N$(R^0)_2$; N$R^0$-C(=O)-$R^0$; N$R^0$-C(=O)-O-$R^0$; N$R^0$-C(=O)-$NH_2$; N$R^0$-C(=O)-NH-$R^0$; N$R^0$-C(=O)-N$(R^0)_2$; NH-S$(=O)_2$OH; NH-S$(=O)_2R^0$; NH-S$(=O)_2$O$R^0$; NH-S$(=O)_2NH_2$; NH-S$(=O)_2$NH$R^0$; NH-S$(=O)_2$N$(R^0)_2$; N$R^0$-S$(=O)_2$OH; N$R^0$-S$(=O)_2R^0$; N$R^0$-S$(=O)_2$O$R^0$; N$R^0$-S$(=O)_2NH_2$; N$R^0$-S$(=O)_2$NH$R^0$; N$R^0$-S$(=O)_2$N$(R^0)_2$; SH; S$R^0$; S(=O)$R^0$; S$(=O)_2R^0$, S$(=O)_2$OH; S$(=O)_2$O$R^0$; S$(=O)_2NH_2$; S$(=O)_2$NH$R^0$; or S$(=O)_2$N$(R^0)_2$;
or $R^{18}$ and $R^{19}$, together with the carbon or nitrogen atoms joining them as ring members, form an aryl or heteroaryl fused with the phenyl ring and in each case unsubstituted or mono- or poly-substituted; or a $C_{3-7}$-cycloalkyl or heterocyclyl fused with the phenyl ring and in each case saturated or unsaturated, unsubstituted or mono- or poly-substituted;
$R^{20}$ and $R^{21}$ independently of one another denote H or $C_{1-8}$-alkyl, saturated or unsaturated, branched or unbranched, unsubstituted or mono- or poly-substituted; $C_{3-7}$-cycloalkyl or heterocyclyl, in each case saturated or unsaturated, unsubstituted or mono- or poly-substituted.

**10.** Tetrahydrothienopyridines according to any one of claims 1 or 3 to 7, wherein $R^{11}$ and $R^{12}$, together with the nitrogen atom joining them as ring member, form one of the following rings

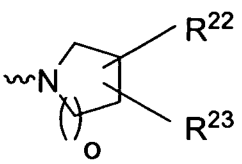
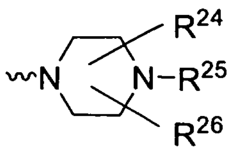
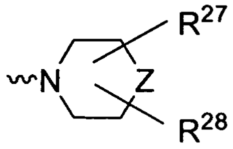
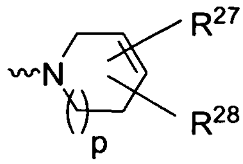
,

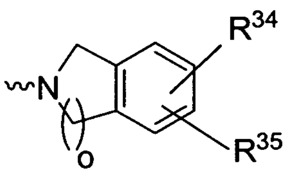
,

wherein

Z = O or S;
o = 0, 1 or 2;
p = 0 or 1;
$R^{22}$, $R^{23}$, $R^{24}$, $R^{25}$ and $R^{26}$ independently of one another denote H; $C_{1-8}$-alkyl, saturated or unsaturated; branched

or unbranched, unsubstituted or mono- or poly-substituted; $C_{3-7}$-cycloalkyl or heterocyclyl, in each case saturated or unsaturated, unsubstituted or mono- or poly-substituted; aryl or heteroaryl, in each case unsubstituted or mono- or poly-substituted; $C_{1-8}$-alkyl-bridged $C_{3-7}$- cycloalkyl or heterocyclyl, in each case saturated or unsaturated, unsubstituted or mono- or poly-substituted, wherein the alkyl chain in each case can be branched or unbranched, saturated or unsaturated, unsubstituted, mono- or poly-substituted; $C_{1-8}$-alkyl-bridged aryl or heteroaryl, in each case unsubstituted or mono- or poly-substituted, wherein the alkyl chain in each case can be branched or unbranched, saturated or unsaturated, unsubstituted or mono- or poly-substituted;

or $R^{22}$ and $R^{23}$, with the carbon atom(s) joining them as ring member(s), form a $C_{3-7}$-cycloalkyl or heterocyclyl, in each case saturated or unsaturated, unsubstituted or mono- or poly-substituted, optionally fused with (hetero)aryl, unsubstituted or mono- or poly-substituted; or form a fused aryl or heteroaryl, unsubstituted or mono- or poly-substituted;

$R^{27}$ and $R^{28}$ independently of one another denote H; $C_{1-8}$-alkyl, saturated or unsaturated; branched or unbranched, unsubstituted or mono- or poly-substituted;

$R^{34}$ and $R^{35}$ independently of one another denote H; F; Cl; Br; I; NO; $NO_2$; CN; $NH_2$; NH-$C_{1-8}$-alkyl; N($C_{1-8}$-alkyl)$_2$; NH-C(=O)$C_{1-8}$-alkyl; NH-C(=O)-aryl; NH-C(=O)-heteroaryl; $C_{1-8}$-alkyl; $CF_3$; C(=O)H; C(=O)$C_{1-8}$-alkyl; C(=O)aryl; C(=O)heteroaryl; $CO_2H$; C(=O)O-$C_{1-8}$-alkyl; C(=O)O-aryl; C(=O)O-heteroaryl; $CONH_2$; C(=O)NH-$C_{1-8}$-alkyl; C(=O)N($C_{1-8}$-alkyl)$_2$; C(=O)NH-aryl; C(=O)N(aryl)$_2$; C(=O)NH-heteroaryl; C(=O)N(heteroaryl)$_2$; C(=O)N($C_{1-8}$-alkyl)(aryl); C(=O)N($C_{1-8}$-alkyl)(heteroaryl); C(=O)N(aryl)(heteroaryl); OH; O-$C_{1-8}$-alkyl; O-$C_{1-8}$-alkyl-OH; $OCF_3$; O-($C_{1-8}$-alkyl)-O-$C_{1-8}$-alkyl; O-benzyl; O-aryl; O-heteroaryl; O-C(=O)$C_{1-8}$-alkyl; O-C(=O)aryl; O-C(=O)heteroaryl; SH; S-$C_{1-8}$-alkyl; $SCF_3$; S-benzyl; S-aryl; S-heteroaryl; aryl; heteroaryl; $C_{3-7}$-cycloalkyl; heterocyclyl; or $C_{1-8}$-alkyl-bridged aryl, heteroaryl, $C_{3-7}$-cycloalkyl or heterocyclyl.

**11.** Tetrahydrothienopyridines according to claim 1 selected from the group

**1** 4-oxo-4-(4-(thiophen-2-yl)-6,7-dihydrothieno[3,2-c]pyridin-5(4H)-yl)-N-(3-(trifluoromethyl)benzyl) butanamide;
**2** 4-oxo-4-(4-phenyl-6,7-dihydrothieno[3,2-c]pyridin-5(4H)-yl)-N-(3-(trifluoromethyl)benzyl)butanamide;
**3** 4-(4-(3-fluorophenyl)-6,7-dihydrothieno[3,2-c]pyridin-5(4H)-yl)-4-oxo-N-(3-(trifluoromethyl)benzyl) butanamide;
**4** 4-oxo-4-(4-m-tolyl-6,7-dihydrothieno[3,2-c]pyridin-5(4H)-yl)-N-(3-(trifluoromethyl)benzyl)butanamide;
**5** 4-oxo-4-(4-p-tolyl-6,7-dihydrothieno[3,2-c]pyridin-5(4H)-yl)-N-(3-(trifluoromethyl)benzyl)butanamide;
**6** 4-(4-(2-fluorophenyl)-6,7-dihydrothieno[3,2-c]pyridin-5(4H)-yl)-4-oxo-N-(3-(trifluoromethyl)benzyl) butanamide;
**7** 4-(4-(4-fluorophenyl)-6,7-dihydrothieno[3,2-c]pyridin-5(4H)-yl)-4-oxo-N-(3-(trifluoromethyl)benzyl) butanamide;
**8** 4-oxo-4-(4-o-tolyl-6,7-dihydrothieno[3,2-c]pyridin-5(4H)-yl)-N-(3-(trifluoromethyl)benzyl)butanamide;
**9** 4-oxo-4-(7-phenyl-4,5-dihydrothieno[2,3-c]pyridin-6(7H)-yl)-N-(3-(trifluoromethyl)benzyl)butanamide;
**10** 4-(2-methyl-4-phenyl-6,7-dihydrothieno[3,2-c]pyridin-5(4H)-yl)-4-oxo-N-(3-(trifluoromethyl)benzyl) butanamide;
**11** N-(4-methylbenzyl)-4-oxo-4-(4-phenyl-6,7-dihydrothieno[3,2-c]pyridin-5(4H)-yl)butanamide;
**12** N-benzyl-4-oxo-4-(4-phenyl-6,7-dihydrothieno[3,2-c]pyridin-5(4H)-yl)butanamide;
**13** 4-oxo-4-(4-phenyl-6,7-dihydrothieno[3,2-c]pyridin-5(4H)-yl)-N-(4-(trifluoromethyl)benzyl)butanamide;
**14** N-(2-methoxybenzyl)-4-oxo-4-(4-phenyl-6,7-dihydrothieno[3,2-c]pyridin-5(4H)-yl)butanamide;
**15** N-(3-methoxybenzyl)-4-oxo-4-(4-phenyl-6,7-dihydrothieno[3,2-c]pyridin-5(4H)-yl)butanamide;
**16** N-(4-methoxybenzyl)-4-oxo-4-(4-phenyl-6,7-dihydrothieno[3,2-c]pyridin-5(4H)-yl)butanamide;
**17** 4-oxo-4-(4-phenyl-6,7-dihydrothieno[3,2-c]pyridin-5(4H)-yl)-N-(2-(trifluoromethyl)benzyl)butanamide;
**18** N-(3-fluorobenzyl)-4-oxo-4-(4-phenyl-6,7-dihydrothieno[3,2-c]pyridin-5(4H)-yl)butanamide;
**19** N-(3-methylbenzyl)-4-oxo-4-(4-phenyl-6,7-dihydrothieno[3,2-c]pyridin-5(4H)-yl)butanamide;
**20** 4-(3-methyl-4-phenyl-6,7-dihydrothieno[3,2-c]pyridin-5(4H)-yl)-4-oxo-N-(3-(trifluoromethyl)benzyl) butanamide;
**21** 4-(4-cyclohexyl-6,7-dihydrothieno[3,2-c]pyridin-5(4H)-yl)-4-oxo-N-(3-(trifluoromethyl)benzyl)butanamide;
**22** 4-(4-isopropyl-6,7-dihydrothieno[3,2-c]pyridin-5(4H)-yl)-4-oxo-N-(3-(trifluoromethyl)benzyl)butanamide;
**23** 4-(4-butyl-6,7-dihydrothieno[3,2-c]pyridin-5(4H)-yl)-4-oxo-N-(3-(trifluoromethyl)benzyl)butanamide;
**24** N-methyl-4-oxo-4-(4-phenyl-6,7-dihydrothieno[3,2-c]pyridin-5(4H)-yl)-N-(3-(trifluoromethyl)benzyl) butanamide;

(continued)

| | |
|---|---|
| **25** | 4-oxo-N-phenethyl-4-(4-phenyl-6,7-dihydrothieno[3,2-c]pyridin-5(4H)-yl)butanamide; |
| **26** | N-(2-methylbenzyl)-4-oxo-4-(4-phenyl-6,7-dihydrothieno[3,2-c]pyridin-5(4H)-yl)butanamide; |
| **27** | 4-oxo-2-phenyl-4-(4-phenyl-6,7-dihydrothieno[3,2-c]pyridin-5(4H)-yl)-N-(3-(trifluoromethyl)benzyl) butanamide; |
| **28** | (R)-2-methyl-4-oxo-4-(4-phenyl-6,7-dihydrothieno[3,2-c]pyridin-5(4H)-yl)-N-(3-(trifluoromethyl)benzyl) butanamide; |
| **29** | 4-oxo-3-phenyl-4-(4-phenyl-6,7-dihydrothieno[3,2-c]pyridin-5(4H)-yl)-N-(3-(trifluoromethyl)benzyl) butanamide; |
| **30** | 4-(6,7-dihydrothieno[3,2-c]pyridin-5(4H)-yl)-4-oxo-3-phenyl-N-(3-(trifluoromethyl)benzyl)butanamide; |
| **31** | rac. (1 S,2R)-2-(4-phenyl-4,5,6,7-tetrahydrothieno[3,2-c]pyridine-5-carbonyl)-N-(3-(trifluoromethyl)benzyl) cyclohexanecarboxamide; |
| **32** | rac. (1 S)-2-(4-phenyl-4,5,6,7-tetrahydrothieno[3,2-c]pyridine-5-carbonyl)-N-(3-(trifluoromethyl)benzyl) cyclohexanecarboxamide; |
| **33** | rac. (1 S,2R)-2-(4-phenyl-4,5,6,7-tetrahydrothieno[3,2-c]pyridine-5-carbonyl)-N-(3-(trifluoromethyl)benzyl) cyclopentanecarboxamide; |
| **34** | (R)-3-methyl-4-oxo-4-(4-phenyl-6,7-dihydrothieno[3,2-c]pyridin-5(4H)-yl)-N-(3-(trifluoromethyl)benzyl) butanamide; |
| **35** | (-)-4-oxo-4-(4-phenyl-6,7-dihydrothieno[3,2-c]pyridin-5(4H)-yl)-N-(3-(trifluoromethyl)benzyl)butanamide; |
| **36** | (+)-4-oxo-4-(4-phenyl-6,7-dihydrothieno[3,2-c]pyridin-5(4H)-yl)-N-(3-(trifluoromethyl)benzyl)butanamide; |
| **37** | 4-(6,7-dihydrothieno[3,2-c]pyridin-5(4H)-yl)-4-oxo-2-phenyl-N-(3-(trifluoromethyl)benzyl)butanamide; |
| **38** | N-(3,5-bis(trifluoromethyl)benzyl)-4-oxo-4-(4-phenyl-6,7-dihydrothieno[3,2-c]pyridin-5(4H)-yl)butanamide; |
| **39** | N-(2-fluoro-5-(trifluoromethyl)benzyl)-4-oxo-4-(4-phenyl-6,7-dihydrothieno-[3,2-c]pyridin-5(4H)-yl) butanamide; |
| **40** | N-(2-fluoro-3-(trifluoromethyl)benzyl)-4-oxo-4-(4-phenyl-6,7-dihydrothieno-[3,2-c]pyridin-5(4H)-yl) butanamide; |
| **41** | N-(3-fluoro-5-(trifluoromethyl)benzyl)-4-oxo-4-(4-phenyl-6,7-dihydrothieno-[3,2-c]pyridin-5(4H)-yl) butanamide; |
| **42** | rac. (1S,2R)-2-(4-phenyl-4,5,6,7-tetrahydrothieno[3,2-c]pyridine-5-carbonyl)-N-(3-(trifluoromethyl)benzyl) cyclopropanecarboxamide; |
| **43** | N-(3,4-difluorobenzyl)-4-oxo-4-(4-phenyl-6,7-dihydrothieno[3,2-c]pyridin-5(4H)-yl)butanamide; |
| **44** | 4-oxo-4-(5-phenyl-4,5-dihydrothieno[2,3-c]pyridin-6(7H)-yl)-N-(3-(trifluoromethyl)benzyl)butanamide; |
| **45** | 4-(1-methyl-4-phenyl-6,7-dihydrothieno[3,4-c]pyridin-5(4H)-yl)-4-oxo-N-(3-(trifluoromethyl)benzyl) butanamide; |
| **46** | N-(4-methoxyphenyl)-4-oxo-4-(4-p-tolyl-6,7-dihydrothieno[3,2-c]pyridin-5(4H)-yl)butanamide; |
| **47** | N-(1 -methyl-1H-indazol-6-yl)-4-oxo-4-(4-p-tolyl-6,7-dihydrothieno[3,2-c]pyridin-5(4H)-yl)butanamide; |
| **48** | N-benzyl-4-oxo-4-(4-p-tolyl-6,7-dihydrothieno[3,2-c]pyridin-5(4H)-yl)butanamide; |
| **49** | 4-oxo-N-phenethyl-4-(4-p-tolyl-6,7-dihydrothieno[3,2-c]pyridin-5(4H)-yl)butanamide; |
| **50** | 4-oxo-N-(pyridin-4-ylmethyl)-4-(4-p-tolyl-6,7-dihydrothieno[3,2-c]pyridin-5(4H)-yl)butanamide; |
| **51** | 4-oxo-N-(3-phenylpropyl)-4-(4-p-tolyl-6,7-dihydrothieno[3,2-c]pyridin-5(4H)-yl)butanamide; |
| **52** | N-(benzo[c][1,2,5]thiadiazol-4-yl)-4-oxo-4-(4-m-tolyl-6,7-dihydrothieno[3,2-c]pyridin-5(4H)-yl)butanamide; |
| **53** | N-(1 -methyl-1H-indazol-6-yl)-4-oxo-4-(4-m-tolyl-6,7-dihydrothieno[3,2-c]-pyridin-5(4H)-yl)butanamide; |
| **54** | 4-oxo-N-(pyridin-2-ylmethyl)-4-(4-m-tolyl-6,7-dihydrothieno[3,2-c]pyridin-5(4H)-yl)butanamide; |
| **55** | 1-(4-methylpiperazin-1-yl)-4-(4-m-tolyl-6,7-dihydrothieno[3,2-c]pyridin-5(4H)-yl)butane-1,4-dione; |
| **56** | 3-methyl-4-oxo-4-(4-phenyl-6,7-dihydrothieno[3,2-c]pyridin-5(4H)-yl)-N-(pyridin-2-ylmethyl)butanamide; |
| **57** | 4-oxo-4-(4-phenyl-6,7-dihydrothieno[3,2-c]pyridin-5(4H)-yl)-N-(thiophen-2-ylmethyl)butanamide; |
| **58** | N-(2-chlorophenyl)-4-(4-(4-fluorophenyl)-6,7-dihydrothieno[3,2-c]pyridin-5(4H)-yl)-4-oxobutanamide; |
| **59** | 4-(4-(4-chlorophenyl)-6,7-dihydrothieno[3,2-c]pyridin-5(4H)-yl)-N-(furan-2-ylmethyl)-4-oxobutanamide; |
| **60** | 4-(4-(4-chlorophenyl)-6,7-dihydrothieno[3,2-c]pyridin-5(4H)-yl)-4-oxo-N-propylbutanamide; |
| **61** | N-(2-chlorobenzyl)-4-oxo-4-(4-m-tolyl-6,7-dihydrothieno[3,2-c]pyridin-5(4H)-yl)butanamide; |
| **62** | N-(2,4-dichlorobenzyl)-4-(4-(3-fluorophenyl)-6,7-dihydrothieno[3,2-c]pyridin-5(4H)-yl)-4-oxobutanamide; |
| **63** | N-(4-fluorobenzyl)-4-(4-(3-fluorophenyl)-6,7-dihydrothieno[3,2-c]pyridin-5(4H)-yl)-4-oxobutanamide; |
| **64** | N-(3,4-dichlorobenzyl)-3-methyl-4-oxo-4-(4-phenyl-6,7-dihydrothieno[3,2-c]pyridin-5(4H)-yl)butanamide; |

(continued)

| | |
|---|---|
| **65** | N-(2,5-difluorobenzyl)-3-methyl-4-oxo-4-(4-phenyl-6,7-dihydrothieno[3,2-c]pyridin-5(4H)-yl)butanamide; |
| **66** | 3-methyl-4-oxo-4-(4-phenyl-6,7-dihydrothieno[3,2-c]pyridin-5(4H)-yl)-N-(3-(trifluoromethyl)benzyl) butanamide; |
| **67** | 1-(4-(4-chlorophenyl)-6,7-dihydrothieno[3,2-c]pyridin-5(4H)-yl)-4-morpholinobutane-1,4-dione; |
| **68** | 1-(4-(4-chlorophenyl)-6,7-dihydrothieno[3,2-c]pyridin-5(4H)-yl)-4-(4-(4-fluorophenyl)piperazin-1-yl) butane-1,4-dione; |
| **69** | 2-methyl-N-(2-(5-methyl-1H-pyrazol-1-yl)ethyl)-4-oxo-4-(4-phenyl-6,7-dihydrothieno[3,2-c]pyridin-5 (4H)-yl)butanamide; |
| **70** | N-(naphthalen-1-ylmethyl)-4-oxo-4-(4-phenyl-6,7-dihydrothieno[3,2-c]pyridin-5(4H)-yl)butanamide; |
| **71** | 4-(4-(4-fluorophenyl)-6,7-dihydrothieno[3,2-c]pyridin-5(4H)-yl)-N-(4-methylbenzyl)-4-oxobutanamide; |
| **72** | N-(benzo[d][1,3]dioxol-5-ylmethyl)-4-(4-(4-fluorophenyl)-6,7-dihydrothieno-[3,2-c]pyridin-5(4H)-yl)-4-oxobutanamide; |
| **73** | 4-(4-(4-fluorophenyl)-6,7-dihydrothieno[3,2-c]pyridin-5(4H)-yl)-4-oxo-N-(2-(trifluoromethyl)benzyl) butanamide; |
| **74** | 4-(4-(4-fluorophenyl)-6,7-dihydrothieno[3,2-c]pyridin-5(4H)-yl)-N-(naphthalen-1-ylmethyl)-4-oxobutanamide; |
| **75** | 4-(4-(4-chlorophenyl)-6,7-dihydrothieno[3,2-c]pyridin-5(4H)-yl)-N-(3-fluorobenzyl)-4-oxobutanamide; |
| **76** | 2-methyl-N-(1-methyl-1H-indazol-6-yl)-4-oxo-4-(4-phenyl-6,7-dihydrothieno[3,2-c]pyridin-5(4H)-yl) butanamide; |
| **77** | N-(4-methoxybenzyl)-2-methyl-4-oxo-4-(4-phenyl-6,7-dihydrothieno[3,2-c]-pyridin-5(4H)-yl)butanamide; |
| **78** | 2-methyl-1-(4-methylpiperazin-1-yl)-4-(4-phenyl-6,7-dihydrothieno[3,2-c]-pyridin-5(4H)-yl)butane-1,4-dione; |
| **79** | N-(2-(1H-indol-3-yl)ethyl)-2-methyl-4-oxo-4-(4-phenyl-6,7-dihydrothieno[3,2-c]pyridin-5(4H)-yl) butanamide; |
| **80** | N-(2-fluorobenzyl)-2-methyl-4-oxo-4-(4-phenyl-6,7-dihydrothieno[3,2-c]pyridin-5(4H)-yl)butanamide; |
| **81** | 2-methyl-4-oxo-4-(4-phenyl-6,7-dihydrothieno[3,2-c]pyridin-5(4H)-yl)-N-(2-(trifluoromethyl)benzyl) butanamide; |
| **82** | 4-oxo-N-(2-(piperidin-1-yl)ethyl)-4-(4-p-tolyl-6,7-dihydrothieno[3,2-c]pyridin-5(4H)-yl)butanamide; |
| **83** | 4-oxo-N-((tetrahydrofuran-2-yl)methyl)-4-(4-p-tolyl-6,7-dihydrothieno[3,2-c]pyridin-5(4H)-yl)butanamide; |
| **84** | N-(4-chlorobenzyl)-4-oxo-4-(4-p-tolyl-6,7-dihydrothieno[3,2-c]pyridin-5(4H)-yl)butanamide; |
| **85** | N-(2,3-dichlorobenzyl)-4-oxo-4-(4-p-tolyl-6,7-dihydrothieno[3,2-c]pyridin-5(4H)-yl)butanamide; |
| **86** | 4-oxo-N-(2-(thiophen-2-yl)ethyl)-4-(4-m-tolyl-6,7-dihydrothieno[3,2-c]pyridin-5(4H)-yl)butanamide; |
| **87** | N-(cyclohexylmethyl)-4-oxo-4-(4-m-tolyl-6,7-dihydrothieno[3,2-c]pyridin-5(4H)-yl)butanamide; |
| **88** | N-(3-chlorophenethyl)-4-oxo-4-(4-m-tolyl-6,7-dihydrothieno[3,2-c]pyridin-5(4H)-yl)butanamide; |
| **89** | N-(3,3-diphenylpropyl)-4-oxo-4-(4-m-tolyl-6,7-dihydrothieno[3,2-c]pyridin-5(4H)-yl)butanamide; |
| **90** | 4-(4-(3-fluorophenyl)-6,7-dihydrothieno[3,2-c]pyridin-5(4H)-yl)-N-(3-morpholinopropyl)-4-oxobutanamide; |
| **91** | 4-(4-(3-fluorophenyl)-6,7-dihydrothieno[3,2-c]pyridin-5(4H)-yl)-4-oxo-N-(pyridin-3-ylmethyl)butanamide; |
| **92** | 4-(4-(3-fluorophenyl)-6,7-dihydrothieno[3,2-c]pyridin-5(4H)-yl)-N-(3-methylbenzyl)-4-oxobutanamide; |
| **93** | 4-(4-(3-fluorophenyl)-6,7-dihydrothieno[3,2-c]pyridin-5(4H)-yl)-N-(4-methylphenethyl)-4-oxobutanamide; |
| **94** | 3-methyl-4-oxo-4-(4-phenyl-6,7-dihydrothieno[3,2-c]pyridin-5(4H)-yl)-N-(4-phenylbutyl)butanamide; |
| **95** | N-(biphenyl-4-ylmethyl)-4-oxo-4-(4-m-tolyl-6,7-dihydrothieno[3,2-c]pyridin-5(4H)-yl)butanamide; |
| **96** | 4-(4-(3-fluorophenyl)-6,7-dihydrothieno[3,2-c]pyridin-5(4H)-yl)-N-(2-methoxybenzyl)-4-oxobutanamide; |
| **97** | N-(4-chlorophenethyl)-4-(4-(3-fluorophenyl)-6,7-dihydrothieno[3,2-c]pyridin-5(4H)-yl)-4-oxobutanamide; |
| **98** | 4-(4-(3-fluorophenyl)-6,7-dihydrothieno[3,2-c]pyridin-5(4H)-yl)-N-((5-methyl-3-phenylisoxazol-4-yl) methyl)-4-oxobutanamide; |
| **99** | N-(2,6-difluorobenzyl)-3-methyl-4-oxo-4-(4-phenyl-6,7-dihydrothieno[3,2-c]pyridin-5(4H)-yl)butanamide; |
| **100** | N-(3,5-difluorobenzyl)-3-methyl-4-oxo-4-(4-phenyl-6,7-dihydrothieno[3,2-c]pyridin-5(4H)-yl)butanamide; |
| **101** | N-(3-chlorobenzyl)-3-methyl-4-oxo-4-(4-phenyl-6,7-dihydrothieno[3,2-c]pyridin-5(4H)-yl)butanamide; |
| **102** | N-(3,5-dimethoxyphenethyl)-3-methyl-4-oxo-4-(4-phenyl-6,7-dihydrothieno[3,2-c]pyridin-5(4H)-yl) butanamide; |
| **103** | N-(3,4-difluorobenzyl)-4-oxo-4-(4-phenyl-6,7-dihydrothieno[3,2-c]pyridin-5(4H)-yl)butanamide; |
| **104** | N-(2,4-dichlorophenethyl)-4-oxo-4-(4-phenyl-6,7-dihydrothieno[3,2-c]pyridin-5(4H)-yl)butanamide; |

(continued)

| | |
|---|---|
| **105** | N-(2-(1H-1,2,4-triazol-1-yl)ethyl)-4-oxo-4-(4-phenyl-6,7-dihydrothieno[3,2-c]pyridin-5(4H)-yl)butanamide; |
| **106** | 4-(4-(4-fluorophenyl)-6,7-dihydrothieno[3,2-c]pyridin-5(4H)-yl)-N-(2-methylbenzyl)-4-oxobutanamide; |
| **107** | N-(4-fluorophenethyl)-4-(4-(4-fluorophenyl)-6,7-dihydrothieno[3,2-c]pyridin-5(4H)-yl)-4-oxobutanamide; |
| **108** | 4-(4-(4-fluorophenyl)-6,7-dihydrothieno[3,2-c]pyridin-5(4H)-yl)-N-((5-methylisoxazol-3-yl)methyl)-4-oxobutanamide; |
| **109** | N-(2-chlorophenethyl)-4-(4-(4-fluorophenyl)-6,7-dihydrothieno[3,2-c]pyridin-5(4H)-yl)-4-oxobutanamide; |
| **110** | 4-(4-(4-chlorophenyl)-6,7-dihydrothieno[3,2-c]pyridin-5(4H)-yl)-N-(2-cyclo-hexenylethyl)-4-oxobutanamide; |
| **111** | 4-(4-(4-chlorophenyl)-6,7-dihydrothieno[3,2-c]pyridin-5(4H)-yl)-N-(3,5-dimethoxybenzyl)-4-oxobutanamide; |
| **112** | 4-(4-(4-chlorophenyl)-6,7-dihydrothieno[3,2-c]pyridin-5(4H)-yl)-N-(3,4-dichlorophenethyl)-4-oxobutanamide; |
| **113** | 4-(4-(4-chlorophenyl)-6,7-dihydrothieno[3,2-c]pyridin-5(4H)-yl)-N-(3-fluorophenethyl)-4-oxobutanamide; |
| **114** | 1-(3-phenylpiperidin-1-yl)-4-(4-p-tolyl-6,7-dihydrothieno[3,2-c]pyridin-5(4H)-yl)butane-1,4-dione; |
| **115** | 1-(4-benzylpiperazin-1-yl)-4-(4-p-tolyl-6,7-dihydrothieno[3,2-c]pyridin-5(4H)-yl)butane-1,4-dione; |
| **116** | 1-(4-(4-methoxyphenyl)piperazin-1-yl)-4-(4-p-tolyl-6, 7-dihydrothieno[3,2-c]pyridin-5(4H)-yl)butane-1,4-dione; |
| **117** | 1-(2-benzylpiperidin-1-yl)-4-(4-m-tolyl-6, 7-d ihydrothieno[3,2-c]pyrid in-5(4H)-yl)butane-1,4-dione; |
| **118** | 1-(4-(2-fluorophenyl)piperazin-1-yl)-4-(4-m-tolyl-6,7-dihydrothieno[3,2-c]-pyridin-5(4H)-yl)butane-1,4-dione; |
| **119** | 1-(4-(cyclohexylmethyl)piperazin-1-yl)-4-(4-m-tolyl-6,7-dihydrothieno[3,2-c]pyridin-5(4H)-yl)butane-1,4-dione; |
| **120** | N-(3-(1H-imidazol-1-yl)propyl)-4-(4-(3-fluorophenyl)-6,7-dihydrothieno[3,2-c]pyridin-5(4H)-yl)-4-oxobutanamide; |
| **121** | 1-(4-(3-fluorophenyl)-6,7-dihydrothieno[3,2-c]pyridin-5(4H)-yl)-4-(3-phenyl-pyrrolidin-1-yl)butane-1,4-dione; |
| **122** | 1-(4-(3-fluorophenyl)-6,7-dihydrothieno[3,2-c]pyridin-5(4H)-yl)-4-(4-(furan-2-carbonyl)piperazin-1-yl) butane-1,4-dione; |
| **123** | N-(3-(3,4-dihydroquinolin-1(2H)-yl)propyl)-3-methyl-4-oxo-4-(4-phenyl-6,7-dihydrothieno[3,2-c]pyridin-5 (4H)-yl)butanamide; |
| **124** | 2-methyl-1-(4-phenyl-6,7-dihydrothieno[3,2-c]pyridin-5(4H)-yl)-4-(piperidin-1-yl)butane-1,4-dione; |
| **125** | 4-(4-(2-methoxyphenyl)piperazin-1-yl)-2-methyl-1-(4-phenyl-6,7-dihydrothieno[3,2-c]pyridin-5(4H)-yl) butane-1,4-dione; |
| **126** | 4-oxo-4-(4-phenyl-6,7-dihydrothieno[3,2-c]pyridin-5(4H)-yl)-N-(2-(pyrazin-2-yl)ethyl)butanamide; |
| **127** | N,N-diethyl-4-oxo-4-(4-phenyl-6,7-dihydrothieno[3,2-c]pyridin-5(4H)-yl)-butanamide; |
| **128** | 1-(4-phenyl-6,7-dihydrothieno[3,2-c]pyridin-5(4H)-yl)-4-(4-(pyridin-2-yl)piperazin-1-yl)butane-1,4-dione; |
| **129** | 1-(4-(4-fluorophenyl)-6,7-dihyd rothieno[3,2-c]pyrid in-5(4H)-yl)-4-(4-(3-methoxyphenyl)piperazin-1-yl) butane-1,4-dione; |
| **130** | 1-(4-isopropylpiperazin-1-yl)-4-(4-p-tolyl-6,7-dihydrothieno[3,2-c]pyridin-5(4H)-yl)butane-1,4-dione; |
| **131** | 1-(4-p-tolyl-6,7-dihydrothieno[3,2-c]pyridin-5(4H)-yl)-4-(4-(3-(trifluoromethyl)phenyl)piperazin-1-yl) butane-1,4-dione; |
| **132** | 1-(4-phenylpiperazin-1-yl)-4-(4-m-tolyl-6,7-dihydrothieno[3,2-c]pyridin-5(4H)-yl)butane-1,4-dione; |
| **133** | 1-(3,5-dimethylpiperidin-1-yl)-4-(4-m-tolyl-6,7-dihydrothieno[3,2-c]pyridin-5(4H)-yl)butane-1,4-dione; |
| **134** | 1-(5,6-dihydropyridin-1 (2H)-yl)-4-(4-(3-fluorophenyl)-6,7-dihydrothieno[3,2-c]pyridin-5(4H)-yl)butane-1,4-dione; |
| **135** | 1-(4-(3-fluorophenyl)-6,7-dihydrothieno[3,2-c]pyridin-5(4H)-yl)-4-(4-(pyrimidin-2-yl)piperazin-1-yl)butane-1,4-dione; |
| **136** | 1-(4-(3-fluorophenyl)-6,7-dihydrothieno[3,2-c]pyridin-5(4H)-yl)-4-(3-methyl-piperidin-1-yl)butane-1,4-dione; |
| **137** | N-ethyl-4-(4-(3-fluorophenyl)-6,7-dihydrothieno[3,2-c]pyridin-5(4H)-yl)-4-oxo-N-(pyridin-4-ylmethyl) butanamide; |

(continued)

| | |
|---|---|
| **138** | 4-(4-acetylpiperazin-1-yl)-2-methyl-1-(4-phenyl-6,7-dihydrothieno[3,2-c]pyridin-5(4H)-yl)butane-1,4-dione; |
| **139** | 4-(2,6-dimethylmorpholino)-2-methyl-1-(4-phenyl-6,7-dihydrothieno[3,2-c]-pyridin-5(4H)-yl)butane-1,4-dione; |
| **140** | 4-(2,5-dihydro-1H-pyrrol-1-yl)-2-methyl-1-(4-phenyl-6,7-dihydrothieno[3,2-c]pyridin-5(4H)-yl)butane-1,4-dione; |
| **141** | N-(2-methoxyphenethyl)-4-oxo-4-(4-phenyl-6,7-dihydrothieno[3,2-c]pyridin-5(4H)-yl)butanamide; |
| **142** | 4-(4-(4-fluorophenyl)-6,7-dihydrothieno[3,2-c]pyridin-5(4H)-yl)-N-isopentyl-4-oxobutanamide; |
| **143** | N-(2,4-dimethoxybenzyl)-4-(4-(4-fluorophenyl)-6,7-dihydrothieno[3,2-c]pyridin-5(4H)-yl)-4-oxobutanamide; |
| **144** | 4-(4-(4-chlorophenyl)-6,7-dihydrothieno[3,2-c]pyridin-5(4H)-yl)-N-((5-methylfuran-2-yl)methyl)-4-oxobutanamide; |
| **145** | 4-(4-(4-chlorophenyl)-6,7-dihydrothieno[3,2-c]pyridin-5(4H)-yl)-4-oxo-N-pentylbutanamide; |
| **146** | 1-(4-benzylpiperidin-1-yl)-4-(4-(4-chlorophenyl)-6,7-dihydrothieno[3,2-c]-pyridin-5(4H)-yl)butane-1,4-dione; |
| **147** | 1-(4-(4-chlorophenyl)-6,7-dihydrothieno[3,2-c]pyridin-5(4H)-yl)-4-(4-methylpiperidin-1-yl)butane-1,4-dione; |
| **148** | 1-(azetidin-1-yl)-4-(4-(4-chlorophenyl)-6,7-dihydrothieno[3,2-c]pyridin-5(4H)-yl)butane-1,4-dione; |
| **149** | 1-(3,4-dihydroisoquinolin-2(1H)-yl)-2-methyl-4-(4-phenyl-6,7-dihydrothieno[3,2-c]pyridin-5(4H)-yl)butane-1,4-dione; |
| **150** | 1-(4-(benzo[d][1,3]dioxol-5-ylmethyl)piperazin-1-yl)-2-methyl-4-(4-phenyl-6,7-dihydrothieno[3,2-c]pyridin-5(4H)-yl)butane-1,4-dione; |
| **151** | N-benzyl-N-ethyl-4-oxo-4-(4-p-tolyl-6,7-dihydrothieno[3,2-c]pyridin-5(4H)-yl)butanamide; |
| **152** | N-methyl-4-oxo-N-phenethyl-4-(4-p-tolyl-6,7-dihydrothieno[3,2-c]pyridin-5(4H)-yl)butanamide; |
| **153** | ethyl1-(4-oxo-4-(4-p-tolyl-6,7-dihydrothieno[3,2-c]pyridin-5(4H)-yl)butanoyl)piperidine-4-carboxylate; |
| **154** | (E)-1-(4-(3-phenylprop-2-enyl)-piperazin-1-yl)-4-(4-p-tolyl-6,7-dihydrothieno[3,2-c]pyridin-5(4H)-yl) butane-1,4-dione; |
| **155** | 1-(2-(thiazol-2-yl)pyrrolidin-1-yl)-4-(4-p-tolyl-6, 7-dihyd rothieno[3,2-c]pyridin-5(4H)-yl)butane-1,4-dione; |
| **156** | N-cyclohexyl-N-ethyl-4-oxo-4-(4-m-tolyl-6,7-dihydrothieno[3,2-c]pyridin-5(4H)-yl)butanamide; |
| **157** | N-ethyl-N-isopropyl-4-oxo-4-(4-m-tolyl-6,7-dihydrothieno[3,2-c]pyridin-5(4H)-yl)butanamide; |
| **158** | 1-(pyrrolidin-1-yl)-4-(4-m-tolyl-6,7-dihydrothieno[3,2-c]pyridin-5(4H)-yl)butane-1,4-dione; |
| **159** | 1-(4-(pyridin-4-yl)piperazin-1-yl)-4-(4-m-tolyl-6, 7-d ihydrothieno[3,2-c]pyridin-5(4H)-yl)butane-1,4-dione; |
| **160** | 1-(2-(pyridin-2-ylmethyl)pyrrolidin-1-yl)-4-(4-m-tolyl-6,7-dihydrothieno[3,2-c]pyridin-5(4H)-yl)butane-1,4-dione; |
| **161** | ethyl 1-(4-(4-(4-fluorophenyl)-6,7-dihydrothieno[3,2-c]pyridin-5(4H)-yl)-4-oxobutanoyl)piperidine-3-carboxylate; |
| **162** | 1-(4-(4-fluorophenyl)-6,7-dihydrothieno[3,2-c]pyridin-5(4H)-yl)-4-(4-(4-(trifluoromethyl)phenyl)piperazin-1-yl)butane-1,4-dione; |
| **163** | 1-(2-(5-bromopyridin-3-yl)pyrrolidin-1-yl)-4-(4-(4-fluorophenyl)-6,7-dihydro-thieno[3,2-c]pyridin-5(4H)-yl) butane-1,4-dione; |
| **164** | 1-(4-ethylpiperazin-1-yl)-4-(4-phenyl-6,7-dihydrothieno[3,2-c]pyridin-5(4H)-yl)butane-1,4-dione; |
| **165** | 1-(2-ethylpiperidin-1-yl)-4-(4-phenyl-6,7-dihydrothieno[3,2-c]pyridin-5(4H)-yl)butane-1,4-dione; |
| **166** | N,N-dibenzyl-4-oxo-4-(4-phenyl-6,7-dihydrothieno[3,2-c]pyridin-5(4H)-yl)butanamide; |
| **167** | N,N-diisobutyl-4-oxo-4-(4-phenyl-6,7-dihydrothieno[3,2-c]pyridin-5(4H)-yl)butanamide; |
| **168** | 1-(3,4-dihydroquinolin-1 (2H)-yl)-4-(4-(4-fluorophenyl)-6,7-dihydrothieno[3,2-c]pyridin-5(4H)-yl)butane-1,4-dione; |
| **169** | 1-(4-(3,4-dichlorobenzyl)piperazin-1-yl)-4-(4-p-tolyl-6,7-dihydrothieno[3,2-c]pyridin-5(4H)-yl)butane-1,4-dione; |
| **170** | 1-(4-p-tolyl-6,7-dihydrothieno[3,2-c]pyridin-5(4H)-yl)-4-(4-(2,4,6-trimethyl-benzyl)piperazin-1-yl)butane-1,4-dione; |
| **171** | 1-(4-(4-bromobenzyl)piperazin-1-yl)-4-(4-m-tolyl-6,7-dihydrothieno[3,2-c]-pyridin-5(4H)-yl)butane-1,4-dione; |

(continued)

| | |
|---|---|
| **172** | 1-(4-(4-chlorobenzyl)piperazin-1-yl)-4-(4-m-tolyl-6,7-dihydrothieno[3,2-c]-pyridin-5(4H)-yl)butane-1,4-dione; |
| **173** | 1-(2-((4,6-dimethylpyridin-2-yl)methyl)pyrrolidin-1-yl)-4-(4-(3-fluorophenyl)-6,7-dihydrothieno[3,2-c] pyridin-5(4H)-yl)butane-1,4-dione; |
| **174** | 1-(4-(3-fluorophenyl)-6,7-dihydrothieno[3,2-c]pyridin-5(4H)-yl)-4-(2-((6-methylpyridin-2-yl)methyl) pyrrolidin-1-yl)butane-1,4-dione; |
| **175** | 1-(2-((5-ethylpyridin-2-yl)methyl)pyrrolidin-1-yl)-4-(4-(3-fluorophenyl)-6,7-dihydrothieno[3,2-c]pyridin-5 (4H)-yl)butane-1,4-dione; |
| **176** | 1-(4-(3-fluorophenyl)-6,7-dihydrothieno[3,2-c]pyridin-5(4H)-yl)-4-(2-(6-methoxypyridin-3-yl)piperidin-1-yl) butane-1,4-dione; |
| **177** | 4-(4-(3-fluorophenyl)-6,7-dihydrothieno[3,2-c]pyridin-5(4H)-yl)-N-methyl-4-oxo-N-(pyridin-3-ylmethyl) butanamide; |
| **178** | N-(2,2-diphenylethyl)-3-methyl-4-oxo-4-(4-phenyl-6,7-dihydrothieno[3,2-c]-pyridin-5(4H)-yl)butanamide; |
| **179** | N-(cyclopropylmethyl)-4-oxo-4-(4-phenyl-6,7-dihydrothieno[3,2-c]pyridin-5(4H)-yl)butanamide; |
| **180** | 1-(4-(3-fluorophenyl)-6,7-dihydrothieno[3,2-c]pyridin-5(4H)-yl)-4-(4-(4-methylbenzyl)piperazin-1-yl) butane-1,4-dione; |
| **181** | 2-methyl-1-(4-phenyl-6,7-dihydrothieno[3,2-c]pyridin-5(4H)-yl)-4-(2-(pyridin-2-ylmethyl)piperidin-1-yl) butane-1,4-dione; |
| **182** | 4-(4-(3,5-dichloropyridin-4-yl)piperazin-1-yl)-2-methyl-1-(4-phenyl-6,7-dihydrothieno[3,2-c]pyridin-5 (4H)-yl)butane-1,4-dione; |
| **183** | 1-(2-((4,6-dimethylpyridin-2-yl)methyl)piperidin-1-yl)-4-(4-phenyl-6,7-dihydrothieno[3,2-c]pyridin-5(4H)-yl) butane-1,4-dione; |
| **184** | N-(4-fluorobenzyl)-N-methyl-4-oxo-4-(4-phenyl-6,7-dihydrothieno[3,2-c]pyridin-5(4H)-yl)butanamide; |
| **185** | 1-(4-(3-fluorophenyl)-6,7-dihydrothieno[3,2-c]pyridin-5(4H)-yl)-4-(2-(pyridin-2-yl)pyrrolidin-1-yl)butane-1,4-dione; |
| **186** | 4-(4-(4-methoxybenzyl)piperazin-1-yl)-2-methyl-1-(4-phenyl-6,7-dihydro-thieno[3,2-c]pyridin-5(4H)-yl) butane-1,4-dione; |
| **187** | 2-methyl-1-(4-phenyl-6,7-dihydrothieno[3,2-c]pyridin-5(4H)-yl)-4-(3-(pyridin-3-yl)pyrrolidin-1-yl)butane-1,4-dione; |
| **188** | 1-(4-(2-fluorobenzyl)piperazin-1-yl)-4-(4-phenyl-6,7-dihydrothieno[3,2-c]-pyridin-5(4H)-yl)butane-1,4-dione; |
| **189** | N-methyl-4-oxo-4-(4-phenyl-6,7-dihydrothieno[3,2-c]pyridin-5(4H)-yl)-N-(2-(pyridin-4-yl)ethyl) butanamide; |
| **190** | N-butyl-N-ethyl-3-methyl-4-oxo-4-(4-phenyl-6,7-dihydrothieno[3,2-c]pyridin-5(4H)-yl)butanamide; |
| **191** | N,3-dimethyl-4-oxo-4-(4-phenyl-6,7-dihydrothieno[3,2-c]pyridin-5(4H)-yl)-N-propylbutanamide; |
| **192** | 4-((S)-2-(methoxymethyl)pyrrolidin-1-yl)-2-methyl-1-(4-phenyl-6,7-dihydro-thieno[3,2-c]pyridin-5(4H)-yl) butane-1,4-dione; |
| **193** | 4-(4-(5-chloro-2-methylphenyl)piperazin-1-yl)-2-methyl-1-(4-phenyl-6,7-dihydrothieno[3,2-c]pyridin-5 (4H)-yl)butane-1,4-dione; |
| **194** | N-(furan-2-ylmethyl)-N-methyl-4-oxo-4-(4-phenyl-6,7-dihydrothieno[3,2-c]-pyridin-5(4H)-yl)butanamide; |
| **195** | 1-((4aR,8aS)-octahydroisoquinolin-2(1H)-yl)-4-(4-phenyl-6,7-dihydrothieno[3,2-c]pyridin-5(4H)-yl)butane-1,4-dione; |
| **196** | 1-(4-phenyl-6,7-dihydrothieno[3,2-c]pyridin-5(4H)-yl)-4-thiomorpholinobutane-1,4-dione; |
| **197** | 1-(4-phenyl-6,7-dihydrothieno[3,2-c]pyridin-5(4H)-yl)-4-(2-(pyridin-3-yl)-pyrrolidin-1-yl)butane-1,4-dione; |
| **198** | N-benzyl-4-(4-(4-fluorophenyl)-6,7-dihydrothieno[3,2-c]pyridin-5(4H)-yl)-N-isopropyl-4-oxobutanamide; |
| **199** | N-benzyl-4-(4-(4-fluorophenyl)-6,7-dihydrothieno[3,2-c]pyridin-5(4H)-yl)-N-methyl-4-oxobutanamide; |
| **200** | N-(3,4-dimethoxyphenethyl)-4-(4-(4-fluorophenyl)-6,7-dihydrothieno[3,2-c]pyridin-5(4H)-yl)-N-methyl-4-oxobutanamide; |
| **201** | 1-(4-(4-fluorophenyl)-6,7-dihydrothieno[3,2-c]pyridin-5(4H)-yl)-4-(4-methyl-2-phenylpiperazin-1-yl) butane-1,4-dione; |
| **202** | 1-(4-(4-chlorophenyl)-6,7-dihydrothieno[3,2-c]pyridin-5(4H)-yl)-4-(4-phenylpiperidin-1-yl)butane-1,4-dione; |
| **203** | 4-(4-(4-chlorophenyl)-6,7-dihydrothieno[3,2-c]pyridin-5(4H)-yl)-4-oxo-N-(2-(pyridin-2-yl)ethyl)butanamide; |

(continued)

| | |
|---|---|
| **204** | N-benzyl-2-methyl-4-oxo-N-phenethyl-4-(4-phenyl-6,7-dihydrothieno[3,2-c]pyridin-5(4H)-yl)butanamide; |
| **205** | 1-(4-(4-fluorophenyl)-6,7-dihydrothieno[3,2-c]pyridin-5(4H)-yl)-4-(2-((6-methylpyridin-2-yl)methyl) piperidin-1-yl)butane-1,4-dione; |
| **206** | 1-(4-(4-fluorophenyl)-6,7-dihydrothieno[3,2-c]pyridin-5(4H)-yl)-4-(4-(3-methylbenzyl)piperazin-1-yl) butane-1,4-dione; |
| **207** | 1-(4-(4-chlorophenyl)-6,7-dihydrothieno[3,2-c]pyridin-5(4H)-yl)-4-(2-(pyridin-4-ylmethyl)piperidin-1-yl) butane-1,4-dione; |
| **208** | 1-(4-(4-chlorophenyl)-6,7-dihydrothieno[3,2-c]pyridin-5(4H)-yl)-4-(2-((5-ethylpyridin-2-yl)methyl)piperidin-1-yl)butane-1,4-dione; |
| **209** | 1-(4-(4-chlorophenyl)-6,7-dihydrothieno[3,2-c]pyridin-5(4H)-yl)-4-(2-((3-methylpyridin-2-yl)methyl) piperidin-1-yl)butane-1,4-dione; |
| **210** | 1-(4-(4-chlorophenyl)-6,7-dihydrothieno[3,2-c]pyridin-5(4H)-yl)-4-(4-(3-chlorophenyl)piperazin-1-yl) butane-1,4-dione; |
| **211** | 1-(4-(4-chlorophenyl)-6,7-dihydrothieno[3,2-c]pyridin-5(4H)-yl)-4-(4-(2,3-dimethylphenyl)piperazin-1-yl) butane-1,4-dione; |
| **212** | 1-(4-(4-chlorophenyl)-6,7-dihydrothieno[3,2-c]pyridin-5(4H)-yl)-4-(4-(3,4-dimethylphenyl)piperazin-1-yl) butane-1,4-dione; |
| **213** | 1-(4-(4-chlorophenyl)-6,7-dihydrothieno[3,2-c]pyridin-5(4H)-yl)-4-(4-(3,4-dichlorophenyl)piperazin-1-yl) butane-1,4-dione; |
| **214** | 1-(4-(4-tert-butylbenzyl)piperazin-1-yl)-4-(4-(4-chlorophenyl)-6,7-dihydro-thieno[3,2-c]pyridin-5(4H)-yl) butane-1,4-dione; |
| **215** | 1-[4-(3,5-dichloro-4-pyridyl)-1-piperazinyl]-2-methyl-4-(4-phenyl-6,7-dihydro-4H-thieno[3,2-c]pyridin-5-yl) butane-1,4-dione; |
| **216** | 1-[4-[(4-methoxyphenyl)methyl]-1-piperazinyl]-4-[4-(p-tolyl)-6, 7-dihydro-4H-thieno[3,2-c]pyridin-5-yl] butane-1,4-dione; |
| **217** | 1-[4-[(2-fluorophenyl)methyl]-1-piperazinyl]-4-[4-(p-tolyl)-6,7-dihydro-4H-thieno[3,2-c]pyridin-5-yl]butane-1,4-dione; |
| **218** | 1-(4-methyl-2-phenyl-1-piperazinyl)-4-[4-(p-tolyl)-6,7-dihydro-4H-thieno[3,2-c]pyridin-5-yl]butane-1,4-dione; |
| **219** | 1-(4-phenyl-1-piperidinyl)-4-[4-(p-tolyl)-6,7-dihydro-4H-thieno[3,2-c]pyridin-5-yl]butane-1,4-dione; |
| **220** | 1-[4-(p-tolyl)-6,7-dihydro-4H-thieno[3,2-c]pyridin-5-yl]-4-[2-(2-pyridyl)-1-pyrrolidinyl]butane-1,4-dione; |
| **221** | 1-[4-(p-tolyl)-6,7-dihydro-4H-thieno[3,2-c]pyridin-5-yl]-4-[3-(3-pyridyl)-1-pyrrolidinyl]butane-1,4-dione; |
| **222** | 4-oxo-4-[4-(p-tolyl)-6,7-dihydro-4H-thieno[3,2-c]pyridin-5-yl]-N-[2-(2-pyridyl)ethyl]butanamide; |
| **223** | 1-[4-[(4-methoxyphenyl)methyl]-1-piperazinyl]-4-[4-(m-tolyl)-6,7-dihydro-4H-thieno[3,2-c]pyridin-5-yl] butane-1,4-dione; |
| **224** | 1-[4-[(2-fluorophenyl)methyl]-1-piperazinyl]-4-[4-(m-tolyl)-6,7-dihydro-4H-thieno[3,2-c]pyridin-5-yl]butane-1,4-dione; |
| **225** | 1-(4-methyl-2-phenyl-1-piperazinyl)-4-[4-(m-tolyl)-6,7-dihydro-4H-thieno[3,2-c]pyridin-5-yl]butane-1,4-dione; |
| **226** | 1-[4-(m-tolyl)-6,7-dihydro-4H-thieno[3,2-c]pyridin-5-yl]-4-(4-phenyl-1-piperidinyl)butane-1,4-dione; |
| **227** | 1-[4-(m-tolyl)-6,7-dihydro-4H-thieno[3,2-c]pyridin-5-yl]-4-[2-(2-pyridyl)-1-pyrrolidinyl]butane-1,4-dione; |
| **228** | 1-[4-(m-tolyl)-6,7-dihydro-4H-thieno[3,2-c]pyridin-5-yl]-4-[3-(3-pyridyl)-1-pyrrolidinyl]butane-1,4-dione; |
| **229** | N-methyl-4-[4-(m-tolyl)-6,7-dihydro-4H-thieno[3,2-c]pyridin-5-yl]-4-oxo-N-[2-(4-pyridyl)ethyl]butanamide; |
| **230** | 1-[4-(3-fluorophenyl)-6,7-dihydro-4H-thieno[3,2-c]pyridin-5-yl]-4-[4-[(4-methoxyphenyl)methyl]-1-piperazinyl]butane-1,4-dione; |
| **231** | 1-[4-(3-fluorophenyl)-6,7-dihydro-4H-thieno[3,2-c]pyridin-5-yl]-4-[4-[(2-fluorophenyl)methyl]-1-piperazinyl] butane-1,4-dione; |
| **232** | 4-[4-(m-tolyl)-6,7-dihydro-4H-thieno[3,2-c]pyridin-5-yl]-4-oxo-N-(p-tolyl)-butanamide; |
| **233** | N-(2,4-dimethylphenyl)-4-[4-(m-tolyl)-6,7-dihydro-4H-thieno[3,2-c]pyridin-5-yl]-4-oxobutanamide; |
| **234** | 4-[4-(3-fluorophenyl)-6,7-dihydro-4H-thieno[3,2-c]pyridin-5-yl]-N-(1-methyl-6-indazolyl)-4-oxobutanamide; |
| **235** | 4-[4-(3-fluorophenyl)-6,7-dihydro-4H-thieno[3,2-c]pyridin-5-yl]-4-oxo-N-(p-tolyl)butanamide; |

(continued)

| | |
|---|---|
| **236** | 4-[4-(3-fluorophenyl)-6,7-dihydro-4H-thieno[3,2-c]pyridin-5-yl]-4-oxo-N-phenylbutanamide; |
| **237** | N-(2-chlorophenyl)-4-[4-(3-fluorophenyl)-6,7-dihydro-4H-thieno[3,2-c]pyridin-5-yl]-4-oxobutanamide; |
| **238** | N-(2,4-dimethylphenyl)-4-[4-(3-fluorophenyl)-6,7-dihydro-4H-thieno[3,2-c]-pyridin-5-yl]-4-oxobutanamide; |
| **239** | 4-[4-(4-chlorophenyl)-6,7-dihydro-4H-thieno[3,2-c]pyridin-5-yl]-N-(1-methyl-6-indazolyl)-4-oxobutanamide; |
| **240** | 4-[4-(4-chlorophenyl)-6,7-dihydro-4H-thieno[3,2-c]pyridin-5-yl]-4-oxo-N-(p-tolyl)butanamide; |
| **241** | 4-[4-(4-chlorophenyl)-6,7-dihydro-4H-thieno[3,2-c]pyridin-5-yl]-N-(4-methoxyphenyl)-4-oxobutanamide; |
| **242** | 4-[4-(4-chlorophenyl)-6,7-dihydro-4H-thieno[3,2-c]pyridin-5-yl]-4-oxo-N-phenylbutanamide; |
| **243** | 4-[4-(4-chlorophenyl)-6,7-dihydro-4H-thieno[3,2-c]pyridin-5-yl]-N-(2,4-dimethylphenyl)-4-oxobutanamide; |
| **244** | 4-[4-(2,6-dimethylphenyl)-6,7-dihydro-4H-thieno[3,2-c]pyridin-5-yl]-4-oxo-N-[[3-(trifluoromethyl)phenyl]methyl]butanamide; |
| **245** | N-(2-cyclohexylethyl)-4-oxo-4-(4-phenyl-6,7-dihydro-4H-thieno[3,2-c]pyridin-5-yl)butanamide; |
| **246** | N-(3,3-dimethylbutyl)-4-oxo-4-(4-phenyl-6,7-dihydro-4H-thieno[3,2-c]pyridin-5-yl)butanamide; |
| **247** | N-(cyclohexylmethyl)-4-oxo-4-(4-phenyl-6,7-dihydro-4H-thieno[3,2-c]pyridin-5-yl)butanamide; |
| **248** | N-[[3-methyl-5-(trifluoromethoxy)phenyl]methyl]-4-oxo-4-(4-phenyl-6,7-dihydro-4H-thieno[3,2-c]pyridin-5-yl)butanamide; |
| **249** | N-[[4-methyl-3-(trifluoromethyl)phenyl]methyl]-4-oxo-4-(4-phenyl-6,7-dihydro-4H-thieno[3,2-c]pyridin-5-yl)butanamide; |
| **250** | N-[[4-fluoro-3-(trifluoromethyl)phenyl]methyl]-4-oxo-4-(4-phenyl-6,7-dihydro-4H-thieno[3,2-c]pyridin-5-yl)butanamide; |
| **251** | 4-[4-(2-ethylphenyl)-6,7-dihydro-4H-thieno[3,2-c]pyridin-5-yl]-4-oxo-N-[[3-(trifluoromethyl)phenyl]methyl]butanamide; |
| **252** | 4-[4-(2-isopropylphenyl)-6,7-dihydro-4H-thieno[3,2-c]pyridin-5-yl]-4-oxo-N-[[3-(trifluoromethyl)phenyl]methyl]butanamide; |
| **253** | N-(3-cyclohexylpropyl)-4-oxo-4-(4-phenyl-6,7-dihydro-4H-thieno[3,2-c]pyridin-5-yl)butanamide; |
| **254** | 4-[4-(3-methyl-2-thienyl)-6,7-dihydro-4H-thieno[3,2-c]pyridin-5-yl]-4-oxo-N-[[3-(trifluoromethyl)phenyl]methyl]butanamide; |
| **255** | (1 S,2S)-2-[oxo-(4-phenyl-6,7-dihydro-4H-thieno[3,2-c]pyridin-5-yl)methyl]-N-[[3-(trifluoromethyl)phenyl]methyl]-1-cyclopropanecarboxamide; |
| **256** | 4-[4-(o-tolyl)-6,7-dihydro-4H-thieno[3,2-c]pyridin-5-yl]-4-oxo-N-[[2-(trifluoromethyl)phenyl]methyl]butanamide; |
| **257** | 4-[4-(o-tolyl)-6,7-dihydro-4H-thieno[3,2-c]pyridin-5-yl]-4-oxo-N-[[4-(trifluoromethyl)phenyl]methyl]butanamide; |
| **258** | 4-(7-butyl-5,7-dihydro-4H-thieno[2,3-c]pyridin-6-yl)-4-oxo-N-[[3-(trifluoro-methyl)phenyl]methyl]butanamide; |
| **259** | 4-[4-(cyclohexylmethyl)-6,7-dihydro-4H-thieno[3,2-c]pyridin-5-yl]-4-oxo-N-[[3-(trifluoromethyl)phenyl]methyl]butanamide; |
| **260** | 4-(4-cyclopropyl-6,7-dihydro-4H-thieno[3,2-c]pyridin-5-yl)-4-oxo-N-[[3-(trifluoromethyl)phenyl]methyl]butanamide; |
| **261** | 4-oxo-4-(4-phenethyl-6,7-dihydro-4H-thieno[3,2-c]pyridin-5-yl)-N-[[3-(trifluoromethyl)phenyl]methyl]butanamide; |
| **262** | 4-[4-(4-fluoro-2-methylphenyl)-6,7-dihydro-4H-thieno[3,2-c]pyridin-5-yl]-4-oxo-N-[[3-(trifluoromethyl)phenyl]methyl]butanamide; |
| **263** | 4-(4-cyclopentyl-6,7-dihydro-4H-thieno[3,2-c]pyridin-5-yl)-4-oxo-N-[[3-(trifluoromethyl)phenyl]methyl]butanamide; |
| **264** | N-[[2-methyl-3-(trifluoromethyl)phenyl]methyl]-4-oxo-4-(4-phenyl-6,7-dihydro-4H-thieno[3,2-c]pyridin-5-yl)butanamide; |
| **265** | 4-oxo-4-[4-(3-phenylpropyl)-6,7-dihydro-4H-thieno[3,2-c]pyridin-5-yl]-N-[[3-(trifluoromethyl)phenyl]methyl]butanamide; |
| **266** | N-[[2-methyl-5-(trifluoromethyl)phenyl]methyl]-4-oxo-4-(4-phenyl-6,7-dihydro-4H-thieno[3,2-c]pyridin-5-yl)butanamide; |
| **267** | N-cyclohexyl-4-oxo-4-(4-phenyl-6,7-dihydro-4H-thieno[3,2-c]pyridin-5-yl)-butanamide; |

(continued)

| | |
|---|---|
| **268** | N-(2,2-dimethylpropyl)-4-oxo-4-(4-phenyl-6,7-dihydro-4H-thieno[3,2-c]pyridin-5-yl)butanamide; |
| **269** | 1-(4-phenyl-6,7-dihydro-4H-thieno[3,2-c]pyridin-5-yl)-4-[7-(trifluoromethyl)-3,4-dihydro-1H-isoquinolin-2-yl]butane-1,4-dione; |
| **270** | 4-oxo-N-pentyl-4-(4-phenyl-6,7-dihydro-4H-thieno[3,2-c]pyridin-5-yl)-butanamide; |
| **271** | 1-(4-phenyl-6,7-dihydro-4H-thieno[3,2-c]pyridin-5-yl)-4-[5-(trifluoromethyl)-3,4-dihydro-1H-isoquinolin-2-yl]butane-1,4-dione; |
| **272** | 4-oxo-4-(4-phenyl-6,7-dihydro-4H-thieno[3,2-c]pyridin-5-yl)-N-[5-(trifluoro-methyl)-1-tetralinyl] butanamide; |
| **273** | 4-oxo-4-(4-phenyl-6,7-dihydro-4H-thieno[3,2-c]pyridin-5-yl)-N-[7-(trifluoro-methyl)-1-tetralinyl] butanamide; |
| **274** | 4-[7-(o-tolyl)-5,7-dihydro-4H-thieno[2,3-c]pyridin-6-yl]-4-oxo-N-[[3-(trifluoromethyl)phenyl]methyl] butanamide; |
| **275** | 4-(7-cyclohexyl-5,7-dihydro-4H-thieno[2,3-c]pyridin-6-yl)-4-oxo-N-[[3-(trifluoromethyl)phenyl]methyl] butanamide; |

or physiologically acceptable salts thereof.

**12.** Medicament comprising at least one compound according to any one of claims 1 to 11 or a compound selected from the group consisting of

- 1-morpholino-4-(4-(thiophen-2-yl)-6,7-dihydrothieno[3,2-c]pyridin-5(4H)-yl)butane-1,4-dione,
- 1-(4-acetylpiperazin-1-yl)-4-(4-(thiophen-2-yl)-6,7-dihydrothieno[3,2-c]pyridin-5(4H)-yl)butane-1,4-dione and
- 1-(3-phenyl-4,5-dihydropyrazol-1-yl)-4-(4-(thiophen-2-yl)-6,7-dihydrothieno-[3,2-c]pyridin-5(4H)-yl)butane-1,4-dione;

in the form of an individual stereoisomer or a mixture thereof, the free compounds and/or physiologically acceptable salts thereof, and suitable additives and/or auxiliary substances and/or optionally further active ingredients.

**13.** Use of a compound according to any one of claims 1 to 11 or of a compound selected from the group consisting of

- 1-morpholino-4-(4-(thiophen-2-yl)-6,7-dihydrothieno[3,2-c]pyridin-5(4H)-yl)butane-1,4-dione,
- 1-(4-acetylpiperazin-1-yl)-4-(4-(thiophen-2-yl)-6,7-dihydrothieno[3,2-c]pyridin-5(4H)-yl)butane-1,4-dione and
- 1-(3-phenyl-4,5-dihydropyrazol-1-yl)-4-(4-(thiophen-2-yl)-6,7-dihydrothieno-[3,2-c]pyridin-5(4H)-yl)butane-1,4-dione;

in the form of an individual stereoisomer or a mixture thereof, the free compound and/or physiologically acceptable salts thereof, in the preparation of a medicament for the treatment of pain, epilepsy, anxiety, dependency, mania, bipolar disorders, migraine, cognitive diseases, dystonia-associated dyskinesias and/or urinary incontinence.

## Revendications

**1.** Tétrahydrothiénopyridines substituées de formule générale (1)

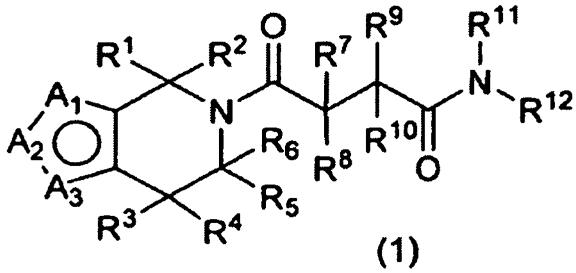

(1)

dans laquelle

$A_1$ représente S, $A_2$ représente $CR^{14}$ et $A_3$ représente $CR^{15}$ ; ou
$A_1$ représente $CR^{13}$, $A_2$ représente S et $A_3$ représente $CR^{15}$ ; ou
$A_1$ représente $CR^{13}$, $A_2$ représente $CR^{14}$ et $A_3$ représente S ;
$R^0$ représente alkyle en $C_{1-8}$, saturé ou insaturé, ramifié ou non ramifié, non substitué ou substitué une ou plusieurs fois ; cycloalkyle en $C_{3-7}$ ou hétérocyclyle, à chaque fois saturé ou insaturé, non substitué ou substitué une ou plusieurs fois ; aryle ou hétéroaryle, à chaque fois non substitué ou substitué une ou plusieurs fois ; cycloalkyle en $C_{3-7}$ ou hétérocyclyle ponté par alkyle en $C_{1-8}$, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; ou aryle ou hétéroaryle ponté par alkyle en $C_{1-8}$, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ;
$R^1$ représente H ; F ; Cl ; Br ; CN ou $R^0$;
$R^2$ représente H ; F ; Cl ; Br ou alkyle en $C_{1-8}$, saturé ou insaturé, ramifié ou non ramifié, non substitué ou substitué une ou plusieurs fois ;
ou $R^1$ et $R^2$ forment ensemble avec l'atome de carbone qui les relie en tant qu'élément de cycle un cycloalkyle en $C_{3-7}$ ou un hétérocyclyle, à chaque fois saturé ou insaturé, non substitué ou substitué une ou plusieurs fois, à chaque fois éventuellement condensé avec (hétéro-)aryle, non substitué ou substitué une ou plusieurs fois ;
$R^3$, $R^4$, $R^5$, $R^6$ représentent indépendamment les uns des autres H ; F ; Cl ; Br ; alkyle en $C_{1-8}$, saturé ou insaturé, ramifié ou non ramifié, non substitué ou substitué une ou plusieurs fois ; ou phényle, non substitué ou substitué une ou plusieurs fois ;
$R^7$ représente H ; F ; Cl ; Br ; CN ; $R^0$ ; $OR^0$ ; O-C(=O)-$R^0$ ; O-C(=O)-O-$R^0$ ; O-(C=O)-NH-$R^0$ ; O-C(=O)-N$(R^0)_2$ ; NH-$R^0$; N$(R^0)_2$ ; NH-C(=O)-$R^0$ ; NH-C(=O)-O-$R^0$ ; NH-C(=O)-NH-$R^0$; NH-C(=O)-N$(R^0)_2$ ; $NR^0$-C(=O)-$R^0$ ; $NR^0$-C(=O)-O-$R^0$ ; $NR^0$-C(=O)-$NH_2$ ; $NR^0$-C(=O)-NH-$R^0$ ; $NR^0$-C(=O)-N$(R^0)_2$ ; NH-S=$)_2$OH ; NH-S$(=O)_2R^0$; NH-S$(=O)_2OR^0$ ; NH-S$(=O)_2NH_2$ ; NH-S$(=O)_2NHR^0$ ; NH-S$(=O)_2N(R^0)_2$ ; $NR^0$-S$(=O)_2$OH ; $NR^0$-S$(=O)_2R^0$ ; $NR^0$-S$(=O)_2OR^0$ ; $NR^0$-S$(=O)_2NH_2$ ; $NR^0$-S$(=O)_2NHR^0$ ; $NR^0$-S$(=O)_2N(R^0)_2$ ; C(=O)-OH ; C(=O)-$OR^0$; C(=O)-$NH_2$ ; C(=O)-NH-$R^0$ ; C(=O)-N$(R^0)_2$ ; S$(=O)_2$-OH ; S$(=O)_2OR^0$ ; S$(=O)_2$-$NH_2$ ; S$(=O)_2$-NH-$R^0$ ; S$(=O)_2$-N$(R^0)_2$ ;
$R^8$ représente H ; F ; C1 ; Br ; CN ou alkyle en $C_{1-8}$ ; saturé ou insaturé, ramifié ou non ramifié, non substitué ou substitué une ou plusieurs fois ;
$R^9$ représente H ; F ; Cl ; Br ; CN ; alkyle en $C_{1-8}$ ; saturé ou insaturé, ramifié ou non ramifié, non substitué ou substitué une ou plusieurs fois ; cycloalkyle en $C_{3-7}$ ou hétérocyclyle, à chaque fois saturé ou insaturé, non substitué ou substitué une ou plusieurs fois ; aryle ou hétéroaryle, à chaque fois non substitué ou substitué une ou plusieurs fois ; cycloalkyle en $C_{3-7}$ ou hétérocyclyle ponté par alkyle en $C_{1-8}$, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; ou aryle ou hétéroaryle ponté par alkyle en $C_{2-8}$, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ; 0-alkyle en $C_{1-8}$, saturé ou insaturé, ramifié ou non ramifié, non substitué ou substitué une ou plusieurs fois ; O-cycloalkyle en $C_{3-7}$ ou O-hétérocyclyle, à chaque fois saturé ou insaturé, non substitué ou substitué une ou plusieurs fois ; cycloalkyle en $C_{3-7}$ ou hétérocycle ponté par 0-alkyle en $C_{1-8}$, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; ou aryle ou hétéroaryle ponté par O-alkyle en $C_{1-8}$, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ; O-C(=O)-$R^0$ ; O-C(=O)-O-R° ; O-(C=O)-NH-$R^0$ ; O-C(=O)-N$(R^0)_2$ ; NH-$R^0$ ; N$(R^0)_2$ ; NH-C(=O)-$R^0$ ; NH-C(=O)-O-$R^0$ ; NH-C(=O)-NH-$R^0$ ; NH-C(=O)-N$(R^0)_2$ ; $NR^0$-C(=O)-$R^0$ ; $NR^0$-C(=O)-$OR^0$ ; $NR^0$-C(=O)-$NH_2$ ; $NR^0$-C(=O)-NH-$R^0$ ; $NR^0$-C(=O)-N$(R^0)_2$ ; NH-S$(=O)_2$OH ; NH-S$(=O)_2R^0$ ; NH-S$(=O)_2OR^0$ ; NH-S $(=O)_2NH_2$ ; NH-S$(=O)_2NHR^0$ ; NH-S$(=O)_2N(R^0)_2$ ; $NR^0$-S$(=O)_2$OH ; $NR^0$-S$(=O)_2R^0$ ; $NR^0$-S$(O)_2OR^0$ ; $NR^0$-S$(=O)_2NH_2$ ; $NR^0$-S$(=O)_2NHR^0$ ; $NR^0$-S$(=O)_2N(R^0)_2$ ; C(=O)-OH ; C(=O)-$OR^0$ ; C(=O)-$NH_2$ ; C(=O)-NH-$R^0$ ; C(=O)-N$(R^0)_2$ ; S$(=O)_2$-OH ; S $(=O)_2OR^0$ ; S$(=O)_2$-$NH_2$ ; S$(=O)_2$-NH-$R^0$ ; S$(=O)_2$-N$(R^0)_2$ ;
$R^{10}$ représente H ; F ; C1 ; Br ; CN ou alkyle en $C_{1-8}$, saturé ou insaturé, ramifié ou non ramifié, non substitué ou substitué une ou plusieurs fois ;
ou $R^7$ et $R^9$ forment ensemble avec les atomes de carbone qui les relient en tant qu'éléments de cycle un cycloalkyle en $C_{3-7}$ ou un hétérocyclyle, à chaque fois saturé ou insaturé, non substitué ou substitué une ou plusieurs fois, éventuellement condensé avec (hétéro- )aryle, non substitué ou substitué une ou plusieurs fois ;
ou $R^7$ et $R^8$ ; ou $R^9$ et $R^{10}$ ; forment ensemble avec les atomes de carbone qui les relient en tant qu'éléments de cycle un cycloalkyle en $C_{3-7}$ ou un hétérocyclyle, à chaque fois saturé ou insaturé, non substitué ou substitué une ou plusieurs fois, à chaque fois éventuellement condensé avec (hétéro-)aryle, non substitué ou substitué

une ou plusieurs fois ;
$R^{11}$ représente H ; alkyle en $C_{1-8}$, saturé ou insaturé, ramifié ou non ramifié, non substitué ou substitué une ou plusieurs fois ; ou cycloalkyle en $C_{3-7}$, saturé ou insaturé, non substitué ou substitué une ou plusieurs fois ;
$R^{12}$ représente alkyle en $C_{2-16}$, saturé ou insaturé ; ramifié ou non ramifié, non substitué ou substitué une ou plusieurs fois ; cycloalkyle en $C_{3-7}$ ou hétérocyclyle, à chaque fois saturé ou insaturé, non substitué ou substitué une ou plusieurs fois ; aryle ou hétéroaryle, non substitué ou substitué une ou plusieurs fois ; cycloalkyle en $C_{3-7}$ ou hétérocyclyle ponté par alkyle en $C_{1-8}$, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; aryle ou hétéroaryle ponté par alkyle en $C_{1-8}$, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ;
ou $R^{11}$ et $R^{12}$ forment ensemble avec l'atome d'azote qui les relie en tant qu'élément de cycle un hétérocyclyle, saturé ou insaturé, non substitué ou substitué une ou plusieurs fois, éventuellement condensé avec (hétéro- )aryle, non substitué ou substitué une ou plusieurs fois ;
$R^{13}$, $R^{14}$ et $R^{15}$ signifient chacun indépendamment les uns des autres H ; F ; Cl ; Br ; I ; NO ; $NO_2$ ; $CF_3$ ; CN ; $R^0$ ; C(=O)H ; C(=O)$R^0$ ; $CO_2H$ ; C(=O)O$R^0$ ; $CONH_2$ ; C(=O)NH$R^0$ ; C(=C)N$(R^0)_2$ ; OH ; O$R^0$ ; O-(alkyle en $C_{1-8}$)-O ; O-C(=O)-$R^0$ ; O-C(=O)-O-$R^0$ ; O-(C=O)-NH-$R^0$ ; O-C(=O)-N$(R^0)_2$ ; O-S$(=O)_2$-$R^0$ ; O-S$(=O)_2$OH ; O-S$(=O)_2$O$R^0$ ; 0-S$(=O)_2NH_2$ ; O-S$(=O)_2$NH$R^0$ ; O-S$(=O)_2$N$(R^0)_2$ ; $NH_2$ ; NH-$R^0$ ; N$(R^0)_2$ ; NH-C(=O)-$R^0$ ; NH-C(=O)-O-$R^0$ ; NH-C(=O)-NH-$R^0$ ; NH-C(=O)-N$(R^0)_2$ ; N$R^0$-C(=O) -$R^0$ ; N$R^0$-C(=O)-O-$R^0$ ; NR°-C(=O)-$NH_2$ ; N$R^0$-C(=O)-NH-$R^0$ ; N$R^0$-C(=O)-N$(R^0)_2$ ; NH-S $(=O)_2$OH ; NH-S$(=O)_2R^0$ ; NH-S$(=O)_2$O$R^0$ ; NH-S$(=O)_2NH_2$ ; NH-S$(=O)_2$NH$R^0$ ; NH-S$(=O)_2$N$(R^0)_2$ ; N$R^0$-S$(=O)_2$OH ; N$R^0$-S$(=O)_2R^0$ ; N$R^0$-S$(=O)_2$O$R^0$ ; N$R^0$-S$(=O)_2NH_2$ ; N$R^0$-S$(=O)_2$NH$R^0$ ; N$R^0$-S$(=O)_2$N$(R^0)_2$ ; SH ; S$R^0$ ; S(=O)$R^0$ ; S$(=O)_2R^0$ ; S$(=O)_2$OH ; S$(=O)_2$O$R^0$ ; S$(=O)_2NH_2$ ; S$(=O)_2$NH$R^0$ ; ou S$(=O)_2$N$(R^0)_2$ ;
« alkyle substitué », « hétérocyclyle substitué » et « cycloalkyle substitué » représentant la substitution d'un ou de plusieurs atomes d'hydrogène à chaque fois indépendamment les uns des autres par F ; C1 ; Br ; I ; CN ; $CF_3$ ; =O ; =NH ; =C$(NH_2)_2$ ; $NO_2$ ; $R^0$ ; C(=O)H ; C(=O)$R^0$ ; $CO_2H$ ; C(=O)O$R^0$ ; $CONH_2$ ; C(=O)NH$R^0$; C(=O)N$(R^0)_2$ ; OH ; O$R^0$ ; O-(alkyle en $C_{1-8}$)-O ; O-C(=O)-$R^0$ ; O-C(=O)-O-$R^0$ ; O-(C=O)-NH-$R^0$ ; O-C(=O)-N$(R^0)_2$ ; OS$(=O)_2$-$R^0$ ; O-S$(=O)_2$OH ; O-S$(=O)_2$O$R^0$ ; O-S$(=O)_2NH_2$ ; OS$(=O)_2$NH$R^0$ ; O-S$(=O)_2$N$(R^0)_2$ ; $NH_2$ ; NH-$R^0$ ; N$(R^0)_2$ ; NH-C(=O)-$R^0$ ; NH-C(=O)-O-$R^0$ ; NH-C(=O)-NH-$R^0$ ; NH-C(=O)-N$(R^0)_2$ ; N$R^0$-C(=O)-$R^0$ ; N$R^0$-C(=O)-O-$R^0$ ; N$R^0$-C(=O)-$NH_2$ ; N$R^0$-C(=O)-NH-$R^0$ ; N$R^0$-C(=O)-N$(R^0)_2$ ; NH-S$(=O)_2$OH ; NH-S$(=O)_2R^0$ ; NH-S$(=O)_2$O$R^0$ ; NH-S$(=O)_2NH_2$ ; NH-S$(=O)_2$NH$R^{10}$ ; NH-S$(=O)_2$N$(R^0)_2$ ; N$R^0$-S$(=O)_2$OH ; N$R^0$-S$(=O)_2R^0$ ; N$R^0$-S$(=O)_2$O$R^0$ ; N$R^0$-S$(=O)_2NH_2$ ; N$R^0$-S$(=O)_2$NH$R^0$ ; N$R^0$-S$(=O)_2$N$(R^0)_2$ ; SH ; S$R^0$ ; S(=O)$R^0$ ; S$(=O)_2R^0$ ; S$(=O)_2$OH ; S$(=O)_2$O$R^0$ ; S$(=O)_2NH_2$ ; S$(=O)_2$NH$R^0$ ; S$(=O)_2$N$(R^0)_2$ ;
« aryle substitué » et « hétéroaryle substitué » représentant la substitution d'un ou de plusieurs atomes d'hydrogène à chaque fois indépendamment les uns des autres par F ; C1 ; Br ; I ; NO ; $NO_2$ ; $CF_3$ ; CN ; $R^0$ ; C(=O)H ; C(=O)$R^0$ ; $CO_2H$ ; C(=O)O$R^0$ ; $CONH_2$ ; C(=O)NH$R^0$ ; C(=O)N$(R^0)_2$ ; OH ; O$R^0$ ; O-(alkyle en $C_{1-8}$)-O ; O-C(=O)-$R^0$ ; O-C(=O)-O-$R^0$ ; O-(C=O)-NH-$R^0$ ; O-C(=O)-N$(R^0)_2$ ; O-S$(=O)_2$-$R^0$ ; O-S$(=O)_2$OH ; O-S$(=O)_2$O$R^0$ ; OS$(=O)_2NH_2$ ; O-S$(=O)_2$NH$R^0$ ; O-S$(=O)_2$N$(R^0)_2$ ; $NH_2$ ; NH-$R^0$ ; N$(R^0)_2$ ; NH-C(=O)-$R^0$ ; NH-C(=O)-O-$R^0$ ; NH-C(=O)-NH-$R^0$ ; NH-C(=O)-N$(R^0)_2$ ; N$R^0$-C(=O)-$R^0$ ; N$R^0$-C(=O)-O-$R^0$ ; N$R^0$-C(=O)-$NH_2$ ; N$R^0$-C(=O)-NH-$R^0$ ; N$R^0$-C(=O)-N$(R^0)_2$ ; NH-S$(=O)_2$OH ; NH-S$(=O)_2R^0$ ; NH-S$(=O)_2$O$R^0$ ; NH-S$(=O)_2NH_2$ ; NH-S$(=O)_2$NH$R^0$ ; NH-S$(=O)_2$N$(R^0)_2$ ; N$R^0$-S$(=O)_2$OH ; N$R^0$-S$(=O)_2R^0$ ; N$R^0$-S$(=O)_2$O$R^0$ ; N$R^0$-S$(=O)_2NH_2$ ; N$R^0$-S$(=O)_2$NH$R^0$ ; N$R^0$-S$(=O)_2$N$(R^0)_2$ ; SH ; S$R^0$ ; S(=O)$R^0$ ; S$(=O)_2R^0$ ; S$(=O)_2$OH ; S$(=O)_2$O$R^0$ ; S$(=O)_2NH_2$ ; S$(=O)_2$NH$R^0$ ; S$(=O)_2$N$(R^0)_2$ ;
à l'exception des composés suivants :

la 1-morpholino-4-(4-(thiophén-2-yl)-6,7-dihydrothiéno[3,2-c]pyridin-5(4H)-yl)butane-1,4-dione,
la 1-(4-acétylpipérazin-1-yl)-4-(4-(thiophén-2-yl)-6,7-dihydrothiéno[3,2-c]pyridin-5(4H)-yl)butane-1,4-dione et
la
1-(3-phényl-4,5-dihydropyrazol-1-yl)-4-(4-(thiophén-2-yl)-6,7-dihydrothiéno[3,2-c]pyridin-5(4H)-yl)butane-1,4-dione ;

sous la forme des composés libres ou de sels d'acides ou de bases physiologiquement compatibles.

**2.** Tétrahydrothiénopyridines selon la revendication 1, **caractérisées en ce que**
$R^{12}$ représente alkyle en $C_{4-16}$, saturé ou insaturé, ramifié ou non ramifié, non substitué ou substitué une ou plusieurs fois ; cycloalkyle en $C_{3-7}$ ou hétérocyclyle, à chaque fois saturé ou insaturé, non substitué ou substitué une ou

plusieurs fois ; aryle ou hétéroaryle, non substitué ou substitué une ou plusieurs fois ; cycloalkyle en $C_{3-7}$ ou hétérocyclyle ponté par alkyle en $C_{1-8}$, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; aryle ou hétéroaryle ponté par alkyle en $C_{1-8}$, à chaque fois non substitué ou substitué une ou plusieurs fois, la chaîne alkyle pouvant à chaque fois être ramifiée ou non ramifiée, saturée ou insaturée, non substituée, substituée une ou plusieurs fois.

**3.** Tétrahydrothiénopyridines selon l'une quelconque des revendications précédentes, **caractérisées en ce que** $R^7$ représente H ; F ; Cl ; Br ; CN ; OH ; $NH_2$ ; alkyle en $C_{1-8}$, O-alkyle en $C_{1-8}$, NH-alkyle en $C_{1-8}$, N(alkyle en $C_{1-8})_2$, à chaque fois saturé ou insaturé, ramifié ou non ramifié, non substitué ou substitué une ou plusieurs fois ; phényle ou hétéroaryle, à chaque fois non substitué ou substitué une ou plusieurs fois ; phényle ou hétéroaryle ponté par alkyle en $C_{1-2}$, à chaque fois non substitué ou substitué une ou plusieurs fois, la chaîne alkyle pouvant à chaque fois être ramifiée ou non ramifiée, saturée ou insaturée, non substituée ou substituée une ou plusieurs fois ;
$R^9$ représente H ; F ; Cl ; Br ; CN ; OH ; $NH_2$ ; alkyle en $C_{1-8}$, O-alkyle en $C_{1-8}$, NH-alkyle en $C_{1-8}$, N(alkyle en $C_{1-8})_2$, à chaque fois saturé ou insaturé, ramifié ou non ramifié, non substitué ou substitué une ou plusieurs fois ; phényle ou hétéroaryle, à chaque fois non substitué ou substitué une ou plusieurs fois ; phényle ou hétéroaryle ponté par alkyle en $C_2$, à chaque fois non substitué ou substitué une ou plusieurs fois, la chaîne alkyle pouvant à chaque fois être ramifiée ou non ramifiée, saturée ou insaturée, non substituée ou substituée une ou plusieurs fois.

**4.** Tétrahydrothiénopyridines selon l'une quelconque des revendications précédentes, **caractérisées en ce que** $R^1$ et $R^2$ signifient à chaque fois H ;
ou $R^2$ signifie H et $R^1$ est différent de H.

**5.** Tétrahydrothiénopyridines selon l'une quelconque des revendications précédentes, qui présentent une des formules générales (1a), (1b) ou (1c), dans lesquelles

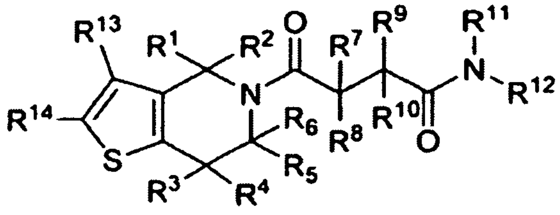

(1a)

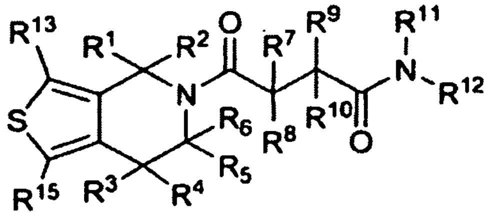

(1b)

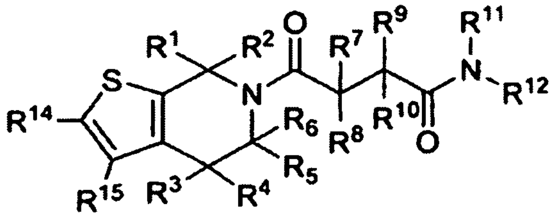

(1c)

$R^{13}$, $R^{14}$ et $R^{15}$ représentent chacun indépendamment les uns des autres H ; F ; Cl ; Br ; I ; NO ; $NO_2$ ; CN ; $NH_2$ ; NH-alkyle en $C_{1-8}$ ; N(alkyle en $C_{1-8})_2$ ; NH-C(=O)-alkyle en $C_{1-8}$ ; NH-C(=O)-aryle ; NH-C(=O)-hétéroaryle ; alkyle en $C_{1-8}$ ; $CF_3$ ; CHO ; C(=O)alkyle en $C_{1-8}$ ; C(=O)aryle ; C(=O)hétéroaryle ; $CO_2H$ ; C(=O)O-alkyle en $C_{1-8}$ ; C(=O)O-aryle; C(=O)O-hétéroaryle ; $CONH_2$ ; C(=O)NH-alkyle en $C_{1-8}$ ; C(=O)N (alkyle en $C_{1-8})_2$ ; C(=O)NH-aryle ; C(=O)N(aryle$)_2$ ; C(=O)NH-hétéroaryle ; C(=O)N(hétéroaryle$)_2$ ; C(=O)N (alkyle en $C_{1-8}$)(aryle) ; C(=O)N(alkyle en $C_{1-8}$) (hétéroaryle) ; C(=O)N(hétéroaryle)(aryle) ; OH ; 0-alkyle en $C_{1-8}$ ; $OCF_3$ ; O-(alkyle en $C_{1-8}$)-O ; O-(alkyle en $C_{1-8}$)-O-alkyle en $C_{1-8}$ ; O-benzyle ; O-aryle ; O-hétéroaryle ; 0-C(=O)alkyle en $C_{1-8}$ ; O-C(=O)aryle ; 0-C(=O)hétéroaryle ; SH ; S-alkyle en $C_{1-8}$ ; $SCF_3$ ; S-benzyle ; S-aryle ; S-hétéroaryle ; aryle ; hétéroaryle ; cycloalkyle en $C_{3-7}$ ; hétérocyclyle ou aryle, hétéroaryle, cycloalkyle en $C_{3-7}$ ou hétérocyclyle ponté par alkyle en $C_{1-8}$.

**6.** Tétrahydrothiénopyridines selon l'une quelconque des revendications précédentes, dans lesquelles

$R^3$, $R^4$, $R^5$ et $R^6$ représentent indépendamment les uns des autres H ; alkyle en $C_{1-8}$, saturé ou insaturé, ramifié ou non ramifié, non substitué ou substitué une ou plusieurs fois ; ou phényle, non substitué ou substitué une ou plusieurs fois.

**7.** Tétrahydrothiénopyridines selon l'une quelconque des revendications précédentes, dans lesquelles
$R^8$ représente H ou alkyle en $C_{1-8}$ ; saturé ou insaturé, ramifié ou non ramifié, non substitué ou substitué une ou plusieurs fois ;
$R^{10}$ représente H ou alkyle en $C_{1-8}$ ; saturé ou insaturé, ramifié ou non ramifié, non substitué ou substitué une ou plusieurs fois.

**8.** Tétrahydrothiénopyridines selon l'une quelconque des revendications précédentes, dans lesquelles
$R^{11}$ représente H ; alkyle en $C_{1-8}$, saturé ou insaturé, ramifié ou non ramifié, cycloalkyle en $C_{3-7}$, saturé ou insaturé, ou benzyle, non substitué ou substitué une ou plusieurs fois.

**9.** Tétrahydrothiénopyridines selon l'une quelconque des revendications précédentes, dans lesquelles
$R^{12}$ représente alkyle en $C_{4-16}$, saturé ou insaturé ; ramifié ou non ramifié, non substitué ou substitué une ou plusieurs fois ; ou est choisi parmi les structures partielles A, B ou C suivantes

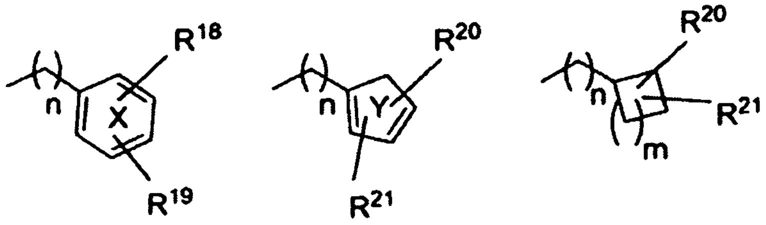

A B C

dans lesquelles

n = 0, 1, 2, 3, 4, 5, 6, 7 ou 8 ;
m = 0, 1, 2 ou 3 ;
le cycle X peut contenir un ou deux atomes N en tant qu'éléments de cycle ;
le cycle Y contient au moins 1 hétéroatome choisi parmi N, O ou S et peut contenir jusqu'à 3 hétéroatomes choisis indépendamment les uns des autres parmi N, O ou S ;
$R^{18}$ et $R^{19}$ signifient indépendamment l'un de l'autre H ; F ; Cl ; Br ; I ; NO ; $NO_2$ ; $CF_3$ ; CN ; $R^0$ ; C(=O)H ; C(=O)$R^0$ ; $CO_2H$ ; C(=O)O$R^0$ ; $CONH_2$ ; C(=O)NH$R^0$ ; C(=O)N($R^0$)$_2$ ; OH ; O$R^0$ ; 0-(alkyle en $C_{1-8}$)-O ; O-C(=O)-$R^0$ ; O-C(=O)-O-$R^0$ ; O-(C=O)-NH-$R^0$ ; O-C(=O)-N($R^0$)$_2$ ; OS(=O)$_2$-$R^0$ ; O-S(=O)$_2$OH ; O-S(=O)$_2$O$R^0$ ; O-S (=O)$_2$NH$_2$ ; 0-S(=O)$_2$NH$R^0$ ; O-S(=O)$_2$N($R^0$)$_2$ ; $NH_2$ ; NH-$R^0$ ; N($R^0$)$_2$ ; NH-C(=O)-$R^0$ ; NH-C(=O)-O-$R^0$ ; NH-C(=O)-NH-$R^0$ ; NH-C(=O)-N($R^0$)$_2$ ; N$R^0$-C(=O)-$R^0$ ; N$R^0$-C (=O)-O-$R^0$ ; N$R^0$-C(=O)-NH$_2$ ; N$R^0$-C(=O)-NH-$R^0$ ; N$R^0$-C(=O)-N($R^0$)$_2$ ; NH-S(=O)$_2$OH ; NH-S(=O)$_2$$R^0$ ; NH-S(=O)$_2$O$R^0$ ; NH-S(=O)$_2$NH$_2$ ; NH-S(=O)$_2$NH$R^0$ ; NH-S(=O)$_2$N($R^0$)$_2$ ; N$R^0$-S(=O)$_2$OH ; N$R^0$-S(=O)$_2$$R^0$ ; N$R^0$-S(=O)$_2$O$R^0$ ; N$R^0$-S(=O)$_2$NH$_2$ N$R^0$-S(=O)$_2$NH$R^0$ ; N$R^0$-S(=O)$_2$N($R^0$)$_2$ ; SH ; S$R^0$ ; S(=O)$R^0$ ; S(=O)$_2$$R^0$ ; S(=O)$_2$OH ; S(=O)$_2$O$R^0$ ; S(=O)$_2$NH$_2$ ; S(=O)$_2$NH$R^0$ ; ou S(=O)$_2$N($R^0$)$_2$ ;
ou $R^{18}$ et $R^{19}$ forment ensemble avec les atomes de carbone ou d'azote qui les relient en tant qu'éléments de cycle un aryle ou hétéroaryle condensé sur le cycle phényle, à chaque fois non substitué ou substitué une ou plusieurs fois ; ou un cycloalkyle en $C_{3-7}$ ou hétérocyclyle condensé sur le cycle phényle, à chaque fois saturé ou insaturé, non substitué ou substitué une ou plusieurs fois ;
$R^{20}$ et $R^{21}$ signifient indépendamment l'un de l'autre H ou alkyle en $C_{1-8}$, saturé ou insaturé, ramifié ou non ramifié, non substitué ou substitué une ou plusieurs fois, cycloalkyle en $C_{3-7}$ ou hétérocyclyle, à chaque fois saturé ou insaturé, non substitué ou substitué une ou plusieurs fois.

**10.** Tétrahydrothiénopyridines selon l'une quelconque des revendications 1 ou 3 à 7, dans lesquelles $R^{11}$ et $R^{12}$ forment ensemble avec l'atome d'azote qui les relie en tant qu'élément de cycle un des cycles suivantes :

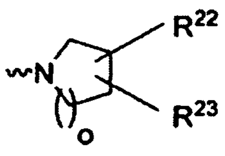 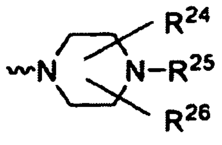 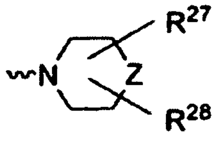 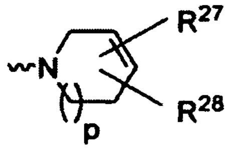 .

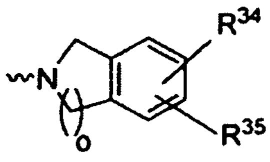

dans lesquels

Z = O ou S ;
o = 0, 1 ou 2 ;
p = 0 ou 1 ;
$R^{22}$, $R^{23}$, $R^{24}$, $R^{25}$ et $R^{26}$ représentent indépendamment les uns des autres H ; alkyle en $C_{1-8}$, saturé ou insaturé, ramifié ou non ramifié, non substitué ou substitué une ou plusieurs fois ; cycloalkyle en $C_{3-7}$ ou hétérocyclyle, à chaque fois saturé ou insaturé, non substitué ou substitué une ou plusieurs fois ; aryle ou hétéroaryle, à chaque fois non substitué ou substitué une ou plusieurs fois ; cycloalkyle en $C_{3-7}$ ou hétérocyclyle ponté par alkyle en $C_{1-8}$, à chaque fois saturé ou insaturé, non substitué ou substitué une ou plusieurs fois, la chaîne alkyle pouvant à chaque fois être ramifiée ou non ramifiée, saturée ou insaturée, non substituée, substituée une ou plusieurs fois ; aryle ou hétéroaryle ponté par alkyle en $C_{1-8}$, à chaque fois non substitué ou substitué une ou plusieurs fois, la chaîne alkyle pouvant à chaque fois être ramifiée ou non ramifiée, saturée ou insaturée, non substituée, substituée une ou plusieurs fois ;
ou $R^{22}$ et $R^{23}$ forment avec le ou les atomes de carbone qui les relient en tant qu'éléments de cycle un cycloalkyle en $C_{3-7}$ ou hétérocyclyle, à chaque fois saturé ou insaturé, non substitué ou substitué une ou plusieurs fois, éventuellement condensé avec (hétéro- )aryle, non substitué ou substitué une ou plusieurs fois ; ou un aryle ou hétéroaryle condensé, non substitué ou substitué une ou plusieurs fois ;
$R^{27}$ et $R^{28}$ signifient indépendamment l' un de l'autre H ; alkyle en $C_{1-8}$, saturé ou insaturé, ramifié ou non ramifié, non substitué ou substitué une ou plusieurs fois ;
$R^{34}$ et $R^{35}$ signifient indépendamment l'un de l'autre H ; F ; Cl ; Br ; I ; NO ; $NO_2$ ; CN ; $NH_2$ ; NH-alkyle en $C_{1-8}$ ; N (alkyle en $C_{1-8})_2$ ; NH-C(=O)alkyle en $C_{1-8}$ ; NH-C(=O)-aryle ; NH-C(=O)-hétéroaryle ; alkyle en $C_{1-8}$ ; $CF_3$ ; C(=O)H ; C(=O)alkyle en $C_{1-8}$ ; C(=O)aryle ; C(=O)hétéroaryle ; $CO_2H$ ; C(=O)O-alkyle en $C_{1-8}$ ; C(=O)O-aryle ; C(=O)O-hétéroaryle ; $CONH_2$ ; C(=O)NH-alkyle en $C_{1-8}$ ; C(=O)N(alkyle en $C_{1-8})_2$ ; C(=O)NH-aryle ; C(=O)N$(aryle)_2$ ; C(=O)NH-hétéroaryle ; C(=O)N$(hétéroaryle)_2$ ; C(=O)N(alkyle en $C_{1-8}$)(aryle) ; C(=O)N(alkyle en $C_{1-8}$) (hétéroaryle) ; C(=O)N(aryle)(hétéroaryle) ; OH ; O-alkyle en $C_{1-8}$ ; O-alkyle en $C_{1-8}$-OH ; $OCF_3$ ; O-(alkyle en $C_{1-8}$)-O-alkyle en $C_{1-8}$ ; O-benzyle ; O-aryle ; O-hétéroaryle ; OC(=O)alkyle en $C_{1-8}$ ; O-C(=O)aryle ; OC(=O)hétéroaryle ; SH ; S-alkyle en $C_{1-8}$ ; $SCF_3$ ; S-benzyle ; S-aryle ; S-hétéroaryle ; aryle ; hétéroaryle ; cycloalkyle en $C_{3-7}$ ; hétérocyclyle ; ou aryle, hétéroaryle, cycloalkyle en $C_{3-7}$ ou hétérocyclyle ponté par alkyle en $C_{1-8}$.

**11.** Tétrahydrothiénopyridines selon la revendication 1, choisies dans le groupe constitué par :

1 le 4-oxo-4-(4-(thiophén-2-yl)-6,7-dihydrothiéno[3,2-c]pyridin-5(4H)-yl)-N-(3-(trifluorométhyl)benzyl)butanamide ;
2 le 4-oxo-4-(4-phényl-6,7-dihydrothiéno[3,2-c]pyridin-5(4H)-yl)-N-(3-(trifluorométhyl)benzyl)butanamide ;
3 le 4-(4-(3-fluorophényl)-6,7-dihydrothiéno[3,2-c]pyridin-5(4H)-yl)-4-oxo-N-(3-(trifluorométhyl)benzyl)butanamide ;
4 le 4-oxo-4-(4-m-tolyl-6,7-dihydrothiéno[3,2-c]pyridin-5(4H)-yl)-N-(3-(trifluorométhyl)benzyl)butanamide ;
5 le 4-oxo-4-(4-p-tolyl-6,7-dihydrothiéno[3,2-c]pyridin-5(4H)-yl)-N-(3-(trifluorométhyl)benzyl)butanamide ;
6 le 4-(4-(2-fluorophényl)-6,7-dihydrothiéno[3,2-c]pyridin-5(4H)-yl)-4-oxo-N-(3-(trifluorométhyl)benzyl)butanamide ;
7 le 4-(4-(4-fluorophényl)-6,7-dihydrothiéno[3,2-c]pyridin-5(4H)-yl)-4-oxo-N-(3-(trifluorométhyl)benzyl)butanamide ;
8 le 4-oxo-4-(4-o-tolyl-6,7-dihydrothiéno[3,2-c]pyridin-5(4H)-yl)-N-(3-(trifluorométhyl)benzyl)butanamide ;
9 le 4-oxo-4-(7-phényl-4,5-dihydrothiéno[2,3-c]pyridin-6(7H)-yl)-N-(3-(trifluorométhyl)benzyl)butanamide ;
10 le 4-(2-méthyl-4-phényl-6,7-dihydrothiéno[3,2-c]pyridin-5(4H)-yl)-4-oxo-N-(3-(trifluorométhyl)benzyl)butanamide ;
11 le N-(4-méthylbenzyl)-4-oxo-4-(4-phényl-6,7-dihydrothiéno[3,2-c]pyridin-5(4H)-yl)butanamide ;
12 le N-benzyl-4-oxo-4-(4-phényl-6,7-dihydrothiéno[3,2-c]pyridin-5(4H)-yl)butanamide ;
13 le 4-oxo-4-(4-phényl-6,7-dihydrothiéno[3,2-c]pyridin-5(4H)-yl)-N-(4-(trifluorométhyl)benzyl)butanamide ;
14 le N-(2-méthoxybenzyl)-4-oxo-4-(4-phényl-6,7-dihydrothiéno[3,2-c]pyridin-5(4H)-yl)butanamide ;
15 le N-(3-méthoxybenzyl)-4-oxo-4-(4-phényl-6,7-dihydrothiéno[3,2-c]pyridin-5(4H)-yl)butanamide ;
16 le N-(4-méthoxybenzyl)-4-oxo-4-(4-phényl-6,7-dihydrothiéno[3,2-c]pyridin-5(4H)-yl)butanamide ;
17 le 4-oxo-4-(4-phényl-6,7-dihydrothiéno[3,2-c]pyridin-5(4H)-yl)-N-(2-(trifluorométhyl)benzyl)butanamide ;
18 le N-(3-fluorobenzyl)-4-oxo-4-(4-phényl-6,7-dihydrothiéno[3,2-c]pyridin-5(4H)-yl)butanamide ;
19 le N-(3-méthylbenzyl)-4-oxo-4-(4-phényl-6,7-dihydrothiéno[3,2-c]pyridin-5(4H)-yl)butanamide ;
20 le 4-(3-méthyl-4-phényl-6,7-dihydrothiéno[3,2-c]pyridin-5(4H)-yl)-4-oxo-N-(3-(trifluorométhyl)benzyl)butanamide ;
21 le 4-(4-cyclohexyl-6,7-dihydrothiéno[3,2-c]pyridin-5(4H)-yl)-4-oxo-N-(3-(trifluorométhyl)benzyl)butanamide ;
22 le 4-(4-isopropyl-6,7-dihydrothiéno[3,2-c]pyridin-5(4H)-yl)-4-oxo-N-(3-(trifluorométhyl)benzyl)butanamide ;
23 le 4-(4-butyl-6,7-dihydrothiéno[3,2-c]pyridin-5(4H)-yl)-4-oxo-N-(3-(trifluorométhyl)benzyl)butanamide ;
24 le N-méthyl-4-oxo-4-(4-phényl-6,7-dihydrothiéno[3,2-c]pyridin-5(4H)-yl)-N-(3-(trifluorométhyl)benzyl)butanamide ;
25 le 4-oxo-N-phénéthyl-4-(4-phényl-6,7-dihydrothiéno[3,2-c]pyridin-5(4H)-yl)butanamide ;
26 le N-(2-méthylbenzyl)-4-oxo-4-(4-phényl-6,7-dihydrothiéno[3,2-c]pyridin-5(4H)-yl)butanamide ;
27 le 4-oxo-2-phényl-4-(4-phényl-6,7-dihydrothiéno[3,2-c]pyridin-5(4H)-yl)-N-(3-(trifluorométhyl)benzyl)butanamide ;
28 le (R)-2-méthyl-4-oxo-4-(4-phényl-6,7-dihydrothiéno[3,2-c]pyridin-5(4H)-yl)-N-(3-(trifluorométhyl)benzyl)butanamide ;
29 le 4-oxo-3-phényl-4-(4-phényl-6,7-dihydrothiéno[3,2-c]pyridin-5(4H)-yl)-N-(3-(trifluorométhyl)benzyl)butanamide ;
30 le 4-(6,7-dihydrothiéno[3,2-c]pyridin-5(4H)-yl)-4-oxo-3-phényl-N-(3-(trifluorométhyl)benzyl)butanamide ;
31 le rac. (1S,2R)-2-(4-phényl-4,5,6,7-tétrahydrothiéno[3,2-c]pyridine-5-carbonyl)-N-(3-(trifluorométhyl)benzyl)cyclohexanecarboxamide ;
32 le rac. (1S)-2-(4-phényl-4,5,6,7-tétrahydrothiéno[3,2-c]pyridine-5-carbonyl)-N-(3-(trifluorométhyl)benzyl)-cyclohexanecarboxamide ;
33 le rac. (1S,2R)-2-(4-phényl-4,5,6,7-tétrahydrothiéno[3,2-c]pyridine-5-carbonyl)-N-(3-(trifluorométhyl)benzyl)cyclopentanecarboxamide ;
34 le (R)-3-méthyl-4-oxo-4-(4-phényl-6,7-dihydrothiéno[3,2-c]pyridin-5(4H)-yl)-N-(3-(trifluorométhyl)benzyl)-butanamide ;
35 le (-)-4-oxo-4-(4-phényl-6,7-dihydrothiéno[3,2-c]pyridin-5(4H)-yl)-N-(3-(trifluorométhyl)benzyl)butanamide ;
36 le (+)-4-oxo-4-(4-phényl-6,7-dihydrothiéno[3,2-c]pyridin-5(4H)-yl)-N-(3-(trifluorométhyl)benzyl)butanamide ;
37 le 4-(6,7-dihydrothiéno[3,2-c]pyridin-5(4H)-yl)-4-oxo-2-phényl-N-(3-(trifluorométhyl)benzyl)butanamide ;
38 le N-(3,5-bis(trifluorométhyl)benzyl)-4-oxo-4-(4-phényl-6,7-dihydrothiéno[3.2-c]pyridin-5(4H)-yl)butanamide ;
39 le N-(2-fluoro-5-(trifluorométhyl)benzyl)-4-oxo-4-(4-phényl-6,7-dihydrothiéno[3,2-c]pyridin-5(4H)-yl)butanamide ;
40 le N-(2-fluoro-3-(trifluorométhyl)benzyl)-4-oxo-4-(4-phényl-6,7-dihydrothiéno[3,2-c]pyridin-5(4H)-yl)butanamide ;
41 le N-(3-fluoro-5-(trifluorométhyl)benzyl)-4-oxo-4-(4-phényl-6,7-dihydrothiéno[3,2-c]pyridin-5(4H)-yl)butanamide ;
42 le rac. (1S,2R)-2-(4-phényl-4,5,6,7-tétrahydrothiéno[3,2-c]pyridine-5-carbonyl)-N-(3-(trifluorométhyl)benzyl)cyclopropanecarboxamide ;
43 le N-(3,4-difluorobenzyl)-4-oxo-4-(4-phényl-6,7-dihydrothiéno[3,2-c]pyridin-5(4H)-yl)butanamide ;
44 le 4-oxo-4-(5-phényl-4,5-dihydrothiéno[2,3-c]pyridin-6(7H)-yl)-N-(3-(trifluorométhyl)benzyl)butanamide ;
45 le 4-(1-méthyl-4-phényl-6,7-dihydrothiéno[3,4-c]pyridin-5(4H)-yl)-4-oxo-N-(3-(trifluorométhyl)benzyl)butanamide ;
46 le N-(4-méthoxyphényl)-4-oxo-4-(4-p-tolyl-6,7-dihydrothiéno[3,2-c]pyridin-5(4H)-yl)butanamide ;
47 le N-(1-méthyl-1H-indazol-6-yl)-4-oxo-4-(4-p-tolyl-6,7-dihydrothiéno[3,2-c]pyridin-5(4H)-yl)butanamide ;
48 le N-benzyl-4-oxo-4-(4-p-tolyl-6,7-dihydrothiéno[3,2-c]pyridin-5(4H)-yl)butanamide ;

49 le 4-oxo-N-phénéthyl-4-(4-p-tolyl-6,7-dihydrothiéno[3,2-c]pyridin-5(4H)-yl)butanamide ;
50 le 4-oxo-N-(pyridin-4-ylméthyl)-4-(4-p-tolyl-6,7-dihydrothiéno[3,2-c]pyridin-5(4H)-yl)butanamide ;
51 le 4-oxo-N-(3-phénylpropyl)-4-(4-p-tolyl-6,7-dihydrothiéno[3,2-c]pyridin-5(4H)-yl)butanamide ;
52 le N-(benzo[c][1,2,5]thiadiazol-4-yl)-4-oxo-4-(4-m-tolyl-6,7-dihydrothiéno[3,2-c]pyridin-5(4H)-yl)butanamide ;
53 le N-(1-méthyl-1H-indazol-6-yl)-4-oxo-4-(4-m-tolyl-6,7-dihydrothiéno[3,2-c]pyridin-5(4H)-yl)butanamide ;
54 le 4-oxo-N-(pyridin-2-ylméthyl)-4-(4-m-tolyl-6,7-dihydrothiéno[3,2-c]pyridin-5(4H)-yl)butanamide ;
55 la 1-(4-méthylpipérazin-1-yl)-4-(4-m-tolyl-6,7-dihydrothiéno[3,2-c]pyridin-5(4H)-yl)butane-1,4-dione ;
56 le 3-méthyl-4-oxo-4-(4-phényl-6,7-dihydrothiéno[3,2-c]pyridin-5(4H)-yl)-N-(pyridin-2-ylméthyl)butanamide ;
57 le 4-oxo-4-(4-phényl-6,7-dihydrothiéno[3,2-c]pyridin-5(4H)-yl)-N-(thiophén-2-ylméthyl)butanamide ;
58 le N-(2-chlorophényl)-4-(4-(4-fluorophényl)-6,7-dihydrothiéno[3,2-c]pyridin-5(4H)-yl)-4-oxobutanamide ;
59 le 4-(4-(4-chlorophényl)-6,7-dihydrothiéno[3,2-c]pyridin-5(4H)-yl)-N-(furan-2-ylméthyl)-4-oxobutanamide ;
60 le 4-(4-(4-chlorophényl)-6,7-dihydrothiéno[3,2-c]pyridin-5(4H)-yl)-4-oxo-N-propylbutanamide ;
61 le N-(2-chlorobenzyl)-4-oxo-4-(4-m-tolyl-6,7-dihydrothiéno[3,2-c]pyridin-5(4H)-yl)butanamide ;
62 le N-(2,4-dichlorobenzyl)-4-(4-(3-fluorophényl)-6,7-dihydrothiéno[3,2-c]pyridin-5(4H)-yl)-4-oxobutanamide ;
63 le N-(4-fluorobenzyl)-4-(4-(3-fluorophényl)-6,7-dihydrothiéno[3,2-c]pyridin-5(4H)-yl)-4-oxobutanamide ;
64 le N-(3,4-dichlorobenzyl)-3-méthyl-4-oxo-4-(4-phényl-6,7-dihydrothiéno[3,2-c]pyridin-5(4H)-yl)butanamide ;
65 le N-(2,5-difluorobenzyl)-3-méthyl-4-oxo-4-(4-phényl-6,7-dihydrothiéno[3,2-c]pyridin-5(4H)-yl)butanamide ;
66 le 3-méthyl-4-oxo-4-(4-phényl-6,7-dihydrothiéno[3,2-c]pyridin-5(4H)-yl)-N-(3-(trifluorométhyl)benzyl)butanamide ;
67 la 1-(4-(4-chlorophényl)-6,7-dihydrothiéno[3,2-c]pyridin-5(4H)-yl)-4-morpholinobutane-1,4-dione ;
68 la 1-(4-(4-chlorophényl)-6,7-dihydrothiéno[3,2-c]pyridin-5(4H)-yl)-4-(4-(4-fluorophényl)pipérazin-1-yl)butane-1,4-dione ;
69 le 2-méthyl-N-(2-(5-méthyl-1H-pyrazol-1-yl)éthyl)-4-oxo-4-(4-phényl-6,7-dihydrothiéno[3,2-c]pyridin-5(4H)-yl)butanamide ;
70 le N-(naphtalén-1-ylméthyl)-4-oxo-4-(4-phényl-6,7-dihydrothiéno[3,2-c]pyridin-5(4H)-yl)butanamide ;
71 le 4-(4-(4-fluorophényl)-6,7-dihydrothiéno[3,2-c]pyridin-5(4H)-yl)-N-(4-méthylbenzyl)-4-oxobutanamide ;
72 le N-(benzo[d][1,3]dioxol-5-ylméthyl)-4-(4-(4-fluorophényl)-6,7-dihydrothiéno[3,2-c]pyridin-5(4H)-yl)-4-oxobutanamide ;
73 le 4-(4-(4-fluorophényl)-6,7-dihydrothiéno[3,2-c]pyridin-5(4H)-yl)-4-oxo-N-(2-(trifluorométhyl)benzyl)butanamide ;
74 le 4-(4-(4-fluorophényl)-6,7-dihydrothiéno[3,2-c]pyridin-5(4H)-yl)-N-(naphtalén-1-ylméthyl)-4-oxobutanamide ;
75 le 4-(4-(4-chlorophényl)-6,7-dihydrothiéno[3,2-c]pyridin-5(4H)-yl)-N-(3-fluorobenzyl)-4-oxobutanamide ;
76 le 2-méthyl-N-(1-méthyl-1H-indazol-6-yl)-4-oxo-4-(4-phényl-6,7-dihydrothiéno[3,2-c]pyridin-5(4H)-yl)butanamide ;
77 le N-(4-méthoxybenzyl)-2-méthyl-4-oxo-4-(4-phényl-6,7-dihydrothiéno[3,2-c]pyridin-5(4H)-yl)butanamide ;
78 la 2-méthyl-1-(4-méthylpipérazin-1-yl)-4-(4-phényl-6,7-dihydrothiéno[3,2-c]pyridin-5(4H)-yl)butane-1,4-dione ;
79 le N-(2-(1H-indol-3-yl)éthyl)-2-méthyl-4-oxo-4-(4-phényl-6,7-dihydrothiéno[3,2-c]pyridin-5(4H)-yl)butanamide ;
80 le N-(2-fluorobenzyl)-2-méthyl-4-oxo-4-(4-phényl-6,7-dihydrothiéno[3,2-c]pyridin-5(4H)-yl)butanamide ;
81 le 2-méthyl-4-oxo-4-(4-phényl-6,7-dihydrothiéno[3,2-c]pyridin-5(4H)-yl)-N-(2-(trifluorométhyl)benzyl)butanamide ;
82 le 4-oxo-N-(2-(pipéridin-1-yl)éthyl)-4-(4-p-tolyl-6,7-dihydrothiéno[3,2-c]pyridin-5(4H)-yl)butanamide ;
83 le 4-oxo-N-((tétrahydrofuran-2-yl)méthyl)-4-(4-p-tolyl-6,7-dihydrothiéno[3,2-c]pyridin-5(4H)-yl)butanamide ;
84 le N-(4-chlorobenzyl)-4-oxo-4-(4-p-tolyl-6,7-dihydrothiéno[3,2-c]pyridin-5(4H)-yl)butanamide ;
85 le N-(2,3-dichlorobenzyl)-4-oxo-4-(4-p-tolyl-6,7-dihydrothiéno[3,2-c]pyridin-5(4H)-yl)butanamide ;
86 le 4-oxo-N-(2-(thiophén-2-yl)éthyl)-4-(4-m-tolyl-6,7-dihydrothiéno[3,2-c]pyridin-5(4H)-yl)butanamide ;
87 le N-(cyclohexylméthyl)-4-oxo-4-(4-m-tolyl-6,7-dihydrothiéno[3,2-c]pyridin-5(4H)-yl)butanamide ;
88 le N-(3-chlorophénéthyl)-4-oxo-4-(4-m-tolyl-6,7-dihydrothiéno[3,2-c]pyridin-5(4H)-yl)butanamide ;
89 le N-(3,3-diphénylpropyl)-4-oxo-4-(4-m-tolyl-6,7-dihydrothiéno[3,2-c]pyridin-5(4H)-yl)butanamide ;
90 le 4-(4-(3-fluorophényl)-6,7-dihydrothiéno[3,2-c]pyridin-5(4H)-yl)-N-(3-morpholinopropyl)-4-oxobutanamide ;
91 le 4-(4-(3-fluorophényl)-6,7-dihydrothiéno[3,2-c]pyridin-5(4H)-yl)-4-oxo-N-(pyridin-3-ylméthyl)butanamide ;
92 le 4-(4-(3-fluorophényl)-6,7-dihydrothiéno[3,2-c]pyridin-5(4H)-yl)-N-(3-méthylbenzyl)-4-oxobutanamide ;
93 le 4-(4-(3-fluorophényl)-6,7-dihydrothiéno[3,2-c]pyridin-5(4H)-yl)-N-(4-méthylphénéthyl)-4-oxobutanamide ;
94 le 3-méthyl-4-oxo-4-(4-phényl-6,7-dihydrothiéno[3,2-c]pyridin-5(4H)-yl)-N-(4-phénylbutyl)butanamide ;
95 le N-(biphényl-4-ylméthyl)-4-oxo-4-(4-m-tolyl-6,7-dihydrothiéno[3,2-c]pyridin-5(4H)-yl)butanamide ;

96 le 4-(4-(3-fluorophényl)-6,7-dihydrothiéno[3,2-c]pyridin-5(4H)-yl)-N-(2-méthoxybenzyl)-4-oxobutanamide ;
97 le N-(4-chlorophénéthyl)-4-(4-(3-fluorophényl)-6,7-dihydrothiéno[3,2-c]pyridin-5(4H)-yl)-4-oxobutanamide ;
98 le 4-(4-(3-fluorophényl)-6,7-dihydrothiéno[3,2-c]pyridin-5(4H)-yl)-N-((5-méthyl-3-phénylisoxazol-4-yl)méthyl)-4-oxobutanamide ;
99 le N-(2,6-difluorobenzyl)-3-méthyl-4-oxo-4-(4-phényl-6,7-dihydrothiéno[3,2-c]pyridin-5(4H)-yl)butanamide ;
100 le N-(3,5-difluorobenzyl)-3-méthyl-4-oxo-4-(4-phényl-6,7-dihydrothiéno[3,2-c]pyridin-5(4H)-yl)butanamide ;
101 le N-(3-chlorobenzyl)-3-méthyl-4-oxo-4-(4-phényl-6,7-dihydrothiéno[3,2-c]pyridin-5(4H)-yl)butanamide ;
102 le N-(3,5-diméthoxyphénéthyl)-3-méthyl-4-oxo-4-(4-phényl-6,7-dihydrothiéno[3,2-c]pyridin-5(4H)-yl)butanamide ;
103 le N-(3,4-difluorobenzyl)-4-oxo-4-(4-phényl-6,7-dihydrothiéno[3,2-c]pyridin-5(4H)-yl)butanamide ;
104 le N-(2,4-dichlorophénéthyl)-4-oxo-4-(4-phényl-6,7-dihydrothiéno[3,2-c]pyridin-5(4H)-yl)butanamide ;
105 le N-(2-(1H-1,2,4-triazol-1-yl)éthyl)-4-oxo-4-(4-phényl-6,7-dihydrothiéno[3,2-c]pyridin-5(4H)-yl)butanamide ;
106 le 4-(4-(4-fluorophényl)-6,7-dihydrothiéno[3,2-c]pyridin-5(4H)-yl)-N-(2-méthylbenzyl)-4-oxobutanamide ;
107 le N-(4-fluorophénéthyl)-4-(4-(4-fluorophényl)-6,7-dihydrothiéno[3,2-c]pyridin-5(4H)-yl)-4-oxobutanamide ;
108 le 4-(4-(4-fluorophényl)-6,7-dihydrothiéno[3,2-c]pyridin-5(4H)-yl)-N-((5-méthylisoxazol-3-yl)méthyl)-4-oxobutanamide ;
109 le N-(2-chlorophénéthyl)-4-(4-(4-fluorophényl)-6,7-dihydrothiéno[3,2-c]pyridin-5(4H)-yl)-4-oxobutanamide ;
110 le 4-(4-(4-chlorophényl)-6,7-dihydrothiéno[3,2-c]pyridin-5(4H)-yl)-N-(2-cyclohexényléthyl)-4-oxobutanamide ;
111 le 4-(4-(4-chlorophényl)-6,7-dihydrothiéno[3,2-c]pyridin-5(4H)-yl)-N-(3,5-diméthoxybenzyl)-4-oxobutanamide ;
112 le 4-(4-(4-chlorophényl)-6,7-dihydrothiéno[3,2-c]pyridin-5(4H)-yl)-N-(3,4-dichlorophénéthyl)-4-oxobutanamide ;
113 le 4-(4-(4-chlorophényl)-6,7-dihydrothiéno[3,2-c]pyridin-5(4H)-yl)-N-(3-fluorophénéthyl)-4-oxobutanamide ;
114 la 1-(3-phénylpipéridin-1-yl)-4-(4-p-tolyl-6,7-dihydrothiéno[3,2-c]pyridin-5(4H)-yl)butane-1,4-dione ;
115 la 1-(4-benzylpipérazin-1-yl)-4-(4-p-tolyl-6,7-dihydrothiéno[3,2-c]pyridin-5(4H)-yl)butane-1,4-dione ;
116 la 1-(4-(4-méthoxyphényl)pipérazin-1-yl)-4-(4-p-tolyl-6,7-dihydrothiéno[3,2-c]pyridin-5(4H)-yl)butane-1,4-dione ;
117 la 1-(2-benzylpipéridin-1-yl)-4-(4-m-tolyl-6,7-dihydrothiéno[3,2-c]pyridin-5(4H)-yl)butane-1,4-dione ;
118 la 1-(4-(2-fluorophényl)pipérazin-1-yl)-4-(4-m-tolyl-6,7-dihydrothiéno[3,2-c]pyridin-5(4H)-yl)butane-1,4-dione ;
119 la 1-(4-(cyclohexylméthyl)pipérazin-1-yl)-4-(4-m-tolyl-6,7-dihydrothiéno[3,2-c]pyridin-5(4H)-yl)butane-1,4-dione ;
120 le N-(3-(1H-imidazol-1-yl)propyl)-4-(4-(3-fluorophényl)-6,7-dihydrothiéno[3,2-c]pyridin-5(4H)-yl)-4-oxobutanamide ;
121 la 1-(4-(3-fluorophényl)-6,7-dihydrothiéno[3,2-c]pyridin-5(4H)-yl)-4-(3-phénylpyrrolidin-1-yl)butane-1,4-dione ;
122 la 1-(4-(3-fluorophényl)-6,7-dihydrothiéno[3,2-c]pyridin-5(4H)-yl)-4-(4-(furane-2-carbonyl)pipérazin-1-yl)-butane-1,4-dione ;
123 le N-(3-(3,4-dihydroquinolin-1(2H)-yl)propyl)-3-méthyl-4-oxo-4-(4-phényl-6,7-dihydrothiéno[3,2-c]pyridin-5(4H)-yl)butanamide ;
124 la 2-méthyl-1-(4-phényl-6,7-dihydrothiéno[3,2-c]pyridin-5(4H)-yl)-4-(pipéridin-1-yl)butane-1,4-dione ;
125 la 4-(4-(2-méthoxyphényl)pipérazin-1-yl)-2-méthyl-1-(4-phényl-6,7-dihydrothiéno[3,2-c]pyridin-5(4H)-yl)-butane-1,4-dione ;
126 le 4-oxo-4-(4-phényl-6,7-dihydrothiéno[3,2-c]pyridin-5(4H)-yl)-N-(2-(pyrazin-2-yl)éthyl)butanamide ;
127 le N,N-diéthyl-4-oxo-4-(4-phényl-6,7-dihydrothiéno[3,2-c]pyridin-5(4H)-yl)butanamide ;
128 la 1-(4-phényl-6,7-dihydrothiéno[3,2-c]pyridin-5(4H)-yl)-4-(4-(pyridin-2-yl)pipérazin-1-yl)butane-1,4-dione ;
129 la 1-(4-(4-fluorophényl)-6,7-dihydrothiéno[3,2-c]pyridin-5(4H)-yl)-4-(4-(3-méthoxyphényl)pipérazin-1-yl)-butane-1,4-dione ;
130 la 1-(4-isopropylpipérazin-1-yl)-4-(4-p-tolyl-6,7-dihydrothiéno[3,2-c]pyridin-5(4H)-yl)butane-1,4-dione ;
131 la 1-(4-p-tolyl-6,7-dihydrothiéno[3,2-c]pyridin-5(4H)-yl)-4-(4-(3-(trifluorométhyl)phényl)pipérazin-1-yl)butane-1,4-dione ;
132 la 1-(4-phénylpipérazin-1-yl)-4-(4-m-tolyl-6,7-dihydrothiéno[3,2-c]pyridin-5(4H)-yl)butane-1,4-dione ;
133 la 1-(3,5-diméthylpipéridin-1-yl)-4-(4-m-tolyl-6,7-dihydrothiéno[3,2-c]pyridin-5(4H)-yl)butane-1,4-dione ;

134 la 1-(5,6-dihydropyridin-1(2H)-yl)-4-(4-(3-fluorophényl)-6,7-dihydrothiéno[3,2-c]pyridin-5(4H)-yl)butane-1,4-dione ;
135 la 1-(4-(3-fluorophényl)-6,7-dihydrothiéno[3,2-c]pyridin-5(4H)-yl)-4-(4-(pyrimidin-2-yl)pipérazin-1-yl)butane-1,4-dione ;
136 la 1-(4-(3-fluorophényl)-6,7-dihydrothiéno[3,2-c]pyridin-5(4H)-yl)-4-(3-méthylpipéridin-1-yl)butane-1,4-dione ;
137 le N-éthyl-4-(4-(3-fluorophényl)-6,7-dihydrothiéno[3,2-c]pyridin-5(4H)-yl)-4-oxo-N-(pyridin-4-ylméthyl)butanamide ;
138 la 4-(4-acétylpipérazin-1-yl)-2-méthyl-1-(4-phényl-6,7-dihydrothiéno[3,2-c]pyridin-5(4H)-yl)butane-1,4-dione ;
139 la 4-(2,6-diméthylmorpholino)-2-méthyl-1-(4-phényl-6,7-dihydrothiéno[3,2-c]pyridin-5(4H)-yl)butane-1,4-dione ;
140 la 4-(2,5-dihydro-1H-pyrrol-1-yl)-2-méthyl-1-(4-phényl-6,7-dihydrothiéno[3,2-c]pyridin-5(4H)-yl)butane-1,4-dione ;
141 le N-(2-méthoxyphénéthyl)-4-oxo-4-(4-phényl-6,7-dihydrothiéno[3,2-c]pyridin-5(4H)-yl)butanamide ;
142 le 4-(4-(4-fluorophényl)-6,7-dihydrothiéno[3,2-c]pyridin-5(4H)-yl)-N-isopentyl-4-oxobutanamide ;
143 le N-(2,4-diméthoxybenzyl)-4-(4-(4-fluorophényl)-6,7-dihydrothiéno[3,2-c]pyridin-5(4H)-yl)-4-oxobutanamide ;
144 le 4-(4-(4-chlorophényl)-6,7-dihydrothiéno[3,2-c]pyridin-5(4H)-yl)-N-((5-méthylfuran-2-yl)méthyl)-4-oxobutanamide ;
145 le 4-(4-(4-chlorophényl)-6,7-dihydrothiéno[3,2-c]pyridin-5(4H)-yl)-4-oxo-N-pentylbutanamide ;
146 la 1-(4-benzylpipéridin-1-yl)-4-(4-(4-chlorophényl)-6,7-dihydrothiéno[3,2-c]pyridin-5(4H)-yl)butane-1,4-dione ;
147 la 1-(4-(4-chlorophényl)-6,7-dihydrothiéno[3,2-c]pyridin-5(4H)-yl)-4-(4-méthylpipéridin-1-yl)butane-1,4-dione ;
148 la 1-(azétidin-1-yl)-4-(4-(4-chlorophényl)-6,7-dihydrothiéno[3,2-c]pyridin-5(4H)-yl)butane-1,4-dione ;
149 la 1-(3,4-dihydroisoquinolin-2(1H)-yl)-2-méthyl-4-(4-phényl-6,7-dihydrothiéno[3,2-c]pyridin-5(4H)-yl)butane-1,4-dione ;
150 la 1-(4-(benzo[d] [1,3]dioxol-5-ylméthyl)pipérazin-1-yl)-2-méthyl-4-(4-phényl-6,7-dihydrothiéno[3,2-c]pyridin-5(4H)-yl)butane-1,4-dione ;
151 le N-benzyl-N-éthyl-4-oxo-4-(4-p-tolyl-6,7-dihydrothiéno[3,2-c]pyridin-5(4H)-yl)butanamide ;
152 le N-méthyl-4-oxo-N-phénéthyl-4-(4-p-tolyl-6,7-dihydrothiéno[3,2-c]pyridin-5(4H)-yl)butanamide ;
153 le 1-(4-oxo-4-(4-p-tolyl-6,7-dihydrothiéno[3,2-c]pyridin-5(4H)-yl)butanoyl)pipéridine-4-carboxylate d'éthyle ;
154 la (E)-1-(4-(3-phénylprop-2-ényl)-pipérazin-1-yl)-4-(4-p-tolyl-6,7-dihydrothiéno[3,2-c]pyridin-5(4H)-yl)butane-1,4-dione ;
155 la 1-(2-(thiazol-2-yl)pyrrolidin-1-yl)-4-(4-p-tolyl-6,7-dihydrothiéno[3,2-c]pyridin-5(4H)-yl)butane-1,4-dione ;
156 le N-cyclohexyl-N-éthyl-4-oxo-4-(4-m-tolyl-6,7-dihydrothiéno[3,2-c]pyridin-5(4H)-yl)butanamide ;
157 le N-éthyl-N-isopropyl-4-oxo-4-(4-m-tolyl-6,7-dihydrothiéno[3,2-c]pyridin-5(4H)-yl)butanamide ;
158 la 1-(pyrrolidin-1-yl)-4-(4-m-tolyl-6,7-dihydrothiéno[3,2-c]pyridin-5(4H)-yl)butane-1,4-dione ;
159 la 1-(4-(pyridin-4-yl)pipérazin-1-yl)-4-(4-m-tolyl-6,7-dihydrothiéno (3,2-c]pyridin-5(4H)-yl)butane-1,4-dione
160 la 1-(2-(pyridin-2-ylméthyl)pyrrolidin-1-yl)-4-(4-m-tolyl-6,7-dihydrothiéno[3,2-c]pyridin-5(4H)-yl)butane-1,4-dione ;
161 le 1-(4-(4-(4-fluorophényl)-6,7-dihydrothiéno[3,2-c]pyridin-5(4H)-yl)-4-oxobutanoyl)pipéridine-3-carboxylate d'éthyle ;
162 la 1-(4-(4-fluorophényl)-6,7-dihydrothiéno[3,2-c]pyridin-5(4H)-yl)-4-(4-(4-(trifluorométhyl)phényl)pipérazin-1-yl)butane-1,4-dione ;
163 la 1-(2-(5-bromopyridin-3-yl)pyrrolidin-1-yl)-4-(4-(4-fluorophényl)-6,7-dihydrothiéno[3,2-c]pyridin-5(4H)-yl)butane-1,4-dione ;
164 la 1-(4-éthylpipérazin-1-yl)-4-(4-phényl-6,7-dihydrothiéno[3,2-c]pyridin-5(4H)-yl)butane-1,4-dione ;
165 la 1-(2-éthylpipéridin-1-yl)-4-(4-phényl-6,7-dihydrothiéno[3,2-c]pyridin-5(4H)-yl)butane-1,4-dione ;
166 le N,N-dibenzyl-4-oxo-4-(4-phényl-6,7-dihydrothiéno[3,2-c]pyridin-5(4H)-yl)butanamide ;
167 le N,N-diisobutyl-4-oxo-4-(4-phényl-6,7-dihydrothiéno[3,2-c]pyridin-5(4H)-yl)butanamide ;
168 la 1-(3,4-dihydroquinolin-1(2H)-yl)-4-(4-(4-fluorophényl)-6,7-dihydrothiéno[3,2-c]pyridin-5(4H)-yl)butane-1,4-dione ;
169 la 1-(4-(3,4-dichlorobenzyl)pipérazin-1-yl)-4-(4-p-tolyl-6,7-dihydrothiéno[3,2-c]pyridin-5(4H)-yl)butane-1,4-dione ;
170 la 1-(4-p-tolyl-6,7-dihydrothiéno[3,2-c]pyridin-5(4H)-yl)-4-(4-(2,4,6-triméthylbenzyl)pipérazin-1-yl)butane-1,4-dione ;
171 la 1-(4-(4-bromobenzyl)pipérazin-1-yl)-4-(4-m-tolyl-6,7-dihydrothiéno[3,2-c]pyridin-5(4H)-yl)butane-1,4-dione ;
172 la 1-(4-(4-chlorobenzyl)pipérazin-1-yl)-4-(4-m-tolyl-6,7-dihydrothiéno[3,2-c]pyridin-5(4H)-yl)butane-1,4-dione ;
173 la 1-(2-((4,6-diméthylpyridin-2-yl)méthyl)pyrrolidin-1-yl)-4-(4-(3-fluorophényl)-6,7-dihydrothiéno[3,2-c]pyridin-5(4H)-yl)butane-1,4-dione ;
174 la 1-(4-(3-fluorophényl)-6,7-dihydrothiéno[3,2-c]pyridin-5(4H)-yl)-4-(2-((6-méthylpyridin-2-yl)méthyl)pyrrolidin-1-yl)butane-1,4-dione ;
175 la 1-(2-((5-éthylpyridin-2-yl)méthyl)pyrrolidin-1-yl)-4-(4-(3-fluorophényl)-6,7-dihydrothiéno[3,2-c]pyridin-5(4H)-yl)butane-1,4-dione ;
176 la 1-(4-(3-fluorophényl)-6,7-dihydrothiéno[3,2-c]pyridin-5(4H)-yl)-4-(2-(6-méthoxypyridin-3-yl)pipéridin-1-yl)butane-1,4-dione ;
177 le 4-(4-(3-fluorophényl)-6,7-dihydrothiéno[3,2-c]pyridin-5(4H)-yl)-N-méthyl-4-oxo-N-(pyridin-3-ylméthyl)butanamide ;
178 le N-(2,2-diphényléthyl)-3-méthyl-4-oxo-4-(4-phényl-6,7-dihydrothiéno[3,2-c]pyridin-5(4H)-yl)butanamide ;
179 le N-(cyclopropylméthyl)-4-oxo-4-(4-phényl-6,7-dihydrothiéno[3,2-c]pyridin-5(4H)-yl)butanamide ;
180 la 1-(4-(3-fluorophényl)-6,7-dihydrothiéno[3,2-c]pyridin-5(4H)-yl)-4-(4-(4-méthylbenzyl)pipérazin-1-yl)butane-1,4-dione ;
181 la 2-méthyl-1-(4-phényl-6,7-dihydrothiéno[3,2-c]pyridin-5(4H)-yl)-4-(2-(pyridin-2-ylméthyl)pipéridin-1-yl)butane-1,4-dione ;
182 la 4-(4-(3,5-dichloropyridin-4-yl)pipérazin-1-yl)-2-méthyl-1-(4-phényl-6,7-dihydrothiéno[3,2-c]pyridin-5(4H)-yl)butane-1,4-dione ;
183 la 1-(2-((4,6-diméthylpyridin-2-yl)méthyl)pipéridin-1-yl)-4-(4-phényl-6,7-dihydrothiéno[3,2-c]pyridin-5(4H)-yl)butane-1,4-dione ;
184 le N-(4-fluorobenzyl)-N-méthyl-4-oxo-4-(4-phényl-6,7-dihydrothiéno[3,2-c]pyridin-5(4H)-yl)butanamide ;
185 la 1-(4-(3-fluorophényl)-6,7-dihydrothiéno[3,2-c]pyridin-5(4H)-yl)-4-(2-(pyridin-2-yl)pyrrolidin-1-yl)butane-1,4-dione ;
186 la 4-(4-(4-méthoxybenzyl)pipérazin-1-yl)-2-méthyl-1-(4-phényl-6,7-dihydrothiéno[3,2-c]pyridin-5(4H)-yl)butane-1,4-dione ;
187 la 2-méthyl-1-(4-phényl-6,7-dihydrothiéno[3,2-c]pyridin-5(4H)-yl)-4-(3-(pyridin-3-yl)pyrrolidin-1-yl)butane-1,4-dione ;
188 la 1-(4-(2-fluorobenzyl)pipérazin-1-yl)-4-(4-phényl-6,7-dihydrothiéno[3,2-c]pyridin-5(4H)-yl)butane-1,4-dione ;
189 le N-méthyl-4-oxo-4-(4-phényl-6,7-dihydrothiéno[3,2-c]pyridin-5(4H)-yl)-N-(2-(pyridin-4-yl)éthyl)butanamide ;
190 le N-butyl-N-éthyl-3-méthyl-4-oxo-4-(4-phényl-6,7-dihydrothiéno[3,2-c]pyridin-5(4H)-yl)butanamide ;
191 le N,3-diméthyl-4-oxo-4-(4-phényl-6,7-dihydrothiéno[3,2-c]pyridin-5(4H)-yl)-N-propylbutanamide ;
192 la 4-((S)-2-(méthoxyméthyl)pyrrolidin-1-yl)-2-méthyl-1-(4-phényl-6,7-dihydrothiéno[3,2-c]pyridin-5(4H)-yl)butane-1,4-dione ;
193 la 4-(4-(5-chloro-2-méthylphényl)pipérazin-1-yl)-2-méthyl-1-(4-phényl-6,7-dihydrothiéno[3,2-c]pyridin-5(4H)-yl)butane-1,4-dione ;
194 le N-(furan-2-ylméthyl)-N-méthyl-4-oxo-4-(4-phényl-6,7-dihydrothiéno[3,2-c]pyridin-5(4H)-yl)butanamide ;
195 la 1-((4aR,8aS)-octahydroisoquinolin-2(1H)-yl)-4-(4-phényl-6,7-dihydrothiéno-[3,2-c]pyridin-5(4H)-yl)-butane-1,4-dione ;
196 la 1-(4-phényl-6,7-dihydrothiéno[3,2-c]pyridin-5(4H)-yl)-4-thiomorpholinobutane-1,4-dione ;
197 la 1-(4-phényl-6,7-dihydrothiéno[3,2-c]pyridin-5(4H)-yl)-4-(2-(pyridin-3-yl)pyrrolidin-1-yl)butane-1,4-dione ;
198 le N-benzyl-4-(4-(4-fluorophényl)-6,7-dihydrothiéno[3,2-c]pyridin-5(4H)-yl)-N-isopropyl-4-oxobutanamide ;
199 le N-benzyl-4-(4-(4-fluorophényl)-6,7-dihydrothiéno[3,2-c]pyridin-5(4H)-yl)-N-méthyl-4-oxobutanamide ;
200 le N-(3,4-diméthoxyphénéthyl)-4-(4-(4-fluorophényl)-6,7-dihydrothiéno[3,2-c]pyridin-5(4H)-yl)-N-méthyl-4-oxobutanamide ;
201 la 1-(4-(4-fluorophényl)-6,7-dihydrothiéno[3,2-c]pyridin-5(4H)-yl)-4-(4-méthyl-2-phénylpipérazin-1-yl)butane-1,4-dione ;
202 la 1-(4-(4-chlorophényl)-6,7-dihydrothiéno[3,2-c]pyridin-5(4H)-yl)-4-(4-phénylpipéridin-1-yl)butane-1,4-dione ;
203 le 4-(4-(4-chlorophényl)-6,7-dihydrothiéno[3,2-c]pyridin-5(4H)-yl)-4-oxo-N-(2-(pyridin-2-yl)éthyl)butanamide ;
204 le N-benzyl-2-méthyl-4-oxo-N-phénéthyl-4-(4-phényl-6,7-dihydrothiéno[3,2-c]pyridin-5(4H)-yl)butanamide ;
205 la 1-(4-(4-fluorophényl)-6,7-dihydrothiéno[3,2-c]pyridin-5(4H)-yl)-4-(2-((6-méthylpyridin-2-yl)méthyl)pipéridin-1-yl)butane-1,4-dione ;
206 la 1-(4-(4-fluorophényl)-6,7-dihydrothiéno[3,2-c]pyridin-5(4H)-yl)-4-(4-(3-méthylbenzyl)pipérazin-1-yl)butane-1,4-dione ;
207 la 1-(4-(4-chlorophényl)-6,7-dihydrothiéno[3,2-c]pyridin-5(4H)-yl)-4-(2-(pyridin-4-ylméthyl)pipéridin-1-yl)butane-1,4-dione ;
208 la 1-(4-(4-chlorophényl)-6,7-dihydrothiéno[3,2-c]pyridin-5(4H)-yl)-4-(2-((5-éthylpyridin-2-yl)méthyl)pipéridin-1-yl)butane-1,4-dione ;
209 la 1-(4-(4-chlorophényl)-6,7-dihydrothiéno[3,2-c]pyridin-5(4H)-yl)-4-(2-((3-méthylpyridin-2-yl)méthyl)pipéridin-1-yl)butane-1,4-dione ;
210 la 1-(4-(4-chlorophényl)-6,7-dihydrothiéno[3,2-c]pyridin-5(4H)-yl)-4-(4-(3-chlorophényl)pipérazin-1-yl)butane-1,4-dione ;
211 la 1-(4-(4-chlorophényl)-6,7-dihydrothiéno[3,2-c]pyridin-5(4H)-yl)-4-(4-(2,3-diméthylphényl)pipérazin-1-yl)butane-1,4-dione ;
212 la 1-(4-(4-chlorophényl)-6,7-dihydrothiéno[3,2-c]pyridin-5(4H)-yl)-4-(4-(3,4-diméthylphényl)pipérazin-1-yl)butane-1,4-dione ;
213 la 1-(4-(4-chlorophényl)-6,7-dihydrothiéno[3,2-c]pyridin-5(4H)-yl)-4-(4-(3,4-dichlorophényl)pipérazin-1-yl)butane-1,4-dione ;
214 la 1-(4-(4-tert-butylbenzyl)pipérazin-1-yl)-4-(4-(4-chlorophényl)-6,7-dihydrothiéno[3,2-c]pyridin-5(4H)-yl)butane-1,4-dione ;
215 la 1-[4-(3,5-dichloro-4-pyridyl)-1-pipérazinyl]-2-méthyl-4-(4-phényl-6,7-dihydro-4H-thiéno[3,2-c]pyridin-5-yl)butane-1,4-dione ;
216 la 1-[4-[(4-méthoxyphényl)méthyl]-1-pipérazinyl]-4-[4-(p-tolyl)-6,7-dihydro-4H-thiéno[3,2-c]pyridin-5-yl]butane-1,4-dione ;
217 la 1-[4-[(2-fluorophényl)méthyl]-1-pipérazinyl]-4-[4-(p-tolyl)-6,7-dihydro-4H-thiéno[3,2-c]pyridin-5-yl]butane-1,4-dione ;
218 la 1-(4-méthyl-2-phényl-1-pipérazinyl)-4-[4-(p-tolyl)-6,7-dihydro-4H-thiéno[3,2-c]pyridin-5-yl]butane-1,4-dione ;
219 la 1-(4-phényl-1-pipéridinyl)-4-[4-(p-tolyl)-6,7-dihydro-4H-thiéno[3,2-c]pyridin-5-yl]butane-1,4-dione ;
220 la 1-[4-(p-tolyl)-6,7-dihydro-4H-thiéno[3,2-c]pyridin-5-yl]-4-[2-(2-pyridyl)-1-pyrrolidinyl]butane-1,4-dione ;
221 la 1-[4-(p-tolyl)-6,7-dihydro-4H-thiéno[3,2-c]pyridin-5-yl]-4-[3-(3-pyridyl)-1-pyrrolidinyl]butane-1,4-dione ;
222 le 4-oxo-4-[4-(p-tolyl)-6,7-dihydro-4H-thiéno[3,2-c]pyridin-5-yl]-N-[2-(2-pyridyl)éthyl]butanamide ;
223 la 1-[4-[(4-méthoxyphényl)méthyl]-1-pipérazinyl]-4-[4-(m-tolyl)-6,7-dihydro-4H-thiéno[3,2-c]pyridin-5-yl]butane-1,4-dione ;
224 la 1-[4-[(2-fluorophényl)méthyl]-1-pipérazinyl]-4-[4-(m-tolyl)-6,7-dihydro-4H-thiéno[3,2-c]pyridin-5-yl]butane-1,4-dione ;
225 la 1-(4-méthyl-2-phényl-1-pipérazinyl)-4-[4-(m-tolyl)-6,7-dihydro-4H-thiéno[3,2-c]pyridin-5-yl]butane-1,4 dione ;
226 la 1-[4-(m-tolyl)-6,7-dihydro-4H-thiéno[3,2-c]pyridin-5-yl]-4-(4-phényl-1-pipéridinyl)butane-1,4-dione ;
227 la 1-[4-(m-tolyl)-6,7-dihydro-4H-thiéno[3,2-c]pyridin-5-yl]-4-[2-(2-pyridyl)-1-pyrrolidinyl]butane-1,4-dione ;
228 la 1-[4-(m-tolyl)-6,7-dihydro-4H-thiéno[3,2-c]pyridin-5-yl]-4-[3-(3-pyridyl)-1-pyrrolidinyl]butane-1,4-dione ;
229 le N-méthyl-4-[4-(m-tolyl)-6,7-dihydro-4H-thiéno[3,2-c]pyridin-5-yl]-4-oxo-N-[2-(4-pyridyl)éthyl]butanamide ;
230 la 1-[4-(3-fluorophényl)-6,7-dihydro-4H-thiéno[3,2-c]pyridin-5-yl]-4-[4-[(4-méthoxyphényl)méthyl]-1-pipérazinyl]butane-1,4-dione ;
231 la 1-[4-(3-fluorophényl)-6,7-dihydro-4H-thiéno[3,2-c]pyridin-5-yl]-4-[4-[(2-fluorophényl)méthyl]-1-pipérazinyl]butane-1,4-dione ;
232 le 4-[4-(m-tolyl)-6,7-dihydro-4H-thiéno[3,2-c]pyridin-5-yl]-4-oxo-N-(p-tolyl)butanamide ;
233 le N-(2,4-diméthylphényl)-4-[4-(m-tolyl)-6,7-dihydro-4H-thiéno[3,2-c]pyridin-5-yl]-4-oxobutanamide ;
234 le 4-[4-(3-fluorophényl)-6,7-dihydro-4H-thiéno[3,2-c]pyridin-5-yl]-N-(1-méthyl-6-indazolyl)-4-oxobutanamide ;
235 le 4-[4-(3-fluorophényl)-6,7-dihydro-4H-thiéno[3,2-c]pyridin-5-yl]-4-oxo-N-(p-tolyl)butanamide ;
236 le 4-[4-(3-fluorophényl)-6,7-dihydro-4H-thiéno[3,2-c]pyridin-5-yl]-4-oxo-N-phénylbutanamide ;
237 le N-(2-chlorophényl)-4-[4-(3-fluorophényl)-6,7-dihydro-4H-thiéno[3,2-c]pyridin-5-yl]-4-oxobutanamide ;
238 le N-(2,4-diméthylphényl)-4-[4-(3-fluorophényl)-6,7-dihydro-4H-thiéno[3,2-c]pyridin-5-yl]-4-oxobutanamide ;
239 le 4-[4-(4-chlorophényl)-6,7-dihydro-4H-thiéno[3,2-c]pyridin-5-yl]-N-(1-méthyl-6-indazolyl)-4-oxobutanamide ;
240 le 4-[4-(4-chlorophényl)-6,7-dihydro-4H-thiéno[3,2-c]pyridin-5-yl]-4-oxo-N-(p-tolyl)butanamide ;
241 le 4-[4-(4-chlorophényl)-6,7-dihydro-4H-thiéno[3,2-c]pyridin-5-yl]-N-(4-méthoxyphényl)-4-oxobutanamide ;
242 le 4-[4-(4-chlorophényl)-6,7-dihydro-4H-thiéno[3,2-c]pyridin-5-yl]-4-oxo-N-phénylbutanamide ;
243 le 4-[4-(4-chlorophényl)-6,7-dihydro-4H-thiéno[3,2-c]pyridin-5-yl]-N-(2,4-diméthylphényl)-4-oxobutanamide ;
244 le 4-[4-(2,6-diméthylphényl)-6,7-dihydro-4H-thiéno[3,2-c]pyridin-5-yl]-4-oxo-N-[[3-(trifluorométhyl)phényl]-méthyl]butanamide ;
245 le N-(2-cyclohexyléthyl)-4-oxo-4-(4-phényl-6,7-dihydro-4H-thiéno[3,2-c]pyridin-5-yl)butanamide ;
246 le N-(3,3-diméthylbutyl)-4-oxo-4-(4-phényl-6,7-dihydro-4H-thiéno[3,2-c]pyridin-5-yl)butanamide ;
247 le N-(cyclohexylméthyl)-4-oxo-4-(4-phényl-6,7-dihydro-4H-thiéno[3,2-c]pyridin-5-yl)butanamide ;
248 le N-[[3-méthyl-5-(trifluorométhoxy)phényl]méthyl]-4-oxo-4-(4-phényl-6,7-dihydro-4H-thiéno[3,2-c]pyridin-5-yl)butanamide ;
249 le N-[[4-méthyl-3-(trifluorométhyl)phényl]méthyl]-4-oxo-4-(4-phényl-6,7-dihydro-4H-thiéno[3,2-c]pyridin-5-yl)butanamide ;
250 le N-[[4-fluoro-3-(trifluorométhyl)phényl]méthyl]-4-oxo-4-(4-phényl-6,7-dihydro-4H-thiéno[3,2-c]pyridin-5-yl)butanamide ;
251 le 4-[4-(2-éthylphényl)-6,7-dihydro-4H-thiéno[3,2-c]pyridin-5-yl]-4-oxo-N-[[3-(trifluorométhyl)phényl]méthyl]butanamide ;
252 le 4-[4-(2-isopropylphényl)-6,7-dihydro-4H-thiéno[3,2-c]pyridin-5-yl]-4-oxo-N-([3-(trifluorométhyl)phényl]-méthyl]butanamide ;
253 le N-(3-cyclohexylpropyl)-4-oxo-4-(4-phényl-6,7-dihydro-4H-thiéno[3,2-c]pyridin-5-yl)butanamide ;
254 le 4-[4-(3-méthyl-2-thiényl)-6,7-dihydro-4H-thiéno[3,2-c]pyridin-5-yl]-4-oxo-N-[[3-(trifluorométhyl)phényl]-méthyl]butanamide ;
255 le (1S,2S)-2-[oxo-(4-phényl-6,7-dihydro-4H-thiéno[3,2-c]pyridin-5-yl)méthyl]-N-[[3-(trifluorométhyl)phényl]méthyl]-l-cyclopropanecarboxamide ;
256 le 4-[4-(o-tolyl)-6,7-dihydro-4H-thiéno[3,2-c]pyridin-5-yl]-4-oxo-N-[[2-(trifluorométhyl)phényl]méthyl]butanamide ;
257 le 4-[4-(o-tolyl)-6,7-dihydro-4H-thiéno[3,2-c]pyridin-5-yl]-4-oxo-N-[[4-(trifluorométhyl)phényl]méthyl]butanamide ;
258 le 4-(7-butyl-5,7-dihydro-4H-thiéno[2,3-c]pyridin-6-yl)-4-oxo-N-[[3-(trifluorométhyl)phényl]méthyl]butanamide ;
259 le 4-[4-(cyclohexylméthyl)-6,7-dihydro-4H-thiéno[3,2-c]pyridin-5-yl]-4-oxo-N-[[3-(trifluorométhyl)phényl]-méthyl]butanamide ;
260 le 4-(4-cyclopropyl-6,7-dihydro-4H-thiéno[3,2-c]pyridin-5-yl)-4-oxo-N-[[3-(trifluorométhyl)phényl]méthyl]-butanamide ;
261 le 4-oxo-4-(4-phénéthyl-6,7-dihydro-4H-thiéno[3,2-c]pyridin-5-yl)-N-[(3-(trifluorométhyl)phényl]méthyl]-butanamide ;
262 le 4-[4-(4-fluoro-2-méthylphényl)-6,7-dihydro-4H-thiéno[3,2-c]pyridin-5-yl]-4-oxo-N-[[3-(trifluorométhyl)-phényl]méthyl]butanamide ;
263 le 4-(4-cyclopentyl-6,7-dihydro-4H-thiéno[3,2-c]pyridin-5-yl)-4-oxo-N-[[3-(trifluorométhyl)phényl]méthyl]-butanamide ;
264 le N-[[2-méthyl-3-(trifluorométhyl)phényl]méthyl]-4-oxo-4-(4-phényl-6,7-dihydro-4H-thiéno[3,2-c]pyridin-5-yl)butanamide ;
265 le 4-oxo-4-[4-(3-phénylpropyl)-6,7-dihydro-4H-thiéno[3,2-c]pyridin-5-yl]-N-[[3-(trifluorométhyl)phényl]-méthyl]butanamide ;
266 le N-[[2-méthyl-5-(trifluorométhyl)phényl]méthyl]-4-oxo-4-(4-phényl-6,7-dihydro-4H-thiéno[3,2-c]pyridin-5-yl)butanamide ;
267 le N-cyclohexyl-4-oxo-4-(4-phényl-6,7-dihydro-4H-thiéno[3,2-c]pyridin-5-yl)butanamide ;
268 le N-(2,2-diméthylpropyl)-4-oxo-4-(4-phényl-6,7-dihydro-4H-thiéno[3,2-c]pyridin-5-yl)butanamide ;
269 la 1-(4-phényl-6,7-dihydro-4H-thiéno[3,2-c]pyridin-5-yl)-4-[7-(trifluorométhyl)-3,4-dihydro-1H-isoquinolin-2-yl]butane-1,4-dione ;
270 le 4-oxo-N-pentyl-4-(4-phényl-6,7-dihydro-4H-thiéno[3,2-c]pyridin-5-yl)butanamide ;
271 la 1-(4-phényl-6,7-dihydro-4H-thiéno[3,2-c]pyridin-5-yl)-4-[5-(trifluorométhyl)-3,4-dihydro-1H-isoquinolin-2-yl]butane-1,4-dione ;
272 le 4-oxo-4-(4-phényl-6,7-dihydro-4H-thiéno[3,2-c]pyridin-5-yl)-N-[5-(trifluorométhyl)-1-tétralinyl]butanamide ;
273 le 4-oxo-4-(4-phényl-6,7-dihydro-4H-thiéno[3,2-c]pyridin-5-yl)-N-[7-(trifluorométhyl)-1-tétralinyl]butanamide ;
274 le 4-[7-(o-tolyl)-5,7-dihydro-4H-thiéno[2,3-c]pyridin-6-yl]-4-oxo-N-[[3-(trifluorométhyl)phényl]méthyl]butanamide ;
275 le 4-(7-cyclohexyl-5,7-dihydro-4H-thiéno[2,3-c]pyridin-6-yl)-4-oxo-N-[[3-(trifluorométhyl)phényl]méthyl]-butanamide ;

ou leurs sels physiologiquement compatibles.

**12.** Médicament contenant au moins un composé selon l'une quelconque des revendications 1 à 11 ou un composé choisi dans le groupe constitué par

- la 1-morpholino-4-(4-(thiophén-2-yl)-6,7-dihydrothiéno[3,2-c]pyridin-5(4H)-yl)butane-1,4-dione,
- la 1-(4-acétylpipérazin-1-yl)-4-(4-(thiophén-2-yl)-6,7-dihydrothiéno[3,2-c]pyridin-5(4H)-yl)butane-1,4-dione et
- la 1-(3-phényl-4,5-dihydropyrazol-1-yl)-4-(4-(thiophén-2-yl)-6,7-dihydrothiéno[3,2-c]pyridin-5(4H)-yl)butane-1,4-dione ;

sous la forme d'un stéréoisomère individuel ou leur mélange, des composés libres et/ou leurs sels physiologiquement compatibles, ainsi que des additifs et/ou adjuvants appropriés et/ou éventuellement des agents actifs supplémentaires.

**13.** Utilisation d'un composé selon l'une quelconque des revendications 1 à 11 ou d'un composé choisi dans le groupe constitué par

- la 1-morpholino-4-(4-(thiophén-2-yl)-6,7-dihydrothiéno[3,2-c]pyridin-5(4H)-yl)butane-1,4-dione,
- la 1-(4-acétylpipérazin-1-yl)-4-(4-(thiophén-2-yl)-6,7-dihydrothiéno[3,2-c]pyridin-5(4H)-yl)butane-1,4-dione et
- la 1-(3-phényl-4,5-dihydropyrazol-1-yl)-4-(4-(thiophén-2-yl)-6,7-dihydrothiéno[3,2-c]pyridin-5(4H)-yl)butane-1,4-dione ;

sous la forme d'un stéréoisomère individuel ou leur mélange, des composés libres et/ou leurs sels physiologiquement compatibles, pour la fabrication d'un médicament pour le traitement de la douleur, de l'épilepsie, des états d'anxiété, de la dépendance, des manies, des troubles bipolaires, de la migraine, des maladies cognitives, des dyskinésies associées à une dystonie et/ou de l'incontinence urinaire.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20G20128277 A
- WO 2008046582 A
- WO 9634870 A


### In der Beschreibung aufgeführte Nicht-Patentliteratur


- **PASSMORE et al.** *J Neurosci.,* 2003, vol. 23 (18), 7227-36
- **BLACKBURN-MUNRO ; JENSEN.** *Eur J Pharmacol.,* 2003, vol. 460 (2-3), 109-16
- **DOST et al.** *Naunyn Schmiedebergs Arch Pharmacol,* 2004, vol. 369 (4), 382-390
- **NIELSEN et al.** *Eur J Pharmacol.,* 2004, vol. 487 (1-3), 93-103
- **GRIBKOFF.** *Expert Opin Ther Targets,* 2003, vol. 7 (6), 737-748
- **KORSGAARD et al.** *J Pharmacol Exp Ther.,* 2005, vol. 14 (1), 282-92
- **WICKENDEN et al.** *Expert Opin Ther Pat,* 2004, vol. 14 (4), 457-469
- **GRIBKOFF.** *Expert Opin Ther Targets,* 2008, vol. 12 (5), 565-81
- **MICELI et al.** *Curr Opin Pharmacol,* 2008, vol. 8 (1), 65-74
- **STRENG et al.** *J Urol,* 2004, vol. 172, 2054-2058
- **HANSEN et al.** *Eur J Pharmacol,* 2007, vol. 570 (1-3), 77-88
- **DENCKER et al.** *Epilepsy Behav,* 2008, vol. 12 (1), 49-53
- **RICHTER et al.** *Br J Pharmacol,* 2006, vol. 149 (6), 747-53
- **BENNETT, G.J. ; XIE, Y.K.** A peripheral mononeuropathy in rat that produces disorders of pain sensation like those seen in man. *Pain,* 1988, vol. 33 (1), 87-107
- **KIM, S.H. ; CHUNG, J.M.** An experimental model for peripheral neuropathy produced by segmental spinal nerve ligation in the rat. *Pain,* 1992, vol. 50 (3), 355-363
- **DE SARRO et al.** *Naunyn-Schmiedeberg's Arch. Pharmacol.,* 2001, vol. 363, 330-336
- **HAMILL OP ; MARTY A ; NEHER E ; SAKMANN B ; SIGWORTH FJ.** Improved patch-clamp techniques for high-resolution current recording from cells and cell-free membrane patches. *Pflugers Arch.,* August 1981, vol. 391 (2), 85-100
- **DUBUISSON, D. ; DENNIS, S.G.** *Pain,* 1977, vol. 4, 161-174